# EUROPEAN PATENT APPLICATION

(11) **EP 4 851 160 A1**
(43) Date of publication of application: **09.12.2026**
(21) Application number: 24921298.6
(22) Date of filing: 02.07.2024
(51) Int. Cl.: C07D 417/14, C07D 498/00, A61K 31/395, A61K 31/425, A61K 31/427, A61P 29/00

(54) **BENZOMORPHOLINE COMPOUND AND USE THEREOF**

(30) Priority: 31.01.2024 CN 202410136721; 25.06.2024 CN 202410829708
(71) Applicant: Shenzhen Crystalo Biopharma Technology Co., Ltd., Shenzhen, Guangdong 518118 (CN)
(72) Inventor: ZHANG, Jin, Shenzhen, Guangdong 518118 (CN); LI, Jian, Shenzhen, Guangdong 518118 (CN); WU, Xiaoyun, Shenzhen, Guangdong 518118 (CN); HU, Han, Shenzhen, Guangdong 518118 (CN); ZHENG, Zhuoping, Shenzhen, Guangdong 518118 (CN); ZHANG, Yuting, Shenzhen, Guangdong 518118 (CN); LIU, Yinzhen, Shenzhen, Guangdong 518118 (CN)
(74) Representative: Murgitroyd & Company
(86) International application number: PCT/CN2024/103136
(87) International publication number: WO 2025/161263

(57) **Abstract**

Disclosed in the present invention are a benzomorpholine compound and a use thereof. Specifically disclosed are a compound I, a stereoisomer thereof, or a pharmaceutically acceptable salt thereof. The compound provided in the present invention has good TRPM3 inhibitory activity and is expected to treat related diseases.

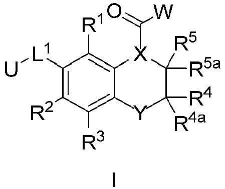

## Description

The present application claims priority of Chinese patent application 2024101367219 filed on January 31, 2024 and Chinese patent application 2024108297081 filed on June 25, 2024. The contents of the above Chinese patent applications are incorporated herein by reference in their entirety.

### TECHNICAL FIELD

The present disclosure relates to a benzomorpholine compound and a use thereof.

### BACKGROUND

The TRP superfamily consists of proteins with six transmembrane domains (6TM) that assemble as homo- or heterotetramers to form cation-permeable ion channels.

TRP channel dysfunction is directly involved in the etiology of various inherited and acquired diseases. Indeed, both loss-of-function and gain-of-function mutations in the TRP channel genes have been identified as the direct cause of inherited diseases, including brachyolmia, hypomagnesemia with secondary hypocalcemia, polycystic kidney disease, mucolipidosis type IV, and familial focal segmental glomerulosclerosis. Moreover, TRP channel function/dysfunction has been directly linked to a wide range of pathological conditions, including chronic pain, hypertension, cancer, and neurodegenerative disorders.

TRPM3 (transient receptor potential melastatin 3) represents a promising pharmacological target. TRPM3 is expressed in a large subset of small-diameter sensory neurons from dorsal root and trigeminal ganglia, and is involved in heat sensing. The neurosteroid pregnenolone sulfate is a potent known activator of TRPM3. The neurosteroid pregnenolone sulfate evoked pain in wild-type mice but not in TRPM3 knock-out mice. It was also recently shown that CFA-induced inflammation and inflammatory pain were eliminated in TRPM3 knock-out mice. Therefore, TRPM3 antagonists may be used as analgesics to counteract pain, such as inflammatory pain.

There is still a great medical need for novel, alternative, and/or better therapeutics for the prevention or treatment of TRPM3-mediated disorders, more specifically for pain (*e.g.,* inflammatory pain). There is an urgent need for therapeutics with good potency on a certain type of pain, low or no side effects, and/or good or better pharmacokinetic or pharmacodynamic properties.

### SUMMARY

The present disclosure provides a class of novel compounds which are antagonists of TRPM3 and may be used as modulators of TRPM3-mediated disorders. The compounds provided by the present disclosure exhibit good TRPM3 inhibitory activity and hold promise for treating related diseases.

The present disclosure provides a compound I, a stereoisomer thereof, or a pharmaceutically acceptable salt thereof, 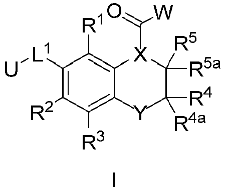 wherein
X is N or CH;
Y is O or -CH₂-;
L¹ is -L^{a}-L^{b}-L^{c}-L ^{d}-;
L^{a}, L^{b}, L^{c}, and L^{d} are each independently a direct bond, 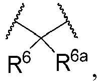 O, S, or 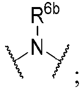 and L^{a}, L^{b}, L^{c}, and L^{d} are not simultaneously a direct bond; the number of O, S, and 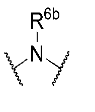 in L^{a}, L^{b}, L^{c}, and L^{d} is 1 or 2;
R⁶, R^{6a}, and R^{6b} are each independently H, C₁-C₆ alkyl, or C₁-C₆ alkyl substituted by one or more R⁶⁻¹;
each R⁶⁻¹ is independently hydroxyl, halogen, amino, -NHC₁-C₆ alkyl, or -N(C₁-C₆ alkyl)₂;
U is 3- to 12-membered heterocycloalkyl, C₆-C₁₀ aryl, 5- to 10-membered heteroaryl, 3- to 8-membered cycloalkyl, C₆-C₁₀ aryl substituted by one or more R⁹⁻¹, 5- to 10-membered heteroaryl substituted by one or more R⁹⁻², or 3- to 8-membered cycloalkyl substituted by one or more R^{9-a};
R⁹⁻¹, R⁹⁻², and R^{9-a} are each independently halogen, cyano, hydroxyl, C₁-C₆ alkyl, C₁-C₆ alkoxy, 3- to 8-membered cycloalkyl, 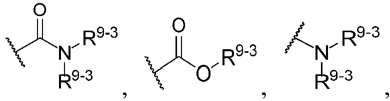 oxo, -O-(3-to 8-membered cycloalkyl), or C₁-C₆ alkyl substituted by one or more R⁹⁻⁴;
each R⁹⁻³ is independently H, C₁-C₆ alkyl, or 3- to 8-membered cycloalkyl;
each R⁹⁻⁴ is independently hydroxyl, halogen, or C₁-C₆ alkoxy;
R¹, R², and R³ are each independently H, halogen, cyano, C₁-C₆ alkyl, C₁-C₆ alkoxy, or C₁-C₆ alkyl substituted by one or more halogens;
R⁴, R^{4a}, R^{5a}, and R⁵ are each independently H, C₁-C₆ alkyl, C₁-C₆ alkoxy, 3- to 8-membered cycloalkyl, or C₁-C₆ alkyl substituted by one or more halogens;
or, R⁴ and R^{4a} together with the carbon atom to which they are attached form a 3- to 8-membered cycloalkyl ring;
W is L²R⁷;
L² is a direct bond, -CH₂-, -NH-, or -N(C₁-C₆ alkyl)-;
R⁷ is 3- to 8-membered cycloalkyl, 3- to 12-membered heterocycloalkyl, 5- to 10-membered heteroaryl, 3- to 12-membered heterocycloalkyl substituted by one or more R⁷⁻¹, 5-to 10-membered heteroaryl substituted by one or more R⁷⁻², C₁-C₆ alkyl substituted by one or more R⁷⁻³, or 3- to 8-membered cycloalkyl substituted by one or more R⁷⁻⁴;
R⁷⁻¹, R⁷⁻², and R⁷⁻⁴ are each independently halogen, hydroxyl, oxo, amino, -NH(C₁-C₆ alkyl), -N(C₁-C₆ alkyl)₂, C₁-C₆ alkyl, C₁-C₆ alkoxy, 3- to 8-membered cycloalkyl, -O-(3- to 8-membered cycloalkyl), 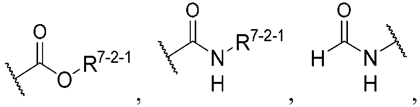 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, amino, C₁-C₆ alkoxy, C₂-C₆ alkenyl, C₂-C₆ alkynyl, -NH(C₁-C₆ alkyl), -N(C₁-C₆ alkyl)₂, 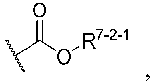 or 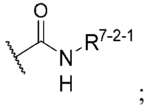
each R⁷⁻³ is independently halogen, hydroxyl, amino, C₁-C₆ alkoxy, -NH(C₁-C₆ alkyl), -N(C₁-C₆ alkyl)₂, 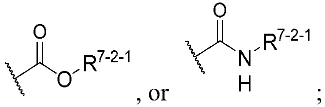
each R⁷⁻²⁻¹ is independently H, C₁-C₆ alkyl, 3- to 8-membered cycloalkyl, or C₁-C₆ alkyl substituted by one or more C₆-C₁₀ aryl;
the heteroatom in the 3- to 12-membered heterocycloalkyl is selected from one or more of N, O, and S; the number of heteroatoms is 1, 2, or 3;
the heteroatom in the 5- to 10-membered heteroaryl is selected from one or more of N, O, and S; the number of heteroatoms is 1, 2, or 3.

In a certain embodiment,
L¹ is 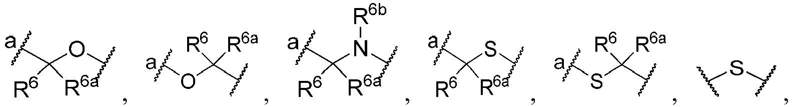 or 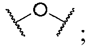 wherein the a-end is attached to U;
U is 3- to 12-membered heterocycloalkyl, C₆-C₁₀ aryl, 5- to 10-membered heteroaryl, C₆-C₁₀ aryl substituted by one or more R⁹⁻¹, or 5- to 10-membered heteroaryl substituted by one or more R⁹⁻²;
R⁹⁻¹ and R⁹⁻² are each independently halogen, cyano, hydroxyl, C₁-C₆ alkyl, C₁-C₆ alkoxy, 3- to 8-membered cycloalkyl, 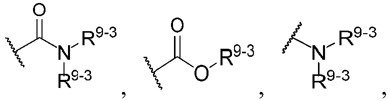 oxo, -0-(3- to 8-membered cycloalkyl), or C₁-C₆ alkyl substituted by one or more R⁹⁻⁴;
R⁷⁻¹, R⁷⁻², and R⁷⁻⁴ are each independently halogen, hydroxyl, oxo, amino, -NH(C₁-C₆ alkyl), -N(C₁-C₆ alkyl)₂, C₁-C₆ alkyl, C₁-C₆ alkoxy, 3- to 8-membered cycloalkyl, -O-(3- to 8-membered cycloalkyl), 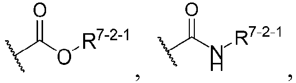 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, amino, C₂-C₆ alkenyl, C₂-C₆ alkynyl, -NHC₁-C₆ alkyl, -N(C₁-C₆ alkyl)₂, 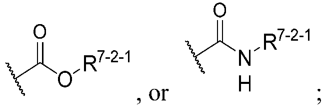
each R⁷⁻²⁻¹ is independently H, C₁-C₆ alkyl, or 3- to 8-membered cycloalkyl.

In a certain embodiment, each C₁-C₆ alkyl may independently be methyl, ethyl, *n-*propyl, isopropyl, *n*-butyl, isobutyl, or *tert-butyl,* preferably methyl.

In a certain embodiment, each C₂-C₆ alkenyl may independently be vinyl, propenyl, allyl, butenyl, or pentenyl, preferably vinyl.

In a certain embodiment, each C₂-C₆ alkynyl may independently be ethynyl, propynyl, propargyl, butynyl, or pentynyl.

In a certain embodiment, each C₁-C₆ alkoxy may independently be methoxy, ethoxy, *n*-propoxy, isopropoxy, *n*-butoxy, isobutoxy, or *tert*-butoxy, preferably methoxy.

In a certain embodiment, each halogen may independently be fluorine, chlorine, bromine, or iodine.

In a certain embodiment, the heteroatom in each 3- to 12-membered heterocycloalkyl may independently be N; the number of heteroatoms may independently be 1 or 2.

In a certain embodiment, each 3- to 12-membered heterocycloalkyl may independently be 4- to 10-membered heterocycloalkyl.

In a certain embodiment, each 3- to 12-membered heterocycloalkyl may independently be a monocyclic ring or a bicyclic ring, wherein the bicyclic ring may be a bridged ring or a spiro ring.

In a certain embodiment, each occurrence of "more" may independently refer to 2 or 3.

In a certain embodiment, each C₆-C₁₀ aryl may independently be phenyl or naphthyl, preferably phenyl.

In a certain embodiment, each 5- to 10-membered heteroaryl may independently be 5-to 6-membered heteroaryl.

In a certain embodiment, the heteroatom in each 5- to 10-membered heteroaryl may independently be N, S, or O; the number of heteroatoms may independently be 1 or 2.

In a certain embodiment, each 5- to 10-membered heteroaryl may independently be a monocyclic ring or a bicyclic ring.

In a certain embodiment, each 3- to 8-membered cycloalkyl may independently be cyclopropyl, cyclobutyl, cyclopentyl, or cyclohexyl, preferably cyclopropyl or cyclobutyl.

In a certain embodiment, the pharmaceutically acceptable salt may be hydrochloride, formate, or trifluoroacetate.

In a certain embodiment, X is N.

In a certain embodiment, Y is O.

In a certain embodiment, L¹ is 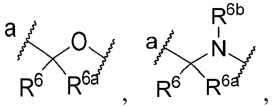 or 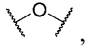 preferably 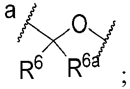 wherein the a-end is attached to U.

In a certain embodiment, the number of O, S, and 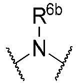 in L^{a}, L^{b}, L^{c}, and L^{d} is 1.

In a certain embodiment, R⁶, R^{6a}, and R^{6b} are each independently H or C₁-C₆ alkyl, preferably H.

In a certain embodiment, U is C₆-C₁₀ aryl, 5- to 10-membered heteroaryl, C₆-C₁₀ aryl substituted by one or more R⁹⁻¹, or 5- to 10-membered heteroaryl substituted by one or more R⁹⁻².

In a certain embodiment, U is C₆-C₁₀ aryl, 5- to 10-membered heteroaryl, 3- to 8-membered cycloalkyl, C₆-C₁₀ aryl substituted by one or more R⁹⁻¹, or 5- to 10-membered heteroaryl substituted by one or more R⁹⁻².

In a certain embodiment, U is 3- to 8-membered cycloalkyl.

In a certain embodiment, R⁹⁻¹ and R⁹⁻² are each independently halogen, hydroxyl, C₁-C₆ alkyl, C₁-C₆ alkoxy, 3- to 8-membered cycloalkyl, 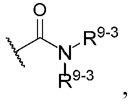 or C₁-C₆ alkyl substituted by one or more R⁹⁻⁴.

In a certain embodiment, R⁹⁻¹, R⁹⁻², and R^{9-a} are each independently halogen, hydroxyl, C₁-C₆ alkyl, C₁-C₆ alkoxy, 3- to 8-membered cycloalkyl, 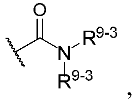 or C₁-C₆ alkyl substituted by one or more R⁹⁻⁴.

In a certain embodiment, R^{9-a} is independently halogen, hydroxyl, C₁-C₆ alkyl, C₁-C₆ alkoxy, 3- to 8-membered cycloalkyl, 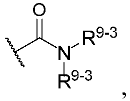 or C₁-C₆ alkyl substituted by one or more R⁹⁻⁴.

In a certain embodiment, each R⁹⁻³ is independently H or C₁-C₆ alkyl, preferably H.

In a certain embodiment, R⁹⁻⁴ is halogen.

In a certain embodiment, R¹, R², and R³ are each independently H or halogen; preferably, R¹ and R³ are H, and R² is H or halogen.

In a certain embodiment, R^{4a} is H.

In a certain embodiment, R⁵ and R^{5a} are H.

In a certain embodiment, R⁴ is H or C₁-C₆ alkyl, preferably H.

In a certain embodiment, L² is a direct bond, -CH₂-, or -NH-, preferably -NH-.

In a certain embodiment, L² is a direct bond, -NH-, or -N(CH₃)-.

In a certain embodiment, L² is -N(CH₃)-.

In a certain embodiment, R⁷ is 3- to 12-membered heterocycloalkyl, 5- to 10-membered heteroaryl, 3- to 12-membered heterocycloalkyl substituted by one or more R⁷⁻¹, 5-to 10-membered heteroaryl substituted by one or more R⁷⁻², or 3- to 8-membered cycloalkyl substituted by one or more R⁷⁻⁴.

In a certain embodiment, R⁷ is C₁-C₆ alkyl substituted by one or more R⁷⁻³.

In a certain embodiment, R⁷⁻³ is hydroxyl or

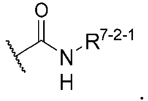

In a certain embodiment, R⁷⁻¹, R⁷⁻², and R⁷⁻⁴ are each independently halogen, amino, -NHC₁-C₆ alkyl, -N(C₁-C₆ alkyl)₂, C₁-C₆ alkyl, 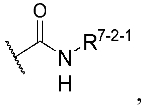 C₁-C₆ alkyl substituted by one or more R⁷⁻¹⁻¹, or C₁-C₆ alkoxy substituted by one or more R⁷⁻¹⁻².

In a certain embodiment, R⁷⁻¹, R⁷⁻², and R⁷⁻⁴ are each independently halogen, amino, -NHC₁-C₆ alkyl, -N(C₁-C₆ alkyl)₂, C₁-C₆ alkyl, 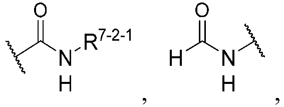 C₁-C₆ alkyl substituted by one or more R⁷⁻¹⁻¹, or C₁-C₆ alkoxy substituted by one or more R⁷⁻¹⁻².

In a certain embodiment, R⁷⁻¹, R⁷⁻², and R⁷⁻⁴ are each independently

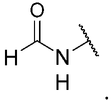

In a certain embodiment, R⁷⁻¹⁻¹ and R⁷⁻¹⁻² are each independently halogen, hydroxyl, C₂-C₆ alkenyl,

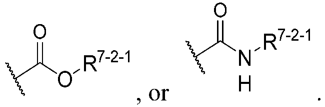

In a certain embodiment, R⁷⁻¹⁻¹ and R⁷⁻¹⁻² are each independently halogen, hydroxyl, C₁-C₆ alkoxy, C₂-C₆ alkenyl,

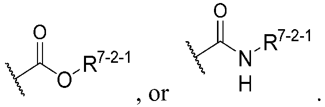

In a certain embodiment, R⁷⁻¹⁻¹ and R⁷⁻¹⁻² are each independently C₁-C₆ alkoxy.

In a certain embodiment, each R⁷⁻²⁻¹ is independently H or C₁-C₆ alkyl.

In a certain embodiment, each R⁷⁻²⁻¹ is independently H, C₁-C₆ alkyl, or C₁-C₆ alkyl substituted by one or more C₆-C₁₀ aryl.

In a certain embodiment, each R⁷⁻²⁻¹ is independently 3- to 8-membered cycloalkyl or C₁-C₆ alkyl substituted by one or more C₆-C₁₀ aryl.

In a certain embodiment, when L² is a direct bond, R⁷ is 3- to 12-membered heterocycloalkyl or 3- to 12-membered heterocycloalkyl substituted by one or more R⁷⁻¹; the 3- to 12-membered heterocycloalkyl is preferably a spiro ring;
preferably, the heteroatom in the 3- to 12-membered heterocycloalkyl may be N, and the number of heteroatoms may be 1 or 2;
more preferably, the N atom in the 3- to 12-membered heterocycloalkyl is attached to L².

In a certain embodiment, R⁷ is 3- to 12-membered heterocycloalkyl or 3- to 12-membered heterocycloalkyl substituted by one or more R⁷⁻¹;
preferably, R⁷ is 5- to 6-membered heterocycloalkyl or 5- to 6-membered heterocycloalkyl substituted by one or more halogens; the heteroatom in the 5- to 6-membered heterocycloalkyl is N, and the number of heteroatoms may be 1 or 2;
more preferably, R⁷ is piperidinyl, tetrahydropyrrolyl, tetrahydropyrrolyl substituted by one or more halogens, or piperidinyl substituted by one or more halogens;
further preferably, R⁷ is piperidinyl substituted by 1 or 2 F or tetrahydropyrrolyl substituted by 1 or 2 F.

In a certain embodiment, L¹ is 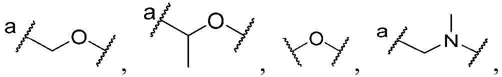 or 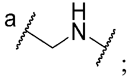 wherein the a-end is attached to U.

In a certain embodiment, U is 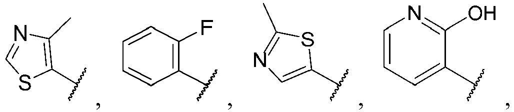 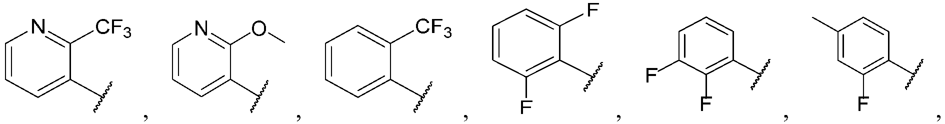 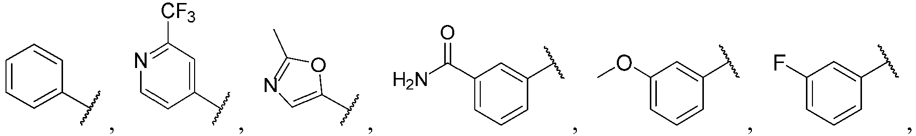 or

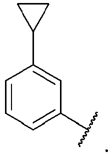

In a certain embodiment, U is

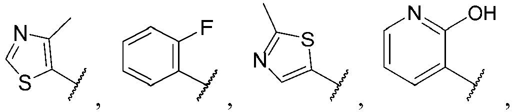

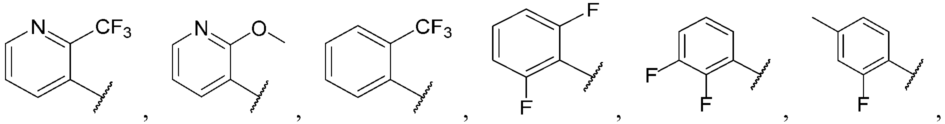

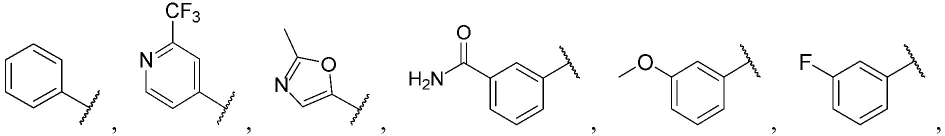

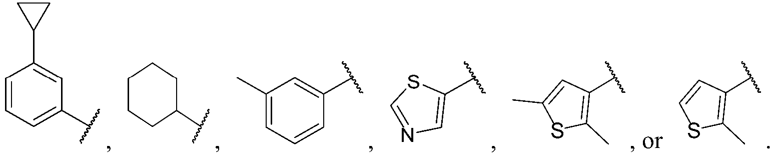

In a certain embodiment, U is

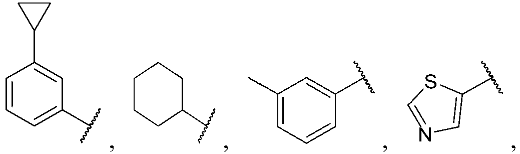

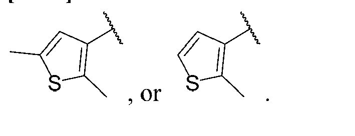

In a certain embodiment, W is 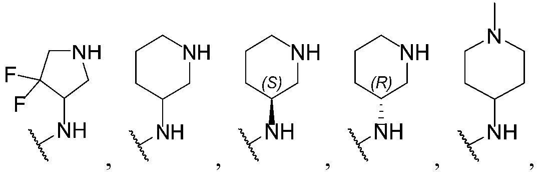 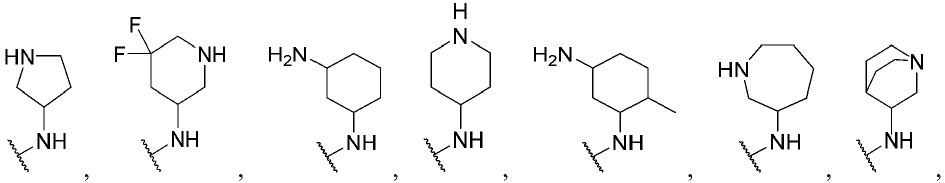 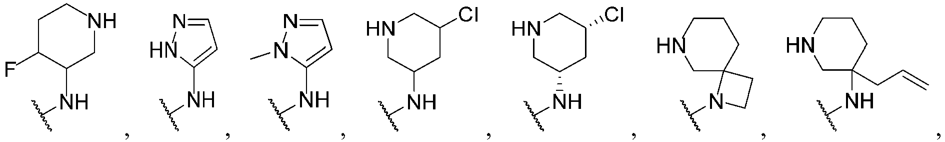 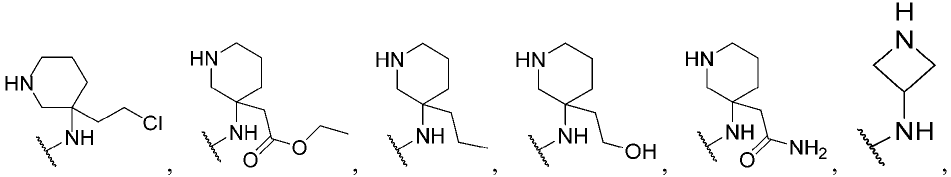 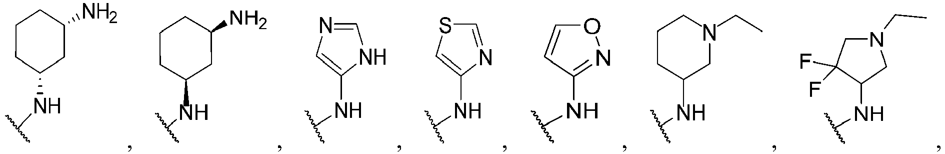 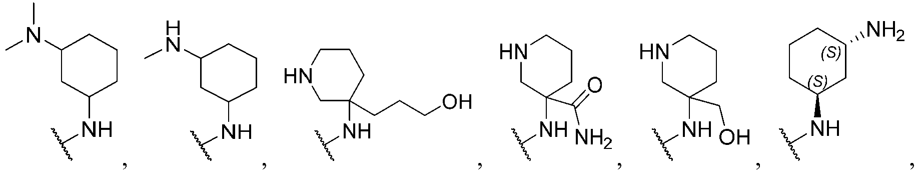 or

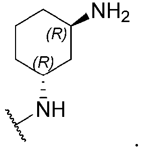

In a certain embodiment, W is

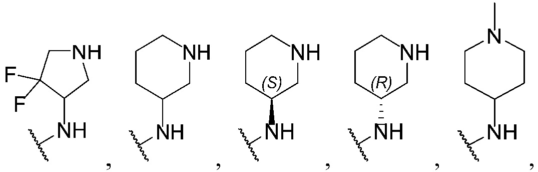

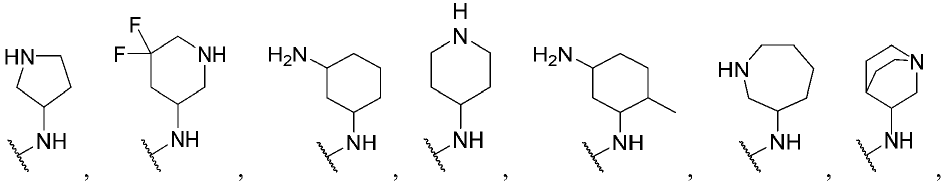

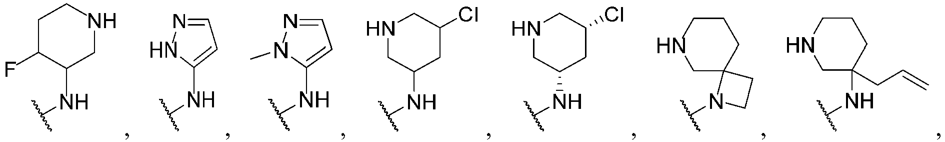

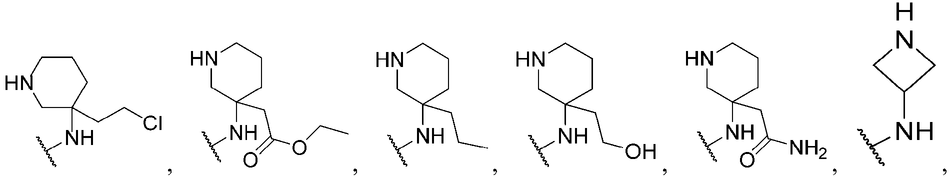

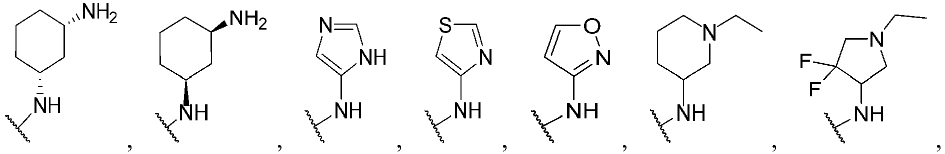

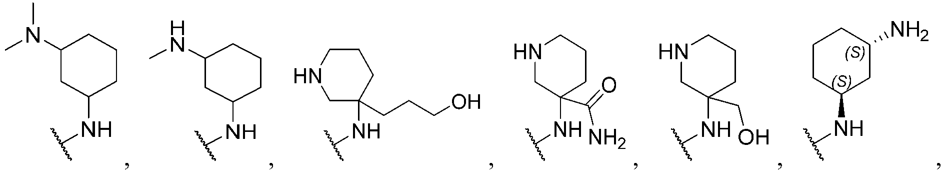

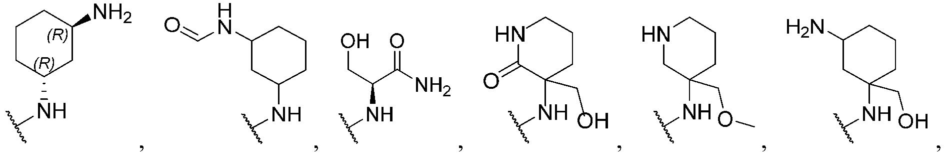

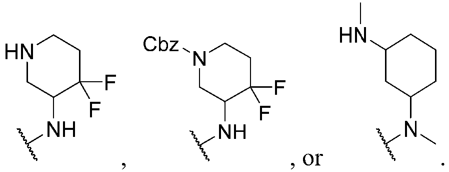

In a certain embodiment, W is

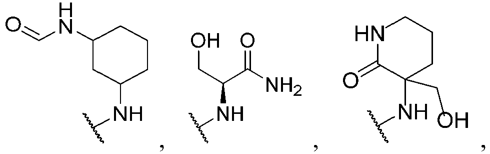

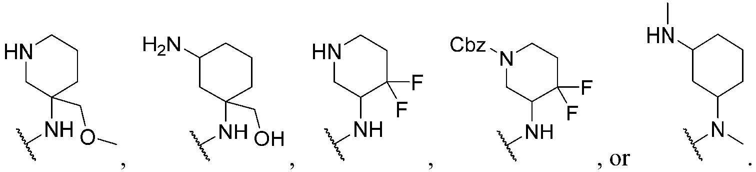

In a certain embodiment,
X is N or CH;
Y is O or -CH₂-;
L¹ is 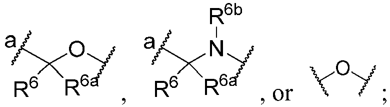 wherein the a-end is attached to U;
R⁶, R^{6a}, and R^{6b} are each independently H or C₁-C₆ alkyl;
U is C₆-C₁₀ aryl, 5- to 10-membered heteroaryl, C₆-C₁₀ aryl substituted by one or more R⁹⁻¹, or 5- to 10-membered heteroaryl substituted by one or more R⁹⁻²;
R⁹⁻¹ and R⁹⁻² are each independently halogen, hydroxyl, C₁-C₆ alkyl, C₁-C₆ alkoxy, 3-to 8-membered cycloalkyl, 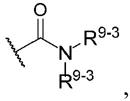 or C₁-C₆ alkyl substituted by one or more halogens;
each R⁹⁻³ is independently H or C₁-C₆ alkyl;
R¹, R², and R³ are each independently H, halogen, or C₁-C₆ alkyl;
R⁴ and R^{4a} are each independently H or C₁-C₆ alkyl;
or, R⁴ and R^{4a} together with the carbon atom to which they are attached form a 3- to 8-membered cycloalkyl ring;
R⁵ and R^{5a} are H;
W is L²R⁷;
L² is a direct bond, -CH₂-, or -NH-;
R⁷ is 3- to 12-membered heterocycloalkyl, 5- to 10-membered heteroaryl, 3- to 12-membered heterocycloalkyl substituted by one or more R⁷⁻¹, 5- to 10-membered heteroaryl substituted by one or more R⁷⁻², or 3- to 8-membered cycloalkyl substituted by one or more R⁷⁻⁴;
R⁷⁻¹, R⁷⁻², and R⁷⁻⁴ are each independently halogen, amino, -NHC₁-C₆ alkyl, C₁-C₆ alkyl, -N(C₁-C₆ alkyl)₂, 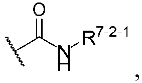 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, C₂-C₆ alkenyl, 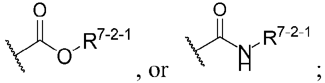
each R⁷⁻²⁻¹ is independently H or C₁-C₆ alkyl;
the heteroatom in the 3- to 12-membered heterocycloalkyl is selected from one or more of N, O, and S; the number of heteroatoms is 1, 2, or 3;
the heteroatom in the 5- to 10-membered heteroaryl is selected from one or more of N, O, and S; the number of heteroatoms is 1, 2, or 3.

In a certain embodiment,
X is N or CH;
Y is O or -CH₂-;
L¹ is 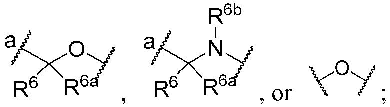 wherein the a-end is attached to U;
R⁶, R^{6a}, and R^{6b} are each independently H or C₁-C₆ alkyl;
U is C₆-C₁₀ aryl, 5- to 10-membered heteroaryl, 3- to 8-membered cycloalkyl, C₆-C₁₀ aryl substituted by one or more R⁹⁻¹, or 5- to 10-membered heteroaryl substituted by one or more R⁹⁻²;
R⁹⁻¹ and R⁹⁻² are each independently halogen, hydroxyl, C₁-C₆ alkyl, C₁-C₆ alkoxy, 3-to 8-membered cycloalkyl, 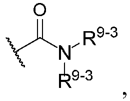 or C₁-C₆ alkyl substituted by one or more halogens;
each R⁹⁻³ is independently H or C₁-C₆ alkyl;
R¹, R², and R³ are each independently H, halogen, or C₁-C₆ alkyl;
R⁴ and R^{4a} are each independently H or C₁-C₆ alkyl;
or, R⁴ and R^{4a} together with the carbon atom to which they are attached form a 3- to 8-membered cycloalkyl ring;
R⁵ and R^{5a} are H;
W is L²R⁷;
L² is a direct bond, -CH₂-, or -NH-;
R⁷ is 3- to 12-membered heterocycloalkyl, 5- to 10-membered heteroaryl, 3- to 12-membered heterocycloalkyl substituted by one or more R⁷⁻¹, 5- to 10-membered heteroaryl substituted by one or more R⁷⁻², C₁-C₆ alkyl substituted by one or more R⁷⁻³, or 3- to 8-membered cycloalkyl substituted by one or more R⁷⁻⁴;
R⁷⁻¹, R⁷⁻², and R⁷⁻⁴ are each independently halogen, amino, -NHC₁-C₆ alkyl, C₁-C₆ alkyl, -N(C₁-C₆ alkyl)₂, 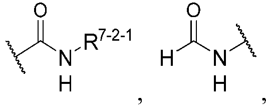 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, C₁-C₆ alkoxy, C₂-C₆ alkenyl, 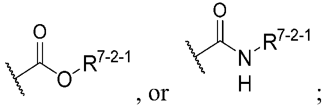
each R⁷⁻³ is independently hydroxyl or 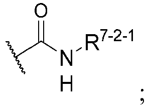 each R⁷⁻²⁻¹ is independently H, C₁-C₆ alkyl, or C₁-C₆ alkyl substituted by one or more C₆-C₁₀ aryl;
the heteroatom in the 3- to 12-membered heterocycloalkyl is selected from one or more of N, O, and S; the number of heteroatoms is 1, 2, or 3;
the heteroatom in the 5- to 10-membered heteroaryl is selected from one or more of N, O, and S; the number of heteroatoms is 1, 2, or 3.

In a certain embodiment, the compound I according to the present disclosure is compound I-1, I-2, or I-3, 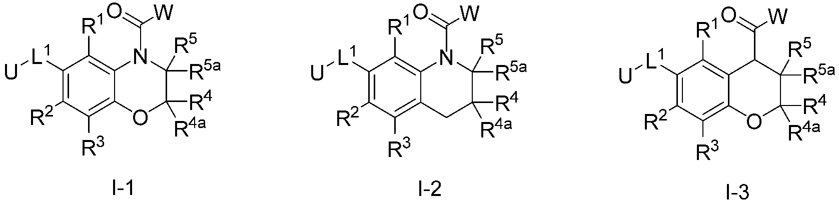 wherein R¹, R², R³, R⁴, R^{4a}, R⁵, R^{5a}, L¹, U, and W are as defined above.

In a certain embodiment, the compound I or the stereoisomer thereof is selected from any one of the following compounds:

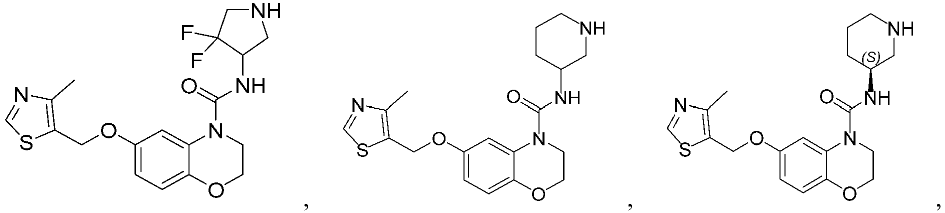

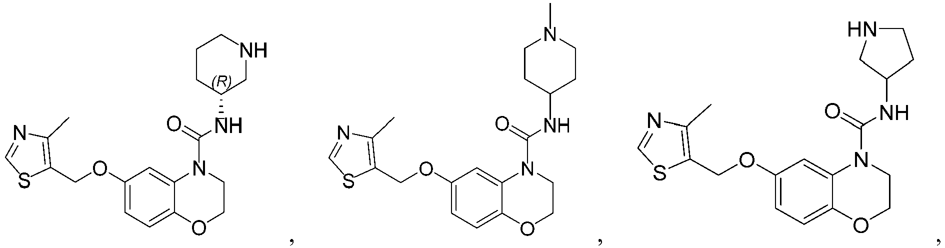

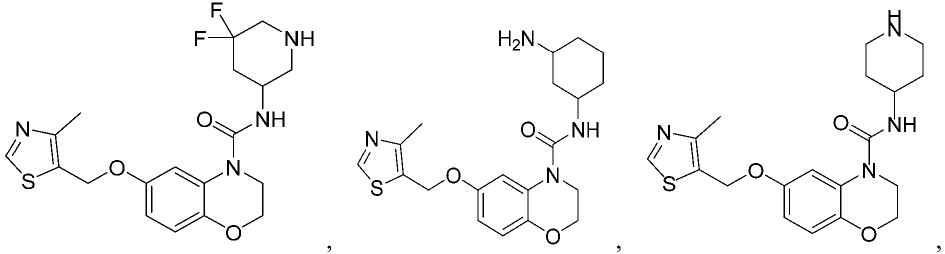

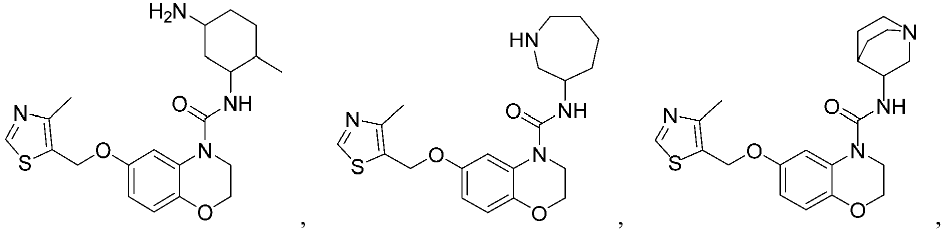

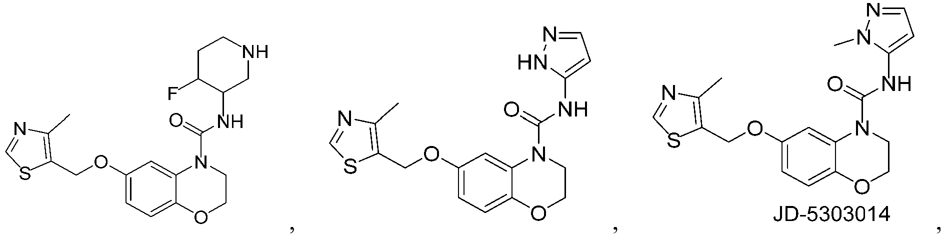

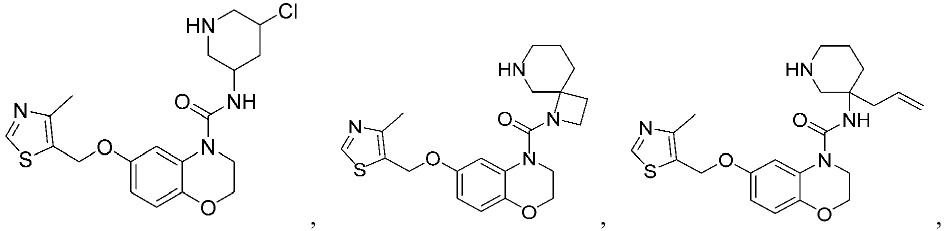

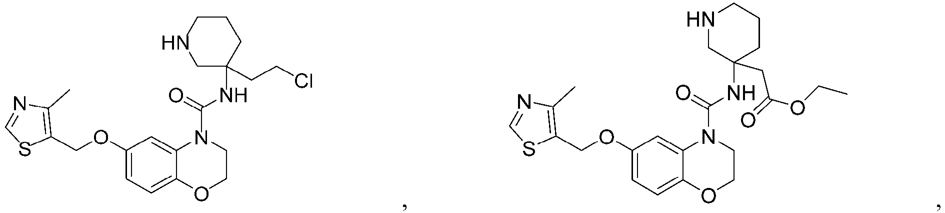

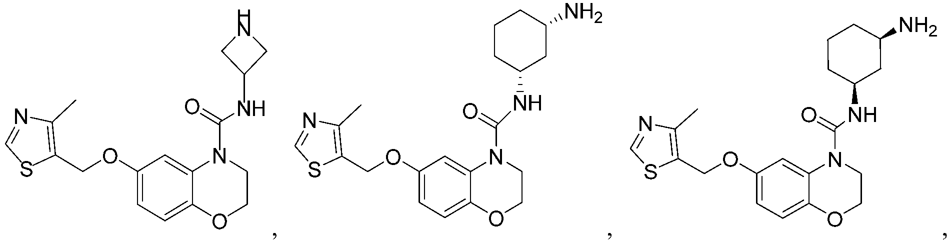

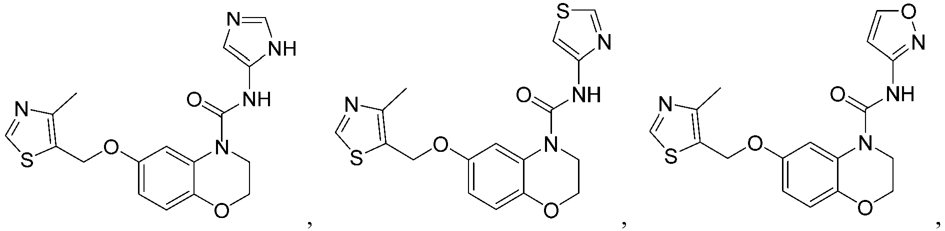

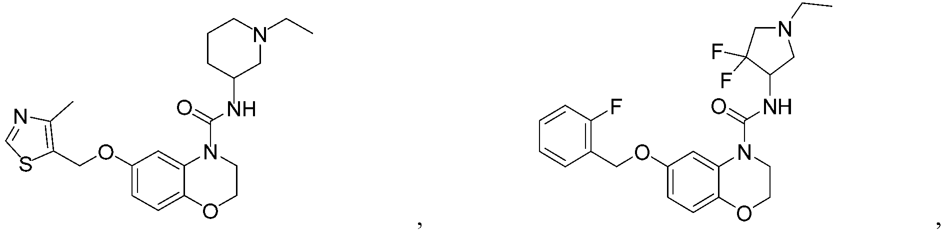

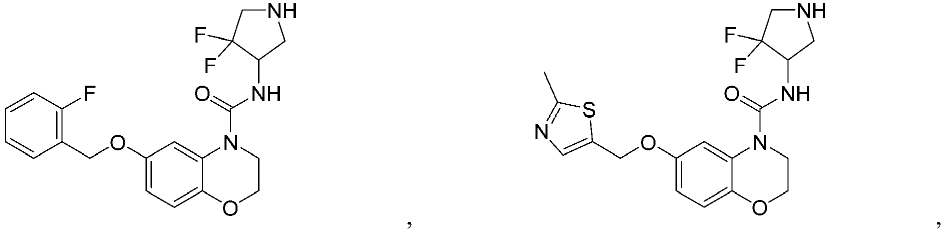

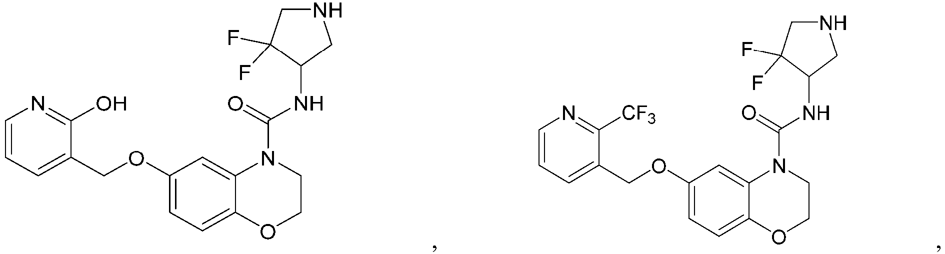

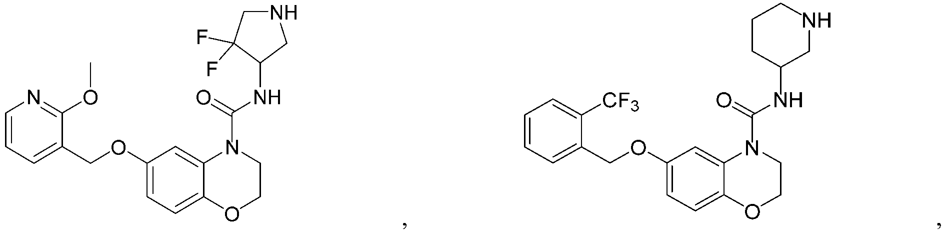

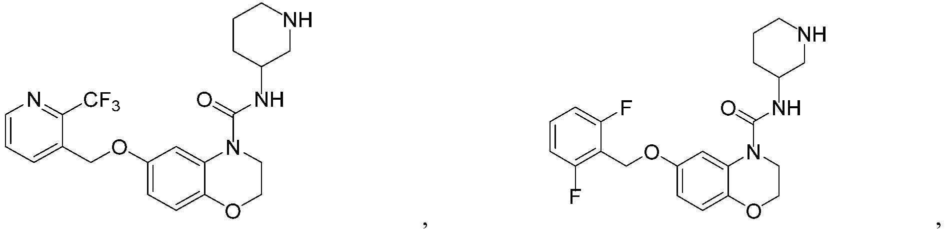

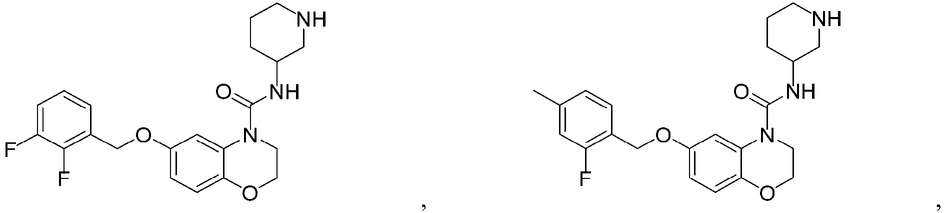

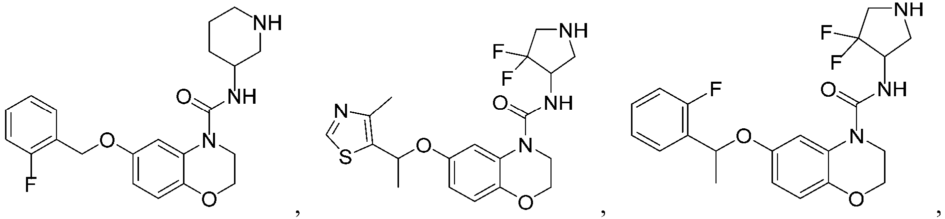

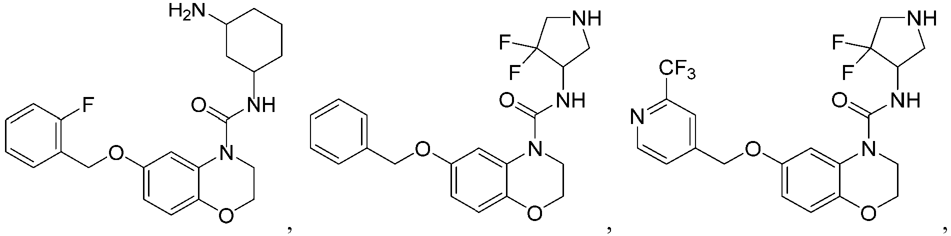

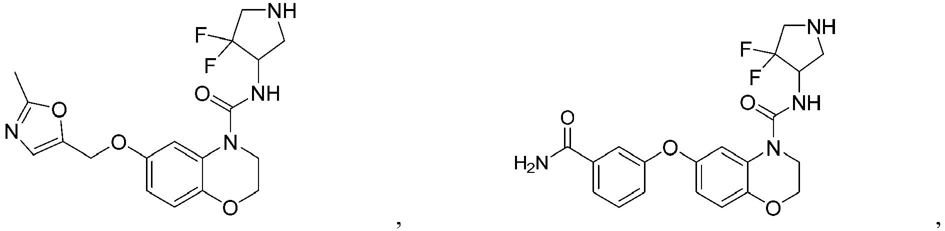

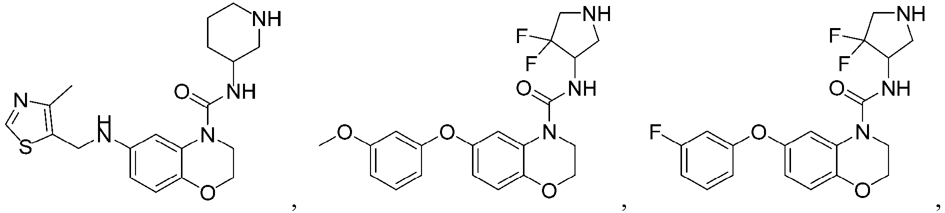

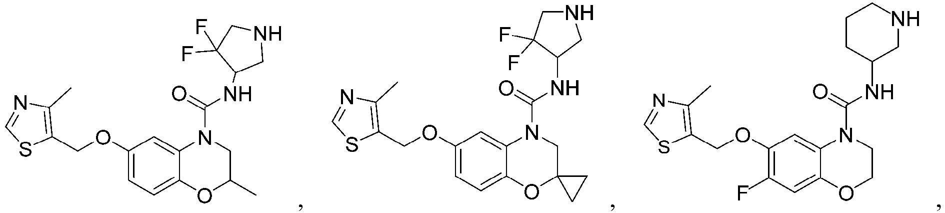

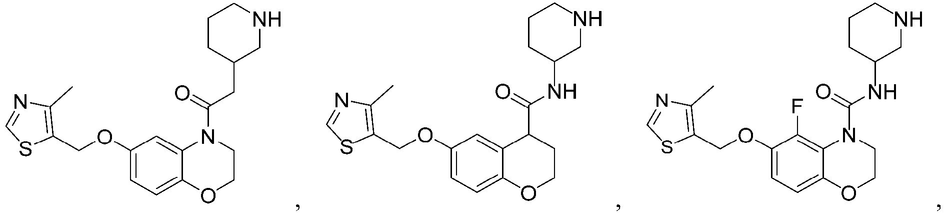

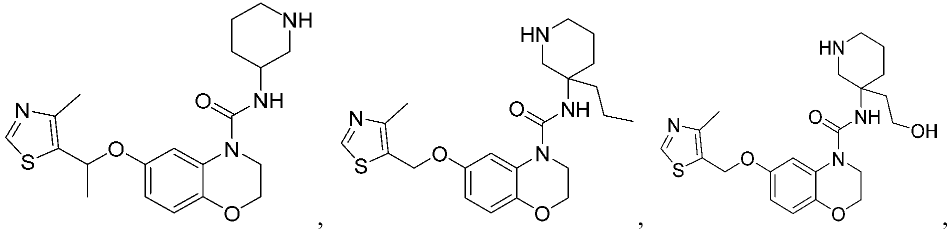

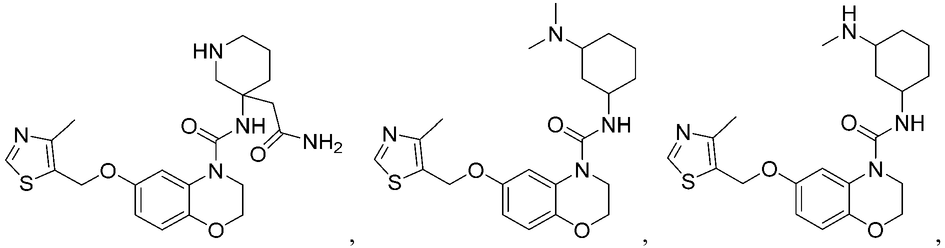

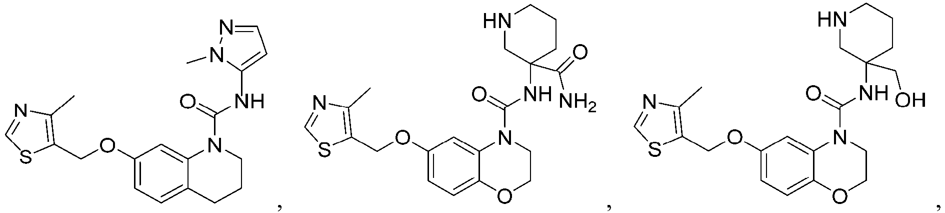

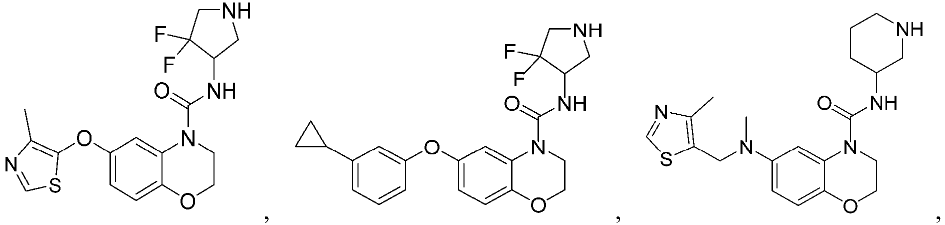

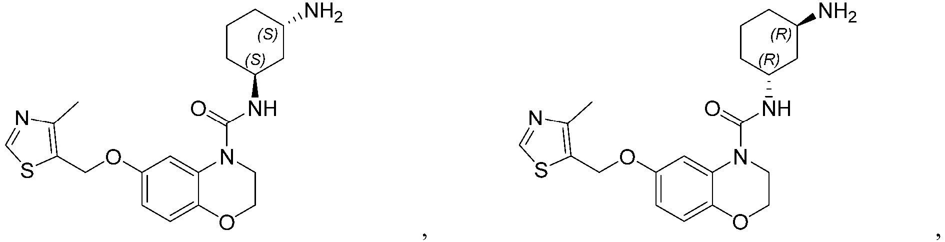

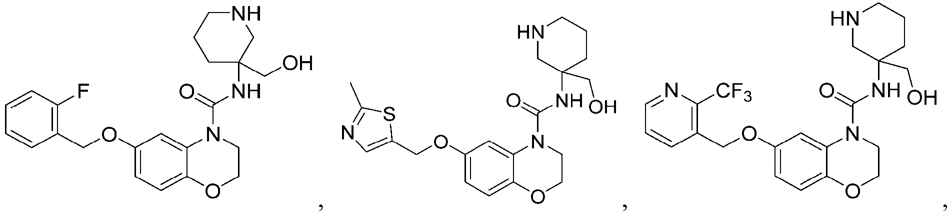

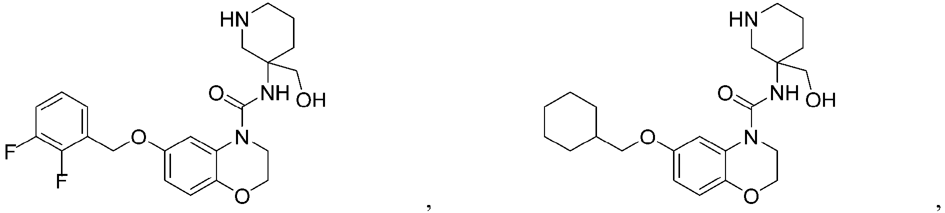

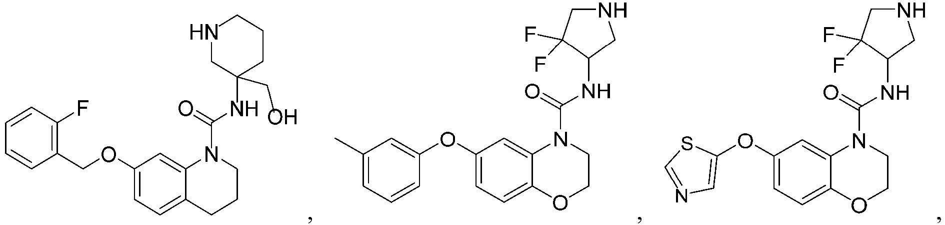

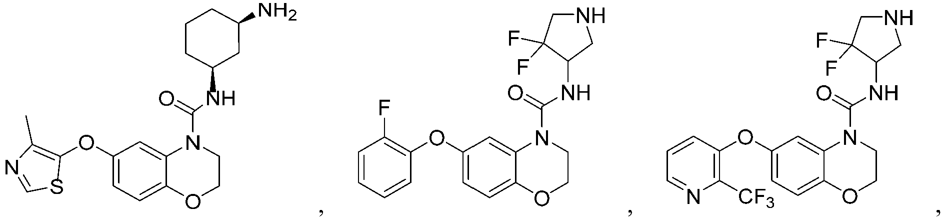

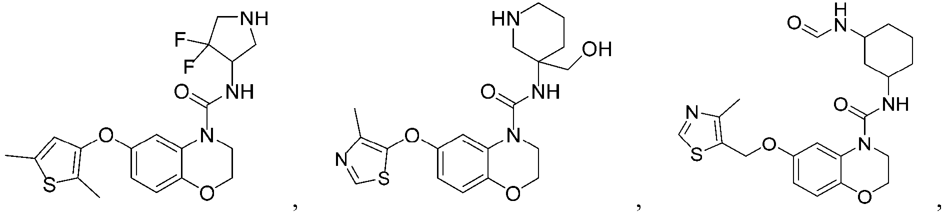

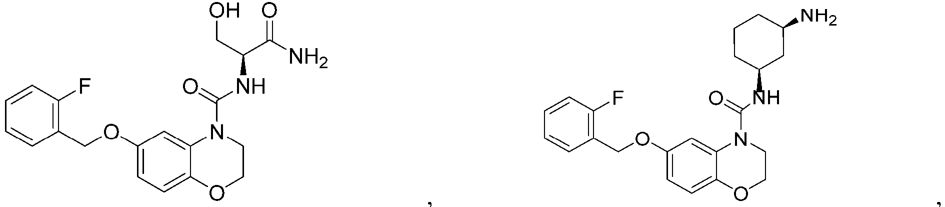

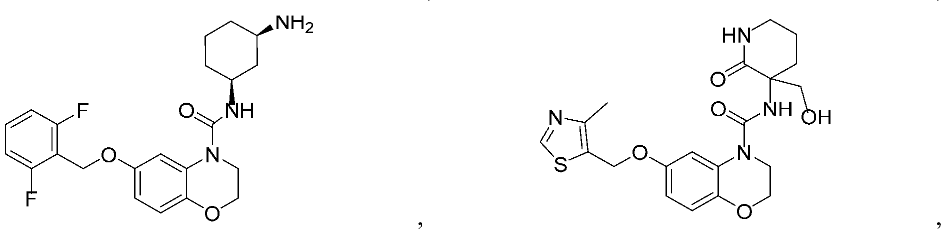

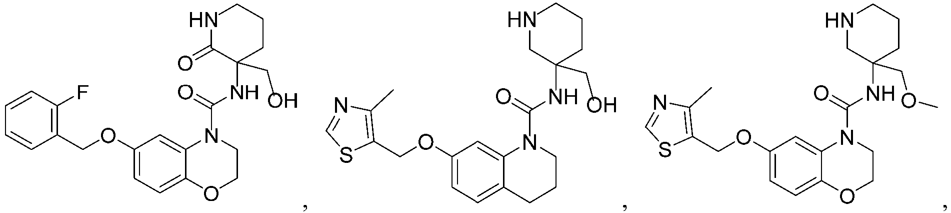

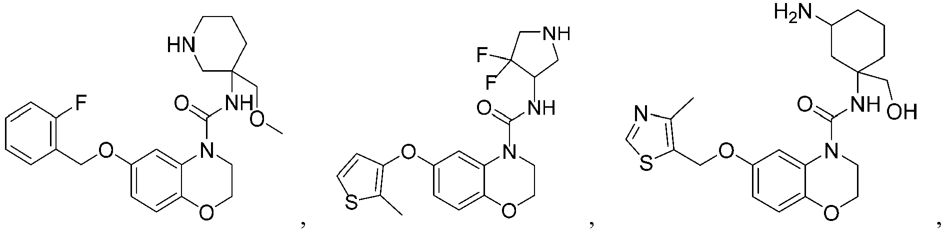

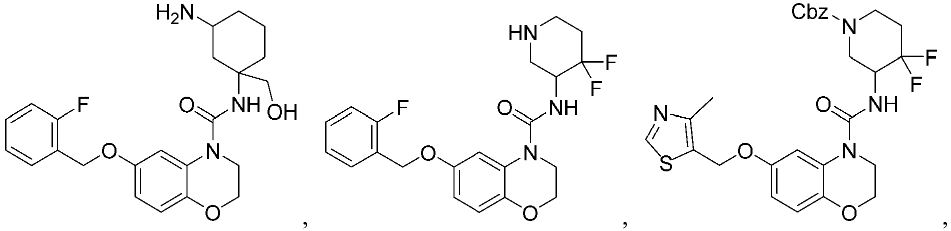

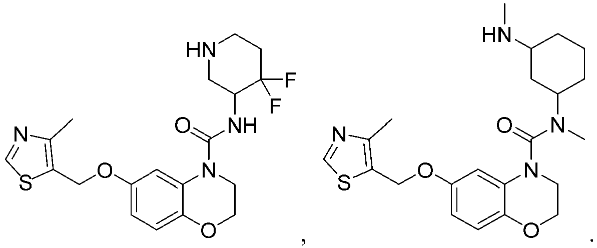

In a certain embodiment, the pharmaceutically acceptable salt of the compound I is selected from any one of the following compounds:

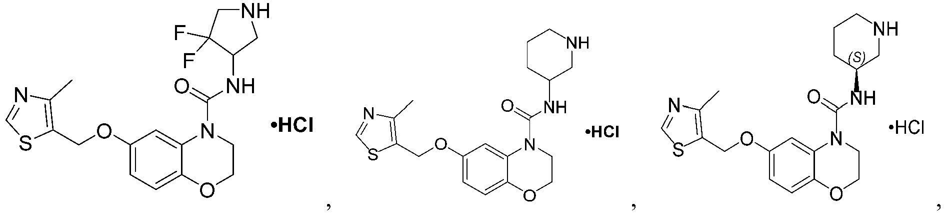

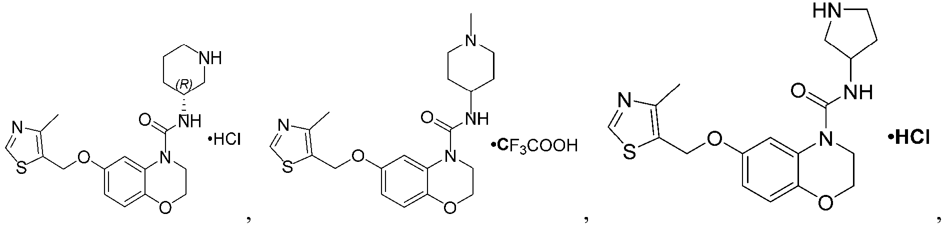

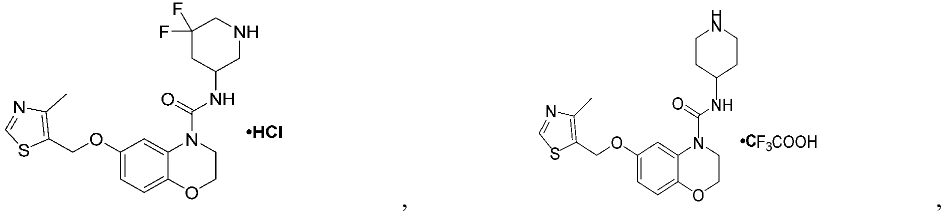

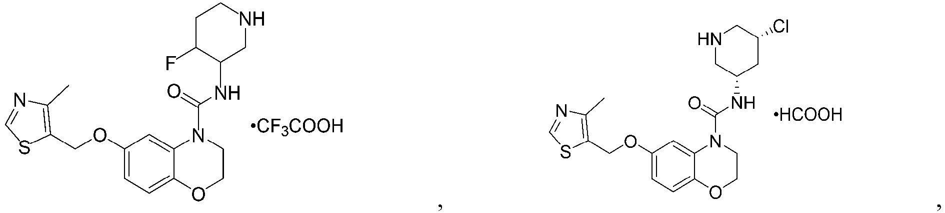

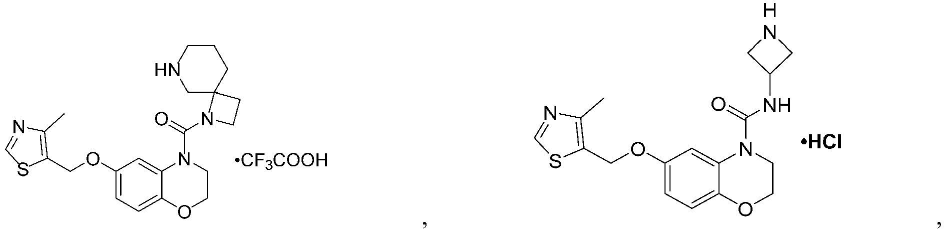

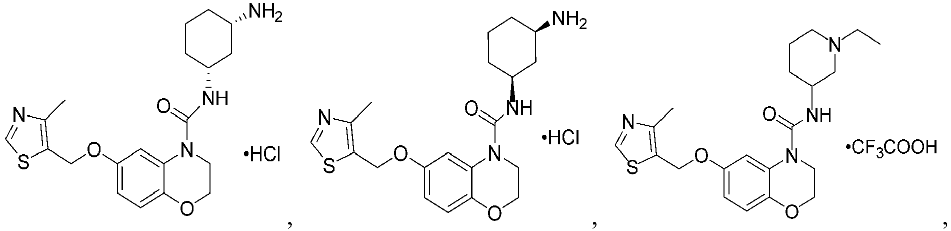

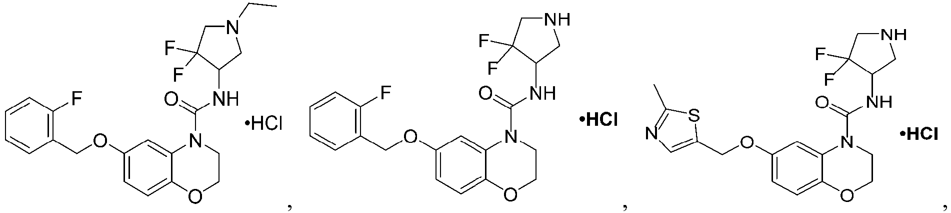

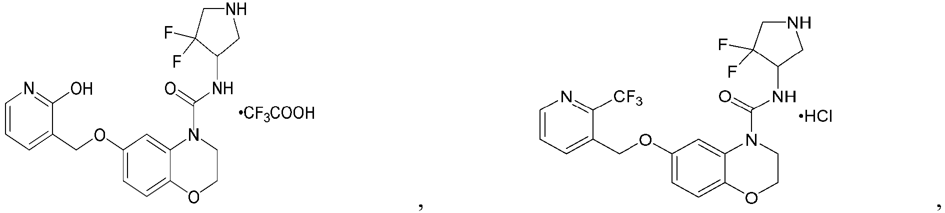

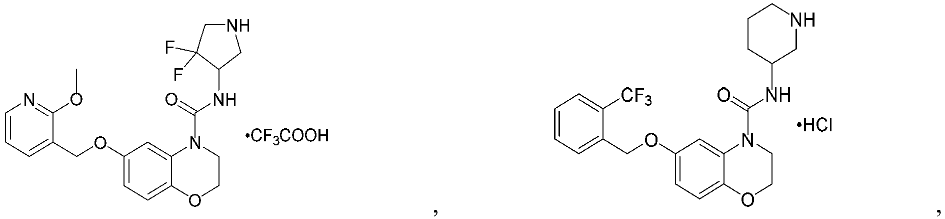

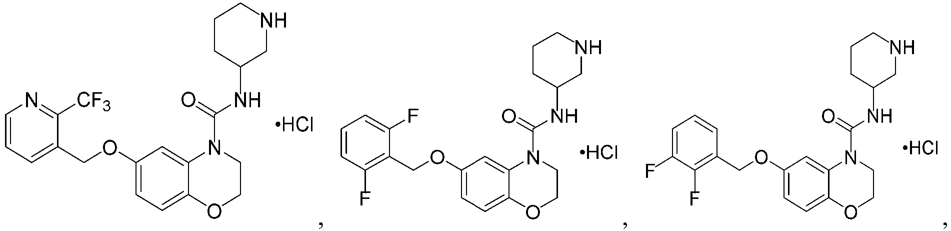

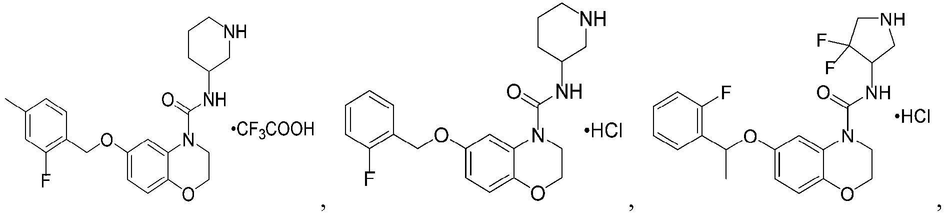

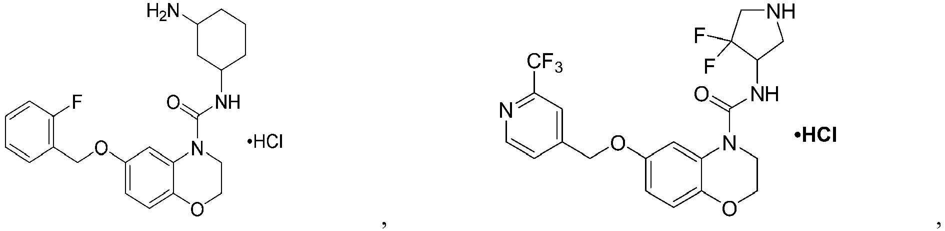

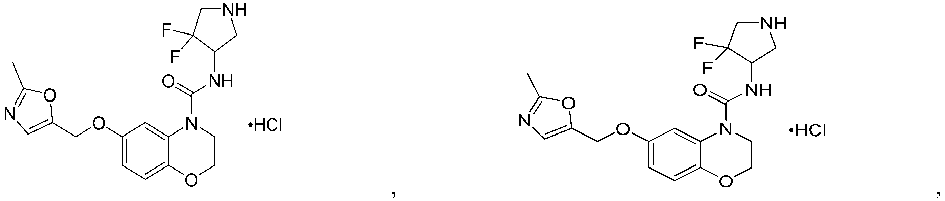

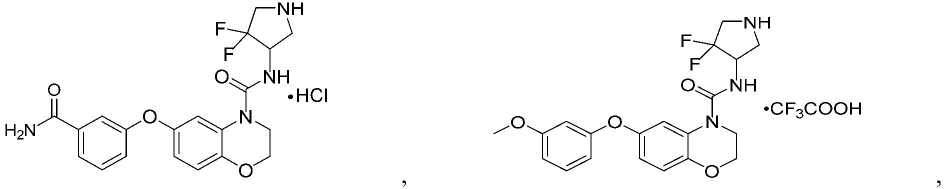

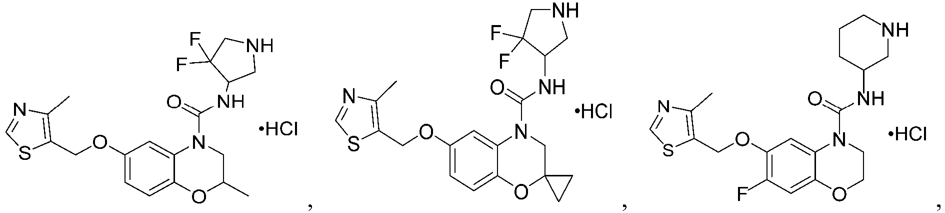

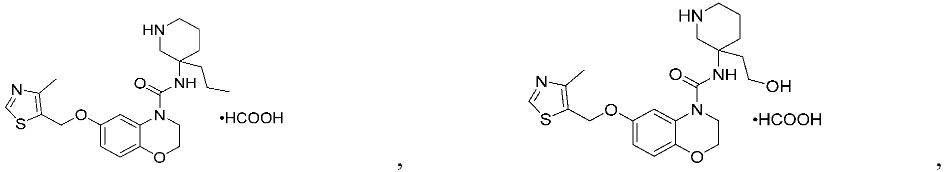

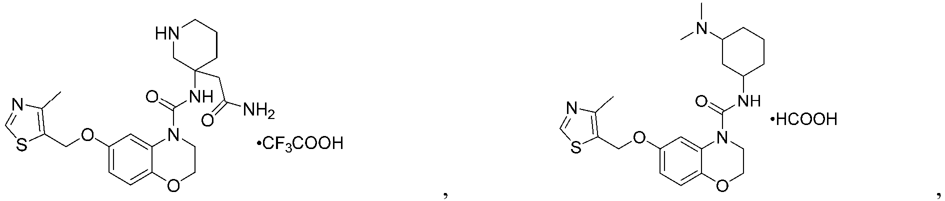

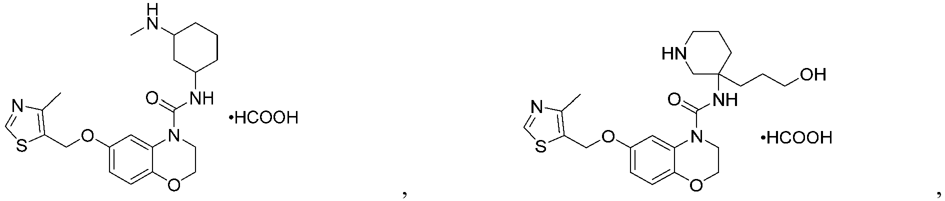

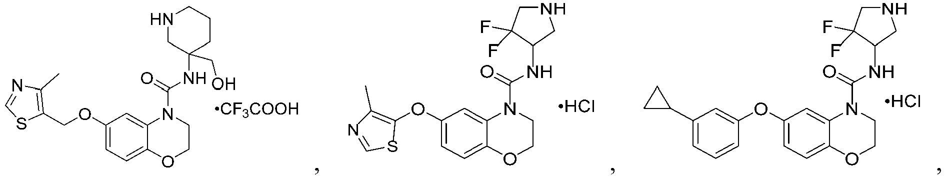

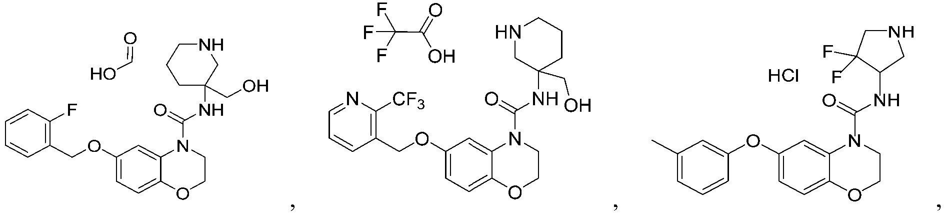

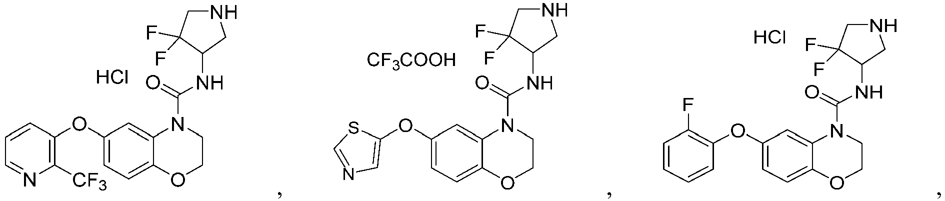

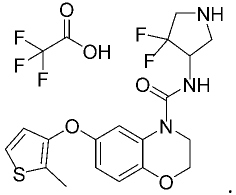

The present disclosure further provides a compound II, 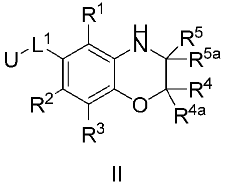 wherein R¹, R², R³, R⁴, R^{4a}, R⁵, R^{5a}, L¹, and U are as defined above.

In another aspect, the present disclosure further provides a pharmaceutical composition comprising the compound I, the stereoisomer thereof, or the pharmaceutically acceptable salt thereof as described above, and at least one pharmaceutically acceptable excipient.

In another aspect, the present disclosure further provides a use of the compound I, the stereoisomer thereof, or the pharmaceutically acceptable salt thereof as described above, or the pharmaceutical composition as described above in the manufacture of a medicament for treating pain; wherein the pain is preferably inflammatory pain.

In another aspect, the present disclosure further provides a use of the compound I, the stereoisomer thereof, or the pharmaceutically acceptable salt thereof as described above, or the pharmaceutical composition as described above in the manufacture of a medicament for preventing and/or treating a TRPM3-mediated disease; wherein the TRPM3-mediated disease is preferably pain, more preferably inflammatory pain.

In another aspect, the present disclosure further provides a use of the compound I, the stereoisomer thereof, or the pharmaceutically acceptable salt thereof as described above, or the pharmaceutical composition as described above in the manufacture of a TRPM3 antagonist. The TRPM3 antagonist may be used *in vivo* in mammals; or *in vitro,* primarily for experimental purposes, such as: serving as a standard sample or control sample for comparison, or being formulated into a kit according to conventional methods in the art to provide rapid detection of TRPM3 antagonistic effects.

### Terminology

Unless otherwise specified, the definitions of groups and terms described in the description and claims of the present disclosure include definitions thereof as examples, exemplary definitions, preferred definitions, definitions recorded in tables, definitions of specific compounds in the examples, *etc.,* which can be arbitrarily combined and integrated with each other. Such combined and integrated definitions of groups and compound structures should fall within the scope of the description of the present disclosure.

Certain chemical groups defined herein are preceded by a shorthand notation to indicate the total number of carbon atoms present in the groups. For example, C₁-C₆ alkyl or C₁₋₆ alkyl refers to an alkyl group as defined below containing a total of 1, 2, 3, 4, 5, or 6 carbon atoms.

Numerical ranges defined herein in substituents, such as 0 to 10, 1 to 6, and 1 to 3, indicate integers within the range; for example, 1 to 6 indicates 1, 2, 3, 4, 5, or 6.

The term "more" refers to 2, 3, 4, 5, 6, 7, 8, 9, 10, or more, preferably 2, 3, or 4.

The term "alkyl" refers to a saturated, linear or branched monovalent hydrocarbon group with a specified number of carbon atoms (*e.g*., C₁-C₆or C₁-C₄). Alkyl groups include, but are not limited to, methyl, ethyl, *n*-propyl, isopropyl, *n*-butyl, isobutyl, *sec*-butyl, *tert-butyl, n*-pentyl, *n*-hexyl, *etc.*

The term "alkenyl" refers to an unsaturated, linear or branched monovalent hydrocarbon group with a specified number of carbon atoms (*e.g.,* C₂-C₆) and one or more (*e.g.,* 1, 2, or 3) carbon-carbon sp² double bonds. Alkenyl groups include, but are not limited to, vinyl, 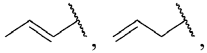 *etc.*

The term "alkynyl" refers to an unsaturated, linear or branched monovalent hydrocarbon group with a specified number of carbon atoms (*e.g.,* C₂-C₆) and one or more (*e.g.,* 1, 2, or 3) carbon-carbon sp³ triple bonds. Alkynyl groups include, but are not limited to, ethynyl, 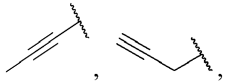 *etc.*

The term "alkoxy" refers to the group R^{X}-O-, and R^{X} is defined in the same way as the term "alkyl". Alkoxy groups include, but are not limited to, methoxy, ethoxy, *n*-propoxy, isopropoxy, *etc.*

The term "halogen" refers to F, Cl, Br, or I.

The term "cycloalkyl" refers to a saturated, cyclic, monovalent hydrocarbon group with a specified number of carbon atoms (*e.g.*, C₃-C₁₀or C₃-C₆), which is a monocyclic ring. Cycloalkyl groups include, but are not limited to, 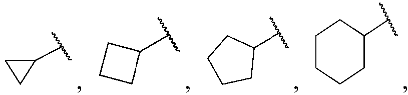 *etc.*

The term "oxo" refers to =O, indicating the substitution of two hydrogen atoms on the same carbon atom by an oxygen atom, *i.e.,* a methylene group is substituted by a carbonyl group; for example, 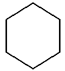 is converted into 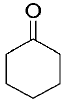 upon oxo substitution.

The term "heterocycloalkyl" refers to a saturated, cyclic, monovalent group with a specified number of ring atoms (*e.g.,* 3- to 12-membered, 4- to 10-membered, or 4- to 6-membered), a specified number of heteroatoms (*e.g*., 1, 2, or 3), and a specified type of heteroatom (one or more of N, O, and S), which is a monocyclic ring or a polycyclic ring, wherein the polycyclic ring may be a bridged ring, a fused ring, or a spiro ring, and may also be a bicyclic ring or a tricyclic ring. The heterocycloalkyl group is attached to the rest of a molecule by a carbon atom or heteroatom. Heterocycloalkyl groups include, but are not limited to, 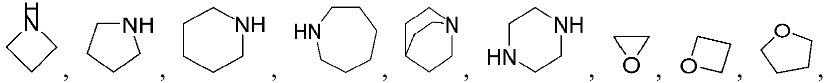 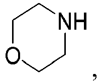 *etc.*

The term "heteroaryl" refers to an unsaturated, cyclic, monovalent group with a specified number of ring atoms (*e.g.,* 5- to 10-membered or 5- to 6-membered), a specified number of heteroatoms (*e.g*., 1, 2, or 3), and a specified type of heteroatom (one or more of N, O, and S), which is a monocyclic ring or a polycyclic ring, wherein the monocyclic rings share two atoms and one bond, and each ring is aromatic. The heteroaryl group is attached to the rest of a molecule by a carbon atom or heteroatom; the heteroaryl group is attached to the rest of a molecule by a ring with or without heteroatoms. Heteroaryl groups include, but are not limited to, 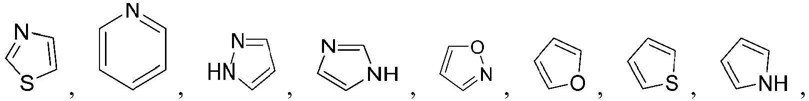 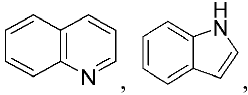 *etc.*

The term "therapeutically effective amount" refers to an amount administered to a patient that is sufficient to effectively treat a disease. The therapeutically effective amount may vary depending on factors such as the type of compound, the type of the disease, the severity of the disease, and the age of the patient, and can be adjusted by those skilled in the art as appropriate.

The term "pharmaceutically acceptable excipient" refers to all substances included in a pharmaceutical preparation other than the active pharmaceutical ingredient, generally classified into two categories: excipients and additives. For details, please refer to the Pharmacopoeia of the People's Republic of China (2020 Editi*on)* and the Handbook of Pharmaceutical Excipients (Paul J. Sheskey, Bruno C. Hancock, Gary P. Moss, David J. Goldfarb, 2020, 9th Edition).

The term "treatment" refers to eliminating the cause of a disease or alleviating symptoms thereof.

The term "prevention" refers to reducing the risk of developing a disease.

The term "patient" refers to any animal in need of treatment or prevention of a disease, typically a mammal, such as a human. Mammals include, but are not limited to, cattle, horses, sheep, pigs, cats, dogs, mice, rats, rabbits, guinea pigs, monkeys, humans, *etc.*

On the basis of not violating common knowledge in the art, the above preferred conditions can be arbitrarily combined to obtain preferred examples of the present disclosure.

The reagents and raw materials used in the present disclosure are all commercially available.

The positive and progressive effects of the present disclosure are that the compounds provided by the present disclosure exhibit good TRPM3 inhibitory activity and hold promise for treating related diseases.

### DETAILED DESCRIPTION OF THE PREFERRED EMBODIMENT

The present disclosure is further illustrated below by means of examples, but the present disclosure is not limited to the scope of the examples. The experimental methods for which specific conditions are not indicated in the following examples are selected according to conventional methods and conditions, or according to the product instructions.

### Example 1: Preparation of N-(4,4-difluorotetrahydro-1H-pyrrol-3-yl)-6-{[(4-methyl-1,3-thiazol-5-yl)methyl]oxy}-3,4-dihydro-2H-benzo[b][1,4]oxazine-4-carboxamide hydrochloride (JD-5303001)

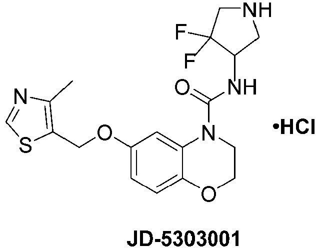

### Step 1: Preparation of 6-(4,4,5,5-tetramethyl-1,3,2-dioxaborolan-2-yl)-3,4-dihydro-2H-benzo[b][1,4]oxazine (JD-5303001-2)

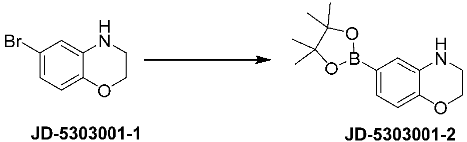

6-Bromo-3,4-dihydro-2*H*-benzo[*b*][1,4]oxazine (**JD-5303001-1**) (10 g, 46.72 mmol, 1.00 eq) was dissolved in dioxane (200 mL), followed by the addition of Pd(dppf)Cl₂ (1.71 g, 2.34 mmol, 0.05 eq), BPD (23.73 g, 93.43 mmol, 2 eq), and KOAc (11.46 g, 116.79 mmol, 2.50 eq), The reaction mixture was stirred at 100°C for 2 hours. The reaction mixture was filtered, and the filtrate was concentrated under reduced pressure to obtain a residue. The residue was purified by column chromatography (SiO₂, petroleum ether/ethyl acetate = 100/1 to 5/1) to obtain 6-(4,4,5,5-tetramethyl-1,3,2-dioxaborolan-2-yl)-3,4-dihydro-2*H-*benzo[*b*][1,4]oxazine (**JD-5303001-2**) (12 g, 45.96 mmol, yield: 98.37%) as a yellow oil.

LC-MS [M+H]⁺ = 262.0.

### Step 2: Preparation of 3,4-dihydro-2H-benzo[b][1,4]oxazin-6-ol (JD-5303001-3)

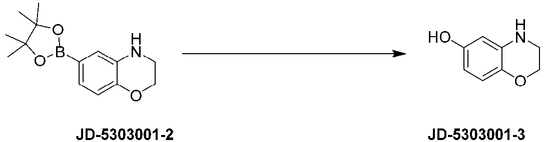

6-(4,4,5,5-Tetramethyl-1,3,2-dioxaborolan-2-yl)-3,4-dihydro-2*H-*benzo[*b*][1,4]oxazine (**JD-5303001-2**) (11 g, 42.13 mmol, 1 eq) was dissolved in a mixture of tetrahydrofuran (110 mL) and water (110 mL), followed by the addition of AcOH (12.65 g, 210.63 mmol, 12.06 mL, 5.0 eq) and H₂O₂ (47.79 g, 421.50 mmol, 40.50 mL, purity: 30%, 10.01 eq). The reaction mixture was stirred at 0°C for 2 hours. The reaction mixture was quenched with Na₂SO₃ (aq., 600 mL) at 0°C and extracted with EtOAc (900 mL, 300 mL * 3). The combined organic phases were dried over anhydrous sodium sulfate, filtered, and concentrated under reduced pressure to obtain a residue. The residue was purified by column chromatography (SiO₂, petroleum ether/ethyl acetate = 50/1 to 1) to obtain 3,4-dihydro-2*H-*benzo[*b*][1,4]oxazin-6-ol (**JD-5303001-3**) (4.17 g, 27.59 mmol, yield: 65.49%) as a brown oil.

LC-MS [M] = 150.1.

### Step 3: Preparation of (4-methyl-1,3-thiazol-5-yl)methanol (JD-5303001b)

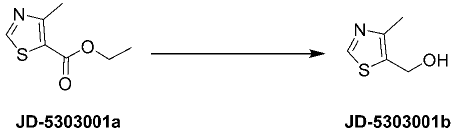

LAH (2.00 M, 29.2 mL, 2.00 eq) was dissolved in THF (50.0 mL), and the mixture was stirred for 30 minutes. Ethyl 4-methyl-1,3-thiazole-5-carboxylate (**JD-5303001a**) was then added thereto, and the reaction mixture was stirred at 0°C for 3 hours. The reaction mixture was quenched with H₂O (2.00 mL) at -10°C, then diluted with 15% NaOH (2.00 mL) and H₂O (6.00 mL), stirred at 25°C for 15 minutes, and concentrated under reduced pressure to obtain a residue. The residue was purified by column chromatography (SiO₂, petroleum ether/ethyl acetate = 1/1) to obtain (4-methyl-1,3-thiazol-5-yl)methanol (**JD-5303001b**) (2.50 g, 19.3 mmol, yield: 66.2%) as a yellow solid.

LC-MS [M+H]⁺ = 130.3.

### Step 4: Preparation of 5-(chloromethyl)-4-methyl-1,3-thiazole (JD-5303001c)

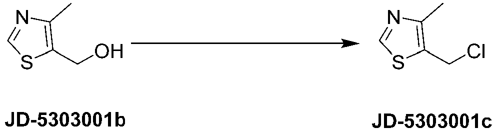

(4-Methyl-1,3-thiazol-5-yl)methanol (**JD-5303001b**) (400 mg, 3.10 mmol, 1.00 eq) and SOCl₂ were dissolved in THF (25.0 mL), and the reaction mixture was stirred at 25°C for 1 hour. The reaction mixture was quenched with H₂O (30.0 mL) and extracted with EtOAc (30.0 mL). The combined organic phases were separated, filtered, and concentrated under reduced pressure to obtain 5-(chloromethyl)-4-methyl-1,3-thiazole (**JD-5303001c**) (500 mg, crude product) as a colorless oil.

### Step 5: Preparation of 6-{[(4-methyl-1,3-thiazol-5-yl)methyl]oxy}-3,4-dihydro-2H-benzo[b][1,4]oxazine (JD-5303001-4)

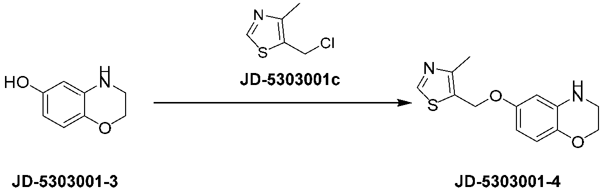

3,4-Dihydro-2*H*-benzo[*b*][1,4]oxazin-6-ol (**JD-5303001-3**) (0.20 g, 1.32 mmol, 1.00 eq) was dissolved in DMF (2 mL), followed by the addition of K₂CO₃ (365 mg, 2.65 mmol, 2.00 eq) and 5-(chloromethyl)-4-methyl-1,3-thiazole (**JD-5303001c**) (292 mg, 1.98 mmol, 1.50 eq). The reaction mixture was stirred at 80°C for 16 hours. The reaction mixture was washed with H₂O (10 mL) and EtOAc (10 mL, 5 mL * 2). The combined organic phases were concentrated under reduced pressure to obtain a residue. The residue was purified by preparative TLC (SiO₂, petroleum ether/ethyl acetate = 1:1) to obtain 6-{[(4-methyl-1,3-thiazol-5-yl)methyl]oxy}-3,4-dihydro-2*H*-benzo[*b*][1,4]oxazine (**JD-5303001-4**) (0.12 g, 457 µmol, yield: 34.5%) as a colorless oil.

LC-MS [M+H]⁺ = 263.1.

### Step 6: Preparation of tert-butyl 3,3-difluoro-4-{[(6-{[(4-methyl-1,3-thiazol-5-yl)methyl]oxy}-3,4-dihydro-2H-benzo[b][1,4]oxazin-4-yl)carbonyl]amino}pyrrolidine-1-carboxylate (JD-5303001-5)

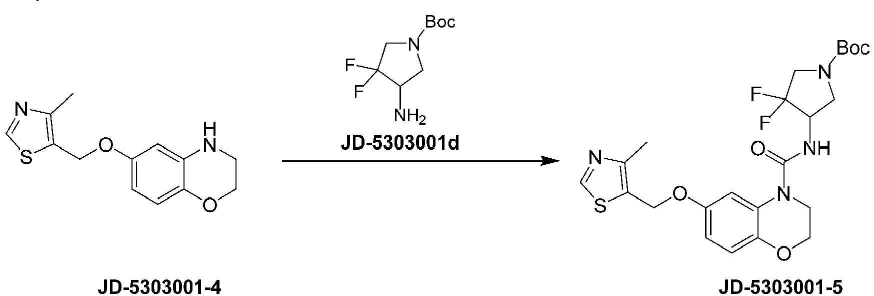

6-{[(4-Methyl-1,3-thiazol-5-yl)methyl]oxy}-3,4-dihydro-2*H*-benzo[*b*][1,4]oxazine (**JD-5303001-4**) (20.3 mg, 91.4 µmol, 1.20 eq) and DIEA (39.4 mg, 304 µmol, 53.1 µL, 4.00 eq) were dissolved in THF (1.00 mL), and the system was purged with N₂ three times. The mixture was cooled to 0°C. BTC (0.09 g, 303 µmol, 0.40 eq) was added thereto, and the mixture was stirred at 25°C for 1 hour. *tert*-Butyl 4-amino-3,3-difluoropyrrolidine-1-carboxylate (**JD-5303001d**) (20.0 mg, 76.2 µmol, 1.00 eq) was then added thereto, and the mixture was stirred at 25°C for 2 hours. TLC (petroleum ether: ethyl acetate = 1:1, Rf = 0.53) indicated complete consumption of the starting material and formation of a new spot. The reaction mixture was quenched with H₂O (5 mL) at 0°C, then extracted with ethyl acetate (15 mL, 5 mL * 3), and concentrated under reduced pressure to obtain a residue. The residue was purified by preparative HPLC to obtain *tert*-butyl 3,3-difluoro-4-{[(6-{[(4-methyl-1,3-thiazol-5-yl)methyl]oxy}-3,4-dihydro-2*H*-benzo[*b*][1,4]oxazin-4-yl)carbonyl]amino}pyrrolidine-1-carboxylate (**JD-5303001-5**) (25.0 mg) as a white solid.

### Step 7: Preparation of N-(4,4-difluorotetrahydro-1H-pyrrol-3-yl)-6-{[(4-methyl-1,3-thiazol-5-yl)methyl]oxy}-3,4-dihydro-2H-benzo[b][1,4]oxazine-4-carboxamide hydrochloride (JD-5303001)

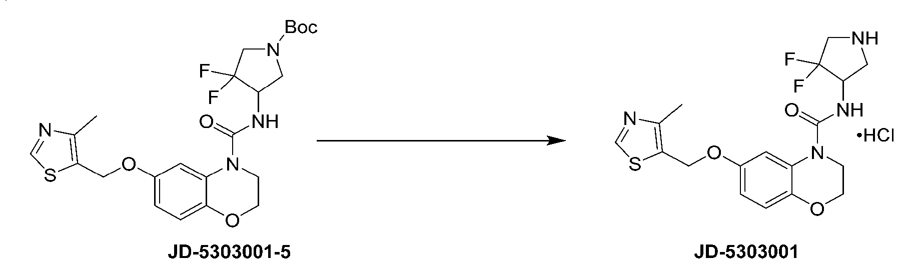

To TFE (1.00 mL) were added tert-butyl 3,3-difluoro-4-{[(6-{[(4-methyl-1,3-thiazol-5-yl)methyl]oxy}-3,4-dihydro-2*H*-benzo[*b*][1,4]oxazin-4-yl)carbonyl]amino}pyrrolidine-1-carboxylate (**JD-5303001-5**) (40.0 mg, 78.3 µmol, 1.00 eq) and TMSCl (17.0 mg, 156 µmol, 19.8 µL, 2 eq), and the mixture was stirred at 25°C for 2 hours. The reaction mixture was filtered and concentrated under reduced pressure to obtain compound **JD-5303001** (23.0 mg, 51.4 µmol, yield: 65.6%, hydrochloride).

LC-MS [M+H]⁺ = 411.0.

**¹H NMR:** (400 MHz, DMSO-*d*₆) δ = 2.40 (s, 3 H), 3.40 - 3.51 (m, 1 H), 3.74 (br s, 5 H), 4.05 - 4.22 (m, 2 H), 4.71 - 4.87 (m, 1 H), 5.18 (s, 2 H), 6.66 (dd, *J =* 8.88, 2.88 Hz, 1 H), 6.79 (d, *J =* 8.88 Hz, 1 H), 7.35 (d, *J =* 2.88 Hz, 1 H), 7.61 (d, *J =* 8.38 Hz, 1 H), 9.10 (s, 1 H), 10.02 - 10.46 (m, 2 H).

### Example 2: Preparation of N-(piperidin-3-yl)-6-{[(4-methyl-1,3-thiazol-5-yl)methyl]oxy}-3,4-dihydro-2H-benzo[b][1,4]oxazine-4-carboxamide hydrochloride (JD-5303002)

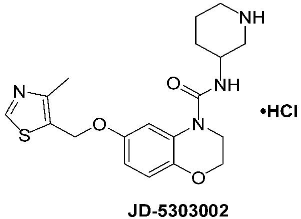

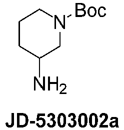

Following the synthetic method of Example 1, compound **JD-5303001d** was replaced with **JD-5303002a** to obtain *N*-(piperidin-3-yl)-6-{[(4-methyl-1,3-thiazol-5-yl)methyl]oxy}-3,4-dihydro-2*H*-benzo[*b*][1,4]oxazine-4-carboxamide hydrochloride (**JD-5303002**) (0.073 g, 181 µmol, yield: 88.5%, purity: 96.4%) as a yellow gum.

LC-MS [M+H]⁺ = 389.2.

¹H NMR (400 MHz, DMSO-*d*₆) δ ppm 1.52 - 1.73 (m, 2 H) 1.86 (br d, *J* = 10.88 Hz, 2 H) 2.40 (s, 3 H) 2.80 (br dd, *J =* 19.39, 9.51 Hz, 2 H) 3.07 - 3.25 (m, 2 H) 3.68 - 3.80 (m, 2 H) 3.89 - 4.01 (m, 1 H) 4.12 (br s, 2 H) 5.18 (s, 2 H) 6.61 (dd, *J* = 8.88, 2.88 Hz, 1 H) 6.76 (d, *J =* 8.88 Hz, 1 H) 7.17 (br d, *J =* 7.38 Hz, 1 H) 7.40 (d, *J =* 2.88 Hz, 1 H) 9.14 - 9.30 (m, 2 H) 9.42 (br s, 1 H).

### Example 3: Preparation of N-[(3S)-piperidin-3-yl]-6-{[(4-methyl-1,3-thiazol-5-yl)methyl]oxy}-3,4-dihydro-2H-benzo[b][1,4]oxazine-4-carboxamide hydrochloride (JD-5303003)

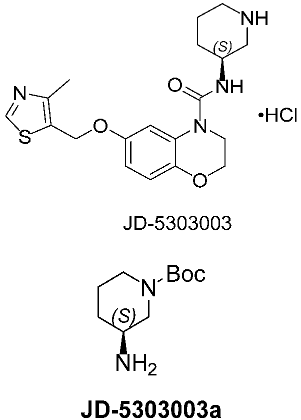

Following the synthetic method of Example 1, compound **JD-5303001d** was replaced with **JD-5303003a** to obtain *N*-[(3*S*)-piperidin-3-yl]-6-{[(4-methyl-1,3-thiazol-5-yl)methyl]oxy}-3,4-dihydro-2*H*-benzo[*b*][1,4]oxazine-4-carboxamide hydrochloride (**JD-5303003**) (48.9 mg, 124 µmol, yield: 71.6%, purity: 99.0%) as a yellow gum.

LC-MS [M+H]⁺ = 389.1.

¹H NMR (400 MHz, DMSO-*d*₆) δ ppm 1.50 - 1.70 (m, 2 H) 1.81 - 1.95 (m, 2 H) 2.39 (s, 3 H) 2.77 (br t, *J =* 9.94 Hz, 2 H) 3.18 (br d, *J =* 12.01 Hz, 1 H) 3.30 (br d, *J =* 10.13 Hz, 1 H) 3.69 (br d, *J =* 2.88 Hz, 2 H) 3.85 - 3.96 (m, 1 H) 4.13 (t, *J =* 4.57 Hz, 2 H) 5.17 (s, 2 H) 6.63 (dd, *J =* 8.88, 2.88 Hz, 1 H) 6.78 (d, *J* = 8.88 Hz, 1 H) 6.96 (d, *J =* 7.38 Hz, 1 H) 7.36 (d, *J* = 2.88 Hz, 1 H) 8.54 - 8.73 (m, 2 H) 8.98 (s, 1 H).

### Example 4: Preparation of N-[(3R)-piperidin-3-yl]-6-{[(4-methyl-1,3-thiazol-5-yl)methyl]oxy}-3,4-dihydro-2H-benzo[b][1,4]oxazine-4-carboxamide hydrochloride (JD-5303004)

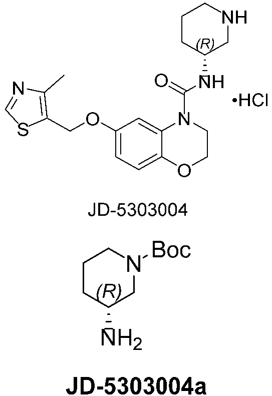

Following the synthetic method of Example 1, compound **JD-5303001d** was replaced with **JD-5303004a** to obtain *N*-[(3*R*)-piperidin-3-yl]-6-{[(4-methyl-1,3-thiazol-5-yl)methyl]oxy}-3,4-dihydro-2*H*-benzo[*b*][1,4]oxazine-4-carboxamide hydrochloride (**JD-5303004**) (39.11 mg, 99.67 µmol, yield: 57.29%, purity: 99%) as a yellow gum.

LC-MS [M+H]⁺ = 389.0.

¹H NMR (400 MHz, DMSO-*d*₆) δ ppm 1.50 - 1.70 (m, 2 H) 1.82 - 1.95 (m, 2 H) 2.39 (s, 3 H) 2.72 - 2.84 (m, 2 H) 3.18 (br d, *J =* 12.38 Hz, 1 H) 3.30 (br d, *J=* 9.76 Hz, 1 H) 3.65 - 3.75 (m, 2 H) 3.82 - 3.95 (m, 1 H) 4.13 (t, *J* = 4.57 Hz, 2 H) 5.17 (s, 2 H) 6.63 (dd, *J* = 8.88, 2.88 Hz, 1 H) 6.78 (d, *J* = 8.88 Hz, 1 H) 6.97 (d, *J* = 7.38 Hz, 1 H) 7.36 (d, *J=* 2.88 Hz, 1 H) 8.52 - 8.76 (m, 2 H) 8.98 (s, 1 H).

### Example 5: Preparation of N-(1-methylpiperidin-4-yl)-6-{[(4-methyl-1,3-thiazol-5-yl)methyl]oxyl-3,4-dihydro-2H-benzo[b][1,4]oxazine-4-carboxamide trifluoroacetate (JD-5303005)

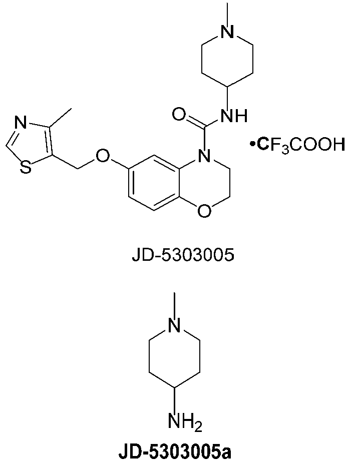

Following the synthetic method of Example 1, compound **JD-5303001d** was replaced with **JD-5303005a** to obtain *N*-(1-methylpiperidin-4-yl)-6-{[(4-methyl-1,3-thiazol-5-yl)methyl]oxy}-3,4-dihydro-2*H*-benzo[*b*][1,4]oxazine-4-carboxamide trifluoroacetate **(JD-5303005)** (71.5 mg, 138 µmol, yield: 36.3%, trifluoroacetate) as a yellow gum.

LC-MS [M+H]⁺ = 403.1.

¹H NMR: (400 MHz, DMSO-*d*₆) δ ppm 1.59 - 1.78 (m, 2 H) 1.92 - 2.06 (m, 2 H) 2.39 (s, 3 H) 2.72 - 2.79 (m, 3 H) 2.99 - 3.11 (m, 2 H) 3.44 (br d, *J =* 11.76 Hz, 2 H) 3.62 - 3.80 (m, 3 H) 4.04 - 4.16 (m, 2 H) 5.16 (s, 2 H) 6.61 (dd, *J* = 8.88, 2.88 Hz, 1 H) 6.74 - 6.80 (m, 1 H) 7.04 (br d, *J =* 7.38 Hz, 1 H) 7.34 - 7.39 (m, 1 H) 8.99 (s, 1 H) 9.27 - 9.57 (m, 1 H).

### Example 6: Preparation of 6-{[(4-methyl-1,3-thiazol-5-yl)methyl]oxy}-N-(tetrahydro-1H-pyrrol-3-yl)-3,4-dihydro-2H-benzo[b][1,4]oxazine-4-carboxamide hydrochloride (JD-5303006)

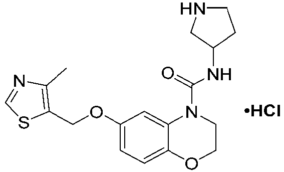

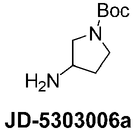

Following the synthetic method of Example 1, compound **JD-5303001d** was replaced with **JD-5303006a** to obtain 6-{[(4-methyl-1,3-thiazol-5-yl)methyl]oxy}-*N*-(tetrahydro-1*H-*pyrrol-3-yl)-3,4-dihydro-2*H*-benzo[*b*][1,4]oxazine-4-carboxamide hydrochloride (**JD-5303006)** (120 mg, 252 µmol, yield: 66.3%) as a white solid.

LC-MS [M+H]⁺ = 375.0.

**¹H NMR:** (400 MHz, DMSO-*d*₆) δ = 1.91 - 2.19 (m, 2 H), 2.41 (s, 3 H), 2.99 - 3.33 (m, 4 H), 3.57 - 3.91 (m, 2 H), 4.00 - 4.23 (m, 2 H), 4.24 - 4.37 (m, 1 H), 5.19 (s, 2 H), 6.50 - 6.82 (m, 2 H), 7.19 - 7.53 (m, 2 H), 9.11 (s, 1 H), 9.26 - 9.57 (m, 2 H).

### Example 7: Preparation of N-(5,5-difluoropiperidin-3-yl)-6-{[(4-methyl-1,3-t̅h̅i̅a̅z̅o̅l̅-̅5̅-̅y̅l̅)̅m̅e̅t̅h̅y̅l̅]̅o̅x̅y̅}̅-̅3̅,̅4̅-̅d̅i̅h̅y̅d̅r̅o̅-̅2̅H̅-̅b̅e̅n̅z̅o̅[̅b̅]̅[̅1̅,̅4̅]̅o̅x̅a̅z̅i̅n̅e̅-̅4̅-̅c̅a̅r̅b̅o̅x̅a̅m̅i̅d̅e̅ hydrochloride (JD-5303007)

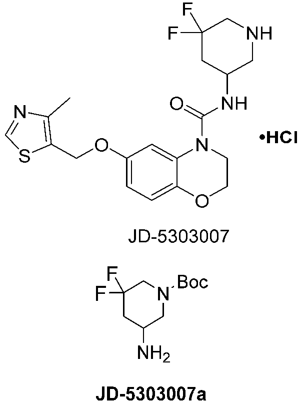

Following the synthetic method of Example 1, compound **JD-5303001d** was replaced with **JD-5303007a** to obtain *N*-(5,5-difluoropiperidin-3-yl)-6-{[(4-methyl-1,3-thiazol-5-yl)methyl]oxy}-3,4-dihydro-2*H*-benzo[*b*][1,4]oxazine-4-carboxamide hydrochloride (**JD-**5303007) (0.045 g, yield: 69.9%, purity: 97.92%) as a brown gum.

LC-MS [M+H]⁺ = 425.2.

¹H NMR (400 MHz, DMSO-*d*₆) δ ppm 2.17 - 2.49 (m, 5 H) 2.99 (br s, 1 H) 3.30 (br d, *J =* 9.76 Hz, 1 H) 3.46 (br d, *J* = 12.76 Hz, 1 H) 3.58 - 3.68 (m, 1 H) 3.70 - 3.79 (m, 2 H) 4.08 - 4.23 (m, 3 H) 5.19 (s, 2 H) 6.63 (dd, *J* = 8.94, 2.94 Hz, 1 H) 6.78 (d, *J=* 8.88 Hz, 1 H) 7.30 - 7.46 (m, 2 H) 9.21 (s, 1 H) 9.83 (br s, 1 H) 10.68 (br s, 1 H).

### Example 8: Preparation of N-(3-aminocyclohexyl)-6-{[(4-methyl-1,3-thiazol-5-yl)methyl]oxy}-3,4-dihydro-2H-benzo[b][1,4]oxazine-4-carboxamide (JD-5303008)

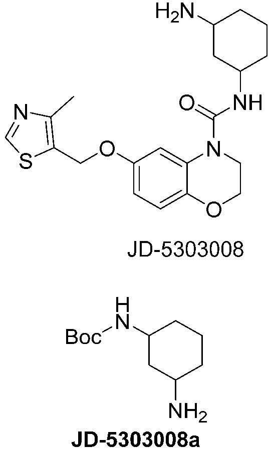

Following the synthetic method of Example 1, compound **JD-5303001d** was replaced with **JD-5303008a** to obtain *N*-(3-aminocyclohexyl)-6-{[(4-methyl-1,3-thiazol-5-yl)methyl]oxy}-3,4-dihydro-2*H*-benzo[*b*][1,4]oxazine-4-carboxamide (**JD-5303008**) (75 mg, yield: 62.4%, purity: 98.8%) as a white solid.

LC-MS ([M+H]⁺) = 403.2.

**¹H NMR** (400 MHz, DMSO-*d*₆) δ = 8.92 (s, 1H), 7.35 (d, *J* = 2.9 Hz, 1H), 6.80 - 6.74 (m, 1H), 6.61 (dd, *J* = 8.8, 2.9 Hz, 1H), 6.39 (d, *J* = 6.8 Hz, 1H), 5.93 - 5.46 (m, 2H), 5.18 (s, 2H), 4.20 - 4.09 (m, 2H), 4.06 - 3.97 (m, 1H), 3.77 - 3.66 (m, 2H), 3.31 - 3.22 (m, 1H), 2.41 (s, 3H), 1.98 - 1.78 (m, 1H), 1.76 - 1.60 (m, 4H), 1.59 - 1.48 (m, 2H), 1.46 - 1.37 (m, 1H).

### Example 9: Preparation of N-(piperidin-4-yl)-6-1[(4-methyl-1,3-thiazol-5-yl)methyl]oxy}-3,4-dihydro-2H-benzo[b][1,4]oxazine-4-carboxamide trifluoroacetate (JD-5303009)

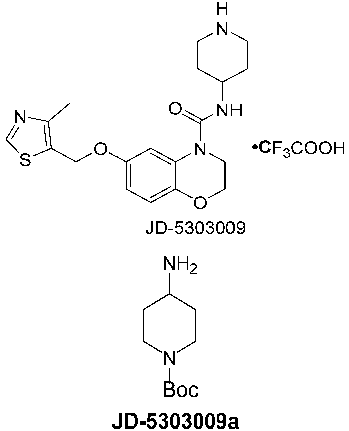

Following the synthetic method of Example 1, **JD-5303001d** was replaced with **JD-5303009a** to obtain *N*-(piperidin-4-yl)-6-{[(4-methyl-1,3-thiazol-5-yl)methyl]oxy}-3,4-dihydro-2*H*-benzo[*b*][1,4]oxazine-4-carboxamide trifluoroacetate (**JD-5303009**) (80 mg, 205.48 µmol, yield: 83.66%, purity: 99.78%) as a white solid.

LC-MS [M+H]⁺ = 389.1.

**¹H NMR:** (400 MHz, DMSO-*d*₆) δ = 9.04 (s, 1 H), 8.80 - 8.92 (m, 1 H), 8.67 - 8.78 (m, 1 H), 7.37 (d, *J =* 2.88 Hz, 1 H), 7.05 - 7.11 (m, 1 H), 6.76 (d, *J =* 8.88 Hz, 1 H), 6.60 (dd, *J =* 8.82, 2.94 Hz, 1 H), 5.15 - 5.18 (m, 2 H), 4.09 - 4.13 (m, 2 H), 3.27 (br d, *J =* 12.26 Hz, 2 H), 3.15 - 3.23 (m, 1 H), 2.87 - 3.05 (m, 4 H), 2.39 (s, 3 H), 1.94 (br d, *J* = 12.26 Hz, 2 H), 1.63 - 1.76 (m, 2 H).

### Example 10: Preparation of N-(5-amino-2-methylcyclohexyl)-6-{[(4-methyl-1,3-thiazol-5-yl)methyl]oxy}-3,4-dihydro-2H-benzo[b][1,4]oxazine-4-carboxamide (JD-5303010)

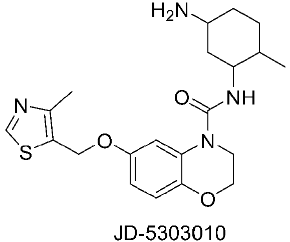

### Step 1: Preparation of tert-butyl (3-amino-4-methylcyclohexyl)carbamate (JD-5303010b)

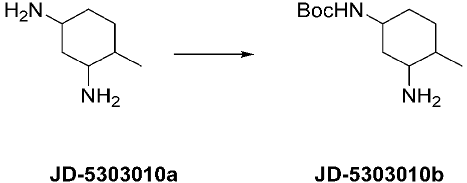

4-Methylcyclohexane-1,3-diamine (**JD-5303010a**) (500 mg, 3.90 mmol, 1.0 eq) was dissolved in dichloromethane (5.0 mL), followed by the addition of triethylamine (592 mg, 5.85 mmol, 1.5 eq) and (Boc)₂O (851 mg, 3.90 mmol, 1.0 eq), and the mixture was reacted at room temperature for 15 hours. The reaction mixture was quenched with water (10 mL) at room temperature and extracted with dichloromethane (30 mL, 10.0 mL * 3). The combined organic phases were concentrated under reduced pressure to obtain a residue. The residue was purified by column chromatography (SiO₂, dichloromethane/methanol = 10/1) to obtain *tert-*butyl (3-amino-4-methylcyclohexyl)carbamate (**JD-5303010b**) (150 mg, yield: 16.8%) as a yellow oil.

LC-MS [M+H]⁺ = 229.2.

**¹H NMR (400 MHz, DMSO-*d*₆):** *δ* 7.00 - 6.43 (m, 1H), 2.99 - 2.52 (m, 2H), 2.31 - 1.78 (m, 2H), 1.73 - 1.56 (m, 2H), 1.53 - 1.44 (m, 1H), 1.40 - 1.34 (m, 10H), 1.12 - 0.74 (m, 6H).

### Step 2: Preparation of N-(5-amino-2-methylcyclohexyl)-6-{[(4-methyl-1,3-thiazol-5-yl)methyl]oxy}-3,4-dihydro-2H-benzo[b][1,4]oxazine-4-carboxamide (JD-5303010)

Following the synthetic method of Example 1, **JD-5303001d** was replaced with **JD-5303010b** to obtain *N*-(5-amino-2-methylcyclohexyl)-6-{[(4-methyl-1,3-thiazol-5-yl)methyl]oxy}-3,4-dihydro-2*H*-benzo[*b*][1,4]oxazine-4-carboxamide (**JD-5303010**) (11.9 mg, yield: 53.7%, purity: 98.82%) as a white solid.

LC-MS [M+H]⁺ = 417.2.

**¹H NMR (400 MHz, DMSO-*d*₆):** *δ* 8.97 (s, 1H), 7.89 (s, 2H), 7.37 - 7.22 (m, 1H), 6.97 - 6.82 (m, 1H), 6.79 - 6.73 (m, 1H), 6.64 - 6.57 (m, 1H), 5.16 (s, 2H), 4.19 - 4.03 (m, 2H), 3.81 - 3.71 (m, 1H), 3.67 - 3.57 (m, 1H), 3.13 - 2.99 (m, 1H), 2.38 (s, 3H), 2.15 - 1.95 (m, 1H), 1.95 - 1.80 (m, 1H), 1.79 - 1.66 (m, 1H), 1.66 - 1.27 (m, 4H), 1.19 - 0.99 (m, 1H), 0.95 - 0.82 (m, 3H).

### Example 11: Preparation of N-(azepan-3-yl)-6-{[(4-methyl-1,3-thiazol-5-yl)methyl]oxy}-3,4-dihydro-2H-benzo[b][1,4]oxazine-4-carboxamide (JD-5303011)

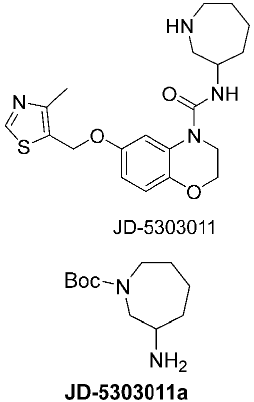

Following the synthetic method of Example 1, **JD-5303001d** was replaced with **JD-5303011a** to obtain *N*-(azepan-3-yl)-6-{[(4-methyl-1,3-thiazol-5-yl)methyl]oxy}-3,4-dihydro-2*H*-benzo[*b*][1,4]oxazine-4-carboxamide (**JD-5303011**) (58 mg, yield: 48.3%, purity: 98.9%) as a white solid.

LC-MS ([M+H]⁺) = 403.2.

**¹H NMR** (300 MHz, DMSO-*d*₆) δ = 8.98 (s, 1H), 7.38 (d, *J =* 2.9 Hz, 1H), 7.06 (d, *J* = 7.6 Hz, 1H), 6.78 (d, *J =* 8.8 Hz, 1H), 6.63 (dd, *J* = 8.9, 2.9 Hz, 1H), 5.17 (s, 2H), 4.13 (t, *J* = 4.4 Hz, 2H), 4.09 - 3.98 (m, 1H), 3.76 - 3.68 (m, 2H), 3.45 - 3.20 (m, 5H), 2.39 (s, 3H), 2.04 - 1.91 (m, 1H), 1.88 - 1.63 (m, 4H), 1.62 - 1.44 (m, 1H).

### Example 12: Preparation of 6-{[(4-methyl-1,3-thiazol-5-yl)methyl]oxy}-N-(4-azabicyclo[2.2.2]octan-2-yl)-3,4-dihydro-2H-benzo[b][1,4]oxazine-4-carboxamide (JD-5303012)

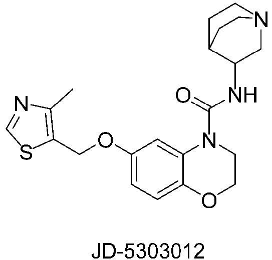

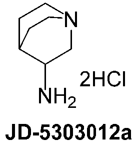

Following the synthetic method of Example 1, **JD-5303001d** was replaced with **JD-5303012a** to obtain 6-{[(4-methyl-1,3-thiazol-5-yl)methyl]oxy}-*N*-(4-azabicyclo[2.2.2]octan-2-yl)-3,4-dihydro-2*H*-benzo[*b*][1,4]oxazine-4-carboxamide (**JD-5303012**) (13.8 mg, yield: 8.7%, purity: 97.71%) as a yellow solid.

LC-MS ([M+H]⁺) = 415.2.

**¹H NMR (300 MHz, CDCl₃):** *δ* 8.70 (s, 1H), 7.42 (d, *J* = 3.0 Hz, 1H), 6.82 (d,*J* = 9.0 Hz, 1H), 6.70 - 6.59 (m, 2H), 5.12 (s, 2H), 4.30 (s, 1H), 4.21 (t, *J* = 4.8 Hz, 2H), 4.00 - 3.91 (m, 2H), 3.86 - 3.71 (m, 2H), 3.55 - 3.43 (m, 1H), 3.28 - 3.03 (m, 3H), 2.47 (s, 3H), 2.38 (d, *J* = 4.8 Hz, 1H), 2.25 - 2.16 (m, 1H), 2.04 - 1.96 (m, 2H), 1.82 - 1.73 (m, 1H).

### Example 13: Preparation of N-(4-fluoropiperidin-3-yl)-6-{[(4-methyl-1,3-thiazol-5-yl)methyl]oxyl-3,4-dihydro-2H-benzo[b][1,4]oxazine-4-carboxamide trifluoroacetate (JD-5303013)

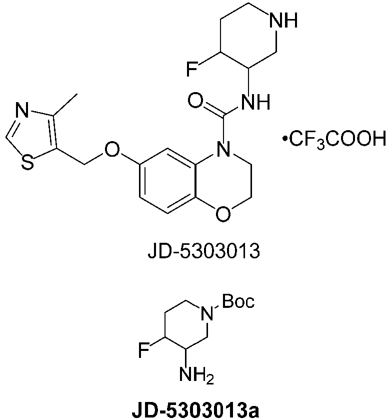

Following the synthetic method of Example 1, **JD-5303001d** was replaced with **JD-5303013a** to obtain *N*-(4-fluoropiperidin-3-yl)-6-{[(4-methyl-1,3-thiazol-5-yl)methyl]oxy}-3,4-dihydro-2*H*-benzo[*b*][1,4]oxazine-4-carboxamide trifluoroacetate (**JD-5303013**) (4.6 mg, 11.3 µmol, yield: 28.6%, purity: 99.6%) as a white solid.

LC-MS [M+H]⁺ = 407.0.

¹H NMR: (400 MHz, DMSO-*d*₆) δ = 8.99 (s, 1 H), 8.66 - 8.93 (m, 2 H), 7.36 (d, *J* = 2.88 Hz, 1 H), 7.14 - 7.19 (m, 1 H), 6.78 - 6.83 (m, 1 H), 6.65 (dd, *J* = 8.94, 2.94 Hz, 1 H), 5.17 (s, 2 H), 4.87 - 5.11 (m, 1 H), 4.06 - 4.27 (m, 3 H), 3.78 (ddd, *J =* 13.35, 5.35, 2.69 Hz, 2 H), 3.17 - 3.29 (m, 2 H), 2.90 - 3.12 (m, 2 H), 2.39 (s, 3 H), 1.90 - 2.28 (m, 2 H).

### Example 14: Preparation of N-(2H-pyrazol-3-yl)-6-{[(4-methyl-1,3-thiazol-5-yl)methyl]oxy}-3,4-dihydro-2H-benzo[b][1,4]oxazine-4-carboxamide (JD-5303014)

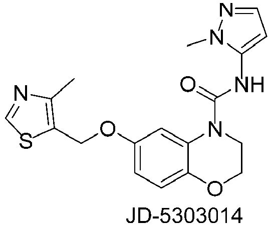

### Step 1: Preparation of 6-{[dimethyl(2-methylprop-2-yl)silyljoxy}-3,4-dihydro-2H-benzo[b][1,4]oxazine (JD-5303014-2)

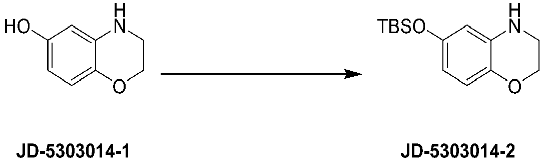

To a solution of 3,4-dihydro-2*H*-benzo[*b*][1,4]oxazin-6-ol (**JD-5303014-1**) (50.0 mg, 330 µmol, 1.00 eq) and TBSCl (74.7 mg, 496 µmol, 61.0 µL, 1.50 eq) in DCM (1.00 mL) was added imidazole (45.0 mg, 661 µmol, 2.00 eq), and the mixture was stirred at 25°C for 3 hours. The reaction mixture was quenched with H₂O (10.0 mL) and extracted with DCM (10.0 mL). The combined organic phases were separated, filtered, and concentrated under reduced pressure to obtain 6-{[dimethyl(2-methylprop-2-yl)silyl]oxy}-3,4-dihydro-2*H-*benzo[b][1,4]oxazine (**JD-5303014-2**) (100 mg, crude product) as a brown oil.

LC-MS [M+H]⁺ = 266.1.

### Step 2: Preparation of 6-{[dimethyl(2-methylprop-2-yl)silyl]oxy}-N-(2-methylpyrazol-3-yl)-3,4-dihydro-2H-benzo[b][1,4]oxazine-4-carboxamide (JD-5303014-3)

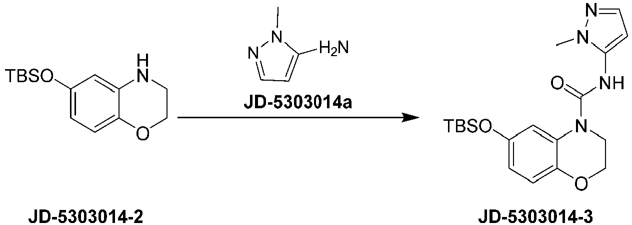

To a solution of 6-{[dimethyl(2-methylprop-2-yl)silyl]oxy}-3,4-dihydro-2*H-*benzo[b][1,4]oxazine (**JD-5303014-2**) (50.0 mg, 188 µmol, 1.00 eq) and 2-methylpyrazol-3-amine (**JD-5303014a**) (21.9 mg, 226 µmol, 1.20 eq) in THF (1.00 mL) were added CDI (36.6 mg, 226 µmol, 1.20 eq) and DIEA (29.2 mg, 226 µmol, 39.3 µL, 1.20 eq), and the mixture was stirred at 25°C for 12 hours. The reaction mixture was quenched with H₂O (10.0 mL) and extracted with EtOAc (10.0 mL). The combined organic phases were separated, filtered, and concentrated under reduced pressure to obtain 6-{[dimethyl(2-methylprop-2-yl)silyl]oxy}-*N-*(2-methylpyrazol-3-yl)-3,4-dihydro-2*H*-benzo[*b*][[1,4]oxazine-4-carboxamide (**JD-5303014-3**) (110 mg, crude product) as a brown oil.

LC-MS [M+H]⁺ = 389.4.

### Step 3: Preparation of 6-hydroxy-N-(2-methylpyrazol-3-yl)-3,4-dihydro-2H-benzo[b][1,4]oxazine-4-carboxamide (JD-5303014-4)

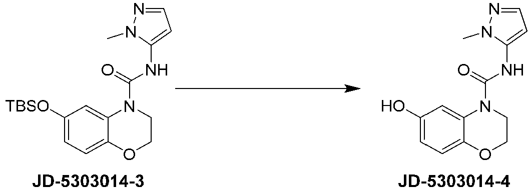

To a solution of 6-{[dimethyl(2-methylprop-2-yl)silyl]oxy}-*N*-(2-methylpyrazol-3-yl)-3,4-dihydro-2*H*-benzo[*b*][1,4]oxazine-4-carboxamide (**JD-5303014-3**) (50.0 mg, 128 µmol, 1.00 eq) in THF (1.00 mL) was added TBAF (50.4 mg, 193 µmol, 1.50 eq), and the mixture was stirred at 25°C for 2 hours. The reaction mixture was quenched with H₂O (10.0 mL) and extracted with EtOAc (10.0 mL). The combined organic phases were separated, filtered, and concentrated under reduced pressure to obtain 6-hydroxy-*N*-(2-methylpyrazol-3-yl)-3,4-dihydro-2*H*-benzo[*b*][1,4]oxazine-4-carboxamide (**JD-5303014-4**) (30.0 mg, crude product) as a brown oil.

LC-MS [M+H]⁺ = 275.0.

### Step 4: Preparation of N-(2H-pyrazol-3-yl)-6-{[(4-methyl-1,3-thiazol-5-yl)methyl]oxy}-3,4-dihydro-2H-benzo[b][1,4]oxazine-4-carboxamide (JD-5303014)

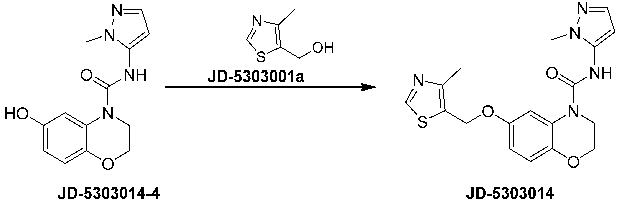

To a solution of 6-hydroxy-*N*-(2-methylpyrazol-3-yl)-3,4-dihydro-2*H-*benzo[*b*][1,4]oxazine-4-carboxamide (**JD-5303014-4**) (30.0 mg, 109 µmol, 1.00 eq) and (4-methyl-1,3-thiazol-5-yl)methanol (**JD-5303001a**) (28.2 mg, 218 µmol, 2.00 eq) in THF (1.00 mL) were added PPh₃ (57.3 mg, 218 µmol, 2.0 eq) and DIAD (44.2 mg, 218 µmol, 42.4 µL, 2.00 eq), and the mixture was stirred at 25°C for 12 hours. The reaction mixture was quenched with H₂O (10.0 mL) and extracted with EtOAc (10.0 mL). The combined organic phases were separated, filtered, and concentrated under reduced pressure to obtain a residue. The residue was purified by preparative HPLC to obtain *N*-(2*H*-pyrazol-3-yl)-6-{[(4-methyl-1,3-thiazol-5-yl)methyl]oxy}-3,4-dihydro-2*H*-benzo[*b*][1,4]oxazine-4-carboxamide **(JD-5303014)** (1.45 mg, 3.76 µmol, yield: 3.44%) as a brown gum.

LC-MS [M+H]⁺ = 386.1.

**¹H NMR (300 MHz, DMSO-*d*₆):** *δ* 9.06 (s, 1H), 8.98 (s, 1H), 7.41 (d, *J* = 2.7 Hz, 1H), 7.33 (d, *J* = 1.8 Hz, 1H), 6.82 (d, *J* = 8.7 Hz, 1H), 6.68 (dd, *J =* 9.0, 3.0 Hz, 1H), 6.11 (d, *J* = 2.1 Hz, 1H), 5.18 (s, 2H), 4.22 (t, *J =* 4.5 Hz, 2H), 3.85 (t, *J* = 4.5 Hz, 2H), 3.65 (s, 3H), 2.38 (s, 3H).

### Example 15: Preparation of N-(5-chloropiperidin-3-yl)-6-{[(4-methyl-1,3-thiazol-5-yl)methyl]oxy}-3,4-dihydro-2H-benzo[b][1,4]oxazine-4-carboxamide formate (JD-5303015)

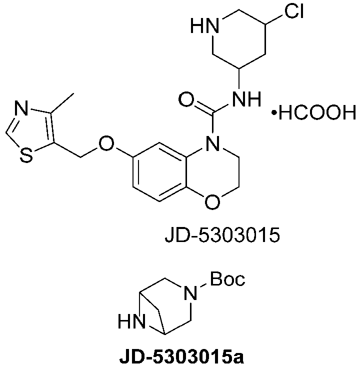

Step 1: Following the synthetic method of Example 1, compound **JD-5303001d** was replaced with **JD-5303015a** to obtain *tert*-butyl 6-[(6-{[(4-methyl-1,3-thiazol-5-yl)methyl]oxy}-3,4-dihydro-2*H*-benzo[*b*][1,4]oxazin-4-yl)carbonyl]-3,6-diazabicyclo[3.1.1]heptane-3-carboxylate (**JD-5303015-1**) (150 mg, 92.2%) as a yellow solid.

**LCMS:** 487.2 ([M+H]⁺).

**¹H NMR (400 MHz, DMSO-*d*₆):** *δ* 8.97 (s, 1H), 7.00 (d, *J* = 2.9 Hz, 1H), 6.80 (d, *J* = 8.8 Hz, 1H), 6.67 (dd, *J* = 8.9, 2.9 Hz, 1H), 5.17 (s, 2H), 4.19 - 4.06 (m, 4H), 3.92 - 3.77 (m, 2H), 3.74 - 3.59 (m, 2H), 3.30 - 3.17 (m, 2H), 2.39 (s, 3H), 1.37 (s, 11H).

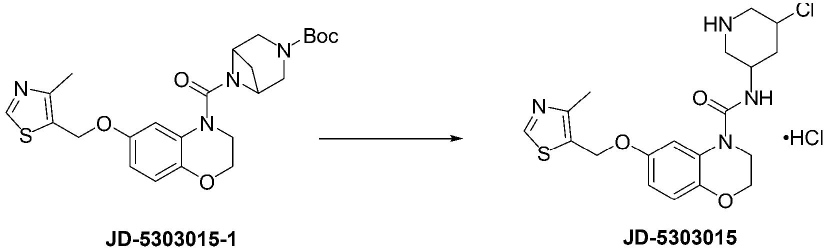

Step 2: *tert*-Butyl 6-[(6-{[(4-methyl-1,3-thiazol-5-yl)methyl]oxy}-3,4-dihydro-2*H-*benzo[*b*][1,4]oxazin-4-yl)carbonyl]-3,6-diazabicyclo[3.1.1]heptane-3-carboxylate **(JD-5303015-1)** (150 mg, 0.31 mmol, 1.0 eq) was dissolved in DCM (2 mL), then dioxane/HCl (1.5 mL, 6.2 mmol, 20.0 eq) was added thereto, and the mixture was reacted at room temperature for 2 hours. The reaction mixture was concentrated under reduced pressure to obtain a residue. The residue was quenched with saturated sodium carbonate solution (3 mL) and extracted with dichloromethane (10 mL * 2). The combined organic phases were washed with brine (10 mL), dried over anhydrous sodium sulfate, and concentrated under reduced pressure to obtain a residue. The residue was purified by prep-HPLC (column: WelchUlC18 150 * 25 mm * 5 mm; mobile phase: [water (0.1% FA)-ACN]; B%: 35% to 45%, 6 minutes) to obtain **JD-5303015** (7 mg, yield: 5.0%, purity: 96.6%) as a white solid.

LC-MS ([M+H]⁺) = 423.1.

**¹H NMR** (300 MHz, MeOD) δ = 8.88 (s, 1H), 8.33 (brs, 0.74H), 7.24 (d, *J* = 2.8 Hz, 1H), 6.80 (d, *J =* 8.9 Hz, 1H), 6.67 (dd, *J* = 9.0, 2.8 Hz, 1H), 5.19 (s, 2H), 4.65 (s, 1H), 4.42 - 4.28 (m, 1H), 4.23 - 4.11 (m, 2H), 3.86 - 3.69 (m, 2H), 3.43 - 3.32 (m, 2H), 2.91 - 2.77 (m, 1H), 2.69 (s, 1H), 2.44 (s, 3H), 2.34 - 2.09 (m, 2H).

### Example 16: Preparation of 6-{[(4-methyl-1,3-thiazol-5-yl)methyl]oxy}-4-(1,6-diazaspiro[3.5]nonane-1-carbonyl)-3,4-dihydro-2H-benzo[b][1,4]oxazine trifluoroacetate (JD-5303016)

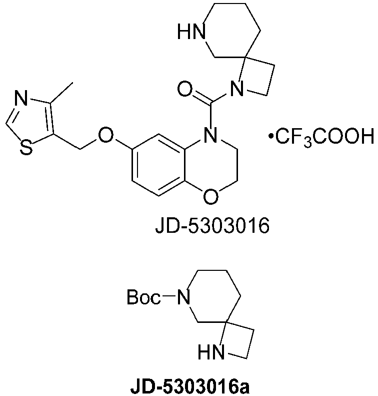

Following the synthetic method of Example 1, **JD-5303001d** was replaced with **JD-5303016a** to obtain 6-{[(4-methyl-1,3-thiazol-5-yl)methyl]oxy}-4-(1,6-diazaspiro[3.5]nonane-1-carbonyl)-3,4-dihydro-2*H*-benzo[*b*][1,4]oxazine trifluoroacetate **(JD-5303016)** (7.3 mg, yield: 7.9%, purity: 97.0%) as a white solid.

LC-MS ([M+H]⁺) = 415.3.

**¹H NMR** (400 MHz, MeOD) δ = 8.96 (s, 1H), 6.85 - 6.78 (m, 2H), 6.72 (dd, *J* = 2.8 Hz, 8.8 Hz, 1H), 5.19 (s, 2H), 4.33 - 4.26 (m, 1H), 4.25 - 4.14 (m, 2H), 3.85 - 3.70(m, 2H), 3.69 - 3.60 (m, 1H), 3.55 - 3.42 (m, 2H), 3.08 - 2.96 (m, 1H), 2.63 - 2.52 (m, 1H), 2.43 (s, 3H), 2.08 - 1.94 (m, 3H), 1.88 - 1.75 (m, 3H).

### Example 17: Preparation of 6-{[(4-methyl-1,3-thiazol-5-yl)methyl]oxy}-N-[3-(prop-2-en-1-yl)piperidin-3-yl]-3,4-dihydro-2H-benzo[b][1,4]oxazine-4-carboxamide (JD-5303017)

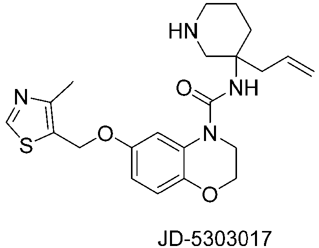

### Step 1: Preparation of tert-butyl 3-amino-3-(prop-2-en-1-yl)piperidine-1-carboxylate (JD-5303017c)

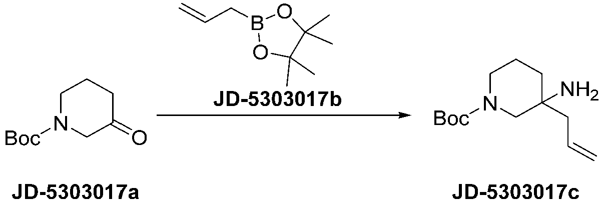

*tert*-Butyl 3-oxopiperidine-1-carboxylate (**JD-5303017a**) (400 mg, 2.0 mmol, 1.0 eq) was dissolved in a solution of NH₃ in MeOH (4.0 mL), and the mixture was reacted at room temperature for 0.5 hours. 4,4,5,5-tetramethyl-2-(prop-2-en-1-yl)-1,3,2-dioxaborolane (**JD-5303017b**) (439 mg, 2.6 mmol, 1.3 eq) was then added thereto, and the reaction system was reacted at room temperature for 11.5 hours. The reaction mixture was added with sodium hydroxide solution (15%) (5 mL) at room temperature to adjust the pH to 10, and extracted with dichloromethane (20 mL, 10.0 mL * 2). The combined organic phases were washed with brine (10 mL), dried over anhydrous sodium sulfate, and concentrated under reduced pressure to obtain a residue. The residue was purified by column chromatography (SiO₂, dichloromethane/methanol = 50/1 to 30/1) to obtain *tert-*butyl 3-amino-3-(prop-2-en-1-yl)piperidine-1-carboxylate (**JD-5303017c**) (310 mg, yield: 64.2%, purity: 92%) as a yellow oil.

LC-MS ([M-56+H]⁺) = 185.1.

**¹H NMR** (300 MHz, DMSO-*d*₆) δ = 5.97 - 5.79 (m, 1H), 5.11 - 4.98 (m, 2H), 3.39 - 2.91 (m, 4H), 2.06 - 1.98 (m, 2H), 1.59 - 1.42 (m, 2H), 1.38 (s, 9H), 1.37 - 1.21 (m, 4H).

### Step 2: Preparation of 6-{[(4-methyl-1,3-thiazol-5-yl)methyl]oxy}-N-[3-(prop-2-en-1-yl)piperidin-3-yl]-3,4-dihydro-2H-benzo[b][1,4]oxazine-4-carboxamide (JD-5303017)

Following the synthetic method of Example 1, compound **JD-5303001d** was replaced with **JD-5303017c** to obtain 6-{[(4-methyl-1,3-thiazol-5-yl)methyl]oxy}-*N*-[3-(prop-2-en-1-yl)piperidin-3-yl]-3,4-dihydro-2*H*-benzo[*b*][1,4]oxazine-4-carboxamide (**JD-5303017**) (25.1 mg, yield: 61.9%, purity: 98.2%) as a white solid.

LC-MS ([M+H]⁺) = 429.3.

**¹H NMR** (300 MHz, DMSO-*d*₆) δ = 8.97 (s, 1H), 8.62 - 7.96 (m, 1H), 7.31 (d, *J=* 3.0 Hz, 1H), 6.77 (d, *J* = 8.7 Hz, 1H), 6.63 (dd, *J* = 9.0, 3.0 Hz, 1H), 6.29 (s, 1H), 5.86 - 5.68 (m, 1H), 5.18 (s, 2H), 5.14 - 5.01 (m, 2H), 4.22 - 4.03 (m, 2H), 3.92 - 3.81 (m, 1H), 3.77 - 3.57 (m, 2H), 3.14 - 3.00 (m, 1H), 2.81 - 2.70 (m, 2H), 2.67 - 2.56 (m, 1H), 2.44 - 2.35 (m, 4H), 2.21 - 2.07 (m, 1H), 1.80 - 1.53 (m, 2H), 1.50 - 1.36 (m, 1H).

### Example 18: Preparation of N-[3-(2-chloroethyl)piperidin-3-yl]-6-{[(4-methyl-1,3-thiazol-5-yl)methyl]oxy}-3,4-dihydro-2H-benzo[b][1,4]oxazine-4-carboxamide (JD-5303018)

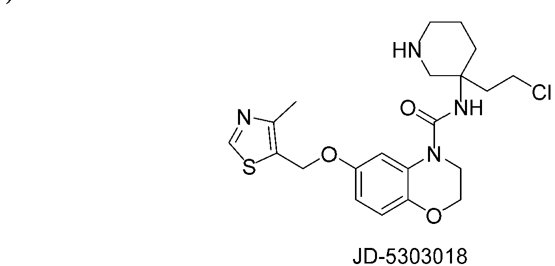

### Step 1: Preparation of tert-butyl 1-[(6-{[(4-methyl-1,3-thiazol-5-yl)methyl]oxy}-3,4-dihydro-2H-benzo[b][1,4]oxazin-4-yl)carbonyl]-1,6-diazaspiro[3.5]nonane-6-carboxylate (JD-5303018-1)

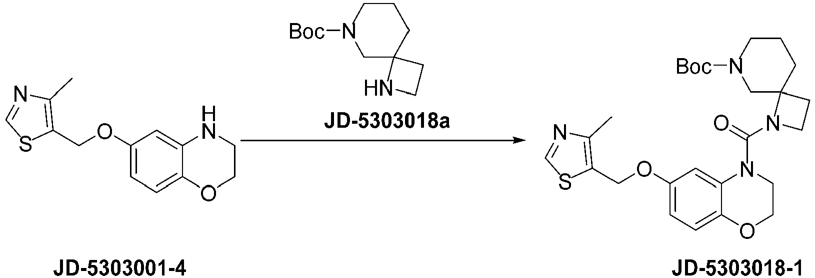

Following the synthetic method of Example 1, **JD-5303001d** was replaced with **JD-5303018a** to obtain *tert*-butyl 1-[(6-{[(4-methyl-1,3-thiazol-5-yl)methyl]oxy}-3,4-dihydro-2*H*-benzo[*b*][1,4]oxazin-4-yl)carbonyl]-1,6-diazaspiro[3.5]nonane-6-carboxylate (**JD-5303018-1**) (150 mg, yield: 87.1%, purity: 92%) as a yellow oil.

LC-MS ([M+H]⁺) = 515.2.

**¹H NMR** (400 MHz, DMSO-*d*₆) δ = 8.97 (s, 1H), 6.84 (d, *J* = 2.9 Hz, 1H), 6.74 (d, *J* = 8.8 Hz, 1H), 6.59 (dd, *J* = 8.9, 2.9 Hz, 1H), 5.20 (s, 2H), 4.23 - 3.34 (m, 10H), 2.39 (s, 3H), 2.33 - 2.19 (m, 1H), 2.02 - 1.85 (m, 3H), 1.69 - 1.57 (m, 1H), 1.44 - 1.40 (m, 1H), 1.39 (s, 9H).

### Step 2: Preparation ofN-[3-(2-chloroethyl)piperidin-3-yl]-6-{[(4-methyl-1,3-thiazol-5-yl)methyl]oxyl-3,4-dihydro-2H-benzo[b][1,4]oxazine-4-carboxamide (JD-5303018)

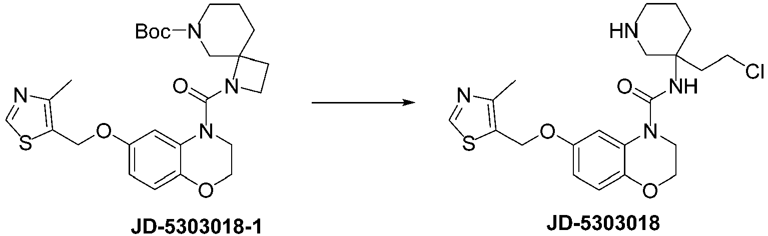

*tert-Butyl* 1-[(6-{[(4-methyl-1,3-thiazol-5-yl)methyl]oxy}-3,4-dihydro-2*H-*benzo[*b*][1,4]oxazin-4-yl)carbonyl]-1,6-diazaspiro[3.5]nonane-6-carboxylate (**JD-5303018-1**) (150 mg, 0.29 mmol, 1.0 eq) was dissolved in DCM (2 mL), then dioxane/HCl (1.46 mL, 5.93 mmol, 20.0 eq) was added thereto, and the mixture was reacted at room temperature for 2 hours. The reaction mixture was concentrated under reduced pressure to obtain a residue. The residue was quenched with saturated sodium carbonate solution (3 mL) and extracted with DCM (10 mL * 2). The combined organic phases were washed with brine (10 mL), dried over anhydrous sodium sulfate, and concentrated under reduced pressure to obtain a residue. The residue was purified by prep-TLC (dichloromethane/methanol = 10/1) to obtain *N*-[3-(2-chloroethyl)piperidin-3-yl]-6-{[(4-methyl-1,3-thiazol-5-yl)methyl]oxy}-3,4-dihydro-2*H-*benzo[*b*][1,4]oxazine-4-carboxamide (**JD-5303018**) (9 mg, yield: 6.8%, purity: 97.3%) as a yellow solid.

LC-MS ([M+H]⁺) = 451.0.

**¹H NMR** (300 MHz, DMSO-*d*₆) δ = 9.16 (s, 2H), 8.99 (s, 1H), 7.27 (d, *J =* 2.9 Hz, 1H), 7.02 (t, *J* = 5.6 Hz, 1H), 6.78 (d, *J* = 8.9 Hz, 1H), 6.63 (dd, *J* = 8.9, 2.9 Hz, 1H), 5.60 (s, 1H), 5.18 (s, 2H), 4.16 - 4.05 (m, 2H), 3.75 - 3.62 (m, 2H), 3.51 (s, 2H), 3.25 - 3.14 (m, 2H), 3.09 - 2.98 (m, 2H), 2.40 (s, 3H), 2.29 - 2.10 (m, 4H).

### Example 19: Preparation of ethyl (3-{[(6-{[(4-methyl-1,3-thiazol-5-yl)methyl]oxy}-3,4-dihydro-2H-benzo[b][1,4]oxazin-4-yl)carbonyl]amino}piperidin-3-yl)acetate (JD-5303019)

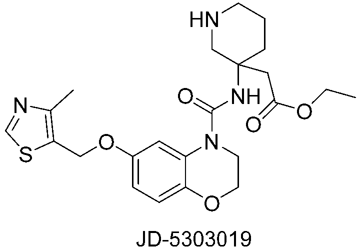

### Step 1: Preparation of ethyl [(3E)-1-{[(2-methylprop-2-yl)oxy]carbonyl}piperidin-3-ylidene]acetate (JD-5303019c)

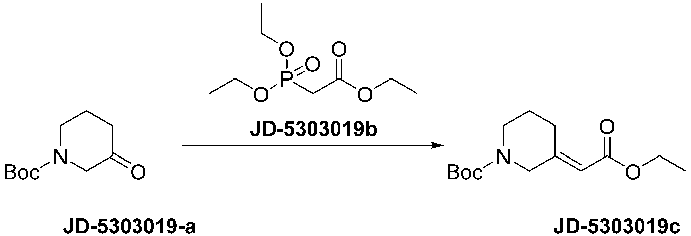

Diethyl (2-ethoxy-2-oxoethyl)phosphonate (**JD-5303019b**) (2.7 g, 12.05 mmol, 1.2 eq) was dissolved in tetrahydrofuran (20 mL), then NaH (482 mg, 12.05 mmol, 1.2 eq) was added thereto at 0°C, and the mixture was stirred for 0.5 hours. *tert*-Butyl 3-oxopiperidine-1-carboxylate (**JD-5303019a**) (2.0 g, 10.04 mmol, 1.0 eq) was then added thereto, and the mixture was stirred at room temperature for 2 hours. The reaction mixture was quenched with saturated ammonium chloride aqueous solution (20 mL) at 0°C and extracted with ethyl acetate (40 mL, 20 mL * 2). The combined organic phases were washed with brine (20 mL), dried over anhydrous sodium sulfate, and concentrated under reduced pressure to obtain a residue. The residue was purified by column chromatography (SiO₂, petroleum ether/ethyl acetate = 200/1 to 50/1) to obtain ethyl [(3*E*)-1-{[(2-methylprop-2-yl)oxy]carbonyl}piperidin-3-ylidene]acetate (**JD-5303019c**) (2.4 g, yield: 88.7%) as a yellow oil.

LC-MS ([M+Na]) = 292.1.

**¹H NMR (400 MHz, CDCl₃):** *δ* = 5.74 (s, 1H), 4.16 (q, *J* = 7.2 Hz, 2H), 3.93 (s, 2H), 3.51 - 3.44 (m, 2H), 2.96 - 2.90 (m, 2H), 1.73 - 1.66 (m, 2H), 1.45 (s, 9H), 1.28 (t, *J* = 7.2 Hz, 3H).

### Step 2: Preparation of ethyl (3-amino-1-{[(2-methylprop-2-yl)oxy]carbonyl}piperidin-3-yl)acetate (JD-5303019d)

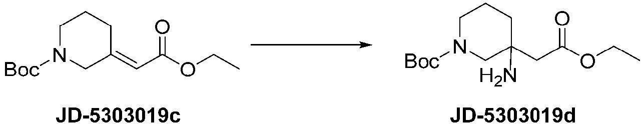

Ethyl [(3*E*)-1-{[(2-methylprop-2-yl)oxy]carbonyl}piperidin-3-ylidene]acetate (**JD-5303019c**) (500 mg, 1.85 mmol, 1.0 eq) was dissolved in ethanol (2.4 mL), and ammonia water (1.2 mL) was added thereto at room temperature. The reaction mixture was stirred at 80°C in a high pressure reactor for 15 hours. The reaction mixture was concentrated under reduced pressure to obtain ethyl (3-amino-1-{[(2-methylprop-2-yl)oxy]carbonyl} piperidin-3-yl)acetate (**JD-5303019d**) (500 mg, crude product) as a green oil.

LC-MS ([M+H]⁺) = 287.3.

### Step 3: Preparation of 6-{[(4-methyl-1,3-thiazol-5-yl)methyl]oxy}-N-[3-(prop-2-en-1-yl)piperidin-3-yl]-3,4-dihydro-2H-benzo[b][1,4]oxazine-4-carboxamide

Following the synthetic method of Example 1, compound **JD-5303001d** was replaced with **JD-5303019d** to obtain 6-{[(4-methyl-1,3-thiazol-5-yl)methyl]oxy}-*N*-[3-(prop-2-en-1-yl)piperidin-3-yl]-3,4-dihydro-2*H*-benzo[*b*][1,4]oxazine-4-carboxamide (**JD-5303019**) (8.9 mg, yield: 30.7%, purity: 95.6%) as a white solid.

LC-MS ([M+H]⁺) = 475.3.

**¹H NMR (300 MHz, DMSO-*d*₆):** *δ* = 8.97 (s, 1H), 8.07 (brs, 1H), 7.31 (d, *J* = 2.7 Hz, 1H), 6.78 (d, *J* = 9.0 Hz, 1H), 6.64 (dd, *J* = 9.0, 3.0 Hz, 1H), 6.38 (s, 1H), 5.18 (s, 2H), 4.22 - 4.06 (m, 2H), 4.02 (q, *J* = 7.2 Hz, 2H), 3.89 - 3.78 (m, 1H), 3.78 - 3.68 (m, 1H), 3.68 - 3.59 (m, 1H), 3.05 (d, *J* = 12.3 Hz, 1H), 2.92 - 2.82 (m, 2H), 2.77 - 2.66 (m, 2H), 2.39 (s, 3H), 2.29 - 2.16 (m, 1H), 1.81 - 1.65 (m, 1H), 1.65 - 1.48 (m, 2H), 1.12 (t, *J* = 7.2 Hz, 3H).

### Example 20: Preparation of N-(azetidin-3-yl)-6-{[(4-methyl-1,3-thiazol-5-yl)methyl]oxy}-3,4-dihydro-2H-benzo[b][1,4]oxazine-4-carboxamide hydrochloride (JD-5303020)

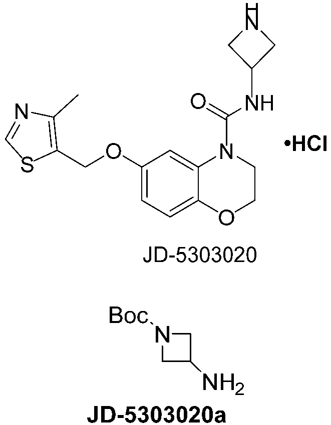

Following the synthetic method of Example 1, **JD-5303001d** was replaced with **JD-5303020a** to obtain *N*-(azetidin-3-yl)-6-{[(4-methyl-1,3-thiazol-5-yl)methyl]oxy}-3,4-dihydro-2*H*-benzo[*b*][1,4]oxazine-4-carboxamide hydrochloride (**JD-5303020**) (55.28 mg, 135.90 µmol, yield: 78.23%, purity: 97.57%, HCl) as a yellow gum.

LC-MS [M+H]⁺ = 361.1.

**¹H NMR:** (400 MHz, DMSO-*d*₆) δ = 2.42 (s, 3 H), 3.67 - 3.80 (m, 2 H), 3.97 - 4.16 (m, 7 H), 5.21 (s, 2 H), 6.64 (dd, *J =* 8.88, 2.88 Hz, 1 H), 6.77 (d, *J* = 8.88 Hz, 1 H), 7.42 (d, *J* = 2.75 Hz, 1 H), 7.92 (br d, *J =* 5.88 Hz, 1 H), 9.25 (br s, 1 H), 9.30 (s, 1 H), 9.38 (br s, 1 H).

### Example 21: Preparation of N-[(1R,3S)-3-aminocyclohexyl]-6-{[(4-methyl-1,3-thiazol-5-yl)methyl]oxy}-3,4-dihydro-2H-benzo[b][1,4]oxazine-4-carboxamide hydrochloride (JD-5303021)

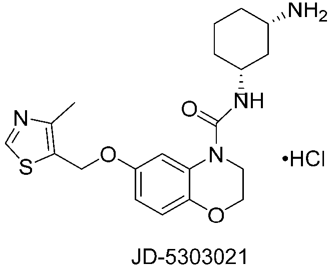

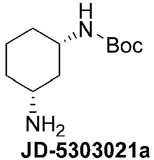

Following the synthetic method of Example 1, compound **JD-5303001d** was replaced with **JD-5303021a** to obtain *N*-[(1*R*,3*S*)-3-aminocyclohexyl]-6-{[(4-methy-1,3-thiazol-5-yl)methyl]oxy}-3,4-dihydro-2*H*-benzo[*b*][1,4]oxazine-4-carboxamide hydrochloride (**JD-5303021**) (60 mg, yield: 74.8%, purity: 99.72%) as a white solid.

LC-MS [M+H]⁺ = 403.2.

**¹H NMR (400 MHz, DMSO-*d*₆):** *δ* 8.98 (s, 1H), 8.00 (s, 2H), 7.36 (d, *J* = 2.8 Hz, 1H), 6.97 (d, *J =* 7.6 Hz, 1H), 6.76 (d, *J =* 8.8 Hz, 1H), 6.60 (dd, *J =* 8.8, 2.8 Hz, 1H), 5.16 (s, 2H), 4.15 - 4.06 (m, 2H), 3.75 - 3.64 (m, 2H), 3.61 - 3.53 (m, 1H), 3.11 - 2.99 (m, 1H), 2.39 (s, 3H), 2.13 (d, *J =* 11.6 Hz, 1H), 1.89 (d, *J =* 12.0 Hz, 1H), 1.82 - 1.72 (m, 2H), 1.41 - 1.26 (m, 2H), 1.25 - 1.11 (m, 2H).

### Example 22: Preparation of N-[(1S,3R)-3-aminocyclohexyl]-6-{[(4-methyl-1,3-thiazol-5-yl)methyl]oxy}-3,4-dihydro-2H-benzo[b][1,4]oxazine-4-carboxamide hydrochloride (JD-5303022)

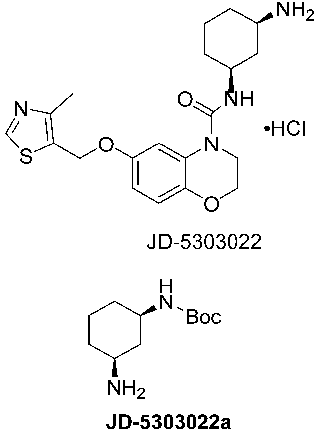

Following the synthetic method of Example 1, compound **JD-5303001d** was replaced with **JD-5303022a** to obtain *N*-[(1*S*,3*R*)-3-aminocyclohexyl]-6-{[(4-methyl-1,3-thiazol-5-yl)methyl]oxy}-3,4-dihydro-2*H*-benzo[*b*][1,4]oxazine-4-carboxamide hydrochloride (**JD-5303022)** (60 mg, yield: 74.8%, purity: 98.32%) as a white solid.

LC-MS [M+H]⁺ = 403.2.

**¹H NMR (300 MHz, DMSO-*d*₆):** *δ* 8.98 (s, 1H), 8.07 (s, 2H),7.36 (d, *J* = 2.91 Hz, 1H), 6.97 (d, *J =* 7.86 Hz, 1H), 6.79 - 6.72 (m, 1H), 6.60 (dd, *J =* 8.85, 2.91 Hz, 1H), 5.16 (s, 2H), 4.16 - 4.06 (m, 2H), 3.75 - 3.62 (m, 2H), 3.62 - 3.53 (m, 1H), 3.14 - 2.98 (m, 1H), 2.39 (s, 3H), 2.21 - 2.07 (m, 1H), 1.96 - 1.85 (m, 1H), 1.83 - 1.68 (m, 2H), 1.44 - 1.23 (m, 4H).

### Example 23: Preparation of N-(1H-imidazol-5-yl)-6-{[(4-methyl-1,3-thiazol-5-yl)methyl]oxy}-3,4-dihydro-2H-benzo[b][1,4]oxazine-4-carboxamide (JD-5303023)

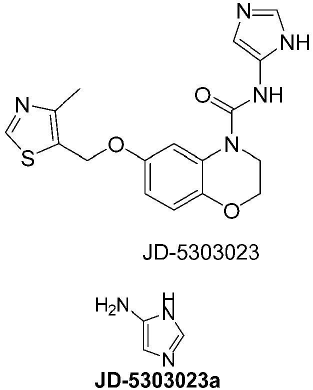

Following the synthetic method of Example 1, compound **JD-5303001d** was replaced with **JD-5303023a** to obtain *N*-(1*H*-imidazol-5-yl)-6-{[(4-methyl-1,3-thiazol-5-yl)methyl]oxy}-3,4-dihydro-2*H*-benzo[*b*][1,4]oxazine-4-carboxamide (**JD-5303023**) (3.00 mg, 8.05 µmol, yield: 2.11%, purity: 99.7%) as a white solid.

LC-MS [M+H]⁺ = 372.1.

**¹H NMR:** (400 MHz, DMSO-*d*₆) δ = 11.70 - 11.90 (m, 1 H), 9.26 - 9.31 (m, 1 H), 8.97 (s, 1 H), 7.35 - 7.48 (m, 2 H), 7.04 (s, 1 H), 6.78 (d, *J =* 8.75 Hz, 1 H), 6.60 - 6.67 (m, 1 H), 5.18 (s, 2 H), 4.11 - 4.19 (m, 2 H), 3.78 - 3.86 (m, 2 H), 2.39 (s, 3 H).

### Example 24: Preparation of 6-{[(4-methyl-1,3-thiazol-5-yl)methyl]oxy}-N-(1,3-thiazol-4-yl)-3,4-dihydro-2H-benzo[b][1,4]oxazine-4-carboxamide (JD-5303024)

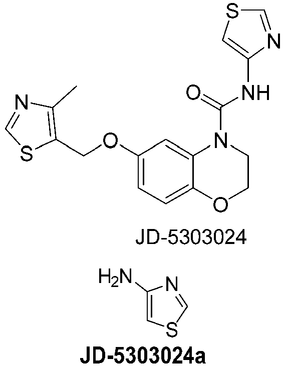

Following the synthetic method of Example 1, **JD-5303001d** was replaced with **JD-**5303024a to obtain 6-{[(4-methyl-1,3-thiazol-5-yl)methyl]oxy}-*N*-(1,3-thiazol-4-yl)-3,4-dihydro-2*H*-benzo[*b*][1,4]oxazine-4-carboxamide (**JD-5303024**) (1.19 mg, 3.06 µmol, yield: 4.01%, purity: 98.62%) as a yellow gum.

LC-MS [M+H]⁺ = 388.9.

¹H NMR: (400 MHz, DMSO-*d*₆) δ ppm 2.38 (s, 3 H) 3.79 - 3.91 (m, 2 H) 4.11 - 4.21 (m, 2 H) 5.18 (s, 2 H) 6.66 (dd, *J* = 8.88, 2.88 Hz, 1 H) 6.80 (d, *J* = 8.88 Hz, 1 H) 7.35 (d, *J* = 2.88 Hz, 1 H) 7.42 (d, *J=* 2.25 Hz, 1 H) 8.95 - 8.97 (m, 1 H) 10.11 (s, 1 H).

### Example 25: Preparation of N-(isoxazol-3-yl)-6-{[(4-methyl-1,3-thiazol-5-yl)methyl]oxy}-3,4-dihydro-2H-benzo[b][1,4]oxazine-4-carboxamide (JD-5303025)

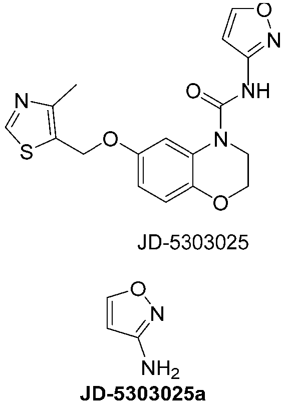

Following the synthetic method of Example 1, compound **JD-5303001d** was replaced with **JD-5303025a** to obtain *N*-(isoxazol-3-yl)-6-{[(4-methyl-1,3-thiazol-5-yl)methyl]oxy}-3,4-dihydro-2*H*-benzo[*b*][1,4]oxazine-4-carboxamide (**JD-5303025**) (3.62 mg, 9.72 µmol, yield: 12.7%, purity: 97.56%) as an off-white solid.

LC-MS [M+H]⁺ = 373.1.

¹H NMR: (400 MHz, DMSO-*d*₆) δ ppm 2.38 (s, 3 H) 3.83 (br d, *J* = 4.38 Hz, 2 H) 4.15 - 4.21 (m, 2 H) 5.18 (s, 2 H) 6.68 (dd, *J* = 8.88, 2.88 Hz, 1 H) 6.81 (dd, *J* = 5.13, 3.63 Hz, 2 H) 7.35 (d, *J =* 2.75 Hz, 1 H) 8.75 (d, *J =* 1.25 Hz, 1 H) 8.98 (s, 1 H) 10.28 (s, 1 H).

### Example 26: Preparation of N-(1-ethylpiperidin-3-yl)-6-{[(4-methyl-1,3-thiazol-5-yl)methyl]oxyl-3,4-dihydro-2H-benzo[b][1,4]oxazine-4-carboxamide trifluoroacetate (JD-5303026)

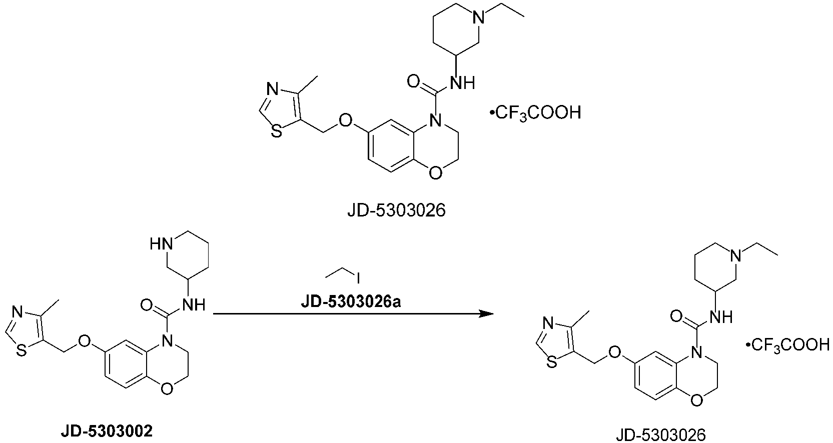

To a solution of *N*-(piperidin-3-yl)-6-{[(4-methyl-1,3-thiazol-5-yl)methyl]oxy}-3,4-dihydro-2*H*-benzo[*b*][1,4]oxazine-4-carboxamide hydrochloride (**JD-5303002**) (50.0 mg, 128 µmol, 1.00 eq) in DMF (2 mL) were added K₂CO₃ (35.5 mg, 257 µmol, 2.00 eq), DIEA (33.2 mg, 257 µmol, 44.8 µL, 2.00 eq), and iodoethane (**JD-5303026a**) (18.0 mg, 115 µmol, 9.26 µL, 0.90 eq), and the mixture was stirred at 25°C for 16 hours. The reaction mixture was quenched with H₂O (2.00 mL) and extracted with EtOAc (4.00 mL, 2.00 mL * 2). The combined organic phases were concentrated under reduced pressure to obtain a residue. The residue was purified by preparative HPLC (column: Welch xextreme C18 150 * 25 mm * 5 µm; mobile phase: [water (TFA)-ACN]; gradient: 13% to 33% B over 10 minutes) to obtain *N*-(1-ethylpiperidin-3-yl)-6-{[(4-methyl-1,3-thiazol-5-yl)methyl]oxy}-3,4-dihydro-2*H-*benzo[*b*][1,4]oxazine-4-carboxamide trifluoroacetate (**JD-5303026**) (20.0 mg, 48.0 µmol, yield: 37.3%, purity: 99.41%) as a yellow gum.

LC-MS [M+H]⁺ = 417.2.

**¹H NMR:** (400 MHz, DMSO-*d*₆) δ = 1.22 (t, *J* = 7.25 Hz, 3 H), 1.46 - 1.83 (m, 2 H), 1.92 (br d, *J =* 11.51 Hz, 2 H), 2.39 (s, 3 H), 2.59 - 2.80 (m, 2 H), 3.11 - 3.22 (m, 2 H), 3.41 - 3.57 (m, 2 H), 3.64 - 3.76 (m, 2 H), 3.93 (dt, *J* = 7.66, 3.86 Hz, 1 H), 4.13 (br t, *J* = 4.25 Hz, 2 H), 5.17 (s, 2 H), 6.63 (dd, *J =* 8.88, 3.00 Hz, 1 H), 6.78 (d, *J* = 8.88 Hz, 1 H), 7.12 (br d, *J =* 7.75 Hz, 1 H), 7.35 (d, *J* = 2.88 Hz, 1 H), 8.99 (s, 1 H), 9.60 (br s, 1 H).

### Example 27: Preparation of N-(1-ethyl-4,4-difluorotetrahydro-1H-pyrrol-3-yl)-6-{[(2-fluorophenyl)methyl]oxy}-3,4-dihydro-2H-benzo[b][1,4] oxazine-4-carboxamide hydrochloride (JD-5303027)

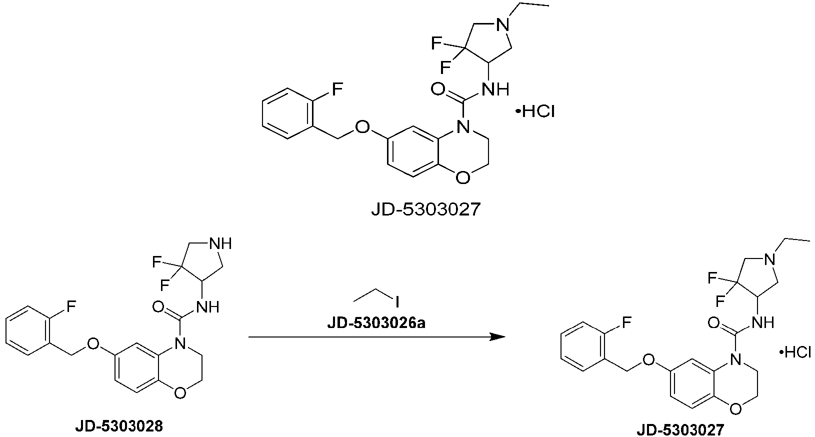

To a solution of *N*-(4,4-difluorotetrahydro-1*H*-pyrrol-3-yl)-6-{[(2-fluorophenyl)methyl]oxyl-3,4-dihydro-2*H*-benzo[*b*][1,4]oxazine-4-carboxamide hydrochloride (**JD-5303028**) (50.0 mg, 122 µmol, 1.00 eq) and iodoethane (**JD-5303026a**) (38.2 mg, 245 µmol, 19.6 µL, 2.00 eq) in DMF (1.00 mL) were added K₂CO₃ (33.9 mg, 245 µmol, 2.00 eq) and DIEA (31.7 mg, 245 µmol, 42.7 µL, 2.00 eq), and the mixture was stirred at 25°C for 12 hours. The reaction mixture was quenched with H₂O (2.00 mL) and extracted with EtOAc (4.00 mL, 2.00 mL * 2). The combined organic phases were separated, filtered, and concentrated under reduced pressure to obtain a residue. The residue was purified by preparative HPLC to obtain *N*-(1-ethyl-4,4-difluorotetrahydro-1*H*-pyrrol-3-yl)-6-{[(2-fluorophenyl)methyl]oxy}-3,4-dihydro-2*H*-benzo[*b*][1,4]oxazine-4-carboxamide hydrochloride (**JD-5303027**) (27.8 mg, 64.0 µmol, yield: 52.1%, purity: 99.41%) as a colorless gum.

LC-MS [M+H]⁺ = 436.2.

¹H NMR: (400 MHz, DMSO-*d*₆) δ ppm 1.18 (t, *J* = 7.19 Hz, 3 H) 3.06 - 3.17 (m, 2 H) 3.32 (br d, *J* = 5.88 Hz, 1 H) 3.67 (ddd, *J* = 10.01, 6.69, 3.31 Hz, 2 H) 3.79 (br dd, *J* = 5.38, 3.00 Hz, 1 H) 3.81 - 3.84 (m, 1 H) 3.94 (br d, *J* = 10.88 Hz, 1 H) 4.10 - 4.20 (m, 2 H) 4.71 - 4.89 (m, 1 H) 5.03 (s, 2 H) 6.68 (dd, *J* = 8.94, 2.94 Hz, 1 H) 6.81 (d, *J* = 8.88 Hz, 1 H) 7.19 - 7.27 (m, 2 H) 7.33 (d, *J* = 2.88 Hz, 1 H) 7.37 - 7.46 (m, 2 H) 7.52 (td, *J* = 7.66, 1.44 Hz, 1 H).

### Example 28: Preparation of 6-{[(4-methyl-1,3-thiazol-5-yl)methyl]oxy}-N-(3-propylpiperidin-3-yl)-3,4-dihydro-2H-benzo[b][1,4]oxazine-4-carboxamide formate (JD-5303056)

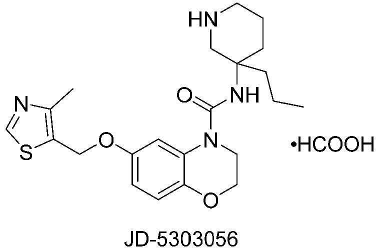

### Step 1: Preparation of tert-butyl 3-{[(6-{[(4-methyl-1,3-thiazol-5-yl)methyl]oxy}-3,4-dihydro-2H-benzo[b][1,4]oxazin-4-yl)carbonyl]amino}-3-propylpiperidine-1-carboxylate (JD-5303056-1)

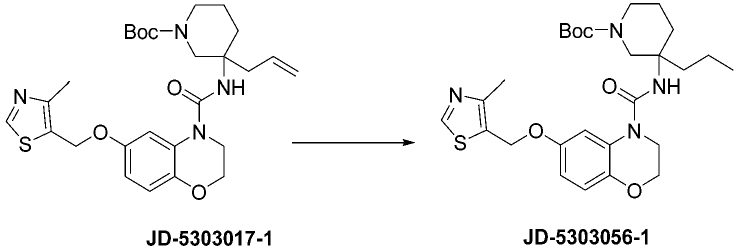

Intermediate **JD-5303017-1** (30 mg, 0.057 mmol, 1.0 eq) of Example 17 was dissolved in MeOH (1.0 mL), then Pd/C (5 mg) (10%) was added thereto, and the suspension was degassed and purged with H₂ three times. The mixture was stirred under a H₂ (15 psi) atmosphere at room temperature for 15 hours. The reaction mixture was filtered, and the filtrate was concentrated to obtain *tert*-butyl 3-{[(6-{[(4-methyl-1,3-thiazol-5-yl)methyl]oxy}-3,4-dihydro-2*H*-benzo[*b*][1,4]oxazin-4-yl)carbonyl]amino}-3-propylpiperidine-1-carboxylate **(JD-5303056-1)** (26 mg, crude product) as a yellow solid.

LC-MS ([M+H]⁺) = 531.2.

### Step 2: Preparation of 6-{[(4-methyl-1,3-thiazol-5-yl)methyl]oxy}-N-(3-propylpiperidin-3-yl)-3,4-dihydro-2H-benzo[b][1,4]oxazine-4-carboxamide formate (JD-5303056)

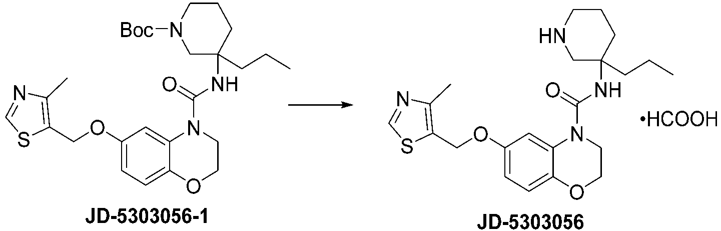

*tert*-Butyl 3-{[(6-{[(4-methyl-1,3-thiazol-5-yl)methyl]oxy}-3,4-dihydro-2*H-*benzo[*b*][1,4]oxazin-4-yl)carbonyl]amino}-3-propylpiperidine-1-carboxylate **(JD-5303056-1)** (26 mg, 0.049 mmol, 1.0 eq) was dissolved in DCM (2 mL), then dioxane/HCl (0.25 mL, 0.98 mmol, 20.0 eq) (4 N) was added thereto, and the mixture was reacted at room temperature for 2 hours. The reaction mixture was concentrated under reduced pressure to obtain a residue. The residue was quenched with sodium hydroxide solution (15%) (3 mL) and extracted with DCM (20 mL, 10 mL * 2). The combined organic phases were washed with brine (10 mL), dried over anhydrous sodium sulfate, and concentrated under reduced pressure to obtain a residue. The residue was purified by prep-HPLC (column: WelchUlC18 150 * 25 mm * 5 µm; mobile phase: [water (0.1% FA)-ACN]; B%: 25% to 35%, 6 minutes) to obtain 6-{[(4-methyl-1,3-thiazol-5-yl)methyl]oxy}-*N*-(3-propylpiperidin-3-yl)-3,4-dihydro-2*H-*benzo[*b*][1,4]oxazine-4-carboxamide formate **(JD-5303056)** (10.1 mg, two-step yield: 39.6%, purity: 98.2%) as a yellow solid.

LC-MS ([M+H]⁺) = 431.2.

**¹H NMR** (400 MHz, CDCl₃) δ = 8.69 (s, 1H), 8.32 (brs, 0.40H), 7.32 (s, 1H), 6.88 - 6.77 (m, 1H), 6.70 - 6.58 (m, 1H), 5.61 (s, 1H), 5.13 (s, 2H), 4.26 (s, 1H), 4.10 (s, 1H), 4.02 - 3.88 (m, 2H), 3.74 - 3.58 (m, 1H), 3.42 - 3.26 (m, 1H), 2.86 - 2.61 (m, 2H), 2.46 (s, 3H), 2.25 - 2.09 (m, 1H), 1.98 - 1.80 (m, 2H), 1.78 - 1.59 (m, 2H), 1.49 - 1.34 (m, 1H), 1.34 - 1.12 (m, 2H), 1.00 - 0.85 (m, 3H).

### Example 29: Preparation of N-[3-(2-hydroxyethyl)piperidin-3-yl]-6-{ [(4-methyl-1,3-thiazol-5-yl)methyl]oxy}-3,4-dihydro-2H-benzo[b][1,4]oxazine-4-carboxamide formate (JD-5303057)

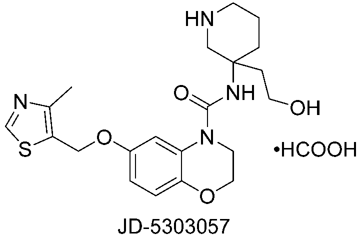

### Step 1: Preparation of tert-butyl 3-(2-hydroxyethyl)-3-{[(6-{[(4-methyl-1,3-thiazol-5-yl)methyl]oxy}-3,4-dihydro-2H-benzo[b][1,4]oxazin-4-yl)carbonyl]amino}piperidine-1-carboxylate (JD-5303057-1)

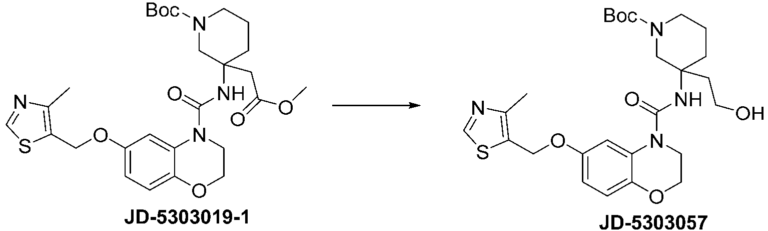

Intermediate **JD-5303019-1** (60 mg, 0.107 mmol, 1.0 eq) of Example 19 was dissolved in tetrahydrofuran (2.0 mL), then LiAlH₄ (1.0 M in THF) (0.16 mL, 0.161 mmol, 1.5 eq) was added thereto at 0°C, and the mixture was reacted at 0°C for 0.5 hours. The reaction mixture was quenched with 15% sodium hydroxide aqueous solution (0.1 mL) at 0°C, then dried over anhydrous sodium sulfate, filtered, and the filtrate was concentrated to obtain a residue. The residue was purified by prep-TLC (dichloromethane/methanol = 20/1) to obtain *tert*-butyl 3-(2-hydroxyethyl)-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303057-1)** (30 mg, yield: 52.3%) as a yellow solid.

**LCMS:** 533.2 ([M+H]⁺).

### Step 2: Preparation of N-[3-(2-hydroxyethyl)piperidin-3-yl]-6-{[(4-methyl-1,3-thiazol-5-yl)methyl]oxy}-3,4-dihydro-2H-benzo[b][1,4]oxazine-4-carboxamide formate (JD-5303057)

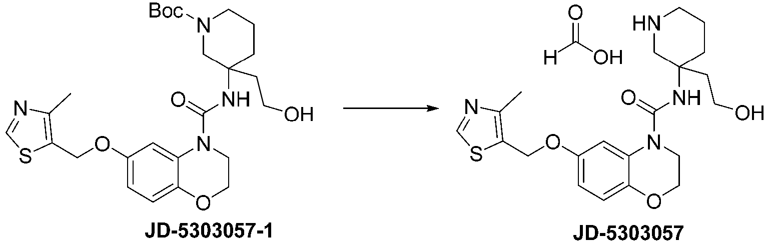

*tert*-Butyl 3-(2-hydroxyethyl)-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303057-1)** (30 mg, 0.056 mmol, 1.0 eq) was dissolved in dichloromethane (1.0 mL), then TFA (128 mg, 1.12 mmol, 20.0 eq) was added thereto, and the mixture was reacted at room temperature for 2 hours. The reaction mixture was then added with potassium carbonate aqueous solution to adjust the pH to 11, and extracted with dichloromethane (10 mL, 5.0 mL * 2). The combined organic phases were concentrated under reduced pressure to obtain a residue. The residue was purified by prep-HPLC (column: WelchUlC18 150 * 19 mm * 5 mm; mobile phase: [water (FA)-ACN]; B%: 25% to 40%, 6 minutes) to obtain *N*-[3-(2-hydroxyethyl)piperidin-3-yl]-6-{[(4-methyl-1,3-thiazol-5-yl)methyl]oxy}-3,4-dihydro-2*H-*benzo[*b*][1,4]oxazine-4-carboxamide formate **(JD-5303057)** (7.0 mg, yield: 28.5%, purity: 97.9%) as a yellow solid.

LC-MS ([M+H]⁺) = 433.2.

**¹H NMR (300 MHz, MeOD):** δ 8.88 (s, 1H), 7.27 (d, *J* = 3.0 Hz, 1H), 6.82 (d,*J* = 9.0 Hz, 1H), 6.69 (dd, *J* = 9.0, 3.0 Hz, 1H), 5.20 (s, 2H), 4.26 - 4.12 (m, 3H), 3.92 - 3.82 (m, 1H), 3.71 (t, *J* = 6.6 Hz, 3H), 3.29 - 3.25 (m, 1H), 3.06 - 2.94 (m, 2H), 2.44 (s, 3H), 2.30 - 2.13 (m, 2H), 2.05 - 1.60 (m, 4H).

### Example 30: Preparation of 2-(3-{[(6-{[(4-methyl-1,3-thiazol-5-yl)methyl]oxy}-3,4-dihydro-2H-benzo[b][1,4]oxazin-4-yl)carbonyl]amino}piperidin-3-yl)acetamide 2,2,2-trifluoroacetate (JD-5303058)

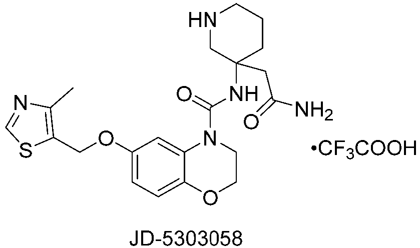

### Step 1: Preparation of tert-butyl 3-(carbamoylmethyl)-3-{[(6-{[(4-methyl-1,3-thiazol-5-yl)methyl]oxy}-3,4-dihydro-2H-benzo[b][1,4]oxazin-4-yl)carbonyl]amino}piperidine-1-carboxylate (JD-5303058-1)

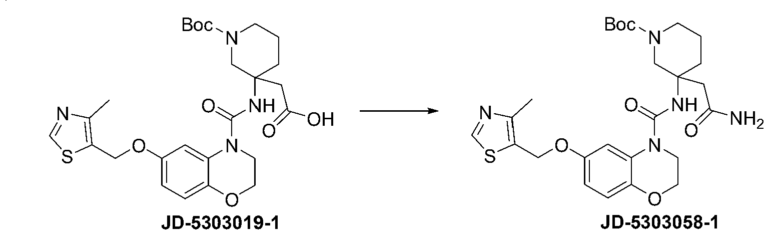

Intermediate **JD-5303019-1** (40 mg, 0.073 mmol, 1.0 eq) of Example 19 was dissolved in dichloromethane (2.0 mL), followed by the addition of DIEA (28 mg, 0.219 mmol, 3.0 eq), HATU (33 mg, 0.088 mmol, 1.2 eq), DMAP (0.85 mg, 0.007 mmol, 0.1 eq), and ammonium chloride (6 mg, 0.11 mmol, 1.5 eq), and the mixture was reacted at room temperature for 3 hours. The reaction mixture was quenched with water (10 mL) at room temperature and extracted with dichloromethane (20 mL, 10.0 mL * 2). The combined organic phases were concentrated under reduced pressure to obtain a residue. The residue was purified by prep-TLC (dichloromethane/methanol = 15/1) to obtain *tert*-butyl 3-(carbamoylmethyl)-3-{[(6-{[(4-methyl-1,3-thiazol-5-yl)methyl]oxy}-3,4-dihydro-2*H-*benzo[b][1,4]oxazin-4-yl)carbonyl]amino}piperidine-1-carboxylate **(JD-5303058-1)** (30 mg, yield: 75.3%) as a yellow solid.

LC-MS ([M+H]⁺) = 546.2.

### Step 2: Preparation of 2-(3-{[(6-{[(4-methyl-1,3-thiazol-5-yl)methyl]oxy}-3,4-dihydro-2H-benzo[b][1,4]oxazin-4-yl)carbonyl]amino}piperidin-3-yl)acetamide 2,2,2-trifluoroacetate (JD-5303058)

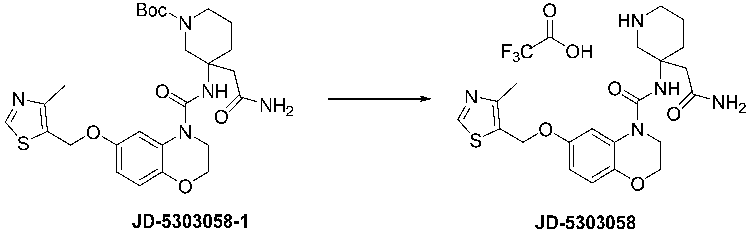

*tert*-Butyl 3-(carbamoylmethyl)-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303058-1)** (50 mg, 0.092 mmol, 1.0 eq) was dissolved in dichloromethane (1.0 mL), then TFA (210 mg, 1.84 mmol, 20.0 eq) was added thereto, and the mixture was reacted at room temperature for 2 hours. The reaction mixture was then concentrated under reduced pressure to obtain a residue. The residue was purified by prep-HPLC (column: WelchUlC18 150 * 19 mm * 5 mm; mobile phase: [water (TFA)-ACN]; B%: 25% to 45%, 6 minutes) to obtain 2-(3-{[(6-{[(4-methyl-1,3-thiazol-5-yl)methyl]oxy}-3,4-dihydro-2*H*-benzo[*b*][1,4]oxazin-4-yl)carbonyl]amino}piperidin-3-yl)acetamide 2,2,2-trifluoroacetate **(JD-5303058)** (16.2 mg, yield: 39.5%, purity: 99.04%) as a yellow solid.

LC-MS ([M+H]⁺) = 446.1.

**¹H NMR (300 MHz, MeOD):** δ 8.94 (s, 1H), 7.33 (d, *J* = 2.7 Hz, 1H), 6.82 (d,*J* = 9.0 Hz, 1H), 6.70 (dd, *J* = 9.0, 3.0 Hz, 1H), 5.22 (s, 2H), 4.31 - 4.17 (m, 3H), 3.88 - 3.77 (m, 2H), 3.38 - 3.32 (m, 1H), 3.09 (d, *J* = 12.9 Hz, 1H), 3.07 - 2.96 (m, 1H), 2.85 (d, *J* = 14.4 Hz, 1H), 2.53 (d, *J* = 14.4 Hz, 1H), 2.45 (s, 3H), 2.33 (d, *J* = 13.2 Hz, 1H), 2.06 - 1.69 (m, 3H).

### Example 31: Preparation of N-[3-(dimethylamino)cyclohexyl]-6-{[(4-methyl-1,3-thiazol-5-yl)methyl]oxy}-3,4-dihydro-2H-benzo[b][1,4]oxazine-4-carboxamide formate (JD-5303059)

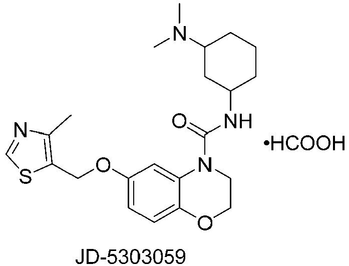

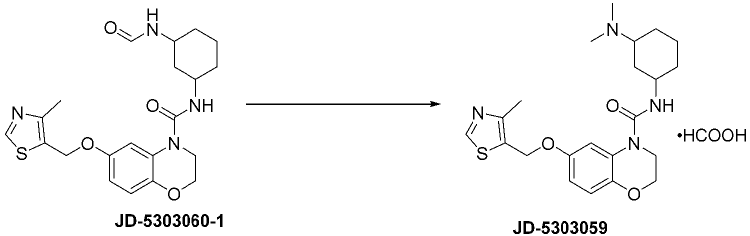

Intermediate **JD-5303060-1** (19 mg, 0.044 mmol, 1.0 eq) of Example 32 was dissolved in THF (1 mL), then BH₃-THF (0.22 mL, 0.22 mmol, 5.0 eq) was added thereto, and the mixture was reacted at room temperature for 15 hours. To the reaction system were added HCl (1 mL) (1 N) and MeOH (2 mL), and the mixture was stirred at room temperature for 20 minutes. The reaction mixture was quenched with saturated sodium carbonate solution (5 mL) and extracted with ethyl acetate (18 mL, 6 mL * 3). The combined organic phases were washed with brine (6 mL), dried over anhydrous sodium sulfate, and concentrated under reduced pressure to obtain a residue. The residue was purified by prep-HPLC (column: WelchUlC18 150 * 25 mm * 5 mm; mobile phase: [water (0.1% FA)-ACN]; B%: 25% to 35%, 6 minutes) to obtain N-[3-(dimethylamino)cyclohexyl]-6-{[(4-methyl-1,3-thiazol-5-yl)methyl]oxy}-3,4-dihydro-2*H*-benzo[*b*][1,4]oxazine-4-carboxamide formate **(JD-5303059)** (2.3 mg, yield: 11.9%, purity: 91.2%) as a yellow solid.

LC-MS ([M+H]⁺) = 431.2.

**¹H** NMR (300 MHz, MeOD) δ = 8.88 (s, 1H), 7.16 - 7.11 (m, 1H), 6.82 (d, *J* = 9.0 Hz, 1H), 6.68 (dd, *J* = 3.0, 9.0 Hz, 1H), 5.47 - 5.30 (m, 1H), 5.20 (s, 2H), 4.27 - 4.14 (m, 2H), 4.14 - 4.07 (m, 1H), 3.90 - 3.62 (m, 2H), 3.31 - 3.30 (m, 6H), 2.93 - 2.75 (m, 1H), 2.44 (s, 3H), 2.40 - 2.34 (m, 2H), 2.18 - 1.96 (m, 2H), 1.76 - 1.69 (m, 2H), 1.60 - 1.49 (m, 2H).

### Example 32: Preparation of N-[3-(methylamino)cyclohexyl]-6-{[(4-methyl-1,3-thiazol-5-yl)methyl]oxy}-3,4-dihydro-2H-benzo[b][1,4]oxazine-4-carboxamide formate (JD-5303060)

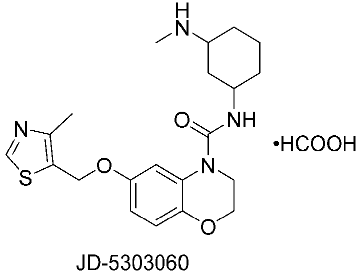

### Step 1: Preparation of N-[3-(formamido)cyclohexyl]-6-{[(4-methyl-1,3-thiazol-5-yl)methyl]oxy}-3,4-dihydro-2H-benzo[b][1,4]oxazine-4-carboxamide (JD-5303060-1)

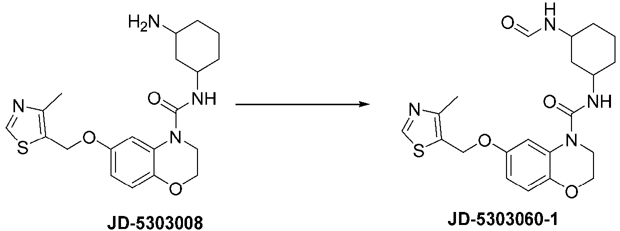

The product **JD-5303008** (70 mg, 0.17 mmol, 1.0 eq) of Example 8 was dissolved in DCM (2 mL), followed by the addition of HCOOH (40 mg, 0.87 mmol, 5.0 eq), HATU (331 mg, 0.87 mmol, 5.0 eq), and DIEA (225 mg, 1.74 mmol, 10.0 eq), and the mixture was reacted at room temperature for 15 hours. The reaction mixture was quenched with saturated potassium carbonate solution (5 mL) and extracted with dichloromethane (20 mL, 10 mL * 2). The combined organic phases were washed with brine (10 mL), dried over anhydrous sodium sulfate, and concentrated under reduced pressure to obtain a residue. The residue was purified by prep-TLC (dichloromethane/methanol = 8/1) to obtain *N*-[3-(formamido)cyclohexyl]-6-{[(4-methyl-1,3-thiazol-5-yl)methyl]oxy}-3,4-dihydro-2*H*-benzo[*b*][1,4]oxazine-4-carboxamide **(JD-5303060-1)** (50 mg, yield: 66.7%, purity: 94%) as a yellow solid.

LC-MS ([M+H]⁺) = 431.3.

**¹H NMR** (400 MHz, DMSO-*d*₆) δ = 8.97 (s, 1H), 8.16 (d, *J* = 7.6 Hz, 1H), 7.95 (s, 1H), 7.32 (d, *J* = 2.8 Hz, 1H), 6.76 (d, *J* = 8.8 Hz, 1H), 6.70 (d, *J* = 7.2 Hz, 1H), 6.59 (dd, *J* = 2.8, 8.8 Hz, 1H), 5.16 (s, 2H), 4.14 - 4.06 (m, 3H), 3.89 - 3.80 (m, 1H), 3.70 - 3.64 (m, 2H), 2.38 (s, 3H), 1.71 - 1.36 (m, 8H).

### Step 2: Preparation of N-[3-(methylamino)cyclohexyl]-6-{[(4-methyl-1,3-thiazol-5-yl)methyl]oxy}-3,4-dihydro-2H-benzo[b][1,4]oxazine-4-carboxamide formate (JD-5303060)

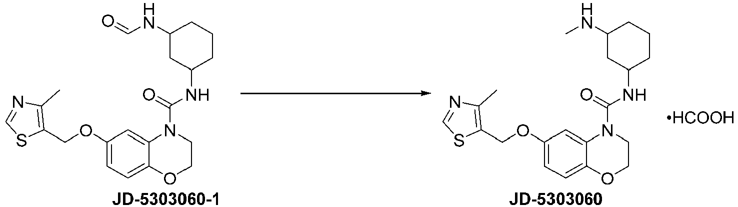

*N*-[3-(Formamido)cyclohexyl]-6-{[(4-methyl-1,3-thiazol-5-yl)methyl]oxy}-3,4-dihydro-2*H*-benzo[*b*][1,4]oxazine-4-carboxamide **(JD-5303060-1)** (50 mg, 0.116 mmol, 1.0 eq) was dissolved in THF (1 mL), then BH₃-THF (0.58 mL, 0.58 mmol, 5.0 eq) was added thereto, and the mixture was reacted at room temperature for 15 hours. To the reaction system were added HCl (2 mL) (1 N) and MeOH (4 mL), and the mixture was stirred at room temperature for 20 minutes. The reaction mixture was quenched with saturated sodium carbonate solution (10 mL) and extracted with ethyl acetate (30 mL, 10 mL * 3). The combined organic phases were washed with brine (10 mL), dried over anhydrous sodium sulfate, and concentrated under reduced pressure to obtain a residue. The residue was purified by prep-HPLC (column: WelchUlC18 150 * 25 mm * 5 mm; mobile phase: [water (0.1% FA)-ACN]; B%: 25% to 35%, 6 minutes) to obtain *N*-[3-(methylamino)cyclohexyl]-6-{[(4-methyl-1,3-thiazol-5-yl)methyl]oxy}-3,4-dihydro-2*H*-benzo[*b*][1,4]oxazine-4-carboxamide formate **(JD-5303060)** (3.1 mg, yield: 5.9%, purity: 99.8%) as a yellow solid.

LC-MS ([M+H]⁺) = 417.2.

**¹H** NMR (400 MHz, MeOD) δ = 8.89 (s, 1H), 8.53 (brs, 0.73H), 7.20 (s, 1H), 6.86 - 6.76 (m, 1H), 6.69 (s, 1H), 5.19 (s, 2H), 4.18 (s, 2H), 4.03 (s, 1H), 3.77 (s, 2H), 2.69 (s, 3H), 2.44 (s, 3H), 2.16 - 1.54 (m, 8H).

### Example 33: Preparation of N-(4,4-difluorotetrahydro-1H-pyrrol-3-yl)-6-{[(2-fluorophenyl)methyl]oxy}-3,4-dihydro-2H-benzo[b][1,4]oxazine-4-carboxamide hydrochloride (JD-5303028)

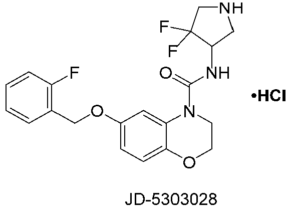

### Step 1: Preparation of 6-{[(2-fluorophenyl)methyl]oxy}-3,4-dihydro-2H-benzo[b][1,4]oxazin-3-one (JD-5303028-2)

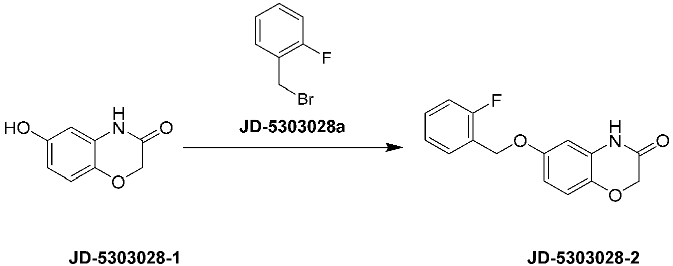

To a solution of DMF (10.0 mL) were added 6-hydroxy-3,4-dihydro-2*H-*benzo[b][1,4]oxazin-3-one **(JD-5303028-1)** (1.00 g, 6.06 mmol, 1.00 eq), K₂CO₃ (1.67 g, 12.1 mmol, 2.00 eq), and 2-(bromomethyl)-1-fluorobenzene **(JD-5303028a)** (1.14 g, 6.06 mmol, 730 µL, 1.00 eq). The reaction mixture was stirred at 25°C for 12 hours. The reaction mixture was quenched with H₂O (10.0 mL) and extracted twice with EtOAc (20.0 mL). The organic phases were separated, filtered, and concentrated under reduced pressure to obtain a residue. The residue was purified by column chromatography (SiO₂, petroleum ether/ethyl acetate = 3/1) to obtain 6-{[(2-fluorophenyl)methyl]oxy}-3,4-dihydro-2*H-*benzo[b][1,4]oxazin-3-one **(JD-5303028-2)** (250 mg, 914 µmol, yield: 15.1%) as a white solid.

LC-MS [M+H]⁺ = 274.1.

### Step 2: Preparation of 6-{[(2-fluorophenyl)methyl]oxy}-3,4-dihydro-2H-benzo[b][1,4]oxazine (JD-5303028-3)

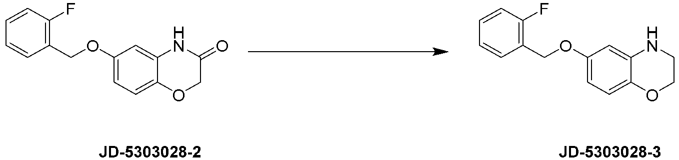

6-{[(2-Fluorophenyl)methyl]oxy}-3,4-dihydro-2*H*-benzo[*b*][1,4]oxazin-3-one (JD-**5303028-2)** (250 mg, 914 µmol, 1.00 eq) was dissolved in a solution of THF (2.50 mL). BH₃-Me₂S (10 M, 457 µL, 5.00 eq) was added thereto at 0°C, and the mixture was stirred at 0°C for 2 hours. The reaction mixture was quenched with MeOH (5.00 mL) at 0°C and concentrated under reduced pressure to obtain a residue. The residue was purified by column chromatography (SiO₂, petroleum ether/ethyl acetate = 2/1) to obtain 6-{[(2-fluorophenyl)methyl]oxy}-3,4-dihydro-2*H*-benzo[*b*][1,4]oxazine **(JD-5303028-3)** (60.0 mg, 231 µmol, yield: 25.2%) as a brown oil.

LC-MS [M+H]⁺ = 260.0.

### Step 3: Preparation of tert-butyl 3,3-difluoro-4-{[(6-{[(2-fluorophenyl)methyl]oxy}-3,4-dihydro-2H-benzo[b][1,4]oxazin-4-yl)carbonyl]amino}pyrrolidine-1-carboxylate (JD-5303028-4)

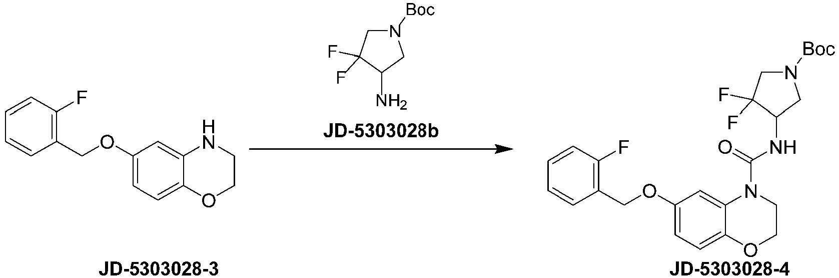

6-{[(2-Fluorophenyl)methyl]oxy}-3,4-dihydro-2*H*-benzo[*b*][1,4]oxazine **(JD-5303028-3)** (50.0 mg, 192 µmol, 1.00 eq) and *tert*-butyl 4-amino-3,3-difluoropyrrolidine-1-carboxylate **(JD-5303028b)** (257 mg, 1.16 mmol, 6.00 eq) were dissolved in DMF (1.00 mL), followed by the addition of CDI (69.7 mg, 430 µmol, 2.00 eq). The mixture was stirred at 65°C for 12 hours. The reaction mixture was filtered and concentrated under reduced pressure to obtain a residue. The residue was purified by preparative HPLC (column: Welch xextreme C18 150 * 25 mm * 5 µm; mobile phase: [water (TFA)-ACN]; gradient: 46% to 76% B over 10 minutes) to obtain *tert*-butyl 3,3-difluoro-4-{[(6-{[(2-fluorophenyl)methyl]oxy}-3,4-dihydro-2*H*-benzo[*b*][1,4]oxazin-4-yl)carbonyl]amino }pyrrolidine-1-carboxylate (**JD-5303028-4)** (85.0 mg, 167 µmol, yield: 86.8%) as a gray solid.

LC-MS [M+Na] = 530.1.

### Step 4: Preparation of N-(4,4-difluorotetrahydro-1H-pyrrol-3-yl)-6-{[(2-fluorophenyl)methyl]oxy}-3,4-dihydro-2H-benzo[b][1,4]oxazine-4-carboxamide hydrochloride (JD-5303028)

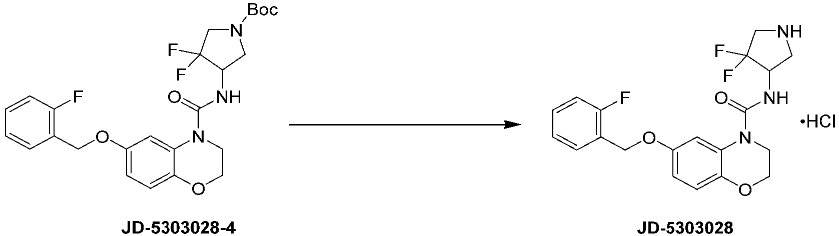

To TFE (1.00 mL) were added *tert*-butyl 3,3-difluoro-4-{[(6-{[(2-fluorophenyl)methyl]oxy}-3,4-dihydro-2*H*-benzo[*b*][1,4]oxazin-4-yl)carbonyl]amino}pyrrolidine-1-carboxylate **(JD-5303028-4)** (30.0 mg, 59.1 µmol, 1.00 eq) and TMSC1 (12.8 mg, 118 µmol, 15.0 µL, 2.00 eq). The mixture was stirred at 25°C for 2 hours. The reaction mixture was filtered and concentrated under reduced pressure to obtain *N*-(4,4-difluorotetrahydro-1*H*-pyrrol-3-yl)-6-{[(2-fluorophenyl)methyl]oxy}-3,4-dihydro-2*H-*benzo[*b*][1,4]oxazine-4-carboxamide hydrochloride **(JD-5303028)** (19.3 mg, 43.5 µmol, yield: 73.6%, purity: 95.83%) as a white solid.

LC-MS [M+Na] = 408.0.

**¹H NMR:** (400 MHz, DMSO-*d*₆) δ = 3.42 - 3.47 (m, 1 H), 3.65 - 3.87 (m, 5 H), 4.07 - 4.26 (m, 2 H), 4.74 - 4.90 (m, 1 H), 5.03 (s, 2 H), 6.67 (dd, *J* = 8.88, 2.88 Hz, 1 H), 6.80 (d, *J* = 8.88 Hz, 1 H), 7.20 - 7.29 (m, 2 H), 7.35 (d, *J* = 2.88 Hz, 1 H), 7.38 - 7.46 (m, 1 H), 7.49 - 7.61 (m, 2 H), 9.87 - 10.31 (m, 1 H).

### Example 34: Preparation of N-(4,4-difluorotetrahydro-1H-pyrrol-3-yl)-6-{[(2-methyl-1,3-thiazol-5-yl)methyl]oxy}-3,4-dihydro-2H-benzo[b][1,4]oxazine-4-carboxamide hydrochloride (JD-5303029)

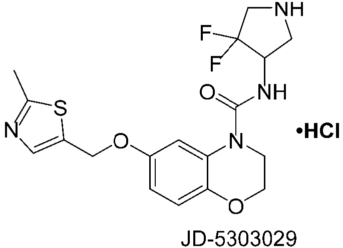

### Step 1: Preparation of (2-methyl-1,3-oxazol-5-yl)methanol (JD-5303029b)

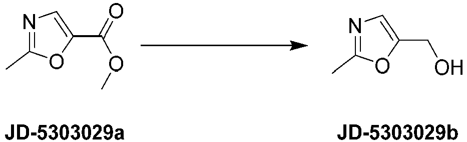

To a solution of methyl 2-methyl-1,3-oxazole-5-carboxylate **(JD-5303029a)** (1.00 g, 7.09 mmol, 1.00 eq) in THF (10.0 mL) was added LAH (2.50 M, 4.25 mL, 1.50 eq) at 0°C, and the mixture was stirred at 0°C for 2 hours. The reaction mixture was quenched with H₂O (0.40 mL) at -10°C, then diluted with 15% NaOH (0.40 mL) and H₂O (1.20 mL), stirred at 25°C for 15 minutes, and filtered and concentrated under reduced pressure to obtain (2-methyl-1,3-oxazol-5-yl)methanol **(JD-5303029b)** (1.00 g, crude product) as a yellow oil.

### Step 2: Preparation of 5-(chloromethyl)-2-methyl-1,3-oxazole (JD-5303029c)

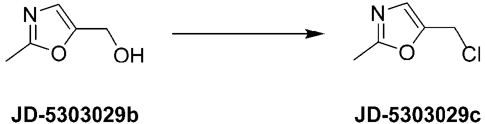

To a solution of (2-methyl-1,3-oxazol-5-yl)methanol **(JD-5303029b)** (1.00 g, 8.84 mmol, 1.00 eq) in THF (10.0 mL) was added SOCl₂ (2.10 g, 17.6 mmol, 1.28 mL, 2.00 eq), and the mixture was stirred at 0°C for 1 hour. The reaction mixture was quenched with Na₂CO₃ to adjust the pH to 8. The reaction mixture was then slowly quenched with warm water, extracted twice with ethyl acetate, and concentrated under reduced pressure to obtain 5-(chloromethyl)-2-methyl-1,3-oxazole **(JD-5303029c)** (1.00 g, crude product) as a yellow oil.

### Step 3: Preparation of N-(4,4-difluorotetrahydro-1H-pyrrol-3-yl)-6-{[(2-methyl-1,3-thiazol-5-yl)methyl]oxy}-3,4-dihydro-2H-benzo[b][1,4]oxazine-4-carboxamide hydrochloride (JD-5303029)

Following the synthetic method of Example 33, compound **JD-5303029c** was replaced with **JD-5303028b** to obtain *N*-(4,4-difluorotetrahydro-1*H*-pyrrol-3-yl)-6-{[(2-methyl-1,3-thiazol-5-yl)methyl]oxy}-3,4-dihydro-2*H*-benzo*[*b][1,4]oxazine-4-carboxamide hydrochloride **(JD-5303029)** (51.2 mg, 124 µmol, yield: 79.6%) as a gray gum.

LC-MS [M+H]⁺ = 411.0.

**¹H NMR:** (400 MHz, DMSO-*d*₆) δ = 2.70 (s, 3 H), 3.47 (br s, 1 H), 3.57 - 3.90 (m, 5 H), 4.03 - 4.24 (m, 2 H), 4.70 - 4.90 (m, 1 H), 5.20 (s, 2 H), 6.65 (dd, *J* = 8.88, 2.88 Hz, 1 H), 6.79 (d, *J* = 8.88 Hz, 1 H), 7.36 (d, *J* = 2.75 Hz, 1 H), 7.64 (br d, *J* = 8.38 Hz, 1 H), 7.82 (s, 1 H), 10.18 - 10.55 (m, 2 H).

### Example 35: Preparation of N-(4,4-difluorotetrahydro-1H-pyrrol-3-yl)-6-{[(2-hydroxypyridin-3-yl)methyl]oxyl-3,4-dihydro-2H-benzo[b][1,4]oxazine-4-carboxamide trifluoroacetate (JD-5303030)

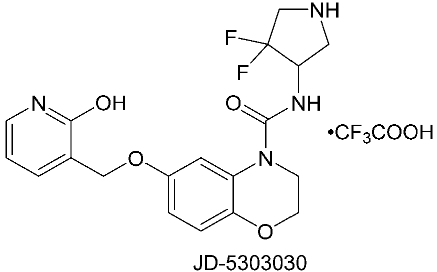

Following the method of Example 37, *N*-(4,4-difluorotetrahydro-1*H*-pyrrol-3-yl)-6-{[(2-hydroxypyridin-3-yl)methyl]oxy}-3,4-dihydro-2*H*-benzo[*b*][1,4]oxazine-4-carboxamide trifluoroacetate **(JD-5303030)** (4.80 mg, 11.3 µmol, yield: 8.45%, purity: 96.2%) was prepared as a white solid in Example 35.

LC-MS [M+H]⁺ = 407.0.

**¹H NMR:** (400 MHz, CHLOROFORM-d) δ = 3.64 (br s, 2 H), 3.71 - 3.79 (m, 2 H), 3.80 - 3.87 (m, 1 H), 3.94 - 4.06 (m, 1 H), 4.19 - 4.29 (m, 2 H), 4.86 (d, *J* = 12.01 Hz, 1 H), 5.00 - 5.09 (m, 1 H), 5.14 (d, *J* = 12.13 Hz, 1 H), 6.46 - 6.55 (m, 2 H), 6.63 (dd, *J* = 8.88, 2.75 Hz, 1 H), 6.79 (d, *J* = 8.88 Hz, 1 H), 7.43 (br d, *J* = 5.50 Hz, 1 H), 7.80 (dd, *J* = 6.94, 1.69 Hz, 1 H), 12.13 - 12.55 (m, 1 H).

### Example 36: Preparation of N-(4,4-difluorotetrahydro-1H-pyrrol-3-yl)-6-({[2-(trifluoromethyl)pyridin-3-yl]methyl}oxy)-3,4-dihydro-2H-benzo[b][1,4]oxazine-4-carboxamide hydrochloride (JD-5303031)

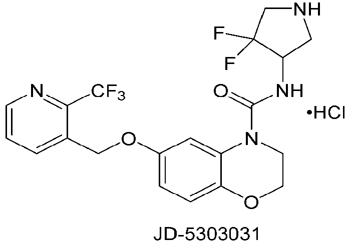

### Step 1: Preparation of 3-(chloromethyl)-2-(trifluoromethyl)pyridine (JD-5303031b)

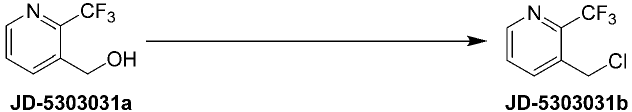

To a solution of [2-(trifluoromethyl)pyridin-3-yl]methanol **(JD-5303031a)** (1.00 g, 5.65 mmol, 1.00 eq) in THF (10.0 mL) was added SOCl₂ (1.34 g, 11.2 mmol, 820 µL, 2.50 eq), and the mixture was stirred at 25°C for 12 hours. TLC (petroleum ether: ethyl acetate = 1:1, Rf = 0.58) indicated complete consumption of the starting material and formation of a new spot. TLC showed that the reaction was complete. The reaction mixture was quenched with Na₂CO₃ to adjust the pH to 8. The reaction mixture was then slowly quenched with warm water, extracted twice with ethyl acetate, and concentrated under reduced pressure to obtain 3-(chloromethyl)-2-(trifluoromethyl)pyridine **(JD-5303031b)** (1.18 g, crude product) as a brown oil.

Step 2: Preparation of *N*-(4,4-difluorotetrahydro-1*H*-pyrrol-3-yl)-6-({[2-(trifluoromethyl)pyridin-3-yl]methylloxy)-3,4-dihydro-2*H*-benzo[*b*][1,4]oxazine-4-carboxamide hydrochloride **(JD-5303031)**

Following the synthetic method of Example 33, compound **JD-5303031b** was replaced with **JD-5303028a** to obtain *N*-(4,4-difluorotetrahydro-1*H*-pyrrol-3-yl)-6-({[2-(trifluoromethyl)pyridin-3-yl]methyl}oxy)-3,4-dihydro-2*H*-benzo[*b*][1,4]oxazine-4-carboxamide hydrochloride **(JD-5303031)** (4.80 mg, 11.3 µmol, yield: 8.45%, purity: 96.2%) as a white solid.

LC-MS [M+H]⁺ = 459.1.

¹H NMR: (400 MHz, DMSO-*d*₆) δ ppm 3.41 - 3.47 (m, 1 H) 3.61 - 3.92 (m, 5 H) 4.06 - 4.24 (m, 2 H) 4.73 - 4.89 (m, 1 H) 5.19 (s, 2 H) 6.67 (dd, *J* = 8.94, 2.94 Hz, 1 H) 6.82 (d, *J* = 8.88 Hz, 1 H) 7.38 (d, *J* = 2.88 Hz, 1 H) 7.58 (d, *J* = 8.38 Hz, 1 H) 7.78 (dd, *J* = 7.88, 4.63 Hz, 1 H) 8.21 (d, *J* = 7.75 Hz, 1 H) 8.72 (d, *J* = 4.00 Hz, 1 H) 10.04 (br s, 2 H).

### Example 37: Preparation of N-(4,4-difluorotetrahydro-1H-pyrrol-3-yl)-6-{[(2-methoxypyridin-3-yl)methyl]oxy}-3,4-dihydro-2H-benzo[b][1,4]oxazine-4-carboxamide trifluoroacetate (JD-5303032)

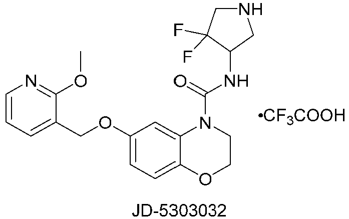

### Step 1: Preparation of 3-(chloromethyl)-2-methoxypyridine (JD-5303032b)

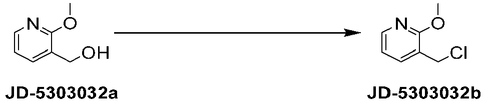

(2-Methoxypyridin-3-yl)methanol **(JD-5303032a)** (1.00 g, 7.19 mmol, 1.00 eq) was dissolved in THF (10.0 mL), then SOCl₂ (1.71 g, 14.3 mmol, 1.04 mL, 2.00 eq) was added thereto, and the mixture was stirred at 0°C for 1 hour. TLC (plate 1: petroleum ether: ethyl acetate = 2:1, Rf = 0.69) indicated complete consumption of the starting material and formation of a new spot. The reaction mixture was quenched with Na₂CO₃ to adjust the pH to 8. The reaction mixture was then slowly quenched with warm water, extracted twice with ethyl acetate, and concentrated under reduced pressure to obtain 3-(chloromethyl)-2-methoxypyridine **(JD-5303032b)** (600 mg, crude product) as a yellow oil.

### Step 2: Preparation of tert-butyl 3,3-difluoro-4-{[(6-{[(2-methoxypyridin-3-yl)methyl]oxy}-3,4-dihydro-2H-benzo[b][1,4]oxazin-4-yl)carbonyl]amino}pyrrolidine-1-carboxylate (JD-5303032-1)

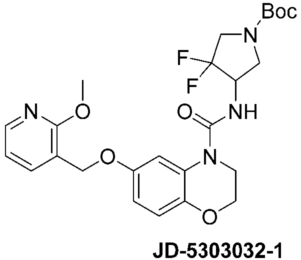

Following the method of Example 33, 3-(chloromethyl)-2-methoxypyridine (**JD-5303032b)** was replaced with **JD-5303028a** to obtain *tert*-butyl 3,3-difluoro-4-{[(6-{[(2-methoxypyridin-3-yl)methyl]oxyl-3,4-dihydro-2*H-*benzo[b][1,4]oxazin-4-yl)carbonyl]amino}pyrrolidine-1-carboxylate **(JD-5303032-1)** (70.0 mg, 134 µmol, yield: 45.7%, purity: 99.09%) as a white solid.

LC-MS [M+H]⁺ = 521.2.

### Step 3: Preparation of N-(4,4-difluorotetrahydro-1H-pyrrol-3-yl)-6-{[(2-methoxypyridin-3-yl)methyl]oxy}-3,4-dihydro-2H-benzo[b][1,4]oxazine-4-carboxamide trifluoroacetate (JD-5303032)

To TFE (2.00 mL) were added compound *tert*-butyl 3,3-difluoro-4-{[(6-{[(2-methoxypyridin-3-yl)methyl]oxyl-3,4-dihydro-2*H*-benzo[*b*][1,4]oxazin-4-yl)carbonyl]amino}pyrrolidine-1-carboxylate **(JD-5303032-1)** (70.0 mg, 134 µmol, 1.00 eq) and TMSCl (29.2 mg, 268 µmol, 34.1 µL, 2.00 eq), and the mixture was stirred at 25°C for 2 hours. The reaction mixture was concentrated under reduced pressure to obtain a residue. The residue was purified by preparative HPLC (column: Welch xextreme C18 150 * 25 mm * 5 µm; mobile phase: [water (TFA)-ACN]; gradient: 14% to 44% B over 14 minutes) to obtain *N*-(4,4-difluorotetrahydro-1*H*-pyrrol-3-yl)-6-{[(2-methoxypyridin-3-yl)methyl]oxy}-3,4-dihydro-2*H*-benzo[*b*][1,4]oxazine-4-carboxamide trifluoroacetate **(JD-5303032)** (24.0 mg, 56.5 µmol, yield: 42.0%, purity: 99.1%) as a white solid.

LC-MS [M+H]⁺ = 421.1.

**¹H NMR** (400 MHz, DMSO-*d*₆) δ = 3.62 - 3.71 (m, 2 H), 3.71 - 3.77 (m, 2 H), 3.78 - 3.85 (m, 2 H), 3.91 (s, 3 H), 4.09 - 4.21 (m, 2 H), 4.77 - 4.87 (m, 1 H), 4.94 (s, 2 H), 6.65 (dd, *J* = 8.94, 2.94 Hz, 1 H), 6.80 (d, *J =* 8.88 Hz, 1 H), 7.01 (dd, *J* = 7.19, 5.07 Hz, 1 H), 7.33 (d, *J* = 2.88 Hz, 1 H), 7.46 (d, *J* = 8.38 Hz, 1 H), 7.76 (dd, *J* = 7.25, 1.75 Hz, 1 H), 8.14 (dd, *J* = 4.94, 1.81 Hz, 1 H).

### Example 38: Preparation of N-(piperidin-3-yl)-6-({[2-(trifluoromethyl)phenyl]methyl}oxy)-3,4-dihydro-2H-benzo[b][1,4]oxazine-4-carboxamide hydrochloride (JD-5303033)

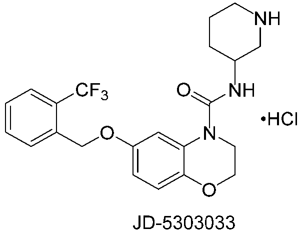

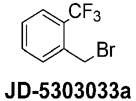

Following the synthetic method of Example 33, compound **JD-5303028a** was replaced with **JD-5303033a** to obtain *N*-(piperidin-3-yl)-6-({[2-(trifluoromethyl)phenyl]methyl}oxy)-3,4-dihydro-2*H*-benzo[*b*][1,4]oxazine-4-carboxamide hydrochloride **(JD-5303033)** (4.87 mg, 11.4 µmol, yield: 61.4%, purity: 98.14%) as an off-white solid.

LC-MS [M+H]⁺ = 436.3.

**¹H NMR:** (400 MHz, DMSO-*d*₆) δ = 1.50 - 1.73 (m, 2 H), 1.81 - 1.91 (m, 2 H), 2.77 (br d, *J* = 1.63 Hz, 2 H), 3.14 (br d, *J* = 12.13 Hz, 1 H), 3.26 (br d, *J* = 11.51 Hz, 1 H), 3.68 - 3.76 (m, 2 H), 3.86 - 3.96 (m, 1 H), 4.10 - 4.17 (m, 2 H), 5.13 (s, 2 H), 6.61 (dd, *J* = 8.88, 2.88 Hz, 1 H), 6.79 (d, *J* = 8.88 Hz, 1 H), 7.03 (d, *J* = 7.38 Hz, 1 H), 7.40 (d, *J* = 2.75 Hz, 1 H), 7.55 - 7.61 (m, 1 H), 7.69 - 7.81 (m, 3 H), 8.71 - 8.94 (m, 2 H).

### Example 39: Preparation of N-(piperidin-3-yl)-6-({[2-(trifluoromethyl)pyridin-3-yl]methyl}oxy)-3,4-dihydro-2H-benzo[b][1,4]oxazine-4-carboxamide hydrochloride (JD-5303034)

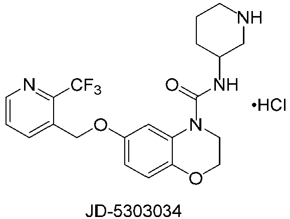

### Step 1: Preparation of 3-(chloromethyl)-2-(trifluoromethyl)pyridine (JD-5303034b)

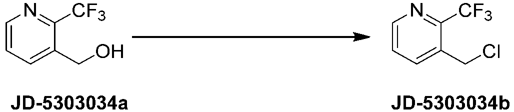

To a solution of [2-(trifluoromethyl)pyridin-3-yl]methanol **(JD-5303034a)** (90.0 mg, 508 µmol, 1.00 eq) in THF (2.00 mL) was added SOCl₂ (120 mg, 1.02 mmol, 73.8 µL, 2.00 eq) at 0°C, and the mixture was stirred at 25°C for 2 hours. TLC (plate 1: petroleum ether: ethyl acetate = 1:1, Rf = 0.58) indicated complete consumption of the starting material and formation of a new spot. The reaction mixture was quenched with Na₂CO₃ to adjust the pH to 8. The reaction mixture was then slowly quenched with warm water and extracted twice with ethyl acetate. The combined organic phases were concentrated under reduced pressure to obtain 3-(chloromethyl)-2-(trifluoromethyl)pyridine **(JD-5303034b)** (50.0 mg, crude product) as a yellow oil.

### Step 2: Preparation of N-(piperidin-3-yl)-6-({[2-(trifluoromethyl)pyridin-3-yl]methyl}oxy)-3,4-dihydro-2H-benzo[b][1,4]oxazine-4-carboxamide hydrochloride (JD-5303034)

Following the synthetic method of Example 33, compound **JD-5303028a** was replaced with **JD-5303034b** to obtain *N*-(piperidin-3-yl)-6-({[2-(trifluoromethyl)pyridin-3-yl]methyl}oxy)-3,4-dihydro-2*H*-benzo[*b*][1,4]oxazine-4-carboxamide hydrochloride (**JD-5303034)** (7.36 mg, 11.46 µmol, yield: 61.47%) as a white solid.

LC-MS [M+H]⁺ = 437.3.

**¹H NMR:** (400 MHz, DMSO-*d*₆) δ = 1.50 - 1.61 (m, 1 H), 1.63 - 1.74 (m, 1 H), 1.86 (br d, *J* = 11.01 Hz, 2 H), 2.78 (q, *J* = 9.30 Hz, 2 H), 3.12 (br d, *J* = 12.13 Hz, 1 H), 3.24 (br d, *J* = 9.88 Hz, 1 H), 3.70 - 3.78 (m, 2 H), 3.87 - 3.97 (m, 1 H), 4.13 (t, *J* = 4.31 Hz, 2 H), 5.18 (s, 2 H), 6.62 (dd, *J* = 8.88, 3.00 Hz, 1 H), 6.79 (d, *J* = 8.88 Hz, 1 H), 7.09 (d, *J* = 7.50 Hz, 1 H), 7.43 (d, *J* = 2.88 Hz, 1 H), 7.78 (dd, *J* = 7.88, 4.75 Hz, 1 H), 8.20 (d, *J* = 7.75 Hz, 1 H), 8.72 (d, *J* = 4.38 Hz, 1 H), 8.93 (br d, *J* = 9.26 Hz, 1 H), 9.14 (br d, *J* = 7.00 Hz, 1 H).

### Example 40: Preparation of 6-{[(2,6-difluorophenyl)methyl]oxy}-N-(piperidin-3-yl)-3,4-dihydro-2H-benzo[b][1,4]oxazine-4-carboxamide hydrochloride (JD-5303035)

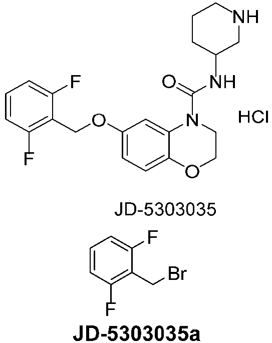

Following the synthetic method of Example 33, compound **JD-5303028a** was replaced with **JD-5303035a** to obtain 6-{[(2,6-difluorophenyl)methyl]oxy}-*N*-(piperidin-3-yl)-3,4-dihydro-2*H*-benzo*[*b][1,4]oxazine-4-carboxamide hydrochloride **(JD-5303035)** (50.0 mg, 123 µmol, yield: 62.4%, purity: 99.81%) as a white solid.

LC-MS [M+H]⁺ = 404.1.

**¹H NMR:** (400 MHz, DMSO-*d*₆)

δ = 1.52 - 1.72 (m, 2 H), 1.81 - 1.92 (m, 2 H), 2.79 (br t, *J* = 9.76 Hz, 2 H), 3.11 (br d, *J* = 12.01 Hz, 1 H), 3.25 (br d, *J* = 10.51 Hz, 1 H), 3.73 (dt, *J* = 9.22, 4.46 Hz, 2 H), 3.87 - 3.98 (m, 1 H), 4.13 (t, *J* = 4.32 Hz, 2 H), 4.99 (s, 2 H), 6.62 (dd, *J* = 8.88, 2.88 Hz, 1 H), 6.78 (d, *J* = 8.88 Hz, 1 H), 7.09 - 7.22 (m, 3 H), 7.39 (d, *J* = 2.88 Hz, 1 H), 7.46 - 7.57 (m, 1 H), 8.89 - 9.27 (m, 2 H).

### Example 41: Preparation of 6-1[(2,3-difluorophenyl)methyl]oxy}-N-(piperidin-3-yl)-3,4-dihydro-2H-benzo[b][1,4]oxazine-4-carboxamide hydrochloride (JD-5303036)

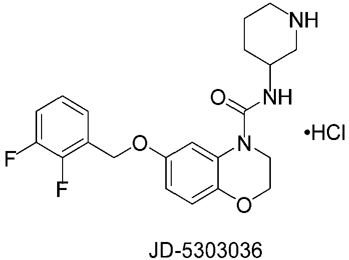

### Step 1: Preparation of 3-(chloromethyl)-1,2-difluorobenzene (JD-5303036b)

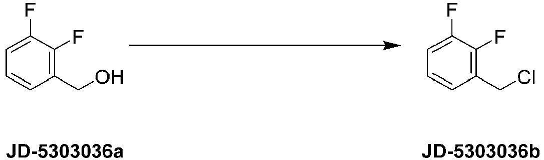

To a solution of (2,3-difluorophenyl)methanol **(JD-5303036a)** (1.50 g, 10.4 mmol, 1.17 mL, 1.00 eq) in THF (15 mL) was added SOCl₂ (2.48 g, 20.8 mmol, 1.51 mL, 2.00 eq) at 25°C, and the mixture was stirred at 25°C for 2 hours. TLC (dichloromethane: methanol = 8:1, Rf = 0.66) indicated complete consumption of the starting material and formation of a new spot. The reaction mixture was concentrated under reduced pressure to obtain 3-(chloromethyl)-1,2-difluorobenzene **(JD-5303036b)** (1.30 g, crude product) as a brown solid.

### Step 2: Preparation of 6-{[(2,3-difluorophenyl)methyl]oxy}-N-(piperidin-3-yl)-3,4-dihydro-2H-benzo[b][1,4]oxazine-4-carboxamide hydrochloride (JD-5303036)

Following the synthetic method of Example 33, compound **JD-5303028a** was replaced with **JD-5303036b** to obtain 6-{[(2,3-difluorophenyl)methyl]oxy}-*N*-(piperidin-3-yl)-3,4-dihydro-2*H*-benzo[*b*][1,4]oxazine-4-carboxamide hydrochloride **(JD-5303036)** (20.6 mg, 49.1 µmol, yield: 61.8%, purity: 96%) as a pink solid.

LC-MS [M+H]⁺ = 404.2.

¹H NMR (400 MHz, DMSO-*d*₆) δ ppm 1.52 - 1.71 (m, 2 H) 1.86 (br d, *J* = 6.75 Hz, 2 H) 2.77 (br t, *J* = 10.88 Hz, 2 H) 3.15 (br d, *J* = 12.51 Hz, 1 H) 3.25 - 3.29 (m, 1 H) 3.71 (br d, *J* = 2.75 Hz, 2 H) 3.85 - 3.97 (m, 1 H) 4.13 (t, *J* = 4.50 Hz, 2 H) 5.07 (s, 2 H) 6.63 (dd, *J* = 8.88, 3.00 Hz, 1 H) 6.78 (d, *J* = 8.88 Hz, 1 H) 7.04 (br d, *J* = 7.38 Hz, 1 H) 7.18 - 7.28 (m, 1 H) 7.30 - 7.38 (m, 1 H) 7.39 - 7.49 (m, 2 H) 8.69 - 8.95 (m, 2 H).

### Example 42: Preparation of 6-{[(2-fluoro-4-methylphenyl)methyl]oxy}-N-(piperidin-3-yl)-3,4-dihydro-2H-benzo[b][1,4]oxazine-4-carboxamide trifluoroacetate (JD-5303037)

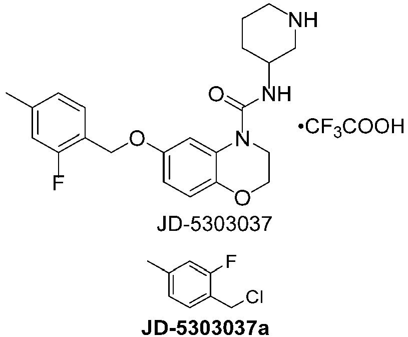

Following the synthetic method of Example 33, compound **JD-5303028a** was replaced with **JD-5303037a** to obtain 6-{[(2-fluoro-4-methylphenyl)methyl]oxy}-*N-*(piperidin-3-yl)-3,4-dihydro-2*H*-benzo[*b*][1,4]oxazine-4-carboxamide trifluoroacetate **(JD-5303037)** (0.9 mg, 2.15 µmol, yield: 5.38%, purity: 95.55%) as a white solid.

LC-MS [M+H]⁺ = 400.1.

**¹H NMR:** (400 MHz, DMSO-*d*₆) δ = 8.34 - 8.70 (m, 2 H), 7.26 - 7.47 (m, 2 H), 6.89 - 7.13 (m, 3 H), 6.77 (d, *J* = 8.88 Hz, 1 H), 6.61 (dd, *J* = 8.88, 2.88 Hz, 1 H), 4.86 - 5.07 (m, 2 H), 4.07 - 4.19 (m, 2 H), 3.83 - 3.95 (m, 1 H), 3.65 - 3.72 (m, 2 H), 2.65 - 2.84 (m, 4 H), 2.32 (s, 3 H), 1.79 - 1.93 (m, 2 H), 1.46 - 1.67 (m, 2 H).

### Example 43: Preparation of 6-{[(2-fluorophenyl)methyl]oxy}-N-(piperidin-3-yl)-3,4-dihydro-2H-benzo[b] [1,4]oxazine-4-carboxamide hydrochloride (JD-5303038)

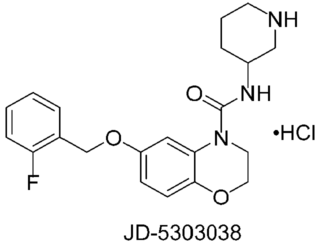

Following the synthetic method of Example 33, compound **JD-5303028b** was replaced with **JD-5303002a** to obtain 6-{[(2-fluorophenyl)methyl]oxy}-*N*-(piperidin-3-yl)-3,4-dihydro-2*H*-benzo[*b*][1,4]oxazine-4-carboxamide hydrochloride **(JD-5303038)** (26.0 mg, 61.6 µmol, yield: 99.7%, HCl) as a yellow gum.

LC-MS [M+H]⁺ = 386.2.

¹H NMR: (400 MHz, DMSO-*d*₆) δ ppm 1.47 - 1.73 (m, 2 H) 1.86 (br d, *J* = 10.13 Hz, 2 H) 2.71 - 2.87 (m, 2 H) 3.07 - 3.30 (m, 2 H) 3.67 - 3.78 (m, 2 H) 3.83 - 3.99 (m, 1 H) 4.07 - 4.17 (m, 2 H) 5.02 (s, 2 H) 6.62 (dd, *J* = 8.88, 2.88 Hz, 1 H) 6.77 (d, *J* = 8.88 Hz, 1 H) 7.08 (d, *J* = 7.50 Hz, 1 H) 7.20 - 7.29 (m, 2 H) 7.37 - 7.45 (m, 2 H) 7.49 - 7.56 (m, 1 H) 8.92 (br d, *J* = 9.25 Hz, 1 H) 9.13 (br d, *J* = 7.50 Hz, 1 H).

### Example 44: Preparation of N-(4,4-difluorotetrahydro-1H-pyrrol-3-yl)-6-{[1-(4-methyl-1,3-thiazol-5-yl)ethyl]oxy}-3,4-dihydro-2H-benzo[b][1,4]oxazine-4-carboxamide trifluoroacetate (JD-5303039)

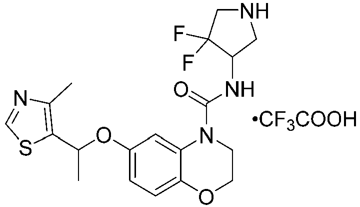

### Step 1: Preparation of 1-(4-methyl-1,3-thiazol-5-yl)ethan-1-ol (JD-5303039b)

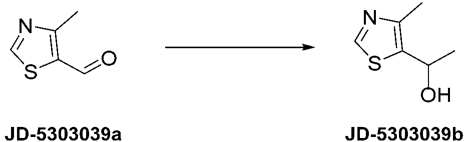

To a solution of 4-methyl-1,3-thiazole-5-carbaldehyde **(JD-5303039a)** (2.50 g, 19.6 mmol, 1.00 eq) in THF (25 mL) was added MeMgBr (3 M, 7.86 mL, 1.20 eq) at 0°C under a N₂ atmosphere, and the mixture was stirred at 0 to 25°C for 16 hours. TLC (petroleum ether: ethyl acetate = 1:1, Rf = 0.42) indicated complete consumption of the starting material and formation of a new spot. TLC showed that the reaction was complete. The reaction mixture was quenched with saturated NH₄Cl (20 mL) at 0°C, then diluted with H₂O (40 mL), and extracted with ethyl acetate (60 mL, 30 mL * 2). The combined organic phases were concentrated under reduced pressure to obtain 1-(4-methyl-1,3-thiazol-5-yl)ethan-1-ol **(JD-5303039b)** (2.65 g, crude product) as a yellow oil.

### Step 2: Preparation of 5-(1-bromoethyl)-4-methyl-1,3-thiazole (JD-5303039c)

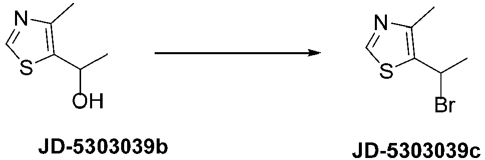

To a solution of 1-(4-methyl-1,3-thiazol-5-yl)ethan-1-ol **(JD-5303039b)** (1.00 g, 6.98 mmol, 1.00 eq) in THF (10 mL) was added PBr₃ (945 mg, 3.49 mmol, 0.50 eq), and the mixture was stirred at 0°C for 2 hours. The reaction mixture was concentrated under reduced pressure to obtain 5-(1-bromoethyl)-4-methyl-1,3-thiazole **(JD-5303039c)** (1.40 g, crude product) as an off-white solid. The reaction mixture was used in the next step without further purification.

### Step 3: Preparation of N-(4,4-difluorotetrahydro-1H-pyrrol-3-yl)-6-{[1-(4-methyl-1,3-thiazol-5-yl)ethyl]oxyl-3,4-dihydro-2H-benzo[b][1,4]oxazine-4-carboxamide trifluoroacetate (JD-5303039)

Following the synthetic method of Example 33, compound **JD-5303028a** was replaced with **JD-5303039c** to obtain *N*-(4,4-difluorotetrahydro-1*H*-pyrrol-3-yl)-6-{[1-(4-methyl-1,3-thiazol-5-yl)ethyl]oxy}-3,4-dihydro-2*H*-benzo[*b*][1,4]oxazine-4-carboxamide trifluoroacetate **(JD-5303039)** (0.12 g, 376 µmol, yield: 5.65%, purity: 91.0%) as a white solid.

LC-MS [M+H]⁺ = 425.1.

¹H NMR (400 MHz, DMSO-*d*₆) δ ppm 1.55 (d, *J* = 6.25 Hz, 3 H) 2.36 (d, *J* = 2.00 Hz, 3 H) 3.29 - 3.47 (m, 2 H) 3.68 - 3.77 (m, 4 H) 4.10 - 4.14 (m, 2 H) 4.69 - 4.90 (m, 1 H) 5.64 (q, *J* = 6.25 Hz, 1 H) 6.57 (dd, *J* = 8.88, 2.25 Hz, 1 H) 6.74 (d, *J* = 8.88 Hz, 1 H) 7.28 (dd, *J* = 4.57, 2.94 Hz, 1 H) 7.39 (br t, *J* = 8.13 Hz, 1 H) 8.89 (s, 1 H) 9.39 - 9.73 (m, 2 H).

### Example 45: Preparation of N-(4,4-difluorotetrahydro-1H-pyrrol-3-yl)-6-1[1-(2-fluorophenyl)ethyl]oxy}-3,4-dihydro-2H-benzo[b][1,4]oxazine-4-carboxamide hydrochloride (JD-5303040)

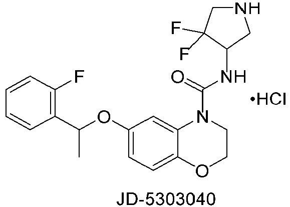

### Step 1: Preparation of 1-(2-fluorophenyl)ethan-1-ol (JD-5303040b)

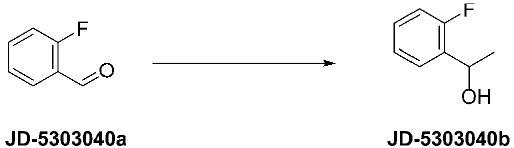

2-(Fluorophenyl)-1-carbaldehyde **(JD-5303040a)** (2.50 g, 20.1 mmol, 2.11 mL, 1.00 eq) was dissolved in THF (25 mL), then MeMgBr (3 M, 8.06 mL, 1.20 eq) was added thereto at 0°C under a N₂ atmosphere, and the mixture was stirred at 0 to 25°C for 16 hours. The reaction mixture was quenched with NH₄Cl (aq., 30 mL) at 0°C, then diluted with H₂O (50 mL), and extracted with ethyl acetate (60 mL, 30 mL * 2). The combined organic phases were washed with NaCl (aq., 20 mL) and concentrated under reduced pressure to obtain 1-(2-fluorophenyl)ethan-1-ol **(JD-5303040b)** (2.30 g, crude product) as a yellow oil.

### Step 2: Preparation of 1-(1-chloroethyl)-2-fluorobenzene (JD-5303040c)

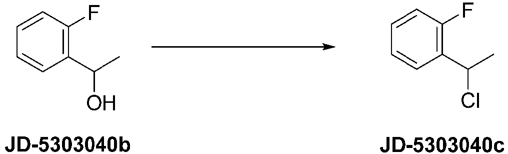

1-(2-Fluorophenyl)ethan-1-ol **(JD-5303040b)** (1.20 g, 8.56 mmol, 1.00 eq) was dissolved in THF (12 mL), then SOCl₂ (3.06 g, 25.6 mmol, 1.87 mL, 3.00 eq) was added thereto, and the reaction mixture was stirred at 25°C for 16 hours. The reaction mixture was quenched with Na₂CO₃ to adjust the pH to 8. The reaction mixture was then slowly quenched with warm water, extracted twice with ethyl acetate, and concentrated under reduced pressure to obtain 1-(1-chloroethyl)-2-fluorobenzene **(JD-5303040c)** (1.28 g, crude product) as a yellow oil.

### Step 3: Preparation of N-(4,4-difluorotetrahydro-1H-pyrrol-3-yl)-6-{[1-(2-fluorophenyl)ethyl]oxy}-3,4-dihydro-2H-benzo[b][1,4]oxazine-4-carboxamide (JD-5303040)

Following the synthetic method of Example 33, compound **JD-5303028a** was replaced with **JD-5303040c** to obtain *N*-(4,4-difluorotetrahydro-1*H*-pyrrol-3-yl)-6-{[1-(2-fluorophenyl)ethyl]oxyl-3,4-dihydro-2*H*-benzo[*b*][1,4]oxazine-4-carboxamide **(JD-5303040)** (7.33 mg, 16.70 µmol, yield: 29.03%, purity: 96%) as a yellow oil.

LC-MS [M+H]⁺ = 422.2.

¹H NMR (400 MHz, DMSO-*d*₆) δ ppm 1.55 (d, *J* = 6.25 Hz, 3 H) 2.36 (d, *J* = 2.00 Hz, 3 H) 3.29 - 3.47 (m, 2 H) 3.68 - 3.77 (m, 4 H) 4.10 - 4.14 (m, 2 H) 4.69 - 4.90 (m, 1 H) 5.64 (q, *J* = 6.25 Hz, 1 H) 6.57 (dd, *J* = 8.88, 2.25 Hz, 1 H) 6.74 (d, *J* = 8.88 Hz, 1 H) 7.28 (dd, *J* = 4.57, 2.94 Hz, 1 H) 7.39 (br t, *J* = 8.13 Hz, 1 H) 8.89 (s, 1 H) 9.39 - 9.73 (m, 2 H).

### Example 46: Preparation of N-(3-aminocyclohexyl)-6-{[(2-fluorophenyl)methyl]oxy}-3,4-dihydro-2H-benzo[b][1,4]oxazine-4-carboxamide hydrochloride (JD-5303041)

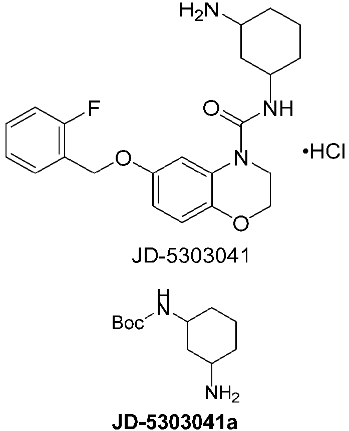

Following the synthetic method of Example 33, compound **JD-5303028b** was replaced with **JD-5303041a** to obtain *N*-(3-aminocyclohexyl)-6-{[(2-fluorophenyl)methyl]oxy}-3,4-dihydro-2*H*-benzo[*b*][1,4]oxazine-4-carboxamide hydrochloride **(JD-5303041)** (10.8 mg, 25.0 µmol, yield: 78.1%, purity: 98.48%) as a yellow solid.

LC-MS [M+H]⁺ = 400.1.

¹H NMR: (400 MHz, DMSO-*d*₆) δ ppm 1.41 - 1.77 (m, 8 H) 1.86 - 2.00 (m, 1 H) 3.64 - 3.75 (m, 2 H) 3.98 (br s, 1 H) 4.05 - 4.17 (m, 2 H) 5.02 (s, 2 H) 6.60 (dd, *J* = 8.88, 3.00 Hz, 1 H) 6.72 - 6.80 (m, 2 H) 7.18 - 7.28 (m, 2 H) 7.31 - 7.46 (m, 2 H) 7.53 (td, *J* = 7.63, 1.50 Hz, 1 H) 7.94 (br s, 3 H).

### Example 47: Preparation of 6-(benzyloxy)-N-(4,4-difluorotetrahydro-1H-pyrrol-3-yl)-3,4-dihydro-2H-benzo[b][1,4]oxazine-4-carboxamide (JD-5303042)

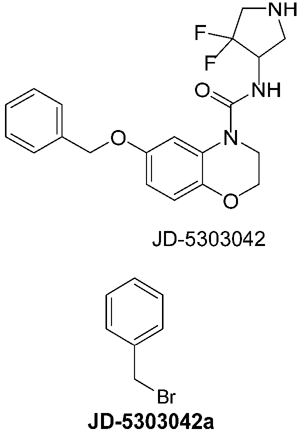

Following the synthetic method of Example 33, compound **JD-5303028a** was replaced with **JD-5303042a** to obtain 6-(benzyloxy)-*N*-(4,4-difluorotetrahydro-1*H*-pyrrol-3-yl)-3,4-dihydro-2*H*-benzo[*b*][1,4]oxazine-4-carboxamide **(JD-5303042)** (7.50 mg, 19.2 µmol, yield: 23.5%, purity: 97.13%) as a white solid.

LC-MS [M+H]⁺ = 390.0.

**¹H NMR:** (400 MHz, DMSO-*d*₆) δ = 2.78 (br dd, *J* = 11.57, 8.44 Hz, 1 H), 2.89 - 3.00 (m, 1 H), 3.15 - 3.26 (m, 2 H), 3.60 - 3.69 (m, 1 H), 3.73 - 3.82 (m, 1 H), 4.05 - 4.19 (m, 2 H), 4.34 (dt, *J* = 16.45, 8.04 Hz, 1 H), 4.98 (s, 2 H), 6.61 (dd, *J* = 8.94, 2.81 Hz, 1 H), 6.77 (d, *J* = 8.88 Hz, 1 H), 7.09 (br d, *J* = 8.50 Hz, 1 H), 7.29 - 7.47 (m, 6 H).

### Example 48: Preparation of N-(4,4-difluorotetrahydro-1H-pyrrol-3-yl)-6-({[2-(trifluoromethyl)pyridin-4-yl]methyl}oxy)-3,4-dihydro-2H-benzo[b][1,4]oxazine-4-carboxamide hydrochloride (JD-5303043)

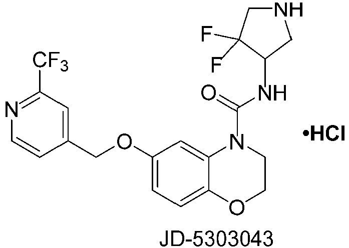

### Step 1: Preparation of [2-(trifluoromethyl)pyridin-4-yl]methanol (JD-5303043b)

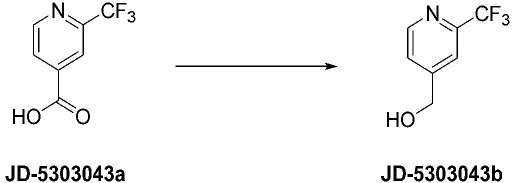

To a solution of 2-(trifluoromethyl)isonicotinic acid **(JD-5303043a)** (1.00 g, 5.23 mmol, 1.00 eq) in THF (10 mL) was dropwise added BH₃-THF (1 M, 10.4 mL, 2.00 eq) at 0°C, and the mixture was stirred at 75°C for 2 hours. The reaction mixture was quenched with MeOH (50 mL) at 0°C under a N₂ atmosphere, stirred at 25°C for 0.5 hours, and concentrated under reduced pressure to obtain [2-(trifluoromethyl)pyridin-4-yl]methanol **(JD-5303043b)** (1.00 g, crude product) as a brown oil.

LC-MS [M+H]⁺ = 178.0.

### Step 2: Preparation of 4-(chloromethyl)-2-(trifluoromethyl)pyridine (JD-5303043c)

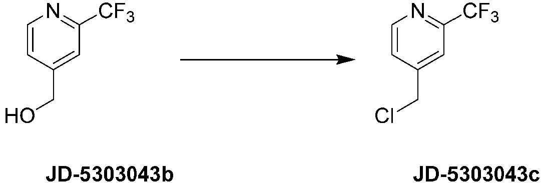

To a solution of [2-(trifluoromethyl)pyridin-4-yl]methanol **(JD-5303043b)** (1.00 g, 5.65 mmol, 1.00 eq) in THF (10 mL) was added SOCl₂ (1.68 g, 14.1 mmol, 1.03 mL, 2.50 eq), and the mixture was stirred at 25°C for 2 hours. The reaction mixture was quenched with Na₂CO₃ aqueous solution to adjust the pH to 8, then slowly quenched with warm water, extracted twice with ethyl acetate, and concentrated under reduced pressure to obtain 4-(chloromethyl)-2-(trifluoromethyl)pyridine **(JD-5303043c)** (0.6 g, crude product) as a brown solid.

### Step 3: Preparation of N-(4,4-difluorotetrahydro-1H-pyrrol-3-yl)-6-({[2-(trifluoromethyl)pyridin-4-yl]methyl}oxy)-3,4-dihydro-2H-benzo[b][1,4]oxazine-4-carboxamide hydrochloride (JD-5303043)

Following the synthetic method of Example 33, **JD-5303028a** was replaced with **JD-5303043c** to obtain *N*-(4,4-difluorotetrahydro-1*H*-pyrrol-3-yl)-6-({[2-(trifluoromethyl)pyridin-4-yl]methyl}oxy)-3,4-dihydro-2*H*-benzo[*b*][1,4]oxazine-4-carboxamide hydrochloride **(JD-5303043)** (0.024 g, 51.7 µmol, yield: 96.4%, purity: 99.0%) as a white solid.

LC-MS [M+H]⁺ = 459.2.

¹H NMR (400 MHz, DMSO-*d*₆) δ ppm 3.41 (br s, 2 H) 3.67 (br s, 4 H) 4.08 - 4.25 (m, 2 H) 4.75 - 4.91 (m, 1 H) 5.20 (s, 2 H) 6.69 (dd, *J* = 8.94, 2.94 Hz, 1 H) 6.83 (d, *J* = 8.88 Hz, 1 H) 7.33 - 7.52 (m, 2 H) 7.74 (d, *J* = 4.88 Hz, 1 H) 7.91 (s, 1 H) 8.77 (d, *J* = 5.00 Hz, 1 H) 9.24 - 10.08 (m, 2 H).

### Example 49: Preparation of N-(4,4-difluorotetrahydro-1H-pyrrol-3-yl)-6-{[(2-methyl-1,3-oxazol-5-yl)methyl]oxy}-3,4-dihydro-2H-benzo[b][1,4]oxazine-4-carboxamide hydrochloride (JD-5303044)

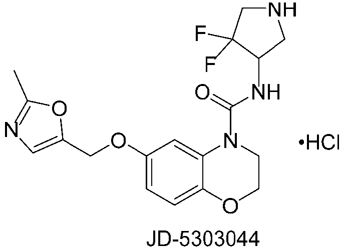

### Step 1: Preparation of (2-methyl-1,3-oxazol-5-yl)methanol (JD-5303044b)

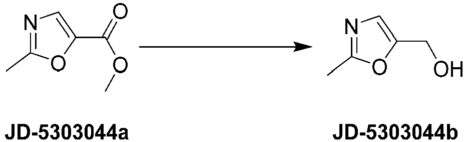

To a solution of methyl 2-methyl-1,3-oxazole-5-carboxylate **(JD-5303044a)** (1.00 g, 7.09 mmol, 1.00 eq) in THF (10.0 mL) was added LAH (2.50 M, 4.25 mL, 1.50 eq) at 0°C, and the mixture was stirred at 0°C for 2 hours. The reaction mixture was quenched with H₂O (0.40 mL) at -10°C, then diluted with 15% NaOH (0.40 mL) and H₂O (1.20 mL), stirred at 25°C for 15 minutes, and filtered and concentrated under reduced pressure to obtain (2-methyl-1,3-oxazol-5-yl)methanol **(JD-5303044b)** (1.00 g, crude product) as a yellow oil.

### Step 2: Preparation of 5-(chloromethyl)-2-methyl-1,3-oxazole (JD-5303044c)

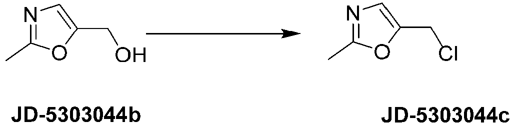

To a solution of (2-methyl-1,3-oxazol-5-yl)methanol **(JD-5303044b)** (1.00 g, 8.84 mmol, 1.00 eq) in THF (10.0 mL) was added SOCl₂ (2.10 g, 17.6 mmol, 1.28 mL, 2.00 eq), and the mixture was stirred at 0°C for 1 hour. The reaction mixture was quenched with Na₂CO₃ to adjust the pH to 8. The reaction mixture was then slowly quenched with warm water, extracted twice with ethyl acetate, and concentrated under reduced pressure to obtain 5-(chloromethyl)-2-methyl-1,3-oxazole **(JD-5303044c)** (1.00 g, crude product) as a yellow oil.

### Step 3: Preparation of N-(4,4-difluorotetrahydro-1H-pyrrol-3-yl)-6-{[(2-methyl-1,3-oxazol-5-yl)methyl]oxyl-3,4-dihydro-2H-benzo[b][1,4]oxazine-4-carboxamide hydrochloride (JD-5303044)

Following the synthetic method of Example 33, **JD-5303028a** was replaced with **JD-5303044c** to obtain *N*-(4,4-difluorotetrahydro-1*H*-pyrrol-3-yl)-6-{[(2-methyl-1,3-oxazol-5-yl)methyl]oxy}-3,4-dihydro-2*H*-benzo[*b*][1,4]oxazine-4-carboxamide hydrochloride **(JD-5303044)** (22.0 mg, 55.7 µmol, yield: 91.95%) as a yellow solid.

LC-MS [M+H]⁺ = 395.0.

**¹H NMR:** (400 MHz, DMSO-*d*₆) δ = 2.41 (s, 3 H), 3.46 (br s, 1 H), 3.62 - 3.84 (m, 5 H), 4.08 - 4.20 (m, 2 H), 4.81 (br d, *J* = 9.26 Hz, 1 H), 4.97 (s, 2 H), 6.65 (dd, *J* = 8.88, 2.88 Hz, 1 H), 6.79 (d, *J* = 8.88 Hz, 1 H), 7.13 (s, 1 H), 7.34 (d, *J* = 2.88 Hz, 1 H), 7.59 (d, *J* = 8.38 Hz, 1 H), 9.95 - 10.38 (m, 2 H).

### Example 50: Preparation of 3-[(4-{[(4,4-difluorotetrahydro-1H-pyrrol-3-yl)amino]carbonyl}-3,4-dihydro-2H-benzo[b][1,4]oxazin-6-yl)oxy]benzene-1-carboxamide hydrochloride (JD-5303045)

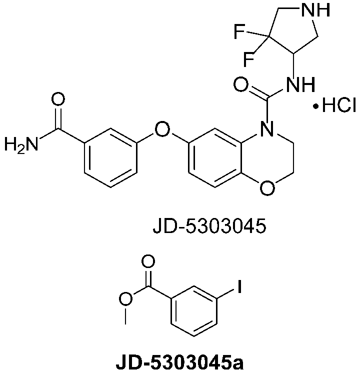

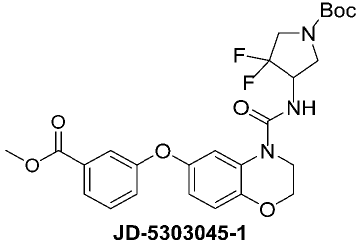

Following the synthetic method of Example 52, compound **JD-5303047a** was replaced with **JD-5303045a** to obtain methyl 3-[(4-{[(4,4-difluoro-1-{[(2-methylprop-2-yl)oxy]carbonyl}tetrahydro-1*H*-pyrrol-3-yl)amino]carbonyl}-3,4-dihydro-2*H-*benzo[b][1,4]oxazin-6-yl)oxy]benzoate **(JD-5303045-1)** (270 mg, 700 µmol, 88.0%) as a yellow gum.

LC-MS [385+Na]⁺ = 408.

### Step 1: Preparation of 3-[(4-{[(4,4-difluoro-1-{[(2-methylprop-2-yl)oxy]carbonyl}tetrahydro-1H-pyrrol-3-yl)amino]carbonyl}-3,4-dihydro-2H-benzo[b][1,4]oxazin-6-yl)oxy]benzoic acid (JD-5303045-2)

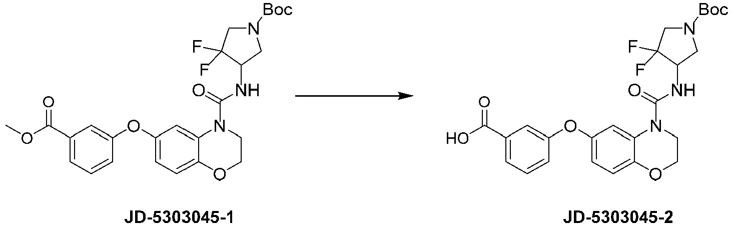

Methyl 3-[(4-{[(4,4-difluoro-1-{[(2-methylprop-2-yl)oxy]carbonyl}tetrahydro-1*H-*pyrrol-3-yl)amino]carbonyl}-3,4-dihydro-2*H*-benzo[*b*][1,4]oxazin-6-yl)oxy]benzoate (**JD-5303045-1)** (120 mg, 224 µmol, 1.00 eq) was dissolved in a mixture of methanol (1.00 mL) and water (1.00 mL), then NaOH (17.9 mg, 449 µmol, 2.00 eq) was added thereto, and the reaction mixture was stirred at 25°C for 16 hours. The reaction mixture was quenched with H₂O (2.00 mL) and extracted with MTBE (4.00 mL, 2.00 mL * 2). The aqueous phase was diluted with hydrochloric acid to adjust the pH to 4, then extracted with ethyl acetate (4.00 mL, 2.00 mL * 2), and concentrated under reduced pressure to obtain 3-[(4-{[(4,4-difluoro-1-{[(2-methylprop-2-yl)oxy]carbonyl}tetrahydro-1*H*-pyrrol-3-yl)amino]carbonyl}-3,4-dihydro-2*H-*benzo[*b*][1,4]oxazin-6-yl)oxy]benzoic acid **(JD-5303045-2)** (100 mg, 192 µmol, yield: 85.5%) as a white solid.

LC-MS [519-99]⁺ = 420.

### Step 2: Preparation of tert-butyl 4-[({6-[(3-carbamoylphenyl)oxy]-3,4-dihydro-2H-benzo[b][1,4]oxazin-4-yl}carbonyl)amino]-3,3-difluoropyrrolidine-1-carboxylate (JD-5303045-3)

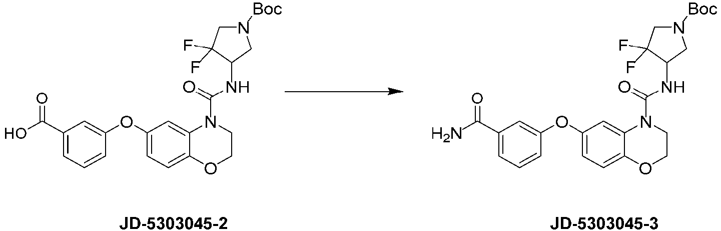

3-[(4-{[(4,4-Difluoro-1-{[(2-methylprop-2-yl)oxy]carbonyl}tetrahydro-1*H*-pyrrol-3-yl)amino]carbonyl}-3,4-dihydro-2*H*-benzo[*b*][1,4]oxazin-6-yl)oxy]benzoic acid **(JD-5303045-2)** (50.0 mg, 96.2 µmol, 1.00 eq) was dissolved in dioxane (2.00 mL), followed by the addition of NH₄HCO₃ (9.13 mg, 115 µmol, 1.26 eq), Boc₂O (27.3 mg, 125 µmol, 28.7 µL, 1.30 eq), and Py (4.72 mg, 59.6 µmol, 4.82 µL, 0.62 eq), and the reaction mixture was stirred at 25°C for 16 hours. The reaction mixture was quenched with H₂O (2.00 mL) and extracted with EtOAc (4.00 mL, 2.00 mL * 2). The combined organic phases were concentrated under reduced pressure to obtain a residue. The residue was purified by preparative HPLC (column: CD04-Welch uultimate C18 150 * 25 * 7 µm; mobile phase: [water (TFA)-ACN]; gradient: 36% to 66% B over 10 minutes) to obtain *tert*-butyl 4-[({6-[(3-carbamoylphenyl)oxy]-3,4-dihydro-2*H*-benzo[*b*][1,4]oxazin-4-yl}carbonyl)amino]-3,3-difluoropyrrolidine-1-carboxylate (**JD-5303045-3)** (20.0 mg, 38.5 µmol, yield: 40.08%) as a white solid.

LC-MS [518-99]⁺ = 419.

### Step 3: Preparation of 3-[(4-{[(4,4-difluorotetrahydro-1H-pyrrol-3-yl)amino]carbonyl}-3,4-dihydro-2H-benzo[b][1,4]oxazin-6-yl)oxy]benzene-1-carboxamide hydrochloride (JD-5303045)

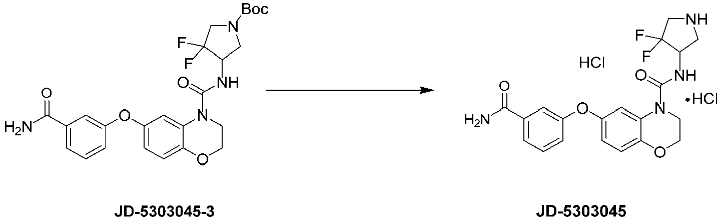

*tert-*Butyl 4-[({6-[(3-carbamoylphenyl)oxy]-3,4-dihydro-2*H*-benzo[*b*][1,4]oxazin-4-yl}carbonyl)amino]-3,3-difluoropyrrolidine-1-carboxylate **(JD-5303045-3)** (20.0 mg, 38.5 µmol, 1.00 eq) was dissolved in TFE (2.00 mL), then TMSCl (8.38 mg, 77.1 µmol, 9.79 µL, 2.00 eq) was added thereto, and the reaction mixture was stirred at 25°C for 2 hours. The reaction mixture was concentrated under reduced pressure to obtain 3-[(4-{[(4,4-difluorotetrahydro-1*H*-pyrrol-3-yl)amino]carbonyl}-3,4-dihydro-2*H*-benzo[*b*][1,4]oxazin-6-yl)oxy]benzene-1-carboxamide hydrochloride **(JD-5303045)** (4.68 mg, 14.3 µmol, yield: 37.1%, purity: 100.00%) as a white solid.

LC-MS [418+H]⁺ = 419.

**¹H NMR:** (400 MHz, DMSO-*d*₆) δ = 3.60 - 3.93 (m, 6 H), 4.14 - 4.29 (m, 2 H), 4.67 - 4.81 (m, 1 H), 6.70 (dd, *J* = 8.76, 2.75 Hz, 1 H), 6.92 (d, *J* = 8.76 Hz, 1 H), 7.11 (dd, *J* = 8.07, 2.31 Hz, 1 H), 7.35 - 7.44 (m, 3 H), 7.45 - 7.54 (m, 2 H), 7.58 (d, *J* = 7.75 Hz, 1 H), 7.98 (s, 1 H), 9.39 - 9.75 (m, 2 H).

### Example 51: Preparation of N-(piperidin-3-yl)-6-{[(4-methyl-1,3-thiazol-5-yl)methyl]amino}-3,4-dihydro-2H-benzo[b][1,4]oxazine-4-carboxamide (JD-5303046)

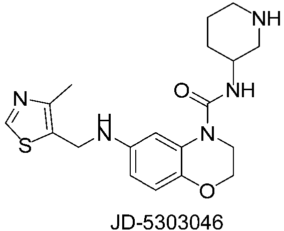

### Step 1: Preparation of tert-butyl 6-nitro-3,4-dihydro-2H-benzo[b][1,4]oxazine-4-carboxylate (JD-5303046-2)

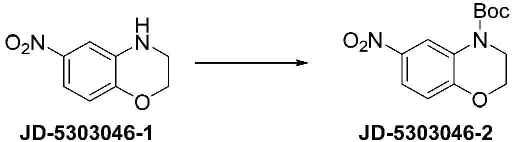

6-Nitro-3,4-dihydro-2*H*-benzo[*b*][1,4]oxazine **(JD-5303046-1)** (0.9 g, 5.0 mmol, 1.0 eq) was dissolved in dioxane (10.0 mL), followed by the addition of Boc₂O (4.4 g, 20.0 mmol, 4.0 eq), TEA (2.5 g, 25.0 mmol, 5.0 eq), and DMAP (61.0 mg, 0.5 mmol, 0.1 eq), and the mixture was reacted at 100°C for 12 hours. The reaction mixture was concentrated under reduced pressure to obtain a residue. The residue was purified by column chromatography (SiO₂, petroleum ether/ethyl acetate = 50/1 to 30/1) to obtain *tert*-butyl 6-nitro-3,4-dihydro-2*H*-benzo[*b*][1,4]oxazine-4-carboxylate **(JD-5303046-2)** (1.2 g, yield: 85.7%, purity: 94%) as a white solid.

LC-MS ([M+H₂O]⁺) = 298.1.

¹H NMR (300 MHz, CDCl₃) δ = 8.79 (s, 1H), 7.88 (dd, *J* = 9.0, 2.7 Hz, 1H), 6.94 (d, *J* = 9.0 Hz, 1H), 4.33 (t, *J* = 4.5 Hz, 2H), 3.91 (t, *J* = 4.8 Hz, 2H), 1.58 (s, 9H).

### Step 2: Preparation of tert-butyl 6-amino-3,4-dihydro-2H-benzo[b][1,4]oxazine-4-carboxylate (JD-5303046-3)

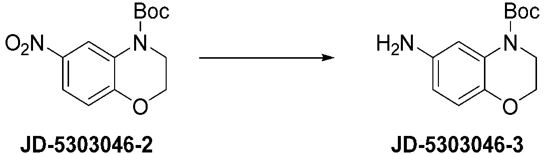

*tert-*Butyl *6*-nitro-3,4-dihydro-2*H*-benzo[*b*][1,4]oxazine-4-carboxylate (**JD-5303046-2)** (1.2 g, 4.3 mmol, 1.0 eq) was dissolved in EtOAc (10.0 mL), then Pd/C (120 mg) (10%) was added thereto, and the mixture was reacted under a H₂ atmosphere at 50°C for 12 hours. The reaction mixture was filtered, and the filtrate was concentrated to obtain *tert*-butyl 6-amino-3,4-dihydro-2*H*-benzo[*b*][1,4]oxazine-4-carboxylate **(JD-5303046-3)** (1.1 g, crude product) as a white solid.

LC-MS ([M+H]⁺) = 251.1.

¹H NMR (400 MHz, DMSO-*d*₆)

δ = 7.12 (s, 1H), 6.52 (d, *J* = 8.8*,* 1H), 6.21 (dd, *J* = 8.4 Hz, 2.4 Hz, 1H), 4.60 (s, 2H), 4.05 (t, *J* = 4.4 Hz, 2H), 3.70 (t, *J* = 4.8 Hz, 2H), 1.48 (s, 9H).

### Step 3: Preparation of tert-butyl 6-{[(4-methyl-1,3-thiazol-5-yl)methyl]amino}-3,4-dihydro-2H-benzo[b][1,4]oxazine-4-carboxylate (JD-5303046-4)

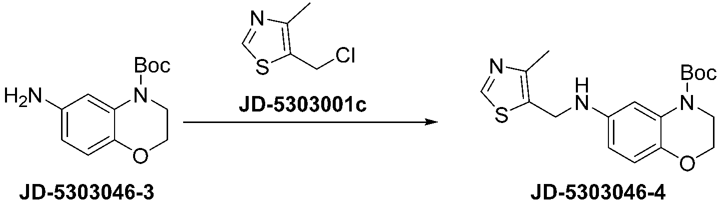

*tert*-Butyl 6-amino-3,4-dihydro-2*H*-benzo[*b*][1,4]oxazine-4-carboxylate **(JD-5303046-3)** (0.9 g, 3.6 mmol, 1.0 eq) was dissolved in THF (10.0 mL), followed by the addition of 5-(chloromethyl)-4-methyl-1,3-thiazole **(JD-5303001c)** (0.58 g, 4.0 mmol, 1.1 eq) and TEA (0.73 g, 7.2 mmol, 2.0 eq), and the mixture was reacted at 60°C for 3 hours. The reaction mixture was quenched with water (20 mL) at room temperature and extracted with ethyl acetate (40 mL, 20.0 mL * 2). The combined organic phases were washed with water (45 mL, 15.0 mL * 3) and brine (20 mL), dried over anhydrous sodium sulfate, and concentrated under reduced pressure to obtain a residue. The residue was purified by column chromatography (SiO₂, petroleum ether/ethyl acetate = 5/1 to 2/1) to obtain *tert*-butyl 6-{[(4-methyl-1,3-thiazol-5-yl)methyl]amino}-3,4-dihydro-2*H*-benzo[*b*][1,4]oxazine-4-carboxylate **(JD-5303046-4)** (0.75 g, two-step yield: 59.2%, purity: 92%) as a yellow solid.

LC-MS ([M+H]⁺) = 362.5.

¹H NMR (400 MHz, CDCl₃) δ = 8.61 (s, 1H), 7.36 (s, 1H), 6.73 (d, *J* = 8.4 Hz, 1H), 6.34 (dd, *J* = 8.8, 2.8 Hz, 1H), 4.40 (s, 2H), 4.17 (t, *J* = 4.4 Hz, 2H), 3.83 (t, *J* = 4.8 Hz, 2H), 2.46 (s, 3H), 1.53 (s, 9H).

### Step 4: Preparation of tert-butyl 6-[5-(4-methyl-1,3-thiazol-5-yl)-3-oxo-1-phenyl-4-aza-2-oxapent-4-yl]-3,4-dihydro-2H-benzo[b][1,4]oxazine-4-carboxylate (JD-5303046-5)

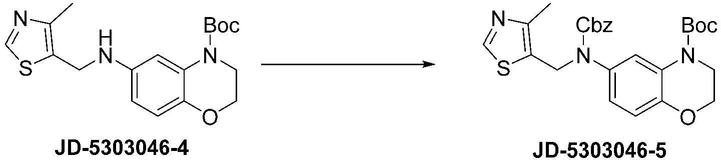

*tert*-Butyl 6-{[(4-methyl-1,3-thiazol-5-yl)methyl]amino}-3,4-dihydro-2*H-*benzo[*b*][1,4]oxazine-4-carboxylate **(JD-5303046-4)** (150 mg, 0.42 mmol, 1.0 eq) was dissolved in THF (3.0 mL), and the mixture was cooled to 0°C. NaH (60%) (33 mg, 0.83 mmol, 2.0 eq) was added thereto, and the mixture was reacted at 0°C for 0.5 hours. CbzCl (127 mg, 0.75 mmol, 1.8 eq) was then added thereto, and the mixture was warmed to room temperature and stirred for 11.5 hours. The reaction mixture was quenched with saturated ammonium chloride aqueous solution (10 mL) at room temperature and extracted with ethyl acetate (30 mL, 10.0 mL * 3). The combined organic phases were washed with brine (10 mL), dried over anhydrous sodium sulfate, and concentrated under reduced pressure to obtain a residue. The residue was purified by column chromatography (SiO₂, petroleum ether/ethyl acetate = 5/1 to 3/1) to obtain *tert*-butyl 6-[5-(4-methyl-1,3-thiazol-5-yl)-3-oxo-1-phenyl-4-aza-2-oxapent-4-yl]-3,4-dihydro-2H-benzo[b][1,4]oxazine-4-carboxylate **(JD-5303046-5)** (180 mg, yield: 87.5%, purity: 92%) as a yellow solid.

LC-MS ([M+H]⁺) = 496.5.

¹H NMR (300 MHz, CDCl₃) δ = 8.60 (s, 1H), 7.82 - 7.62 (m, 1H), 7.29 (s, 5H), 6.77 (d, *J* = 8.7 Hz, 1H), 6.58 (s, 1H), 5.16 (s, 2H), 4.92 (s, 2H), 4.24 (t, *J* = 4.2 Hz, 2H), 3.84 (t, *J* = 4.5 Hz, 2H), 2.17 (s, 3H), 1.49 (s, 9H).

### Step 5: Preparation of benzyl (3,4-dihydro-2H-benzo[b][1,4]oxazin-6-yl)[(4-methyl-1,3-thiazol-5-yl)methyl]carbamate (JD-5303046-6)

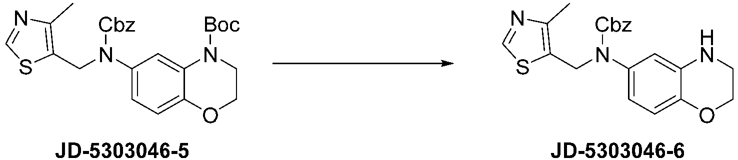

*tert-Butyl* 6-[5-(4-methyl-1,3-thiazol-5-yl)-3-oxo-1-phenyl-4-aza-2-oxapent-4-yl]-3,4-dihydro-2*H*-benzo[*b*][1,4]oxazine-4-carboxylate **(JD-5303046-5)** (180 mg, 0.36 mmol, 1.0 eq) was dissolved in DCM (3 mL), then dioxane/HCl (0.91 mL, 3.63 mmol, 10.0 eq) (4 N) was added thereto, and the mixture was reacted at room temperature for 12 hours. The mixture was concentrated under reduced pressure to obtain a residue. The residue was quenched with sodium hydroxide solution (15%) (3 mL) and extracted with DCM (20 mL, 10 mL * 2). The combined organic phases were washed with brine (10 mL), dried over anhydrous sodium sulfate, and concentrated under reduced pressure to obtain benzyl (3,4-dihydro-2*H*-benzo[*b*][1,4]oxazin-6-yl)[(4-methyl-1,3-thiazol-5-yl)methyl]carbamate **(JD-5303046-6)** (125 mg, crude product) as a yellow solid.

LC-MS ([M+H]⁺) = 396.6.

### Step 6: Preparation of benzyl (4-{[(1-{[(2-methylprop-2-yl)oxy]carbonyl}piperidin-3-yl)amino]carbonyl}-3,4-dihydro-2H-benzo[b][1,4]oxazin-6-yl)[(4-methyl-1,3-thiazol-5-yl)methyl]carbamate (JD-5303046-7)

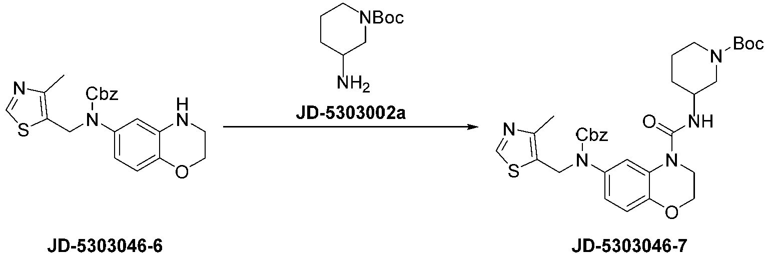

Benzyl (3,4-dihydro-2*H*-benzo[*b*][1,4]oxazin-6-yl)[(4-methyl-1,3-thiazol-5-yl)methyl]carbamate **(JD-5303046-6)** (125 mg, 0.32 mmol, 1.0 eq) and TEA (96 mg, 0.95 mmol, 3.0 eq) were dissolved in DCM (1.0 mL), then (COCl₂)₃ (47 mg, 0.16 mmol, 0.5 eq) was added thereto, and the mixture was reacted at room temperature for 0.5 hours. A solution of *tert*-butyl 3-aminopiperidine-1-carboxylate **(JD-5303002a)** (76 mg, 0.38 mmol, 1.2 eq) in DCM (0.5 mL) was then added thereto, and the mixture was stirred at room temperature for 12 hours. The reaction system was quenched with water (5 mL) and extracted with DCM (20 mL, 10 mL * 2). The combined organic phases were washed with brine (10 mL), dried over anhydrous sodium sulfate, and concentrated under reduced pressure to obtain benzyl (4-{[(1-{[(2-methylprop-2-yl)oxy]carbonyl}piperidin-3-yl)amino]carbonyl}-3,4-dihydro-2*H-*benzo[*b*][1,4]oxazin-6-yl)[(4-methyl-1,3-thiazol-5-yl)methyl]carbamate **(JD-5303046-7)** (300 mg, crude product) as a yellow solid.

LC-MS ([M+H]⁺) = 622.4.

### Step 7: Preparation of N-(piperidin-3-yl)-6-{[(4-methyl-1,3-thiazol-5-yl)methyl]amino}-3,4-dihydro-2H-benzo[b][1,4]oxazine-4-carboxamide formate (JD-5303046)

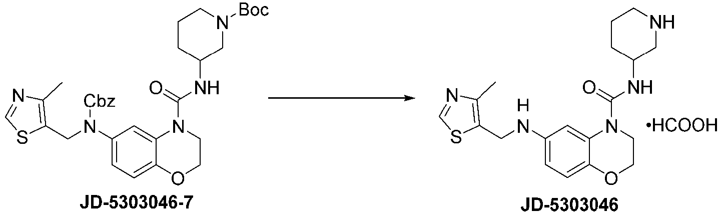

Benzyl (4-{[(1-{[(2-methylprop-2-yl)oxy]carbonyl}piperidin-3-yl)amino]carbonyl}-3,4-dihydro-2*H*-benzo[*b*][1,4]oxazin-6-yl)[(4-methyl-1,3-thiazol-5-yl)methyl]carbamate **(JD-5303046-7)** (300 mg, 0.48 mmol, 1.0 eq) was dissolved in DCM (3 mL), then TMSI (483 mg, 2.42 mmol, 5.0 eq) was added thereto, and the mixture was reacted at room temperature for 12 hours. The reaction mixture was quenched with water (3 mL) and extracted with DCM (20 mL, 10 mL * 2) to remove impurities. The aqueous phase was added with sodium hydroxide solution (3 mL, 15%) to adjust the pH to 9, and extracted with DCM (20 mL, 10 mL * 2). The combined organic phases were washed with brine (10 mL), dried over anhydrous sodium sulfate, and concentrated under reduced pressure to obtain a residue. The residue was purified by prep-HPLC (column: WelchUIC18 150 * 25 mm * 5 mm; mobile phase: [water (0.1% FA)-ACN]; B%: 15% to 25%, 6 minutes) to obtain *N*-(piperidin-3-yl)-6- {[(4-methyl-1,3-thiazol-5-yl)methyl]amino}-3,4-dihydro-2*H*-benzo[*b*][1,4]oxazine-4-carboxamide formate **(JD-5303046)** (29.9 mg, three-step yield: 18.3%, purity: 97.9%) as a yellow solid.

LC-MS ([M-1.34FA+H]⁺) = 388.3.

¹H NMR (300 MHz, DMSO-*d*₆) δ = 8.81 (s, 1H), 8.31 (brs, 1H), 6.87 (d, *J* = 2.4 Hz, 1H), 6.75 (d, *J* = 7.5 Hz, 1H), 6.61 (d, *J* = 8.7 Hz, 1H), 6.27 (dd, *J* = 8.7, 2.4 Hz, 1H), 5.82 (s, 1H), 4.29 (s, 2H), 4.05 (t, *J* = 4.2 Hz, 2H), 3.86 - 3.73 (m, 1H), 3.69 - 3.60 (m, 2H), 3.20 - 2.93 (m, 2H), 2.76 - 2.57 (m, 2H), 2.38 (s, 3H), 1.88 - 1.70 (m, 2H), 1.65 - 1.39 (m, 2H).

### Example 52: Preparation of N-(4,4-difluorotetrahydro-1H-pyrrol-3-yl)-6-[(3-methoxyphenyl)oxy]-3,4-dihydro-2H-benzo[b][1,4]oxazine-4-carboxamide trifluoroacetate (JD-5303047)

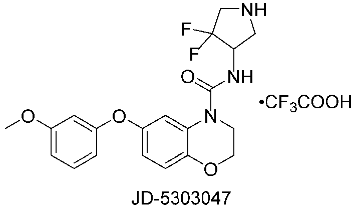

### Step 1: Preparation of tert-butyl 6-bromo-3,4-dihydro-2H-benzo[b][1,4]oxazine-4-carboxylate (JD-5303047-1)

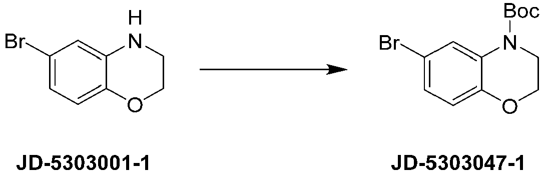

6-Bromo-3,4-dihydro-2*H*-benzo[*b*][1,4]oxazine **(JD-5303001-1)** (13.0 g, 60.7 mmol, 1.00 eq) was dissolved in THF (130 mL), followed by the addition of Boc₂O (26.5 g, 121 mmol, 27.9 mL, 2.00 eq), DMAP (1.48 g, 12.1 mmol, 0.20 eq), and TEA (18.4 g, 182 mmol, 25.4 mL, 3.00 eq), and the mixture was stirred at 25°C for 4 hours. The reaction mixture was concentrated under reduced pressure to obtain a residue. The residue was purified by column chromatography (SiO₂, petroleum ether/ethyl acetate = 100/1 to 0/1) to obtain *tert*-butyl 6-bromo-3,4-dihydro-2H-benzo[b][1,4]oxazine-4-carboxylate **(JD-5303047-1)** (15.0 g, 47.7 mmol, yield: 78.6%) as a white solid.

LC-MS [313-55]⁺ = 258.

### Step 2: Preparation of tert-butyl 6-[(3-methoxyphenyl)oxy]-3,4-dihydro-2H-benzo[b][1,4]oxazine-4-carboxylate (JD-5303047-2)

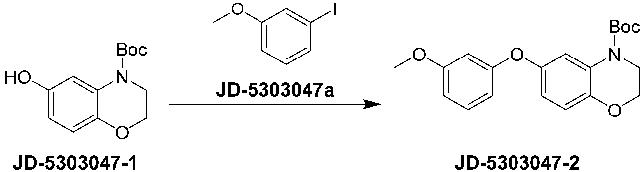

To a solution of *tert*-butyl 6-hydroxy-3,4-dihydro-2*H*-benzo[*b*][1,4]oxazine-4-carboxylate **(JD-5303047-1)** (250 mg, 994 µmol, 1.00 eq) and 3-iodo-1-methoxybenzene **(JD-5303047a)** (465 mg, 1.99 mmol, 236 µL, 2.00 eq) in dioxane (2.50 mL) were added Cs₂CO₃ (972 mg, 2.98 mmol, 3.00 eq) and t-BuXPhos Pd G3 (79.0 mg, 99.4 µmol, 0.10 eq). The mixture was stirred at 80°C for 16 hours. The reaction mixture was quenched with H₂O (10.0 mL) and extracted with EtOAc (20.0 mL). The organic phase was concentrated under reduced pressure to obtain a crude product. The crude product was purified by column chromatography (SiO₂, petroleum ether/ethyl acetate = 5/1) to obtain *tert*-butyl 6-[(3-methoxyphenyl)oxy]-3,4-dihydro-2*H*-benzo[*b*][1,4]oxazine-4-carboxylate **(JD-5303047-2)** (60.0 mg, 167 µmol, yield: 16.8%) as a yellow oil.

LC-MS [357-55]⁺ = 302.

### Step 3: Preparation of 6-[(3-methoxyphenyl)oxy]-3,4-dihydro-2H-benzo[b][1,4]oxazine (JD-5303047-3)

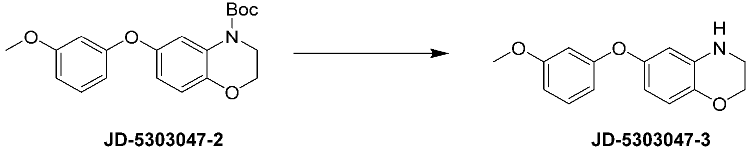

*tert*-Butyl 6-[(3-methoxyphenyl)oxy]-3,4-dihydro-2*H*-benzo[*b*][1,4]oxazine-4-carboxylate **(JD-5303047-2)** (60.0 mg, 167 µmol, 1.00 eq) was dissolved in TFE (1.00 mL), followed by the addition of TMSCl (36.4 mg, 335 µmol, 42.6 µL, 2.00 eq). The mixture was stirred at 25°C for 2 hours. The reaction mixture was filtered and concentrated under reduced pressure to obtain 6-[(3-methoxyphenyl)oxy]-3,4-dihydro-2*H*-benzo[*b*][1,4]oxazine **(JD-5303047-3)** (40.0 mg, crude product) as a colorless oil.

LC-MS [257+H]⁺ = 258.

### Step 4: Preparation of tert-butyl 3,3-difluoro-4-[({6-[(3-methoxyphenyl)oxy]-3,4-dihydro-2H-benzo[b][1,4]oxazin-4-yl}carbonyl)amino]pyrrolidine-1-carboxylate (JD-5303047-4)

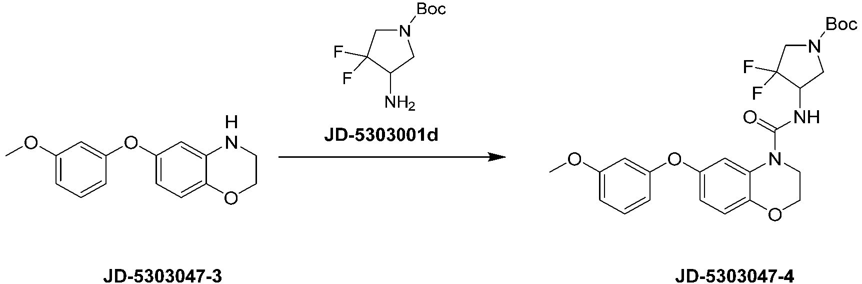

To a solution of 6-[(3-methoxyphenyl)oxy]-3,4-dihydro-2*H*-benzo[*b*][1,4]oxazine **(JD-5303047-3)** (40.0 mg, 155 µmol, 1.00 eq) and *tert*-butyl 4-amino-3,3-difluoropyrrolidine-1-carboxylate **(JD-5303001d)** (69.1 mg, 310 µmol, 2.00 eq) in DMF (1.00 mL) was added CDI (50.4 mg, 310 µmol, 2.00 eq), and the mixture was stirred at 65°C for 12 hours. LC-MS indicated complete consumption of the starting material and formation of a major peak with desired mass. The reaction mixture was quenched with H₂O (10.0 mL) and extracted with EtOAc (20.0 mL). The organic phase was concentrated under reduced pressure to obtain a crude product. The crude product was subjected to HPLC (column: CD01-Phenomenex Luna C18 150 * 25 * 10 µm; mobile phase: [water (FA)-ACN]; gradient: 54% to 84% B over 11 minutes) to obtain *tert*-butyl 3,3-difluoro-4-[({6-[(3-methoxyphenyl)oxy]-3,4-dihydro-2*H-*benzo[*b*][1,4]oxazin-4-yl}carbonyl)amino]pyrrolidine-1-carboxylate **(JD-5303047-4)** (20.0 mg, 39.5 µmol, yield: 25.4%) as a white solid.

LC-MS [505+Na]⁺ = 528.

### Step 5: Preparation of N-(4,4-difluorotetrahydro-1H-pyrrol-3-yl)-6-[(3-methoxyphenyl)oxy]-3,4-dihydro-2H-benzo[b][1,4]oxazine-4-carboxamide trifluoroacetate (JD-5303047)

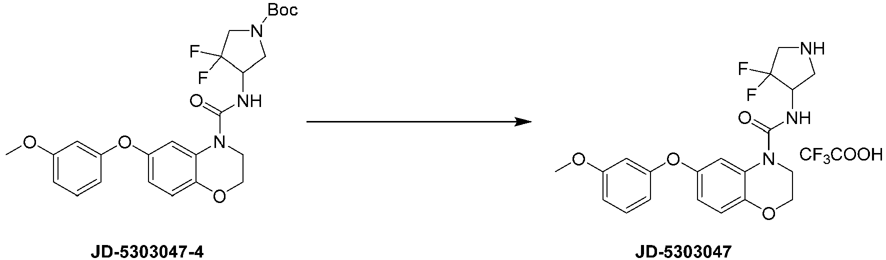

*tert-*Butyl 3,3-difluoro-4-[({6-[(3-methoxyphenyl)oxy]-3,4-dihydro-2*H-*benzo[*b*][1,4]oxazin-4-yl}carbonyl)amino]pyrrolidine-1-carboxylate **(JD-5303047-4)** (20.0 mg, 39.5 µmol, 1.00 eq) was dissolved in TFE (1.00 mL), followed by the addition of TMSCl (8.60 mg, 79.1 µmol, 10.0 µL, 2.00 eq). The mixture was stirred at 25°C for 2 hours. The crude product was subjected to HPLC (column: CD01-Phenomenex Luna C18 150 * 25 * 10 µm; mobile phase: [water (TFA)-ACN]; gradient: 21% to 51% B over 10 minutes) to obtain *N-*(4,4-difluorotetrahydro-1*H*-pyrrol-3-yl)-6-[(3-methoxyphenyl)oxy]-3,4-dihydro-2*H-*benzo[*b*][1,4]oxazine-4-carboxamide trifluoroacetate **(JD-5303047)** (14.1 mg, 34.9 µmol, yield: 88.2%, purity: 99.89%) as a white solid.

LC-MS [405+H]⁺ = 406.

¹H NMR: (400 MHz, DMSO-*d*₆) δ = 3.29 - 3.36 (m, 2 H), 3.65 - 3.71 (m, 2 H), 3.72 (s, 3 H), 3.77 - 3.86 (m, 2 H), 4.12 - 4.30 (m, 2 H), 4.65 - 4.85 (m, 1 H), 6.44 - 6.54 (m, 2 H) 6.60 - 6.73 (m, 2 H), 6.90 (d, *J* = 8.76 Hz, 1 H), 7.23 (t, *J* = 8.13 Hz, 1 H), 7.37 - 7.47 (m, 2 H), 9.33 (br s, 2 H).

### Example 53: Preparation of N-(4,4-difluorotetrahydro-1H-pyrrol-3-yl)-6-[(3-fluorophenyl)oxy]-3,4-dihydro-2H-benzo[b][1,4]oxazine-4-carboxamide (JD-5303048)

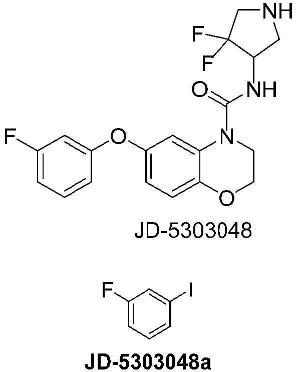

Following the synthetic method of Example 52, compound **JD-5303047a** was replaced with **JD-5303048a** to obtain *N*-(4,4-difluorotetrahydro-1*H*-pyrrol-3-yl)-6-[(3-fluorophenyl)oxy]-3,4-dihydro-2*H*-benzo[*b*][1,4]oxazine-4-carboxamide hydrochloride **(JD-5303048)** (3.84 mg, 9.24 µmol, yield: 47.7%) as an off-white solid.

LCMS [393+H]⁺ = 394.

¹H NMR (400 MHz, DMSO-*d*₆) δ = ppm 3.36 - 3.43 (m, 1 H) 3.59 - 3.91 (m, 5 H) 4.14 - 4.29 (m, 2 H) 4.66 - 4.84 (m, 1 H) 6.72 (dd, *J* = 8.82, 2.81 Hz, 1 H) 6.76 - 6.83 (m, 2 H) 6.86 - 6.96 (m, 2 H) 7.31 - 7.41 (m, 1 H) 7.48 (d, *J* = 2.88 Hz, 1 H) 7.60 (br d, *J* = 8.25 Hz, 1 H) 9.87 (br s, 2 H).

### Example 54: Preparation of N-(piperidin-3-yl)-6-{[1-(4-methyl-1,3-thiazol-5-yl)ethyl]oxy}-3,4-dihydro-2H-benzo[b][1,4]oxazine-4-carboxamide (JD-5303055)

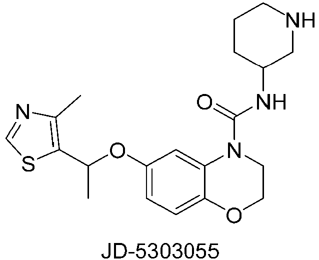

Following the synthetic method of Example 33, **JD-5303028a** was replaced with **JD-5303039c** and **JD-5303028b** was replaced with **JD-5303002a** to obtain N-(piperidin-3-yl)-6-{[1-(4-methyl-1,3-thiazol-5-yl)ethyl]oxy}-3,4-dihydro-2*H*-benzo[*b*][1,4]oxazine-4-carboxamide **(JD-5303055)** (25.2 mg, 56.4 µmol, yield: 94.5%, purity: 98%, hydrochloride) as a white solid.

LC-MS [M+H]⁺ = 403.1.

¹H NMR (400 MHz, METHANOL-*d*₄) δ 1.59 - 1.65 (m, 1H), 1.66 (d, *J* = 6.38 Hz, 3H), 1.76 - 1.91 (m, 1H), 1.99 - 2.12 (m, 2H), 2.41 - 2.47 (m, 3H), 2.79 - 3.01 (m, 2H), 3.34 - 3.38 (m, 1H), 3.49 (dt, *J* = 12.04, 3.80 Hz, 1H), 3.67 - 3.81 (m, 2H), 3.99 (tt, *J* = 10.69, 3.94 Hz, 1H), 4.11 - 4.22 (m, 2H), 5.65 (q, *J* = 6.30 Hz, 1H), 6.60 (ddd, *J* = 8.94, 2.81, 0.88 Hz, 1H), 6.76 (d, *J* = 9.01 Hz, 1H), 7.17 - 7.23 (m, 1H), 8.96 (s, 1H).

### Example 55: Preparation of N-(4,4-difluorotetrahydro-1H-pyrrol-3-yl)-2-methyl-6-{[(4-methyl-1,3-thiazol-5-yl)methyl]oxy}-3,4-dihydro-2H-benzo[b][1,4]oxazine-4-carboxamide hydrochloride (JD-5303049)

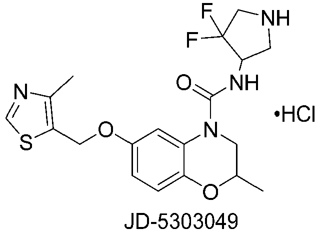

### Step 1: Preparation of 2-methyl-6-(4,4,5,5-tetramethyl-1,3,2-dioxaborolan-2-yl)-3,4-dihydro-2H-benzo[b][1,4]oxazin-3-one (JD-5303049-2)

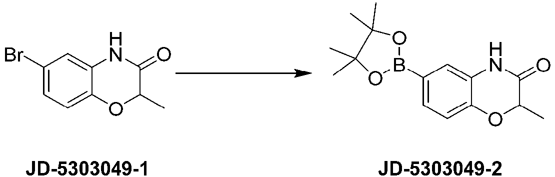

To a solution of 6-bromo-2-methyl-3,4-dihydro-2*H*-benzo[*b*][1,4]oxazine **(JD-5303049-1)** (2.50 g, 10.3 mmol, 1.00 eq) in dioxane (25.0 mL) were added BPD (5.25 g, 20.6 mmol, 2.00 eq), KOAc (2.53 g, 25.8 mmol, 2.50 eq), and PdCl₂(dppf) (377 mg, 516 µmol, 0.05 eq), and the mixture was stirred under a N₂ atmosphere at 100°C for 2 hours. The reaction mixture was quenched with H₂O (20.0 mL) and extracted with EtOAc (40.0 mL, 20.0 mL * 2). The combined organic phases were washed with NaCl (aq., 2.00 mL) and concentrated under reduced pressure to obtain 2-methyl-6-(4,4,5,5-tetramethyl-1,3,2-dioxaborolan-2-yl)-3,4-dihydro-2*H*-benzo[*b*][1,4]oxazin-3-one **(JD-5303049-2)** (3.00 g, crude product) as a black solid.

LC-MS [M] = 289.8.

### Step 2: Preparation of 6-hydroxy-2-methyl-3,4-dihydro-2H-benzo[b][1,4]oxazin-3-one (JD-5303049-3)

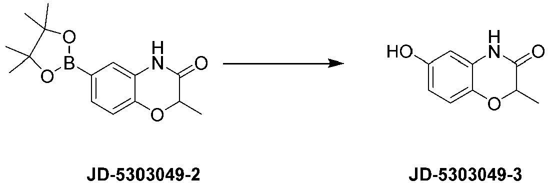

2-Methyl-6-(4,4,5,5-tetramethyl-1,3,2-dioxaborolan-2-yl)-3,4-dihydro-2*H-*benzo[*b*][1,4]oxazin-3-one **(JD-5303049-2)** (1.50 g, 5.19 mmol, 1.00 eq) was dissolved in THF (15.0 mL) and H₂O (7.50 mL), and the mixture was controlled at 0°C. H₂O₂ (2.71 g, 23.9 mmol, 5.53 mL, purity: 30%, 4.61 eq) and AcOH (623 mg, 10.3 mmol, 593 µL, 2.00 eq) were then added thereto, and the mixture was stirred at 0°C for 2 hours. The reaction mixture was quenched with Na₂CO₃ (aq., 16.0 mL) at 0°C, then extracted with EtOAc (32.0 mL, 16.00 mL * 2), and concentrated under reduced pressure to obtain a residue. The residue was purified by column chromatography (SiO₂, petroleum ether/ethyl acetate = 50/1 to 2/1) to obtain 6-hydroxy-2-methyl-3,4-dihydro-2*H*-benzo[*b*][1,4]oxazin-3-one **(JD-5303049-3)** (650 mg, 3.63 mmol, yield: 69.9%) as a white solid.

LC-MS [M+H]⁺ = 180.0.

### Step 3: Preparation of 2-methyl-6-{[(4-methyl-1,3-thiazol-5-yl)methyl]oxy}-3,4-dihydro-2H-benzo[b][1,4]oxazin-3-one (JD-5303049-4)

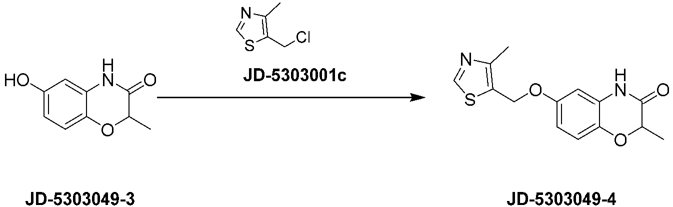

A mixture of 6-hydroxy-2-methyl-3,4-dihydro-2*H*-benzo[*b*][1,4]oxazin-3-one **(JD-5303049-3)** (500 mg, 2.79 mmol, 1.00 eq), K₂CO₃ (771 mg, 5.58 mmol, 2.00 eq), and 5-(chloromethyl)-4-methyl-1,3-thiazole **(JD-5303001c)** (411 mg, 2.79 mmol, 1.00 eq) in DMF (5.00 mL) was stirred at 50°C for 2 hours. The reaction mixture was quenched with H₂O (6.00 mL) and extracted with acetic acid (12.0 mL, 6.00 mL * 2). The combined organic phases were washed with NaCl (aq., 2.00 mL) and concentrated under reduced pressure to obtain a residue. The residue was purified by column chromatography (SiO₂, petroleum ether/ethyl acetate = 50/1 to 2/1) to obtain 2-methyl-6-{[(4-methyl-1,3-thiazol-5-yl)methyl]oxy}-3,4-dihydro-2*H*-benzo[*b*][1,4]oxazin-3-one **(JD-5303049-4)** (400 mg, 1.38 mmol, yield: 49.3%) as a white solid.

LC-MS [M+H]⁺ = 291.0.

### Step 4: Preparation of 2-methyl-6-{[(4-methyl-1,3-thiazol-5-yl)methyl]oxy}-3,4-dihydro-2H-benzo[b][1,4]oxazine (JD-5303049-5)

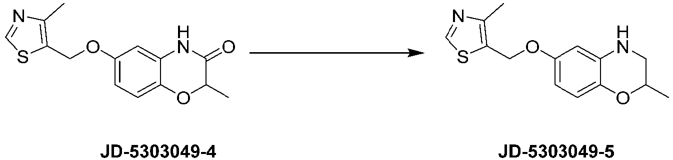

To a solution of 2-methyl-6-{[(4-methyl-1,3-thiazol-5-yl)methyl]oxy}-3,4-dihydro-2*H*-benzo[*b*][1,4]oxazin-3-one **(JD-5303049-4)** (300 mg, 1.03 mmol, 1.00 eq) in THF (3.00 mL) was added BH₃-Me₂S (10 M, 258 µL, 2.50 eq) at 0°C, and the mixture was stirred at 0°C for 2 hours. The reaction mixture was quenched with MeOH (5.00 mL) at 0°C and concentrated under reduced pressure to obtain 2-methyl-6-{[(4-methyl-1,3-thiazol-5-yl)methyl]oxy}-3,4-dihydro-2H-benzo[b][1,4]oxazine **(JD-5303049-5)** (300 mg, crude product) as a yellow solid.

LC-MS [M+H]⁺ = 277.0.

### Step 5: Preparation of tert-butyl 3,3-difluoro-4-{[(2-methyl-6-{[(4-methyl-1,3-thiazol-5-yl)methyl]oxyl-3,4-dihydro-2H-benzo[b][1,4]oxazin-4-yl)carbonyl]amino}pyrrolidine-1-carboxylate (JD-5303049-5)

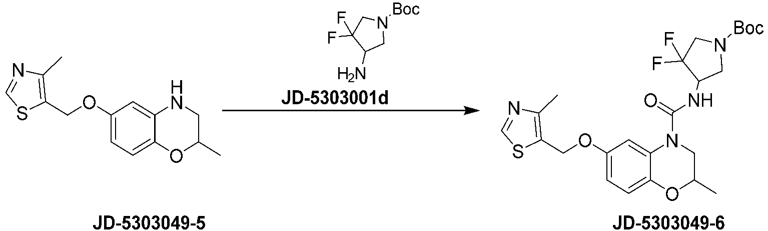

To a solution of *tert-*butyl 4-amino-3,3-difluoropyrrolidine-1-carboxylate **(JD-5303001d)** (160 mg, 723 µmol, 2.00 eq) in DMF (4.00 mL) was added CDI (130 mg, 806 µmol, 2.23 eq), and the mixture was stirred at 65°C for 1 hour. 2-Methyl-6-{[(4-methyl-1,3-thiazol-5-yl)methyl]oxy}-3,4-dihydro-2*H*-benzo[*b*][1,4]oxazine **(JD-5303049-5)** (100 mg, 361 µmol, 1.00 eq) was added thereto at 25°C, and the mixture was stirred at 65°C for 2 hours. The reaction mixture was concentrated and purified by HPLC (column: Welch xextreme C18 150 * 25 mm * 5 µm; mobile phase: [water (TFA)-ACN]; gradient: 40% to 70% B over 10 minutes) to obtain a crude product, which was then purified by HPLC to obtain *tert*-butyl 3,3-difluoro-4-{[(2-methyl-6-{[(4-methyl-1,3-thiazol-5-yl)methyl]oxy}-3,4-dihydro-2*H-*benzo[*b*][1,4]oxazin-4-yl)carbonyl]amino}pyrrolidine-1-carboxylate **(JD-5303049-5)** (120 mg, 228 µmol, yield: 63.2%) as a white solid.

LC-MS [M+H]⁺ = 525.1.

### Step 6: Preparation of N-(4,4-difluorotetrahydro-1H-pyrrol-3-yl)-2-methyl-6-{[(4-methyl-1,3-thiazol-5-yl)methyl]oxyl-3,4-dihydro-2H-benzo[b][1,4]oxazine-4-carboxamide hydrochloride (JD-5303049)

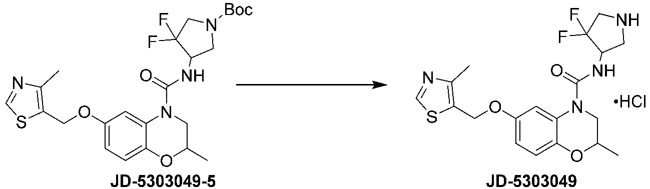

*tert*-Butyl 3,3-difluoro-4-{[(2-methyl-6-{[(4-methyl-1,3-thiazol-5-yl)methyl]oxy}-3,4-dihydro-2*H*-benzo[*b*][1,4]oxazin-4-yl)carbonyl]amino}pyrrolidine-1-carboxylate **(JD-5303049-5)** (100 mg, 190 µmol, 1.00 eq) was dissolved in TFE (2.00 mL), followed by the addition of TMSCl (41.4 mg, 381 µmol, 48.3 µL, 2.00 eq), and the mixture was stirred at 25°C for 1 hour. The reaction mixture was concentrated under reduced pressure to obtain N-(4,4-difluorotetrahydro-1*H*-pyrrol-3-yl)-2-methyl-6-{[(4-methyl-1,3-thiazol-5-yl)methyl]oxy}-3,4-dihydro-2*H*-benzo[*b*][1,4]oxazine-4-carboxamide hydrochloride **(JD-5303049)** (35.1 mg, 82.7 µmol, yield: 43.3%, purity: 98.89%) as a white gum.

LC-MS [M+H]⁺ = 425.0.

**¹H NMR:** (400 MHz, DMSO-*d*₆) δ = 1.26 (t, *J* = 6.13 Hz, 3 H), 2.39 (s, 3 H), 2.81 (td, *J* = 11.69, 8.38 Hz, 1 H), 2.89 - 3.03 (m, 1 H), 3.12 - 3.26 (m, 3 H), 3.87 - 4.03 (m, 1 H), 4.07 - 4.24 (m, 1 H), 4.28 - 4.46 (m, 1 H), 5.16 (s, 2 H), 6.63 (dd, *J* = 8.88, 2.88 Hz, 1 H), 6.78 (dd, *J* = 8.82, 2.69 Hz, 1 H), 7.09 - 7.20 (m, 1 H), 7.30 (dd, *J* = 6.38, 2.88 Hz, 1 H), 8.98 (s, 1 H).

### Example 56: Preparation of N-(4,4-difluorotetrahydro-1H-pyrrol-3-yl)-6-{[(4-methyl-1,3-thiazol-5-yl)methyl]oxy}-3,4-dihydrospiro[benzo[b][1,4]oxazine-2,1'-cyclopropane]-4-carboxamide hydrochloride (JD-5303050)

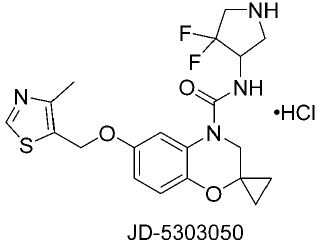

### Step 1: Preparation of 1-[(4-bromo-2-nitrophenyl)oxy]cyclopropane-1-carboxylic acid (JD-5303050-2)

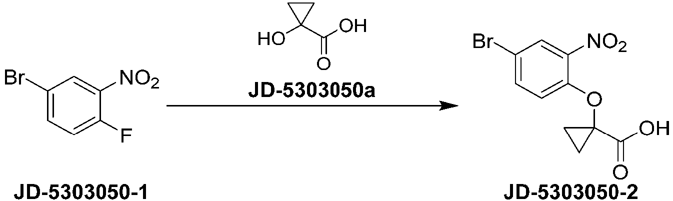

To a solution of 1-hydroxycyclopropane-1-carboxylic acid **(JD-5303050a)** (16.1 g, 73.4 mmol, 9.05 mL, 1.50 eq) in DMF (100 mL) was added NaH (4.90 g, 122 mmol, purity: 60%, 2.50 eq) under a N₂ atmosphere at 0°C, and the mixture was stirred at 0°C for 1 hour. 4-Bromo-1-fluoro-2-nitrobenzene **(JD-5303050-1)** (5.00 g, 48.9 mmol, 1.00 eq) was then added thereto, and the mixture was stirred at 25°C for another 15 hours. The reaction mixture was quenched with H₂O (300 mL) at 0°C and extracted with EtOAc (150 mL * 3). The aqueous phase was added with 2 M HCl to adjust the pH to 6, and extracted with EtOAc (300 mL, 150 mL * 2). The combined organic phases were washed with brine (10 mL * 3), dried over anhydrous MgSO₄, filtered, and concentrated under reduced pressure to obtain a residue. The residue was purified by column chromatography (SiO₂, petroleum ether/ethyl acetate = 50/1 to 2/1) to obtain 1-[(4-bromo-2-nitrophenyl)oxy]cyclopropane-1-carboxylic acid **(JD-5303050-2)** (11.0 g, 36.4 mmol, yield: 74.3%) as a yellow solid.

LC-MS [M] = 301.9.

### Step 2: Preparation of 6-bromo-3,4-dihydrospiro[benzo[b][1,4]oxazine-2,1'-cyclopropane]-3-one (JD-5303050-3)

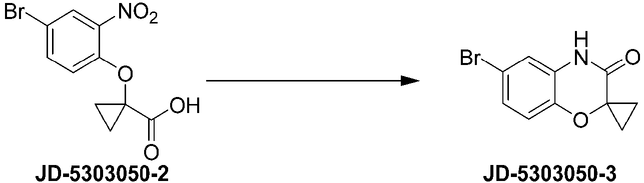

To a solution of 1-[(4-bromo-2-nitrophenyl)oxy]cyclopropane-1-carboxylic acid **(JD-5303050-2)** (4.00 g, 13.2 mmol, 1.00 eq) in ethanol (28.0 mL) and water (4.00 mL) were added NH₄Cl (2.83 g, 52.9 mmol, 4.00 eq) and Fe (2.96 g, 52.9 mmol, 4.00 eq), and the mixture was stirred at 90°C for 16 hours. The reaction mixture was filtered, and the filtrate was concentrated under reduced pressure to obtain a residue. The residue was purified by column chromatography (SiO₂, petroleum ether/ethyl acetate = 50/1 to 2/1) to obtain 6-bromo-3,4-dihydrospiro[benzo[*b*][1,4]oxazine-2,1'-cyclopropane]-3-one **(JD-5303050-3)** (2.20 g, 8.66 mmol, yield: 65.3%) as a white solid.

LC-MS [M+2] = 256.1.

### Step 3: Preparation of 6-(4,4,5,5-tetramethyl-1,3,2-dioxaborolan-2-yl)-3,4-dihydrospiro[benzo[b][1,4]oxazine-2,1'-cyclopropane]-3-one (JD-5303050-4)

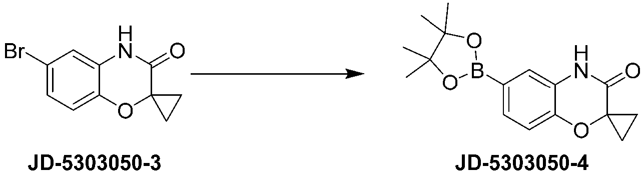

6-Bromo-3,4-dihydrospiro[benzo[*b*][1,4]oxazine-2,1'-cyclopropane]-3-one **(JD-5303050-3)** (2.20 g, 8.66 mmol, 1.00 eq) was dissolved in dioxane (25.0 mL), followed by the addition of BPD (4.40 g, 17.3 mmol, 2.00 eq), KOAc (2.12 g, 21.6 mmol, 2.50 eq), and PdCl₂(dppf) (316 mg, 432 µmol, 0.05 eq), and the mixture was stirred under a N₂ atmosphere at 100°C for 2 hours. The reaction mixture was quenched with H₂O (25.0 mL), then extracted with EtOAc (50.0 mL, 25.0 mL * 2), and concentrated under reduced pressure to obtain 6-(4,4,5,5-tetramethyl-1,3,2-dioxaborolan-2-yl)-3,4-dihydrospiro[benzo[b][1,4]oxazine-2,1'-cyclopropane]-3-one **(JD-5303050-4)** (3.00 g, crude product) as a black solid.

LC-MS [M+H]⁺ = 302.1.

### Step 4: Preparation of 6-hydroxy-3,4-dihydrospiro[benzo[b][1,4]oxazine-2,1'-cyclopropane]-3-one (JD-5303050-5)

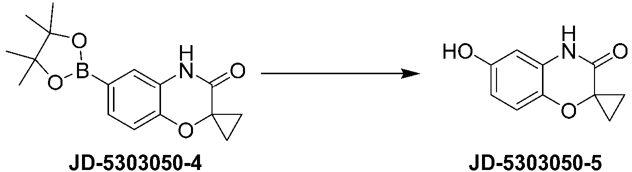

6-(4,4,5,5-Tetramethyl-1,3,2-dioxaborolan-2-yl)-3,4-dihydrospiro[benzo[*b*][1,4]oxazine-2,1'-cyclopropane]-3-one **(JD-5303050-4)** (2.60 g, 8.63 mmol, 1.00 eq) was dissolved in THF (26.0 mL) and H₂O (13.0 mL), and the mixture was controlled at approximately 0°C. H₂O₂ (5.18 g, 45.6 mmol, 4.39 mL, purity: 30%, 5.29 eq) and AcOH (5.18 g, 86.3 mmol, 4.94 mL, 10.0 eq) were then added thereto, and the mixture was stirred at 25°C for 2 hours. The reaction mixture was quenched with Na₂SO₃ (aq., 50.0 mL), then extracted with EtOAc (100 mL, 50.0 mL * 2), and concentrated under reduced pressure to obtain a residue. The residue was purified by column chromatography (SiO₂, petroleum ether/ethyl acetate = 50/1 to 2/1) to obtain 6-hydroxy-3,4-dihydrospiro[benzo[*b*][1,4]oxazine-2,1'-cyclopropane]-3-one **(JD-5303050-5)** (1.20 g, 6.28 mmol, yield: 72.7%) as a white solid.

LC-MS [M+H]⁺ = 192.1.

### Step 5: Preparation of 6-{[(4-methyl-1,3-thiazol-5-yl)methyl]oxy}-3,4-dihydrospiro[benzo[b][1,4]oxazine-2,1'-cyclopropane]-3-one (JD-5303050-6)

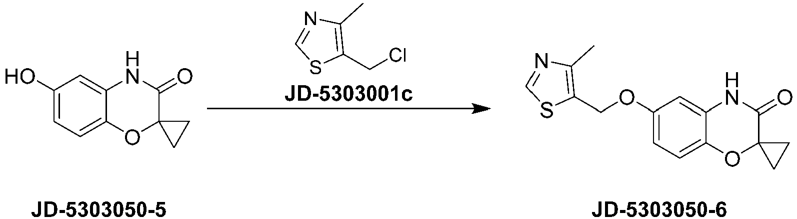

6-Hydroxy-3,4-dihydrospiro[benzo[*b*][1,4]oxazine-2,1'-cyclopropane]-3-one **(JD-5303050-5)** (1.00 g, 5.23 mmol, 1.00 eq) was dissolved in DMF (10.0 mL), followed by the addition of K₂CO₃ (1.45 g, 10.4 mmol, 2.00 eq) and 5-(chloromethyl)-4-methyl-1,3-thiazole **(JD-5303001c)** (772 mg, 5.23 mmol, 1.00 eq), and the mixture was stirred at 50°C for 16 hours. The reaction mixture was quenched with H₂O (20.0 mL) and extracted with EtOAc (40.0 mL, 20.0 mL * 2). The combined organic phases were washed with NaCl (aq., 3.00 mL) and concentrated under reduced pressure to obtain a residue. The residue was purified by column chromatography (SiO₂, petroleum ether/ethyl acetate = 50/1 to 2/1) to obtain 6-{[(4-methyl-1,3-thiazol-5-yl)methyl]oxy}-3,4-dihydrospiro[benzo[*b*][1,4]oxazine-2,1'-cyclopropane]-3-one **(JD-5303050-6)** (600 mg, 1.98 mmol, yield: 37.9%) as an off-white solid.

LC-MS [M+H]⁺ = 303.2.

### Step 6: Preparation of 6-{[(4-methyl-1,3-thiazol-5-yl)methyl]oxy}-3,4-dihydrospiro[benzo[b][1,4]oxazine-2,1'-cyclopropane] (JD-5303050-7)

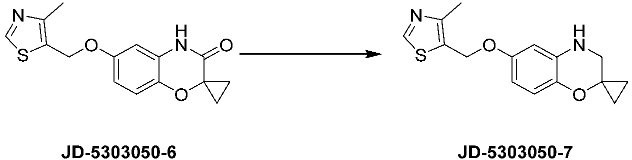

6-{[(4-Methyl-1,3-thiazol-5-yl)methyl]oxy}-3,4-dihydrospiro[benzo[*b*][1,4]oxazine-2,1'-cyclopropane]-3-one **(JD-5303050-6)** (100 mg, 330 µmol, 1.00 eq) was dissolved in THF (2.00 mL), followed by the addition of BH₃-Me₂S (10 M, 82.6 µL, 2.50 eq) at 0°C, and the mixture was stirred at 25°C for 6 hours. The reaction mixture was quenched with methanol (5.00 mL) at 0°C and concentrated under reduced pressure to obtain a residue. The residue was purified by preparative HPLC (column: CD04-Welch uultimate C18 150 * 25 * 7 µm; mobile phase: [water (TFA)-ACN]; gradient: 25% to 45% B over 10 minutes) to obtain 6-{[(4-methyl-1,3-thiazol-5-yl)methyl]oxy}-3,4-dihydrospiro[benzo[b][1,4]oxazine-2,1'-cyclopropane] **(JD-5303050-7)** (30.0 mg, 104 µmol, yield: 31.4%) as a brown oil.

LC-MS [M+H]⁺ = 289.0.

### Step 7: Preparation of tert-butyl 3,3-difluoro-4-{[(6-{[(4-methyl-1,3-thiazol-5-yl)methyl]oxy}-3,4-dihydrospiro[benzo[b][1,4]oxazine-2,1'-cyclopropane]-4-yl)carbonyl]amino}pyrrolidine-1-carboxylate (JD-5303050-8)

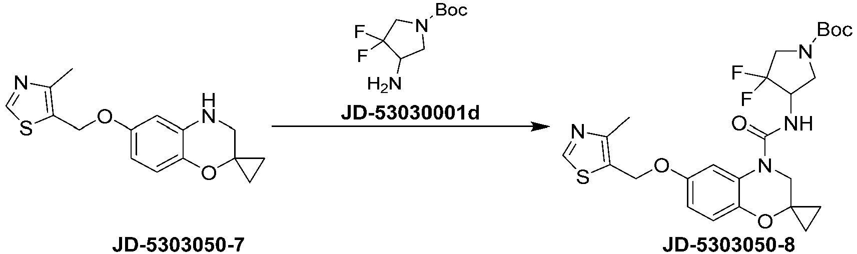

*tert*-Butyl 4-amino-3,3-difluoropyrrolidine-1-carboxylate **(JD-5303001d)** (46.2 mg, 208 µmol, 2.00 eq) and CDI (37.6 mg, 232 µmol, 2.23 eq) were dissolved in DMF (1.00 mL), and the mixture was stirred at 65°C for 1 hour. 6-{[(4-Methyl-1,3-thiazol-5-yl)methyl]oxy}-3,4-dihydrospiro[benzo[*b*][1,4]oxazine-2,1'-cyclopropane] **(JD-5303050-7)** (30.0 mg, 104 µmol, 1.00 eq) was then added thereto, and the mixture was reacted at 65°C for 16 hours. The reaction mixture was quenched with H₂O (2.00 mL) and extracted with EtOAc (4.00 mL, 2.00 mL * 2). The combined organic phases were concentrated under reduced pressure to obtain a residue. The residue was purified by HPLC (column: CD04-Welch uultimate C18 150 * 25 * 7 µm; mobile phase: [water (TFA)-ACN]; gradient: 38% to 68% B over 10 minutes) to obtain *tert*-butyl 3,3-difluoro-4-{[(6-{[(4-methyl-1,3-thiazol-5-yl)methyl]oxy}-3,4-dihydrospiro[benzo[*b*][1,4]oxazine-2,1'-cyclopropane]-4-yl)carbonyl]amino}pyrrolidine-1-carboxylate **(JD-5303050-8)** (30.0 mg, 55.9 µmol, yield: 53.7%) as a white solid.

LC-MS [M+H]⁺ = 537.1.

### Step 8: Preparation of N-(4,4-difluorotetrahydro-1H-pyrrol-3-yl)-6-1[(4-methyl-1,3-thiazol-5-yl)methyl]oxy}-3,4-dihydrospiro[benzo[b][1,4]oxazine-2,1'-cyclopropane]-4-carboxamide hydrochloride (JD-5303050)

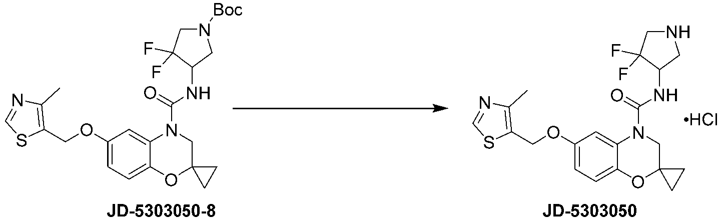

To a solution of *tert*-butyl 3,3-difluoro-4-{[(6-{[(4-methyl-1,3-thiazol-5-yl)methyl]oxyl-3,4-dihydrospiro[benzo[*b*][1,4]oxazine-2,1'-cyclopropane]-4-yl)carbonyl]amino}pyrrolidine-1-carboxylate **(JD-5303050-8)** (30.0 mg, 55.9 µmol, 1.00 eq) in TFE (3.00 mL) was added TMSCl (12.1 mg, 111 µmol, 14.1 µL, 2.00 eq), and the mixture was stirred at 25°C for 2 hours. The reaction mixture was concentrated under reduced pressure to obtain *N*-(4,4-difluorotetrahydro-1*H*-pyrrol-3-yl)-6-{[(4-methyl-1,3-thiazol-5-yl)methyl]oxy}-3,4-dihydrospiro[benzo[*b*][1,4]oxazine-2,1'-cyclopropane]-4-carboxamide hydrochloride **(JD-5303050)** (18.0 mg, 18.33 µmol, yield: 32.7%, purity: 100%) as a yellow gum.

LC-MS [M+H]⁺ = 437.0.

**¹H NMR:** (400 MHz, DMSO-*d*₆) δ = 0.68 - 0.84 (m, 2 H), 0.90 (s, 2 H) 2.40 (s, 3 H), 3.47 (br s, 1 H), 3.68 (br s, 3 H), 3.85 (br s, 2 H), 4.79 (dt, *J* = 11.48, 8.58 Hz, 1 H), 5.20 (s, 2 H), 6.68 (d, *J* = 2.88 Hz, 1 H), 6.72 - 6.79 (m, 1 H), 7.36 (d, *J* = 2.88 Hz, 1 H), 7.60 (d, *J=* 8.38 Hz, 1 H), 9.10 (s, 1 H), 10.08 - 10.41 (m, 2 H).

### Example 57: Preparation of 7-fluoro-N-(piperidin-3-yl)-6-{[(4-methyl-1,3-thiazol-5-yl)methyl]oxy}-3,4-dihydro-2H-benzo[2,1-b][1,4]oxazine-4-carboxamide hydrochloride (JD-5303051)

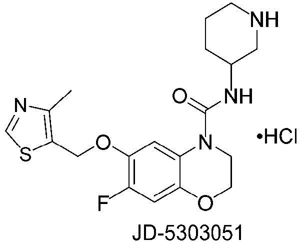

### Step 1: Preparation of methyl [(5-fluoro-4-methoxy-2-nitrophenyl)oxy]acetate (JD-5303051-2)

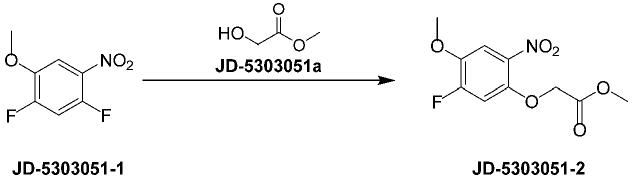

To a solution of methyl 2-hydroxyacetate **(JD-5303051a)** (4.29 g, 47.5 mmol, 3.67 mL, 0.90 eq) in THF (100 mL) was added NaH (1.90 g, 47.5 mmol, purity: 60%, 0.90 eq). The mixture was stirred at 0°C for 0.5 hours. 2,4-Difluoro-1-methoxy-5-nitrobenzene **(JD-5303051-1)** (10.0 g, 52.8 mmol, 1.00 eq) was added thereto, and the mixture was allowed to stand at 25°C for 2 hours. The reaction mixture was quenched with NH₄Cl (10.0 mL) at 0°C, then diluted with H₂O (30.0 mL), and extracted with EtOAc (30 mL). The organic phases were separated, filtered, and concentrated under reduced pressure to obtain a residue. The residue was purified by column chromatography (SiO₂, petroleum ether/ethyl acetate = 1/1) to obtain methyl [(5-fluoro-4-methoxy-2-nitrophenyl)oxy]acetate **(JD-5303051-2)** (1.80 g, 6.94 mmol, yield: 13.1%) as a yellow oil.

LC-MS [M+Na] = 282.0.

### Step 2: Preparation of 7-fluoro-6-methoxy-3,4-dihydro-2H-benzo[2,1-b][1,4]oxazin-3-one (JD-5303051-3)

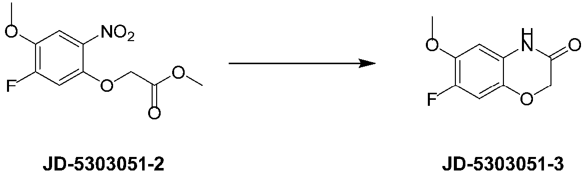

To methyl [(5-fluoro-4-methoxy-2-nitrophenyl)oxy]acetate **(JD-5303051-2)** (1.80 g, 6.94 mmol, 1.00 eq) was added Fe powder (3.88 g, 69.4 mmol, 10.0 eq), followed by the addition of EtOH (20.0 mL) and AcOH (20.0 mL), respectively, and the mixture was stirred at 50°C for 12 hours. The reaction mixture was filtered and concentrated under reduced pressure to obtain 7-fluoro-6-methoxy-3,4-dihydro-2*H*-benzo[2,1-*b*][1,4]oxazin-3-one **(JD-5303051-3)** (1.40 g, crude product) as a brown solid.

LC-MS [M+H]⁺ = 198.1.

### Step 3: Preparation of 7-fluoro-6-hydroxy-3,4-dihydro-2H-benzo[2,1-b][1,4]oxazin-3-one (JD-5303051-4)

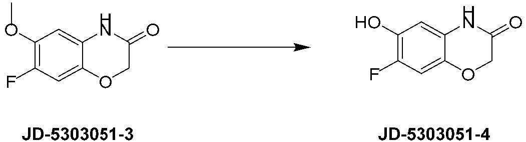

To a solution of 7-fluoro-6-methoxy-3,4-dihydro-2*H*-benzo[2,1-*b*][1,4]oxazin-3-one **(JD-5303051-3)** (1.40 g, 7.10 mmol, 1.00 eq) in DCM (15.0 mL) was added BBr₃ (8.89 g, 35.5 mmol, 3.42 mL, 5.00 eq), and the mixture was stirred at -78°C for 3 hours. The reaction mixture was quenched with MeOH (10 mL) at -78°C, diluted with H₂O (10.0 mL), extracted with DCM (20.0 mL, 10.0 mL * 2), and concentrated under reduced pressure to obtain 7-fluoro-6-hydroxy-3,4-dihydro-2*H*-benzo[2,1-*b*][1,4]oxazin-3-one **(JD-5303051-4)** (500 mg, crude product) as a brown solid.

LC-MS [M-H]⁻ = 182.8.

### Step 4: Preparation of 7-fluoro-6-{[(4-methyl-1,3-thiazol-5-yl)methyl]oxy}-3,4-dihydro-2H-benzo[2,1-b][1,4]oxazin-3-one (JD-5303051-5)

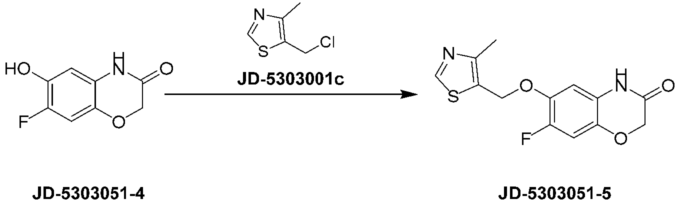

To a solution of 7-fluoro-6-hydroxy-3,4-dihydro-2*H*-benzo[2,1-*b*][1,4]oxazin-3-one **(JD-5303051-4)** (500 mg, 2.73 mmol, 1.00 eq) and 5-(chloromethyl)-4-methyl-1,3-thiazole **(JD-5303001c)** (362 mg, 2.46 mmol, 0.90 eq) in DMF (5.00 mL) was added K₂CO₃ (754 mg, 5.46 mmol, 2.00 eq), and the mixture was stirred at 25°C for 4 hours. The reaction mixture was quenched with H₂O (10.0 mL) and extracted with EtOAc (20.0 mL). The combined organic phases were separated, filtered, and concentrated under reduced pressure to obtain 7-fluoro-6-1[(4-methyl-1,3-thiazol-5-yl)methyl]oxyl-3,4-dihydro-2*H*-benzo[2,1-*b*][1,4]oxazin-3-one **(JD-5303051-5)** (800 mg, crude product) as a brown solid.

LC-MS [M+H]⁺ = 295.0.

### Step 5: Preparation of 7-fluoro-6-{[(4-methyl-1,3-thiazol-5-yl)methyl]oxy}-3,4-dihydro-2H-benzo[2,1-b][1,4]oxazine (JD-5303051-6)

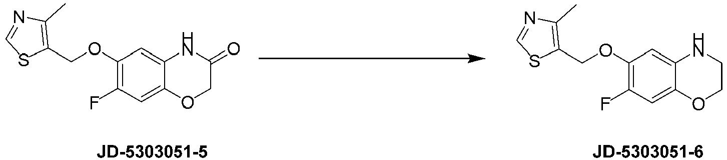

7-Fluoro-6-{[(4-methyl-1,3-thiazol-5-yl)methyl]oxy}-3,4-dihydro-2*H*-benzo[2,1-*b*][1,4]oxazin-3-one **(JD-5303051-5)** (200 mg, 679 µmol, 1.00 eq) was dissolved in THF (2.00 mL), followed by the addition of BMS (10 M, 339 µL, 5.00 eq). The mixture was stirred at 25°C for 2 hours. The reaction mixture was quenched with MeOH (5.00 mL) at 0°C and concentrated under reduced pressure to obtain 7-fluoro-6-{[(4-methyl-1,3-thiazol-5-yl)methyl]oxy}-3,4-dihydro-2*H*-benzo[2,1-*b*][1,4]oxazine **(JD-5303051-6)** (520 mg, crude product) as a brown oil.

LC-MS [M+H]⁺ = 281.0.

### Step 6: Preparation of tert-butyl 3-{[(7-fluoro-6-{[(4-methyl-1,3-thiazol-5-yl)methyl]oxy}-3,4-dihydro-2H-benzo[2,1-b][1,4]oxazin-4-yl)carbonyl]amino}piperidine-1-carboxylate (JD-5303051-7)

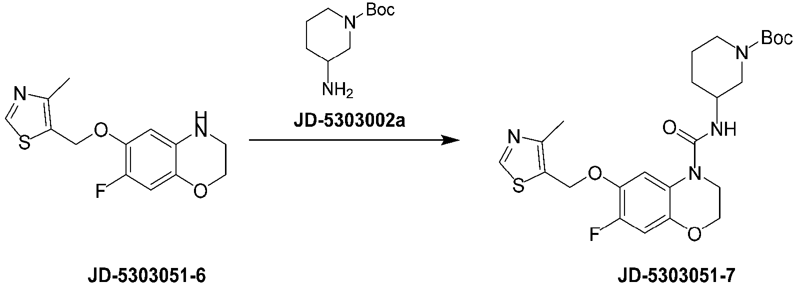

To a solution of 7-fluoro-6-{[(4-methyl-1,3-thiazol-5-yl)methyl]oxy}-3,4-dihydro-2*H*-benzo[2,1-*b*][1,4]oxazine **(JD-5303051-6)** (50.0 mg, 178 µmol, 1.00 eq) and *tert-butyl 3-*aminopiperidine-1-carboxylate **(JD-5303002a)** (107 mg, 535 µmol, 3.00 eq) in THF (1.00 mL) were added BTC (26.4 mg, 89.1 µmol, 0.50 eq) and DIEA (69.1 mg, 535 µmol, 93.2 µL, 3.00 eq), and the mixture was stirred at 90°C for 12 hours. The reaction mixture was quenched with H₂O (10.0 mL) and extracted with EtOAc (10.0 mL). The combined organic phases were separated, filtered, and concentrated under reduced pressure to obtain a residue. The residue was purified by preparative HPLC to obtain *tert*-butyl 3-{[(7-fluoro-6-{[(4-methyl-1,3-thiazol-5-yl)methyl]oxy}-3,4-dihydro-2*H*-benzo[2,1-*b*][1,4]oxazin-4-yl)carbonyl]amino}piperidine-1-carboxylate **(JD-5303051-7)** (5.00 mg, 9.87 µmol, yield: 5.53%) as a white solid.

LC-MS [M+H]⁺ = 507.1.

### Step 7: Preparation of 7-fluoro-N-(piperidin-3-yl)-6-{[(4-methyl-1,3-thiazol-5-yl)methyl]oxy}-3,4-dihydro-2H-benzo [2,1-b][1,4]oxazine-4-carboxamide hydrochloride (JD-5303051)

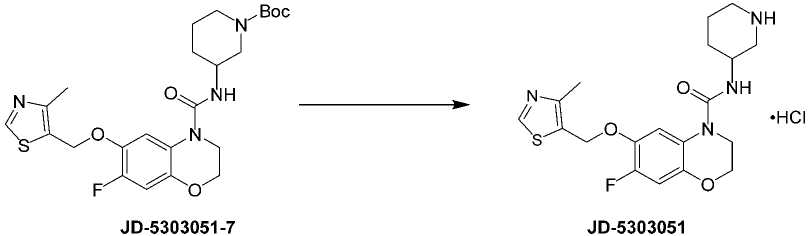

To a solution of *tert*-butyl 3-{[(7-fluoro-6-{[(4-methyl-1,3-thiazol-5-yl)methyl]oxy}-3,4-dihydro-2*H*-benzo[2,1-*b*][1,4]oxazin-4-yl)carbonyl]amino}piperidine-1-carboxylate **(JD-5303051-7)** (5.00 mg, 9.87 µmol, 1.00 eq) in TFE (1.00 mL) was added TMSCl (2.14 mg, 19.7 µmol, 2.51 µL, 2.00 eq). The mixture was stirred at 25°C for 2 hours. The reaction mixture was filtered and concentrated under reduced pressure to obtain a residue. The residue was purified by preparative HPLC to obtain 7-fluoro-*N*-(piperidin-3-yl)-6-{[(4-methyl-1,3-thiazol-5-yl)methyl]oxy}-3,4-dihydro-2*H*-benzo[2,1-*b*][1,4]oxazine-4-carboxamide hydrochloride **(JD-5303051)** (3.66 mg, 8.26 µmol, yield: 83.7%, purity: 98.52%, HCl) as a yellow solid.

LC-MS [M+H]⁺ = 407.1.

¹H NMR: (400 MHz, METHANOL-*d*₄) δ ppm 1.54 - 1.90 (m, 2 H) 1.97 - 2.14 (m, 2 H) 2.44 (s, 3 H) 2.78 - 2.99 (m, 2 H) 3.34 (br d, *J* = 3.50 Hz, 1 H) 3.49 (br dd, *J* = 11.94, 3.69 Hz, 1 H) 3.66 - 3.83 (m, 2 H) 3.92 - 4.05 (m, 1 H) 4.13 - 4.26 (m, 2 H) 5.24 (s, 2 H) 6.69 (d, *J* = 11.76 Hz, 1 H) 7.39 (d, *J* = 8.63 Hz, 1 H) 8.93 (s, 1 H).

### Example 58: Preparation of 2-(piperidin-3-yl)-1-(6-{[(4-methyl-1,3-thiazol-5-yl)methyl]oxy}-3,4-dihydro-2H-benzo[b][1,4]oxazin-4-yl)ethan-1-one (JD-5303052)

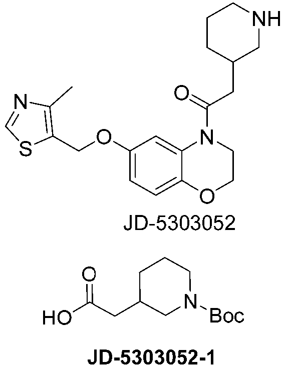

Following the synthetic method of Example 1, compound **JD-5303001d** was replaced with **JD-5303052-1** to obtain 2-(piperidin-3-yl)-1-(6-{[(4-methyl-1,3-thiazol-5-yl)methyl]oxy}-3,4-dihydro-2*H*-benzo[*b*][1,4]oxazin-4-yl)ethan-1-one **(JD-5303052)** (16 mg, yield: 44.7%, purity: 99.2%) as a yellow solid.

LC-MS ([M+H]⁺) = 388.1.

**¹H NMR** (400 MHz, DMSO-*d*₆) δ = 8.92 (s, 1H), 7.52 (s, 1H), 6.82 (d, *J* = 8.9 Hz, 1H), 6.76 (dd, *J=* 8.9, 2.9 Hz, 1H), 5.21 (s, 2H), 4.27 - 4.16 (m, 2H), 3.92 - 3.77 (m, 2H), 3.32 - 3.25 (m, 1H), 3.22 - 3.17 (m, 1H), 2.82 - 2.73 (m, 1H), 2.69 - 2.59 (m, 3H), 2.41 (s, 3H), 2.35 - 2.23 (m, 1H), 1.85 - 1.66 (m, 3H), 1.32 - 1.26 (m, 1H).

### Example 59: Preparation of N-(piperidin-3-yl)-6-{[(4-methyl-1,3-thiazol-5-yl)methyl]oxy}-3,4-dihydro-2H-chromene-4-carboxamide (JD-5303053)

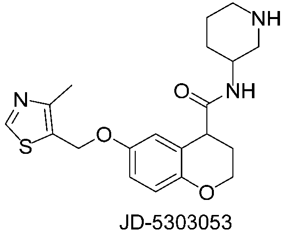

### Step 1: Preparation of 6-bromo-3,4-dihydro-2H-chromene-4-carbonitrile (JD-5303053-2)

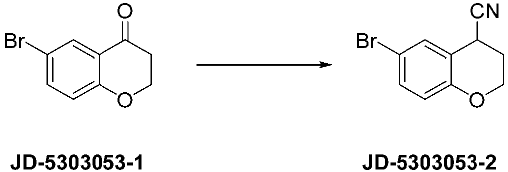

6-bromo-3,4-dihydro-2*H*-chromen-4-one **(JD-5303053-1)** (5.0 g, 22.02 mmol, 1.0 eq) and TosMIC (6.45 g, 33.03 mmol, 1.5 eq) were dissolved in DME (250 mL) and EtOH (10 mL), then the mixture was cooled to 0°C under a N₂ atmosphere, followed by the addition of t-BuOK (5.43 g, 48.44 mmol, 2.2 eq). The mixture was reacted at room temperature for 15 hours. The reaction mixture was quenched with ammonium chloride aqueous solution (40 mL) at room temperature and extracted with ethyl acetate (60 mL, 30.0 mL * 2). The combined organic phases were concentrated under reduced pressure to obtain a residue. The residue was purified by column chromatography (SiO₂, petroleum ether/ethyl acetate = 30/1) to obtain 6-bromo-3,4-dihydro-2*H*-chromene-4-carbonitrile **(JD-5303053-2)** (2.4 g, yield: 45.7%) as a white solid.

**¹H NMR** (400 MHz, CDCl₃) δ = 7.42 (d, *J* = 2.4 Hz, 1H), 7.31 (dd, *J* = 8.8, 2.4 Hz, 1H), 6.75 (d, *J* = 8.8 Hz, 1H), 4.38 - 4.29 (m, 1H), 4.27 - 4.20 (m, 1H), 3.99 (t, *J* = 6.0 Hz, 1H), 2.39 - 2.28 (m, 2H).

### Step 2: Preparation of methyl 6-bromo-3,4-dihydro-2H-chromene-4-carboxylate (JD-5303053-3)

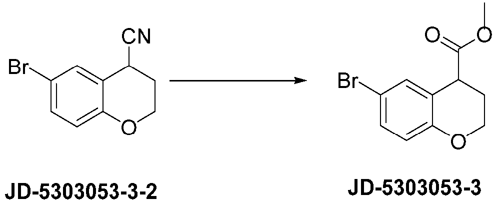

6-Bromo-3,4-dihydro-2*H*-chromene-4-carbonitrile **(JD-5303053-2)** (600 mg, 2.52 mmol, 1.0 eq) was dissolved in MeOH (14 mL), followed by the addition of H₂SO₄ (6.18 g, 63.0 mmol, 25.0 eq). The mixture was reacted at 100°C under a N₂ atmosphere for 15 hours. The reaction mixture was quenched with water (10 mL) at room temperature and extracted with ethyl acetate (30 mL, 15.0 mL * 2). The combined organic phases were washed with saturated Na₂CO₃ solution (10 mL) and brine (10 mL), dried over anhydrous sodium sulfate, and concentrated under reduced pressure to obtain a residue. The residue was purified by column chromatography (SiO₂, petroleum ether/ethyl acetate = 5/1 to 3/1) to obtain methyl 6-bromo-3,4-dihydro-2*H*-chromene-4-carboxylate **(JD-5303053-3)** (550 mg, yield: 80.5%) as a colorless oil.

**LC-MS** ([M+H]⁺) = 271.0.

**¹H NMR** (300 MHz, CDCl₃) δ = 7.37 (dd, *J* = 2.4, 0.6 Hz, 1H), 7.24 (dd, *J* = 8.7, 2.4 Hz, 1H), 6.72 (d, *J=* 8.7 Hz, 1H), 4.30 - 4.21 (m, 2H), 3.80 - 3.70 (m, 4H), 2.38 - 2.27 (m, 1H), 2.16 - 2.00 (m, 1H).

### Step 3: Preparation of methyl 6-(4,4,5,5-tetramethyl-1,3,2-dioxaborolan-2-yl)-3,4-dihydro-2H-chromene-4-carboxylate (JD-5303053-4)

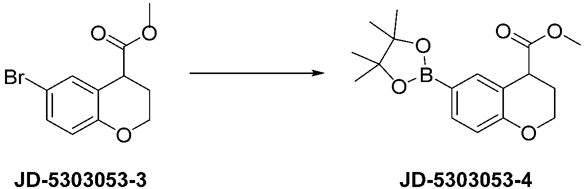

Methyl 6-bromo-3,4-dihydro-2*H*-chromene-4-carboxylate **(JD-5303053-3)** (550 mg, 2.03 mmol, 1.0 eq) was dissolved in dioxane (8.0 mL), followed by the addition of BPD (1.03 g, 4.06 mmol, 2.0 eq), AcOK (400 mg, 4.06 mmol, 2.0 eq), and Pd(dppf)Cl₂ (73 mg, 0.10 mmol, 0.05 eq), and the mixture was reacted at 100°C for 3 hours. The reaction mixture was quenched with water (10 mL) at room temperature and extracted with ethyl acetate (30 mL, 15.0 mL * 2). The combined organic phases were washed with brine (10 mL), dried over anhydrous sodium sulfate, and concentrated under reduced pressure to obtain a residue. The residue was purified by column chromatography (SiO₂, petroleum ether/ethyl acetate = 50/1 to 15/1) to obtain methyl 6-(4,4,5,5-tetramethyl-1,3,2-dioxaborolan-2-yl)-3,4-dihydro-2*H-*chromene-4-carboxylate **(JD-5303053-4)** (900 mg, crude product, containing BPD residue by NMR) as a yellow oil.

**LC-MS** ([M+H]⁺) = 319.0.

**¹H NMR** (300 MHz, CDCl₃) *δ =* 7.66 (s, 1H), 7.61 (dd, *J* = 8.1, 1.5 Hz, 1H), 6.83 (d, *J* = 8.1 Hz, 1H), 4.32 - 4.24 (m, 2H), 3.85 - 3.78 (m, 1H), 3.73 (s, 3H), 2.37 - 2.24 (m, 1H), 2.18 - 2.06 (m, 1H), 1.32 (s, 12H).

### Step 4: Preparation of methyl 6-hydroxy-3,4-dihydro-2H-chromene-4-carboxylate (JD-5303053-5)

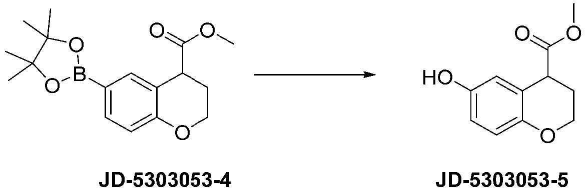

Methyl 6-(4,4,5,5-tetramethyl-1,3,2-dioxaborolan-2-yl)-3,4-dihydro-2*H*-chromene-4-carboxylate **(JD-5303053-4)** (800 mg, 2.51 mmol, 1.0 eq) was dissolved in THF (4.0 mL) and H₂O (4.0 mL), followed by the addition of AcOH (755 mg, 12.57 mmol, 5.0 eq) and H₂O₂ (2.85 g, 25.14 mmol, 10.0 eq) (30%) at 0°C, and the mixture was reacted at room temperature for 15 hours. The reaction mixture was quenched with saturated Na₂SO₃ aqueous solution (10 mL) at 0°C and extracted with ethyl acetate (30 mL, 15.0 mL * 2). The combined organic phases were washed with brine (10 mL), dried over anhydrous sodium sulfate, and concentrated under reduced pressure to obtain a residue. The residue was purified by column chromatography (SiO₂, petroleum ether/ethyl acetate = 20/1 to 5/1) to obtain methyl 6-hydroxy-3,4-dihydro-2*H*-chromene-4-carboxylate **(JD-5303053-5)** (350 mg, yield: 66.8%) as a yellow oil.

**LC-MS** ([M+H]⁺) = 209.0.

**¹H NMR** (400 MHz, DMSO-*d*₆) *δ* = 8.87 (s, 1H), 6.63 - 6.50 (m, 3H), 4.15 - 4.02 (m, 2H), 3.81 (t, *J* = 5.6 Hz, 1H), 3.67 (s, 3H), 2.18 - 2.08 (m, 1H), 2.07 - 1.95 (m, 1H).

### Step 5: Preparation of methyl 6-{[(4-methyl-1,3-thiazol-5-yl)methyl]oxy}-3,4-dihydro-2H-chromene-4-carboxylate (JD-5303053-6)

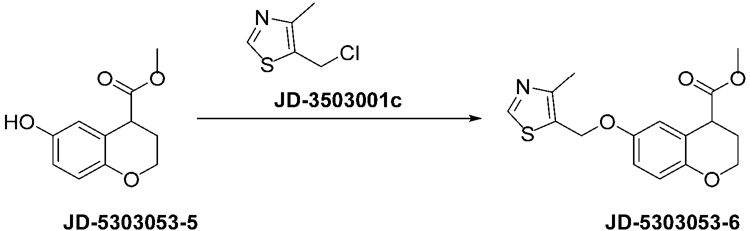

Methyl 6-hydroxy-3,4-dihydro-2*H*-chromene-4-carboxylate **(JD-5303053-5)** (100 mg, 0.48 mmol, 1.0 eq) was dissolved in DMF (2.0 mL), followed by the addition of 5-(chloromethyl)-4-methyl-1,3-thiazole **(JD-5303001c)** (92 mg, 0.62 mmol, 1.3 eq) and K₂CO₃ (133 mg, 0.96 mmol, 2.0 eq), and the mixture was reacted at 50°C for 2 hours. The reaction mixture was quenched with water (10 mL) at room temperature and extracted with ethyl acetate (30 mL, 15.0 mL * 2). The combined organic phases were washed with water (24 mL, 8.0 mL * 3) and brine (10 mL), dried over anhydrous sodium sulfate, and concentrated under reduced pressure to obtain a residue. The residue was purified by column chromatography (SiO₂, petroleum ether/ethyl acetate = 10/1 to 3/1) to obtain methyl 6-{[(4-methyl-1,3-thiazol-5-yl)methyl]oxy}-3,4-dihydro-2*H*-chromene-4-carboxylate **(JD-5303053-6)** (100 g, yield: 65.2%) as a yellow oil.

**LC-MS** ([M+H]⁺) = 320.1.

**¹H NMR** (400 MHz, CDCl₃) *δ* = 8.70 (s, 1H), 6.90 - 6.75 (m, 3H), 5.11 (s, 2H), 4.28 - 4.15 (m, 2H), 3.82 - 3.76 (m, 1H), 3.73 (s, 3H), 2.47 (s, 3H), 2.37 - 2.26 (m, 1H), 2.15 - 2.05 (m, 1H).

### Step 6: Preparation of 6-{[(4-methyl-1,3-thiazol-5-yl)methyl]oxy}-3,4-dihydro-2H-chromene-4-carboxylic acid (JD-5303053-7)

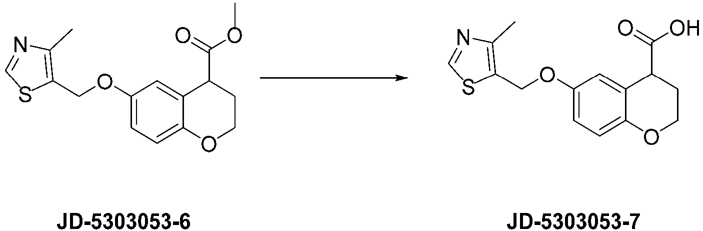

Methyl 6-{[(4-methyl-1,3-thiazol-5-yl)methyl]oxy}-3,4-dihydro-2*H*-chromene-4-carboxylate **(JD-5303053-6)** (100 mg, 0.31 mmol, 1.0 eq) was dissolved in MeOH (0.5 mL), THF (0.5 mL), and H₂O (0.5 mL), followed by the addition of LiOH (39 mg, 0.93 mmol, 3.0 eq), and the mixture was reacted at room temperature for 2 hours. The reaction mixture was added with saturated potassium bisulfate aqueous solution at room temperature to adjust the pH to 3, and diluted with ethyl acetate (5 mL). The mixture was filtered, and the filter cake was collected to obtain 6-{[(4-methyl-1,3-thiazol-5-yl)methyl]oxy}-3,4-dihydro-2*H-*chromene-4-carboxylic acid **(JD-5303053-7)** (70 mg, yield: 73.1%) as a white solid.

**LC-MS** ([M+H]⁺) = 306.1.

**¹H NMR** (300 MHz, DMSO-*d*₆) *δ* = 12.68 (brs, 1H), 8.97 (s, 1H), 6.92 - 6.79 (m, 2H), 6.74 - 6.67 (m, 1H), 5.18 (s, 2H), 4.20 - 4.00 (m, 2H), 3.72 (t, *J* = 5.4 Hz, 1H), 2.39 (s, 3H), 2.22 - 2.10 (m, 1H), 2.08 - 1.93 (m, 1H).

### Step 7: Preparation of tert-butyl 3-{[(6-{[(4-methyl-1,3-thiazol-5-yl)methyl]oxy}-3,4-dihydro-2H-chromen-4-yl)carbonyl]amino}piperidine-1-carboxylate (JD-5303053-8)

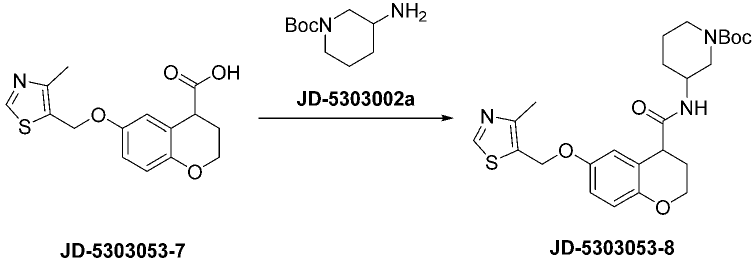

6-{[(4-Methyl-1,3-thiazol-5-yl)methyl]oxy}-3,4-dihydro-2*H*-chromene-4-carboxylic acid **(JD-5303053-7)** (60 mg, 0.20 mmol, 1.0 eq) was dissolved in DCM (2.0 mL), followed by the addition of (COCl)₂ (75 mg, 0.59 mmol, 3.0 eq) and a drop of DMF at 0°C, and the mixture was reacted at room temperature for 0.5 hours. The reaction mixture was concentrated to obtain a residue. The residue was then diluted with DCM (2 mL), followed by the addition of compound **6** (40 mg, 0.15 mmol, 1.0 eq) and TEA (46 mg, 0.46 mmol, 3.0 eq) at room temperature, and the mixture was reacted at room temperature for 1 hour. The reaction system was quenched with water (15 mL) and extracted with dichloromethane (10 mL * 2). The combined organic phases were washed with brine (10 mL), dried over anhydrous sodium sulfate, and concentrated under reduced pressure to obtain a residue. The residue was purified by column chromatography (SiO₂, dichloromethane/methanol = 200/1 to 100/1) to obtain *tert*-butyl 3-{[(6-{[(4-methyl-1,3-thiazol-5-yl)methyl]oxy}-3,4-dihydro-2*H*-chromen-4-yl)carbonyl]amino}piperidine-1-carboxylate **(JD-5303053-8)** (70 mg, yield: 73.4%) as a yellow oil.

**LC-MS** ([M+H]⁺) = 488.2.

**¹H NMR** (300 MHz, CDCl₃) *δ* = 8.72 (d, *J* = 1.5 Hz, 1H), 6.90 - 6.77 (m, 2H), 6.67 (d, *J* = 2.1 Hz, 1H), 5.75 - 5.60 (m, 1H), 5.18 - 5.05 (m, 2H), 4.27 - 4.16 (m, 1H), 4.13 - 3.90 (m, 2H), 3.63 - 3.56 (m, 1H), 3.50 - 3.40 (m, 1H), 3.38 - 3.15 (m, 3H), 2.48 (d, *J* = 3.3 Hz, 3H), 2.44 - 2.32 (m, 1H), 2.27 - 1.74 (m, 3H), 1.60 - 1.49 (m, 2H), 1.41 (d, *J* = 2.1 Hz, 9H).

### Step 8: Preparation of N-(piperidin-3-yl)-6-{[(4-methyl-1,3-thiazol-5-yl)methyl]oxy}-3,4-dihydro-2H-chromene-4-carboxamide (JD-5303053)

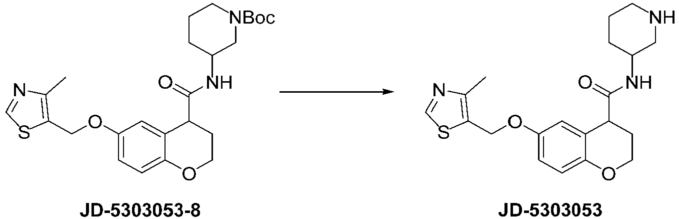

*tert*-Butyl 3-{[(6-{[(4-methyl-1,3-thiazol-5-yl)methyl]oxy}-3,4-dihydro-2*H-*chromen-4-yl)carbonyl]amino}piperidine-1-carboxylate **(JD-5303053-8)** (70 mg, 0.14 mmol, 1.0 eq) was dissolved in DCM (2.5 mL), followed by the addition of dioxane/HCl (4 M, 0.7 mL, 2.8 mmol, 20.0 eq), and the mixture was reacted at room temperature for 2 hours. The reaction mixture was concentrated under reduced pressure to obtain a residue. The residue was added with sodium bicarbonate to adjust the pH to 9, and extracted with DCM (10 mL * 2). The combined organic phases were washed with brine (8 mL), dried over anhydrous sodium sulfate, and concentrated under reduced pressure to obtain a residue. The residue was purified by prep-TLC (dichloromethane/methanol = 8/1) to obtain *N*-(piperidin-3-yl)-6-{[(4-methyl-1,3-thiazol-5-yl)methyl]oxy}-3,4-dihydro-2*H*-chromene-4-carboxamide **(JD-5303053)** (23 mg, yield: 41.0%, purity: 94.0%) as a white solid.

**LC-MS** ([M+H]⁺) = 388.2.

**¹H NMR** (300 MHz, CDCl₃) *δ* = 8.70 (d, *J* = 0.9 Hz, 1H), 6.90 - 6.80 (m, 2H), 6.78 - 6.70 (m, 1H), 6.55 (brs, 1H), 5.11 (s, 2H), 4.28 - 4.12 (m, 2H), 4.12 - 4.00 (m, 1H), 3.70 - 3.56 (m, 1H), 3.09 - 2.97 (m, 2H), 2.89 - 2.79 (m, 2H), 2.78 - 2.64 (m, 1H), 2.46 (s, 3H), 2.42 - 2.27 (m, 1H), 2.19 - 2.02 (m, 1H), 1.80 - 1.52 (m, 4H).

### Example 60: Preparation of 5-fluoro-N-(piperidin-3-yl)-6-{[(4-methyl-1,3-thiazol-5-yl)methyl]oxy}-3,4-dihydro-2H-benzo[b][1,4]oxazine-4-carboxamide (JD-5303054)

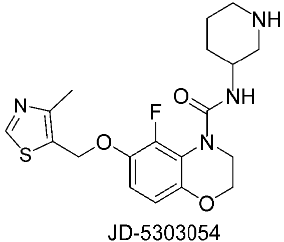

### Step 1: Preparation of 5-fluoro-3,4-dihydro-2H-benzo[b][1,4]oxazin-3-one (JD-5303054-2)

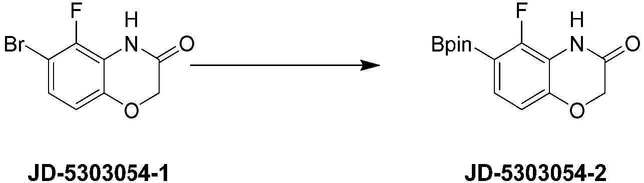

To a solution of 6-bromo-5-fluoro-3,4-dihydro-2*H*-benzo[*b*][1,4]oxazin-3-one **(JD-5303054-1)** (900 mg, 3.66 mmol, 1.00 eq) and BPD (1.86 g, 7.32 mmol, 2.00 eq) in dioxane (9.00 mL) were added KOAc (897 mg, 9.15 mmol, 2.50 eq) and Pd(dppf)Cl₂ (535 mg, 731 µmol, 0.20 eq), and the mixture was stirred at 100°C for 2 hours. The reaction mixture was quenched with H₂O (20.0 mL) and extracted with EtOAc (20.0 mL). The combined organic phases were separated, filtered, and concentrated under reduced pressure to obtain 5-fluoro-3,4-dihydro-2*H*-benzo[*b*][1,4]oxazin-3-one **(JD-5303054-2)** (2.90 g, crude product) as a black solid.

LC-MS [M+H]⁺ = 168.1.

### Step 2: Preparation of 5-fluoro-6-hydroxy-3,4-dihydro-2H-benzo[b][1,4]oxazin-3-one (JD-5303054-3)

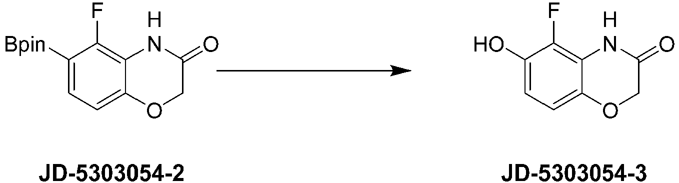

To a solution of 5-fluoro-3,4-dihydro-2*H*-benzo[*b*][1,4]oxazin-3-one **(JD-5303054-2)** (2.90 g, 9.89 mmol, 1.00 eq) in THF (29.0 mL) and H₂O (29.0 mL) was added NaBO₃ (4.57 g, 29.6 mmol, 5.71 mL, 3.00 eq), and the mixture was stirred at 0°C for 3 hours. The reaction mixture was washed with H₂O (40.0 mL) and extracted with EtOAc (50.0 mL). The organic phases were separated, filtered, and concentrated under reduced pressure to obtain a residue. The residue was purified by column chromatography (SiO₂, petroleum ether/ethyl acetate = 1/1) to obtain 5-fluoro-6-hydroxy-3,4-dihydro-2*H*-benzo[*b*][1,4]oxazin-3-one **(JD-5303054-3)** (450 mg, 2.46 mmol, yield: 24.8%) as a yellow solid.

LC-MS [M+H]⁺ = 184.0.

### Step 3: Preparation of 5-fluoro-6-1[(4-methyl-1,3-thiazol-5-yl)methyl]oxyl-3,4-dihydro-2H-benzo[b][1,4]oxazin-3-one (JD-5303054-4)

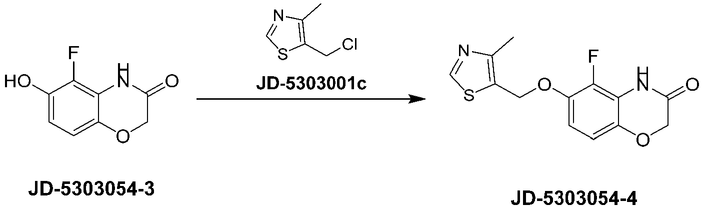

To a solution of 5-fluoro-6-hydroxy-3,4-dihydro-2*H*-benzo[*b*]1,4]oxazin-3-one **(JD-5303054-3)** (253 mg, 1.72 mmol, 0.90 eq) and 5-(chloromethyl)-4-methyl-1,3-thiazole **(JD-5303001c)** (253 mg, 1.72 mmol, 0.9 eq) in DMF (3.00 mL) was added K₂CO₃ (528 mg, 3.82 mmol, 2.00 eq). The mixture was stirred at 25°C for 2 hours. The reaction mixture was washed with H₂O (10.0 mL) and extracted with EtOAc (20.0 mL). The organic phases were separated, filtered, and concentrated under reduced pressure to obtain a residue. The residue was purified by preparative HPLC to obtain 5-fluoro-6-{[(4-methyl-1,3-thiazol-5-yl)methyl]oxy}-3,4-dihydro-2*H*-benzo[*b*][1,4]oxazin-3-one **(JD-5303054-4)** (60.0 mg, 203 µmol, yield: 10.6%) as a white solid.

LC-MS [M+H]⁺ = 295.0.

### Step 4: Preparation of 5-fluoro-6-1[(4-methyl-1,3-thiazol-5-yl)methyl]oxyl-3,4-dihydro-2H-benzo[b][1,4]oxazine (JD-5303054-5)

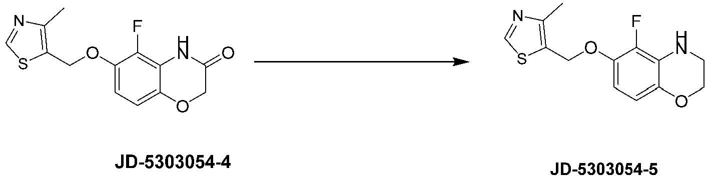

To a solution of 5-fluoro-6-{[(4-methyl-1,3-thiazol-5-yl)methyl]oxy}-3,4-dihydro-2*H*-benzo[*b*][1,4]oxazin-3-one **(JD-5303054-4)** (80.0 mg, 271 µmol, 1.00 eq) in THF (1.00 mL) was added BMS (10 M, 135 µL, 5.00 eq), and the mixture was stirred at 50°C for 8 hours. The reaction mixture was quenched with MeOH (5 mL) at 0°C and concentrated under reduced pressure to obtain a residue. The residue was purified by preparative HPLC to obtain 5-fluoro-6-{[(4-methyl-1,3-thiazol-5-yl)methyl]oxy}-3,4-dihydro-2*H*-benzo[*b*][1,4]oxazine **(JD-5303054-5)** (10.0 mg, 35.6 µmol, yield: 13.12%) as a white solid.

LC-MS [M+H]⁺ = 281.1.

### Step 5: Preparation of tert-butyl 3-{[(5-fluoro-6-{[(4-methyl-1,3-thiazol-5-yl)methyl]oxy}-3,4-dihydro-2H-benzo[b][1,4]oxazin-4-yl)carbonyl]amino}piperidine-1-carboxylate (JD-5303054-6)

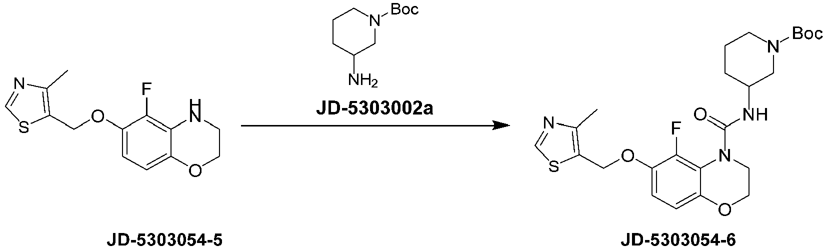

To a solution of 5-fluoro-6-{[(4-methyl-1,3-thiazol-5-yl)methyl]oxy}-3,4-dihydro-2*H*-benzo[*b*][1,4]oxazine (JD-5303054-5) (5.00 mg, 17.8 µmol, 1.00 eq) and *tert*-butyl 3-aminopiperidine-1-carboxylate **(JD-5303002a)** (10.7 mg, 53.5 µmol, 3.00 eq) in THF (1.00 mL) were added DIEA (4.61 mg, 35.6 µmol, 6.21 µL, 2.00 eq) and BTC (2.12 mg, 7.13 µmol, 0.40 eq). The mixture was stirred at 25°C for 12 hours. The reaction mixture was quenched with H₂O (10.0 mL) and extracted with EtOAc (10.0 mL). The organic phases were separated, filtered, and concentrated under reduced pressure to obtain *tert*-butyl 3-{[(5-fluoro-6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303054-6)** (15.0 mg, crude product) as a colorless oil.

LC-MS [M+H]⁺ = 507.2.

### Step 6: Preparation of 5-fluoro-N-(piperidin-3-yl)-6-{[(4-methyl-1,3-thiazol-5-yl)methyl]oxy}-3,4-dihydro-2H-benzo[b][1,4]oxazine-4-carboxamide (JD-5303054)

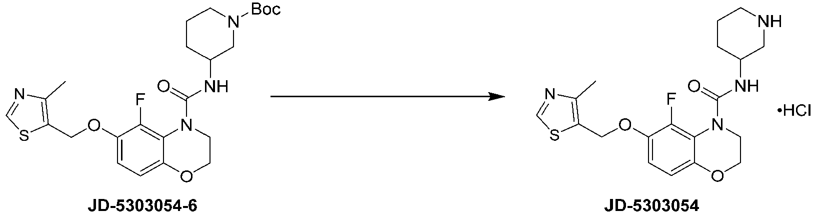

To a solution of *tert*-butyl 3-{[(5-fluoro-6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303054-6)** (15.0 mg, 29.6 µmol, 1.00 eq) in TFE (1.00 mL) was added TMSCl (6.43 mg, 59.2 µmol, 7.52 µL, 2.00 eq), and the mixture was stirred at 25°C for 2 hours. The reaction mixture was filtered and concentrated under reduced pressure to obtain a residue. The residue was purified by preparative HPLC to obtain 5-fluoro-*N*-(piperidin-3-yl)-6-{[(4-methyl-1,3-thiazol-5-yl)methyl]oxy}-3,4-dihydro-2*H*-benzo[*b*][1,4]oxazine-4-carboxamide **(JD-5303054)** (9.66 mg, 21.8 µmol, yield: 73.6%, purity: 99.64%, HCl) as a yellow solid.

LC-MS [M+H]⁺ = 407.2.

¹H NMR: (400 MHz, DMSO-*d*₆) δ ppm 1.42 - 1.71 (m, 2 H) 1.85 (br d, *J* = 10.51 Hz, 2 H) 2.37 (s, 3 H) 2.63 - 2.84 (m, 2 H) 3.12 - 3.30 (m, 2 H) 3.69 (br s, 1 H) 3.77 - 3.93 (m, 2 H) 4.12 (t, *J* = 4.38 Hz, 2 H) 5.25 (s, 2 H) 6.64 (dd, *J=* 9.19, 1.56 Hz, 1 H) 6.97 (t, *J* = 8.94 Hz, 1 H) 7.18 (d, *J=* 7.38 Hz, 1 H) 8.54 - 8.80 (m, 2 H) 8.99 (s, 1 H).

### Example 61: Preparation of N-(2-methylpyrazol-3-yl)-7-{[(4-methyl-1,3-thiazol-5-yl)methyl]oxy}-1,2,3,4-tetrahydroquinoline-1-carboxamide (JD-5303072)

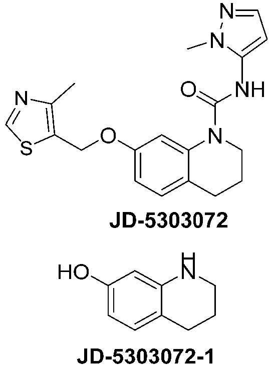

Following the synthetic method of Example 1, compound **JD-5303001-1** was replaced with **JD-5303072-1** and compound **JD-5303001d** was replaced with **JD-5303014a** to obtain *N*-(2-methylpyrazol-3-yl)-7-{[(4-methyl-1,3-thiazol-5-yl)methyl]oxy}-1,2,3,4-tetrahydroquinoline-1-carboxamide **(JD-5303072)** (6.8 mg, 27.28 mmol, yield: 81.39%) as a white solid.

LC-MS [389+H]⁺ = 384.0.

**¹H NMR:** (400 MHz, MeOD) δ 8.75 (s, 1 H), 7.39 (s, 1 H) 6.94 (d, *J* = 8.40 Hz, 1 H) 6.43 - 6.39 (m, 2 H) 6.21 (s, 1 H) 4.61 (d, *J=* 6.00 Hz, 4 H) 3.74 (s, 3 H) 2.70 - 2.76 (m, 2 H) 2.46 (s, 3 H) 1.93 - 2.00 (m, 2 H).

### Example 62: Preparation of N-[3-(3-hydroxypropyl)piperidin-3-yl]-6-{[(4-methyl-1,3-thiazol-5-yl)methyl]oxyl-3,4-dihydro-2H-benzo[b][1,4]oxazine-4-carboxamide formate (JD-5303073)

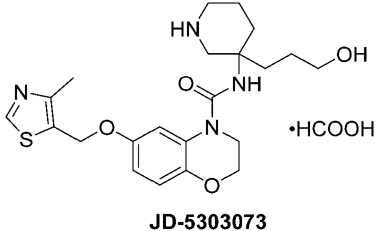

### Step 1: Preparation of tert-butyl 3-1[(6-1[(4-methyl-1,3-thiazol-5-yl)methyl]oxyl-3,4-dihydro-2H-benzo[b][1,4]oxazin-4-yl)carbonyl]amino}-3-(prop-2-en-1-yl)piperidine-1-carboxylate (JD-5303073-1)

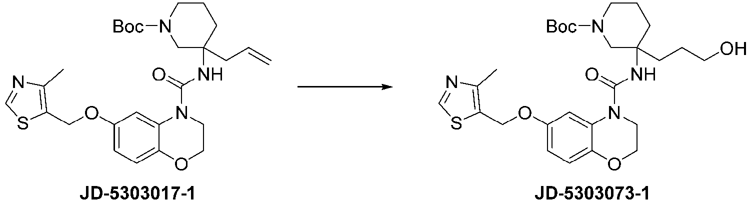

Intermediate *tert*-butyl 3-{[(6-{[(4-methyl-1,3-thiazol-5-yl)methyl]oxy}-3,4-dihydro-2*H*-benzo[*b*][1,4]oxazin-4-yl)carbonyl]amino}-3-(prop-2-en-1-yl)piperidine-1-carboxylate **(JD-5303017-1)** (60 mg, 0.113 mmol, 1.0 eq) of Example 17 was dissolved in THF (2 mL), then BH₃-DMS (0.17 mL, 0.339 mmol, 3.0 eq) (2 N) was added thereto at 0°C, and the mixture was stirred at room temperature for 12 hours. The reaction mixture was then cooled to 0°C, followed by the addition of NaOH (1.0 mL) (3 N), H₂O₂ (1.0 mL), and EtOH (1.0 mL), and the mixture was stirred at room temperature for 4 hours. The reaction mixture was quenched with water (5 mL) and extracted with EtOAc (20 mL, 10 mL * 2). The combined organic phases were washed with brine (10 mL), dried over anhydrous sodium sulfate, and concentrated under reduced pressure to obtain *tert*-butyl 3-{[(6-{[(4-methyl-1,3-thiazol-5-yl)methyl]oxy}-3,4-dihydro-2*H*-benzo[*b*][1,4]oxazin-4-yl)carbonyl]amino}-3-(prop-2-en-1-yl)piperidine-1-carboxylate **(JD-5303073-1)** (60 mg, crude product) as a white solid.

LC-MS ([M+H]⁺) = 547.5.

### Step 2: Preparation of N-[3-(3-hydroxypropyl)piperidin-3-yl]-6-{[(4-methyl-1,3-thiazol-5-yl)methyl]oxy}-3,4-dihydro-2H-benzo[b][1,4]oxazine-4-carboxamide formate (JD-5303073)

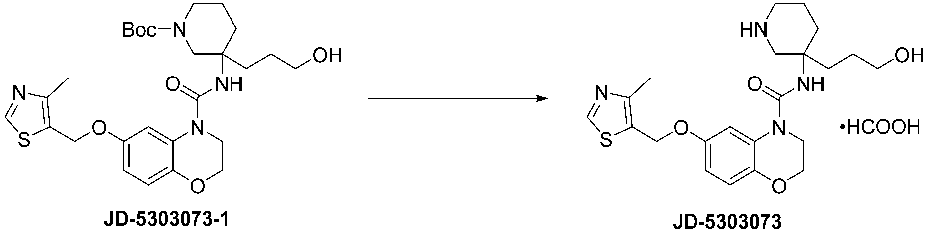

*tert*-Butyl 3-{[(6-{[(4-methyl-1,3-thiazol-5-yl)methyl]oxy}-3,4-dihydro-2*H-*benzo[*b*][1,4]oxazin-4-yl)carbonyl]amino}-3-(prop-2-en-1-yl)piperidine-1-carboxylate **(JD-5303073-1)** (60 mg, 0.110 mmol, 1.0 eq, crude product) was dissolved in DCM (2 mL), then TFA (0.6 mL) was added thereto, and the mixture was reacted at room temperature for 2 hours. The reaction mixture was concentrated under reduced pressure to obtain a residue. The residue was added with sodium hydroxide solution (15%) (5 mL) at room temperature, stirred at room temperature for 1 hour, and extracted with DCM (20 mL, 10 mL * 2). The combined organic phases were washed with brine (10 mL), dried over anhydrous sodium sulfate, and concentrated under reduced pressure to obtain a residue. The residue was purified by prep-HPLC (column: WelchUlC18 150 * 25 mm * 5 mm; mobile phase: [water (0.1% FA)-ACN]; B%: 25% to 35%, 6 minutes) to obtain *N*-[3-(3-hydroxypropyl)piperidin-3-yl]-6-{[(4-methyl-1,3-thiazol-5-yl)methyl]oxy}-3,4-dihydro-2*H*-benzo[*b*][1,4]oxazine-4-carboxamide formate **(JD-5303073)** (11.3 mg, two-step yield: 19.6%, purity: 98.6%) as a white solid.

LC-MS ([M-FA+H]⁺) = 447.3.

**¹H NMR** (400 MHz, MeOD) δ = 8.88 (s, 1H), 8.38 (brs, 1.34H), 7.30 - 7.25 (m, 1H), 6.82 (d, *J* = 8.8 Hz, 1H), 6.72 - 6.67 (m, 1H), 5.19 (s, 2H), 4.29 - 4.14 (m, 3H), 3.95 - 3.86 (m, 1H), 3.74 - 3.64 (m, 1H), 3.59 - 3.53 (m, 2H), 3.36 - 3.31 (m, 1H), 3.07 - 2.92 (m, 2H), 2.44 (s, 3H), 2.18 - 2.05 (m, 2H), 2.01 - 1.80 (m, 2H), 1.66 - 1.44 (m, 4H).

### Example 63: Preparation of 3-{[(6-{[(4-methyl-1,3-thiazol-5-yl)methyl]oxy}-3,4-dihydro-2H-benzo[b][1,4]oxazin-4-yl)carbonyl]amino}piperidine-3-carboxamide (JD-5303074)

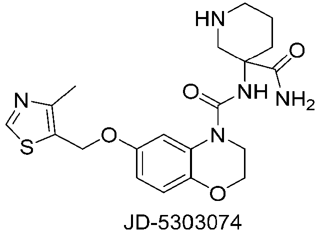

### Step 1: Preparation of benzyl 2,4-dioxo-1,3,7-triazaspiro[4.5]decane-7-carboxylate (JD-5303074-2)

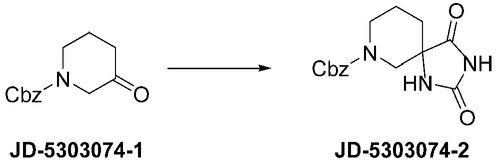

Benzyl 3-oxopiperidine-1-carboxylate **(JD-5303074-1)** (3.0 g, 12.86 mmol, 1.0 eq) was dissolved in MeOH (22.5 mL) and H₂O (22.5 mL), followed by the addition of (NH₄)₂CO₃ (2.6 g, 27.0 mmol, 2.1 eq) and TMSCN (2.55 g, 25.72 mmol, 2.0 eq), and the mixture was reacted at 80°C for 15 hours. The reaction mixture was concentrated under reduced pressure to obtain a residue. The residue was filtered, and the filter cake was collected to obtain benzyl 2,4-dioxo-1,3,7-triazaspiro[4.5]decane-7-carboxylate **(JD-5303074-2)** (3.4 g, yield: 87.1%, purity: 92%) as a yellow solid.

LC-MS ([M+H]⁺) = 304.4.

**¹H NMR** (400 MHz, DMSO-*d*₆) δ = 10.71 (s, 1H), 8.52 (d, *J* = 21.6 Hz, 1H), 7.45 - 7.26 (m, 5H), 5.17 - 5.00 (m, 2H), 3.90 - 3.60 (m, 2H), 3.30 - 3.11 (m, 1H), 3.05 (dd, *J* = 13.2, 6.8 Hz, 1H), 1.93 - 1.76 (m, 1H), 1.76 - 1.56 (m, 3H).

### Step 2: Preparation of tert-butyl 7-[(benzyloxy)carbonyl]-1-{[(2-methylprop-2-yl)oxy]carbonyl}-2,4-dioxo-1,3,7-triazaspiro[4.5]decane-3-carboxylate (JD-5303074-3)

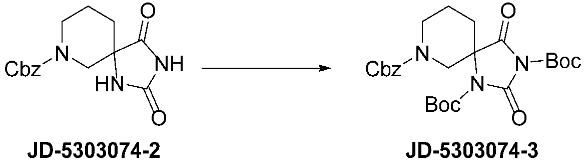

Benzyl 2,4-dioxo-1,3,7-triazaspiro[4.5]decane-7-carboxylate **(JD-5303074-2)** (2.2 g, 7.25 mmol, 1.0 eq) was dissolved in DME (70.0 mL), followed by the addition of TEA (734 mg, 7.25 mmol, 1.0 eq), (Boc)₂O (6.33 g, 29.0 mmol, 4.0 eq), and DMAP (89 mg, 0.725 mmol, 0.1 eq), and the mixture was reacted at room temperature for 15 hours. The reaction mixture was concentrated under reduced pressure to obtain a residue. The residue was purified by column chromatography (SiO₂, petroleum ether/ethyl acetate = 5/1 to 3/1) to obtain *tert*-butyl 7-[(benzyloxy)carbonyl]-1-{[(2-methylprop-2-yl)oxy]carbonyl}-2,4-dioxo-1,3,7-triazaspiro[4.5]decane-3-carboxylate **(JD-5303074-3)** (1.5 g, yield: 41.0%, purity: 94%) as a white solid.

LC-MS ([M+H₂O]⁺) = 521.3.

**¹H NMR** (400 MHz, CDCl₃)

δ = 7.39 - 7.27 (m, 5H), 5.18 - 4.95 (m, 2H), 4.42 - 4.29 (m, 1H), 4.20 (d, *J* = 13.6 Hz, 1H), 3.96 - 3.77 (m, 1H), 2.92 - 2.59 (m, 2H), 2.41 - 2.25 (m, 1H), 1.94 (d, *J* = 13.2 Hz, 1H), 1.74 - 1.66 (m, 1H), 1.59 - 1.52 (m, 18H).

### Step 3: Preparation of 3-amino-1-[(benzyloxy)carbonyl]piperidine-3-carboxylic acid (JD-5303074-4)

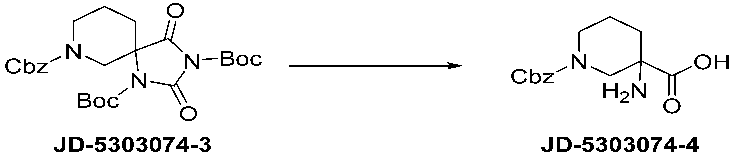

*tert*-Butyl 7-[(benzyloxy)carbonyl]-1-{[(2-methylprop-2-yl)oxy]carbonyl}-2,4-dioxo-1,3,7-triazaspiro[4.5]decane-3-carboxylate **(JD-5303074-3)** (1.5 g, 2.98 mmol, 1.0 eq) was dissolved in THF (15.0 mL) and H₂O (24.0 mL), followed by the addition of LiOH (1.0 g, 23.83 mmol, 8.0 eq), and the mixture was reacted at room temperature for 15 hours. The reaction mixture was added with saturated KHSO₄ solution at room temperature to adjust the pH to 3, and extracted with ethyl acetate (20 mL, 10.0 mL * 2). The combined organic phases were washed with brine (100 mL), dried over anhydrous sodium sulfate, and concentrated under reduced pressure to obtain a residue. The residue was filtered (dichloromethane/methanol = 10/1 (30 mL, 10.0 mL * 3)), and the collected filtrate was concentrated under reduced pressure to obtain 3-amino-1-[(benzyloxy)carbonyl]piperidine-3-carboxylic acid **(JD-5303074-4)** (1.0 g, crude product) as a white solid.

LC-MS ([M+H]⁺) = 279.3.

### Step 4: Preparation of methyl 3-amino-1-[(benzyloxy)carbonyl]piperidine-3-carboxylate (JD-5303074-5)

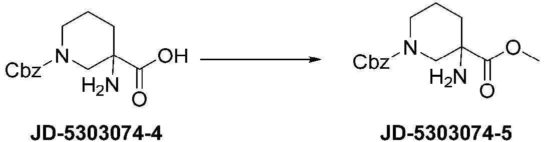

3-Amino-1-[(benzyloxy)carbonyl]piperidine-3-carboxylic acid **(JD-5303074-4)** (1.0 g, crude product, 1.0 eq) was dissolved in MeOH (8.0 mL), followed by the addition of MeSO₃H (2.0 mL), and the mixture was reacted at 65°C for 15 hours. The reaction mixture was concentrated under reduced pressure, then added with saturated sodium carbonate solution to adjust the pH to 9, and extracted with dichloromethane/methanol (10/1) (30 mL, 10.0 mL * 3). The combined organic phases were washed with brine (10 mL), dried over anhydrous sodium sulfate, and concentrated under reduced pressure to obtain methyl 3-amino-1-[(benzyloxy)carbonyl]piperidine-3-carboxylate **(JD-5303074-5)** (400 mg, two-step yield: 45.9%, purity: 92%) as a white solid.

LC-MS ([M+H]⁺) = 293.1.

### Step 5: Preparation of benzyl 3-(methoxycarbonyl)-3-{[(6-{[(4-methyl-1,3-thiazol-5-yl)methyl]oxy}-3,4-dihydro-2H-benzo[b][1,4]oxazin-4-yl)carbonyl]amino}piperidine-1-carboxylate (JD-5303074-6)

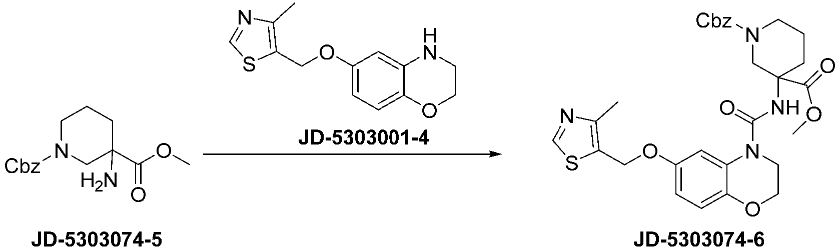

6-{[(4-Methyl-1,3-thiazol-5-yl)methyl]oxy}-3,4-dihydro-2*H*-benzo[*b*][1,4]oxazine **(JD-5303001-4)** (310 mg, 1.18 mmol, 1.0 eq) and TEA (598 mg, 5.91 mmol, 5.0 eq) were dissolved in DCM (3.0 mL), followed by the addition of (COCl₂)₃ (175 mg, 0.59 mmol, 0.5 eq), and the mixture was reacted at room temperature for 0.5 hours. A solution of methyl 3-amino-1-[(benzyloxy)carbonyl]piperidine-3-carboxylate **(JD-5303074-5)** (380 mg, 1.30 mmol, 1.1 eq) in DCM (1 mL) was then added thereto, and the mixture was stirred at room temperature for 48 hours. The reaction system was quenched with water (10 mL) and extracted with DCM (20 mL, 10 mL * 2). The combined organic phases were washed with brine (10 mL), dried over anhydrous sodium sulfate, and concentrated under reduced pressure to obtain a residue. The residue was purified by column chromatography (SiO₂, dichloromethane/methanol = 150/1 to 100/1) to obtain benzyl 3-(methoxycarbonyl)-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303074-6)** (350 mg, yield: 51.0%, purity: 92%) as a yellow solid.

LC-MS ([M+H]⁺) = 581.2.

### Step 6: Preparation of 1-[(benzyloxy)carbonyl]-3-{[(6-{[(4-methyl-1,3-thiazol-5-yl)methyl]oxy}-3,4-dihydro-2H-benzo[b][1,4]oxazin-4-yl)carbonyl]amino}piperidine-3-carboxylic acid (JD-5303074-7)

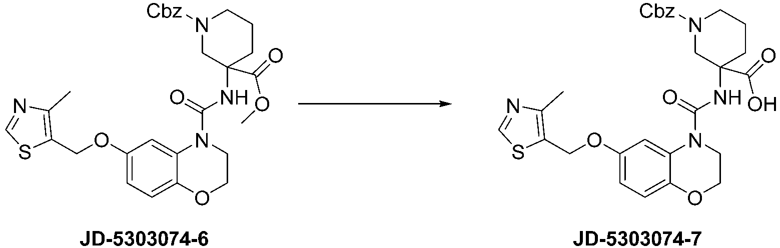

Benzyl 3-(methoxycarbonyl)-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303074-6)** (100 mg, 0.17 mmol, 1.0 eq) was dissolved in MeOH (1.0 mL), THF (1 mL), and H₂O (1 mL), followed by the addition of LiOH (22 mg, 0.52 mmol, 3.0 eq), and the mixture was reacted at room temperature for 15 hours. The reaction system was added with saturated KHSO₄ solution to adjust the pH to 3, and extracted with DCM (20 mL, 10 mL * 2). The combined organic phases were washed with brine (10 mL), dried over anhydrous sodium sulfate, and concentrated under reduced pressure to obtain 1-[(benzyloxy)carbonyl]-3-{[(6-{[(4-methyl-1,3-thiazol-5-yl)methyl]oxy}-3,4-dihydro-2*H*-benzo[*b*][1,4]oxazin-4-yl)carbonyl] amino }piperidine-3-carboxylic acid **(JD-5303074-7)** (90 mg, crude product) as a yellow solid.

**LC-MS** ([M+H]⁺) = 567.2.

### Step 7: Preparation of benzyl 3-carbamoyl-3-{[(6-{[(4-methyl-1,3-thiazol-5-yl)methyl]oxy}-3,4-dihydro-2H-benzo[b][1,4]oxazin-4-yl)carbonyl]amino}piperidine-1-carboxylate (JD-5303074-8)

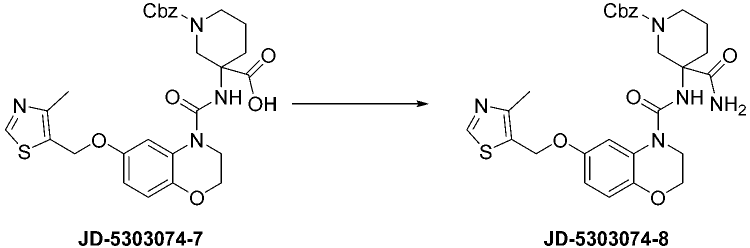

1-[(Benzyloxy)carbonyl]-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-3-carboxylic acid **(JD-5303074-7)** (90 mg, 0.16 mmol, 1.0 eq) was dissolved in DCM (3 mL), followed by the addition of DIEA (62 mg, 0.48 mmol, 3.0 eq), HATU (73 mg, 0.2 mmol, 1.2 eq), DMAP (2 mg, 0.016 mmol, 0.1 eq), and NH₄Cl (13 mg, 0.24 mmol, 1.5 eq), and the mixture was reacted at room temperature for 3 hours. The reaction system was quenched with water (10 mL) and extracted with DCM (20 mL, 10 mL * 2). The combined organic phases were washed with brine (10 mL), dried over anhydrous sodium sulfate, and concentrated under reduced pressure to obtain a residue. The residue was purified by prep-TLC (dichloromethane/methanol = 15/1) to obtain benzyl 3-carbamoyl-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303074-8)** (50 mg, two-step yield: 51.3%, purity: 93%) as a yellow solid.

**LC-MS** ([M+H]⁺) = 565.9.

### Step 7: Preparation of 3-{[(6-{[(4-methyl-1,3-thiazol-5-yl)methyl]oxy}-3,4-dihydro-2H-benzo[b][1,4]oxazin-4-yl)carbonyl]amino}piperidine-3-carboxamide (JD-5303074)

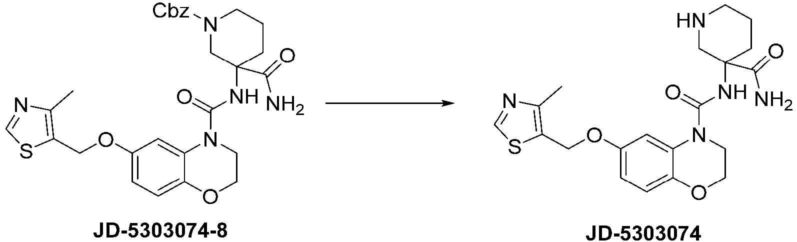

Benzyl 3-carbamoyl-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303074-8)** (45 mg, 0.08 mmol, 1.0 eq) was dissolved in DCM (2 mL), then TMSI (48 mg, 0.24 mmol, 3.0 eq) was added thereto, and the mixture was reacted at room temperature for 4 hours. The reaction mixture was quenched with water (0.5 mL), then added with PTSA (10 mg), and stirred for 5 minutes. The reaction mixture was then added with saturated sodium bicarbonate solution to adjust the pH to 9, and extracted with DCM (15 mL, 5 mL * 2). The combined organic phases were washed with brine (5 mL), dried over anhydrous sodium sulfate, and concentrated under reduced pressure to obtain a residue. The residue was purified by prep-HPLC (column: WelchUlC18 150 * 25 mm * 5 mm; mobile phase: [water (0.1% H₃PO₄)-ACN]; B%: 25% to 45%, 6 minutes) to obtain a residue, which was then added with sodium hydroxide solution (15%) to adjust the pH to 9, and extracted with DCM (15 mL, 5 mL * 2). The combined organic phases were washed with brine (5 mL), dried over anhydrous sodium sulfate, and concentrated under reduced pressure to obtain 3-{[(6-{[(4-methyl-1,3-thiazol-5-yl)methyl]oxy}-3,4-dihydro-2*H*-benzo[*b*][1,4]oxazin-4-yl)carbonyl]amino}piperidine-3-carboxamide **(JD-5303074)** (3.5 mg, yield: 10.1%, purity: 95.2%) as a white solid.

LC-MS ([M+H]⁺) = 432.3.

**¹H NMR** (400 MHz, MeOD) δ = 8.88 (s, 1H), 7.39 (d, *J* = 2.8 Hz, 1H), 6.82 (d,*J* = 8.8 Hz, 1H), 6.68 (dd, *J* = 8.8, 2.8 Hz, 1H), 5.21 (s, 2H), 4.20 (t, *J* = 4.0 Hz, 2H), 3.83 - 3.76 (m, 2H), 3.40 (d, *J* = 12.4 Hz, 1H), 3.00 (d, *J* = 12.8 Hz, 1H), 2.82 (q, *J* = 5.0 Hz, 2H), 2.44 (s, 3H), 2.09 - 2.02 (m, 1H), 2.00 - 1.92 (m, 1H), 1.71 - 1.63 (m, 2H).

### Example 64: Preparation of N-[3-(hydroxymethyl)piperidin-3-yl]-6-{[(4-methyl-1,3-thiazol-5-yl)methyl]oxy}-3,4-dihydro-2H-benzo[b][1,4]oxazine-4-carboxamide trifluoroacetate (JD-5303075)

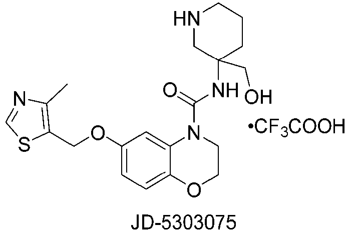

Following the synthetic method of Example 63: Step 1: Preparation of benzyl 2,4-dioxo-1,3,7-triazaspiro[4.5]decane-7-carboxylate **(JD-5303074-2)**

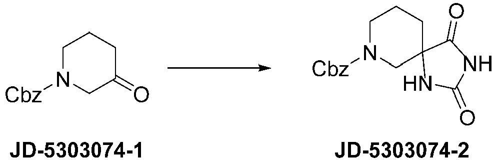

Benzyl 3-oxopiperidine-1-carboxylate **(JD-5303074-1)** (3.0 g, 12.86 mmol, 1.0 eq) was dissolved in MeOH (22.5 mL) and H₂O (22.5 mL), followed by the addition of (NH₄)₂CO₃ (2.6 g, 27.0 mmol, 2.1 eq) and TMSCN (2.55 g, 25.72 mmol, 2.0 eq), and the mixture was reacted at 80°C for 15 hours. The reaction mixture was concentrated under reduced pressure to obtain a residue. The residue was filtered, and the filter cake was collected to obtain benzyl 2,4-dioxo-1,3,7-triazaspiro[4.5]decane-7-carboxylate **(JD-5303074-2)** (3.4 g, yield: 87.1%, purity: 92%) as a yellow solid.

LC-MS ([M+H]⁺) = 304.4.

**¹H NMR** (400 MHz, DMSO-*d*₆) δ = 10.71 (s, 1H), 8.52 (d, *J* = 21.6 Hz, 1H), 7.45 - 7.26 (m, 5H), 5.17 - 5.00 (m, 2H), 3.90 - 3.60 (m, 2H), 3.30 - 3.11 (m, 1H), 3.05 (dd, *J* = 13.2, 6.8 Hz, 1H), 1.93 - 1.76 (m, 1H), 1.76 - 1.56 (m, 3H).

### Step 2: Preparation of tert-butyl 7-[(benzyloxy)carbonyl]-1-{[(2-methylprop-2-yl)oxy]carbonyl}-2,4-dioxo-1,3,7-triazaspiro[4.5]decane-3-carboxylate (JD-5303074-3)

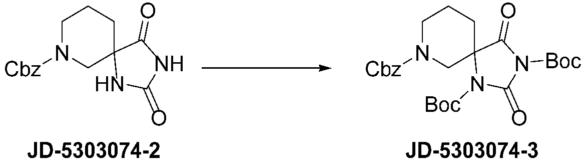

Benzyl 2,4-dioxo-1,3,7-triazaspiro[4.5]decane-7-carboxylate **(JD-5303074-2)** (2.2 g, 7.25 mmol, 1.0 eq) was dissolved in DME (70.0 mL), followed by the addition of TEA (734 mg, 7.25 mmol, 1.0 eq), (Boc)₂O (6.33 g, 29.0 mmol, 4.0 eq), and DMAP (89 mg, 0.725 mmol, 0.1 eq), and the mixture was reacted at room temperature for 15 hours. The reaction mixture was concentrated under reduced pressure to obtain a residue. The residue was purified by column chromatography (SiO₂, petroleum ether/ethyl acetate = 5/1 to 3/1) to obtain *tert-butyl* 7-[(benzyloxy)carbonyl]-1-{[(2-methylprop-2-yl)oxy]carbonyl}-2,4-dioxo-1,3,7-triazaspiro[4.5]decane-3-carboxylate **(JD-5303074-3)** (1.5 g, yield: 41.0%, purity: 94%) as a white solid.

LC-MS ([M+H₂O]⁺) = 521.3.

**¹H NMR** (400 MHz, CDCl₃)

δ = 7.39 - 7.27 (m, 5H), 5.18 - 4.95 (m, 2H), 4.42 - 4.29 (m, 1H), 4.20 (d, *J*= 13.6 Hz, 1H), 3.96 - 3.77 (m, 1H), 2.92 - 2.59 (m, 2H), 2.41 - 2.25 (m, 1H), 1.94 (d, *J* = 13.2 Hz, 1H), 1.74 - 1.66 (m, 1H), 1.59 - 1.52 (m, 18H).

### Step 3: Preparation of 3-amino-1-[(benzyloxy)carbonyl]piperidine-3-carboxylic acid (JD-5303074-4)

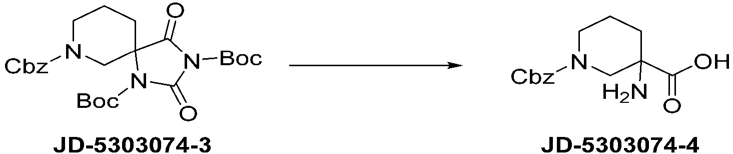

*tert-Butyl* 7-[(benzyloxy)carbonyl]-1-{[(2-methylprop-2-yl)oxy]carbonyl}-2,4-dioxo-1,3,7-triazaspiro[4.5]decane-3-carboxylate **(JD-5303074-3)** (1.5 g, 2.98 mmol, 1.0 eq) was dissolved in THF (15.0 mL) and H₂O (24.0 mL), followed by the addition of LiOH (1.0 g, 23.83 mmol, 8.0 eq), and the mixture was reacted at room temperature for 15 hours. The reaction mixture was added with saturated KHSO₄ solution at room temperature to adjust the pH to 3, and extracted with ethyl acetate (20 mL, 10.0 mL * 2). The combined organic phases were washed with brine (100 mL), dried over anhydrous sodium sulfate, and concentrated under reduced pressure to obtain a residue. The residue was filtered (dichloromethane/methanol = 10/1 (30 mL, 10.0 mL * 3)), and the collected filtrate was concentrated under reduced pressure to obtain 3-amino-1-[(benzyloxy)carbonyl]piperidine-3-carboxylic acid **(JD-5303074-4)** (1.0 g, crude product) as a white solid.

LC-MS ([M+H]⁺) = 279.3.

### Step 4: Preparation of methyl 3-amino-1-[(benzyloxy)carbonyl]piperidine-3-carboxylate (JD-5303074-5)

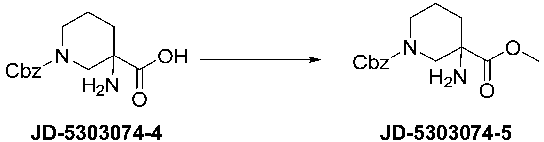

3-Amino-1-[(benzyloxy)carbonyl]piperidine-3-carboxylic acid (JD-5303074-4) (1.0 g, crude product, 1.0 eq) was dissolved in MeOH (8.0 mL), followed by the addition of MeSO₃H (2.0 mL), and the mixture was reacted at 65°C for 15 hours. The reaction mixture was concentrated under reduced pressure, then added with saturated sodium carbonate solution to adjust the pH to 9, and extracted with dichloromethane/methanol (10/1) (30 mL, 10.0 mL * 3). The combined organic phases were washed with brine (10 mL), dried over anhydrous sodium sulfate, and concentrated under reduced pressure to obtain methyl 3-amino-1-[(benzyloxy)carbonyl]piperidine-3-carboxylate **(JD-5303074-5)** (400 mg, two-step yield: 45.9%, purity: 92%) as a white solid.

LC-MS ([M+H]⁺) = 293.1.

Following the synthetic method of Example 1: Step 5: Preparation of 5-(chloromethyl)-4-methyl-1,3-thiazole **(JD-5303001c)**

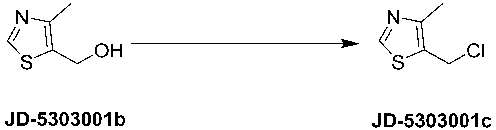

(4-Methyl-1,3-thiazol-5-yl)methanol **(JD-5303001b)** (400 mg, 3.10 mmol, 1.00 eq) and SOCl₂ were dissolved in THF (25.0 mL), and the reaction mixture was stirred at 25°C for 1 hour. The reaction mixture was quenched with H₂O (30.0 mL) and extracted with EtOAc (30.0 mL). The combined organic phases were separated, filtered, and concentrated under reduced pressure to obtain 5-(chloromethyl)-4-methyl-1,3-thiazole **(JD-5303001c)** (500 mg, crude product) as a colorless oil.

### Step 6: Preparation of 6-{[(4-methyl-1,3-thiazol-5-yl)methyl]oxy}-3,4-dihydro-2H-benzo[b][1,4]oxazine (JD-5303001-4)

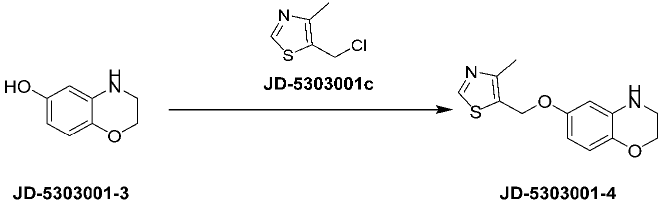

3,4-Dihydro-2*H*-benzo[*b*][1,4]oxazin-6-ol **(JD-5303001-3)** (0.20 g, 1.32 mmol, 1.00 eq) was dissolved in DMF (2 mL), followed by the addition of K₂CO₃ (365 mg, 2.65 mmol, 2.00 eq) and 5-(chloromethyl)-4-methyl-1,3-thiazole **(JD-5303001c)** (292 mg, 1.98 mmol, 1.50 eq). The reaction mixture was stirred at 80°C for 16 hours. The reaction mixture was washed with H₂O (10 mL) and EtOAc (10 mL, 5 mL * 2). The combined organic phases were concentrated under reduced pressure to obtain a residue. The residue was purified by preparative TLC (SiO₂, petroleum ether/ethyl acetate = 1:1) to obtain 6-{[(4-methyl-1,3-thiazol-5-yl)methyl]oxy}-3,4-dihydro-2*H*-benzo[*b*][1,4]oxazine **(JD-5303001-4)** (0.12 g, 457 µmol, yield: 34.5%) as a colorless oil.

LC-MS [M+H]⁺ = 263.1.

### Step 7: Preparation of benzyl 3-(methoxycarbonyl)-3-{[(6-{[(4-methyl-1,3-thiazol-5-yl)methyl]oxy}-3,4-dihydro-2H-benzo[b][1,4]oxazin-4-yl)carbonyl]amino}piperidine-1-carboxylate (JD-5303074-6)

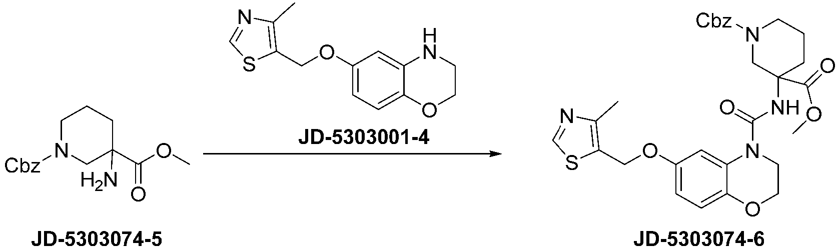

6-{[(4-Methyl-1,3-thiazol-5-yl)methyl]oxy}-3,4-dihydro-2*H*-benzo[*b*][1,4]oxazine **(JD-5303001-4)** (310 mg, 1.18 mmol, 1.0 eq) and TEA (598 mg, 5.91 mmol, 5.0 eq) were dissolved in DCM (3.0 mL), followed by the addition of (COCl₂)₃ (175 mg, 0.59 mmol, 0.5 eq), and the mixture was reacted at room temperature for 0.5 hours. A solution of methyl 3-amino-1-[(benzyloxy)carbonyl]piperidine-3-carboxylate **(JD-5303074-5)** (380 mg, 1.30 mmol, 1.1 eq) in DCM (1 mL) was then added thereto, and the mixture was stirred at room temperature for 48 hours. The reaction system was quenched with water (10 mL) and extracted with DCM (20 mL, 10 mL * 2). The combined organic phases were washed with brine (10 mL), dried over anhydrous sodium sulfate, and concentrated under reduced pressure to obtain a residue. The residue was purified by column chromatography (SiO₂, dichloromethane/methanol = 150/1 to 100/1) to obtain benzyl 3-(methoxycarbonyl)-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303074-6)** (350 mg, yield: 51.0%, purity: 92%) as a yellow solid.

LC-MS ([M+H]⁺) = 581.2.

### Step 8: Preparation of methyl 3-{[(6-{[(4-methyl-1,3-thiazol-5-yl)methyl]oxy}-3,4-dihydro-2H-benzo[b][1,4]oxazin-4-yl)carbonyl]amino}piperidine-3-carboxylate (JD-5303075-1)

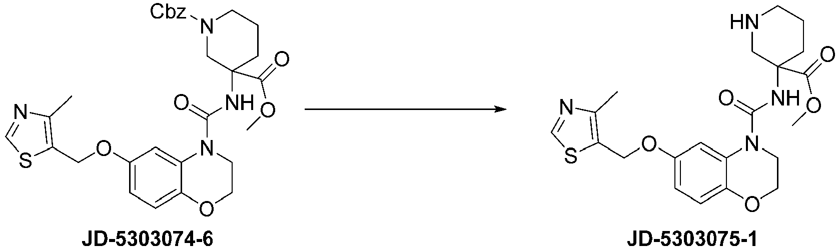

Benzyl 3-(methoxycarbonyl)-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate **(JD-5303074-6)** (150 mg, 0.258 mmol, 1.2 eq) was dissolved in DCM (2 mL), then TMSI (155 mg, 0.774 mmol, 3.0 eq) was added thereto, and the mixture was stirred at room temperature for 3 hours. The reaction mixture was quenched with water (5 mL) and extracted with DCM (10 mL, 5 mL * 2). The aqueous phase was then added with saturated sodium bicarbonate solution to adjust the pH to 9, and extracted with DCM/MeOH (8/1) (27 mL, 9 mL * 3). The combined organic phases were washed with brine (10 mL), dried over anhydrous sodium sulfate, and concentrated under reduced pressure to obtain methyl 3-{[(6-{[(4-methyl-1,3-thiazol-5-yl)methyl]oxy}-3,4-dihydro-2*H*-benzo[*b*][1,4]oxazin-4-yl)carbonyl] amino }piperidine-3-carboxylate **(JD-5303075-1)** (70 mg, yield: 60.8%, purity: 90%) as a yellow solid.

LC-MS ([M+H]⁺) = 447.4.

### Step 9: Preparation of N-[3-(hydroxymethyl)piperidin-3-yl]-6-{[(4-methyl-1,3-thiazol-5-yl)methyl]oxy}-3,4-dihydro-2H-benzo[b][1,4]oxazine-4-carboxamide trifluoroacetate (JD-5303075)

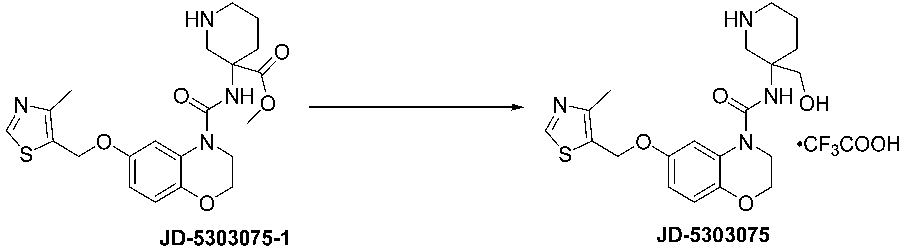

Methyl 3-{[(6-{[(4-methyl-1,3-thiazol-5-yl)methyl]oxy}-3,4-dihydro-2*H-*benzo[*b*][1,4]oxazin-4-yl)carbonyl]amino}piperidine-3-carboxylate **(JD-5303075-1)** (70 mg, 0.157 mmol, 1.0 eq) was dissolved in THF (2 mL), and the mixture was cooled to 0°C. LiAlH₄ (1.0 M in THF) (0.314 mL, 0.314 mmol, 2.0 eq) was then added thereto, and the mixture was reacted at 0°C for 1 hour. The reaction mixture was quenched by dropwise addition of NaOH solution (15%) (0.1 mL) at 0°C and dried over Na₂SO₄. The mixture was filtered, and the collected filtrate was concentrated under reduced pressure to obtain a residue. The residue was purified by prep-HPLC (column: WelchUlC18 150 * 25 mm * 5 mm; mobile phase: [water (0.1% TFA)-ACN]; B%: 25% to 45%, 6 minutes) to obtain *N*-[3-(hydroxymethyl)piperidin-3-yl]-6-{[(4-methyl-1,3-thiazol-5-yl)methyl]oxy}-3,4-dihydro-2*H-*benzo[b][1,4]oxazine-4-carboxamide trifluoroacetate **(JD-5303075)** (20.7 mg, yield: 31.5%, purity: 95.7%) as a pink solid.

LC-MS ([M-TFA+H]⁺) = 419.2.

**¹H NMR** (300 MHz, DMSO-*d*₆) *δ* = 8.97 (s, 1H), 8.29 (d, *J* = 2.4 Hz, 1H), 7.36 (d, *J* = 2.7, Hz, 1H), 6.78 (d, *J* = 9.0 Hz, 1H), 6.62 (dd, *J* = 9.0, 3.0 Hz, 1H), 6.24 (d, *J* = 5.1 Hz, 1H), 5.17 (s, 2H), 4.21 - 4.08 (m, 2H), 3.81 - 3.71 (m, 1H), 3.71 - 3.61 (m, 2H), 3.49 - 3.33 (m, 2H), 3.05 - 2.89 (m, 1H), 2.76 - 2.52 (m, 2H), 2.39 (s, 3H), 2.17 - 2.08 (m, 1H), 1.66 - 1.37 (m, 3H).

Chiral resolution of JD-5303075: JD-5303075 was resolved into JD-5303075A and JD-5303075B using the following method.

Resolution method of JD-5303075

### 2.1 Instrumentation: Shimadzu HPLC (HPLC-Z)

Column: Chiralpak IC (50 * 4.6 mm, 3 µm)
Mobile phase: A: hexane (0.1% diethanolamine), B: isopropanol/methyl naphthyl chloride = 2/1
Gradient: 60% B
Flow rate: 1 mL/min
Back pressure: 100 bar
Column temperature: 35°C
Wavelength: 220 nm
Cycle time: approximately 7 minutes

2.2 Sample preparation: The compound was dissolved in approximately 150 mL of ethanol/hydroxyethyl cellulose with an injection volume of 12 mL per run.

Post-processing: The separated mixture was dried via rotary evaporation at 40°C to obtain enantiomers JD-5303075A and JD-5303075B.

MS and NMR of enantiomer JD-5303075A:
LC-MS ([M-TFA+H]⁺) = 419.2.

**¹H NMR** (300 MHz, DMSO-*d*₆) *δ* = 8.97 (s, 1H), 7.31 (d, *J* = 3.0 Hz, 1H), 6.80 (d, *J* = 9.0, Hz, 1H), 6.38 (d, *J* = 3.0 Hz, 1H), 6.35 (dd, *J* = 9.0, 3.0 Hz, 1H), 6.24 (d, *J* = 5.1 Hz, 1H), 5.17 (s, 2H), 4.21 - 4.08 (m, 2H), 3.81 - 3.71 (m, 1H), 3.71 - 3.61 (m, 2H), 3.75 - 3.69 (m, 2H), 3.67 - 3.31 (m, 1H), 2.96 - 2.75 (m, 2H), 2.50 (s, 3H), 2.51 - 2.39 (m, 1H), 1.42 - 1.29 (m, 3H).

MS and NMR of enantiomer JD-5303075B:
LC-MS ([M-TFA+H]⁺) = 419.2.

¹H NMR (300 MHz, DMSO-*d*₆) *δ* = 8.97 (s, 1H), 7.31 (d, *J=* 2.1 Hz, 1H), 6.79 (d, *J* = 6.9, Hz, 1H), 6.63 (d, *J* = 2.1 Hz, 1H), 6.35 (dd, *J* = 9.0, 3.0 Hz, 1H), 6.24 (d, *J* = 5.1 Hz, 1H), 5.17 (s, 2H), 4.15 - 4.11 (m, 2H), 3.81 - 3.71 (m, 1H), 3.71 - 3.61 (m, 2H), 3.75 - 3.68 (m, 2H), 3.67 - 3.31 (m, 1H), 2.94 - 2.75 (m, 2H), 2.50 (s, 3H), 2.44 - 2.38 (m, 1H), 1.40 - 1.29 (m, 3H).

### Example 65: Preparation of N-(4,4-difluorotetrahydro-1H-pyrrol-3-yl)-6-[(4-methyl-1,3-thiazol-5-yl)oxy]-3,4-dihydro-2H-benzo[b][1,4]oxazine-4-carboxamide hydrochloride (JD-5303076)

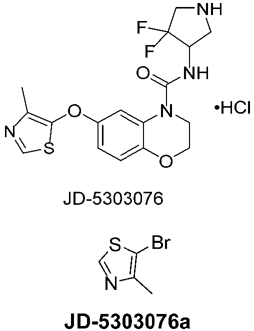

Following the synthetic method of Example 52, compound **JD-5303047a** was replaced with **JD-5303076a** to obtain *N*-(4,4-difluorotetrahydro-1*H*-pyrrol-3-yl)-6-[(4-methyl-1,3-thiazol-5-yl)oxy]-3,4-dihydro-2*H*-benzo[*b*][1,4]oxazine-4-carboxamide hydrochloride **(JD-5303076)** (14.6 mg, 36.9 µmol, yield: 45.8%, purity: 96.49%) as an off-white solid.

LC-MS [396+1]⁺ = 397.

**¹H NMR:** (400 MHz, DMSO-*d*₆) δ = 9.59 - 10.24 (m, 2 H), 8.68 (s, 1 H), 7.45 - 7.68 (m, 2 H), 6.87 (d, *J* = 8.88 Hz, 1 H), 6.68 (dd, *J* = 8.88, 3.00 Hz, 1 H), 4.69 - 4.89 (m, 1 H), 4.17 - 4.27 (m, 2 H), 3.64 - 3.88 (m, 5 H), 3.35 - 3.46 (m, 1 H), 2.22 (s, 3 H).

### Example 66: Preparation of 6-[(3-cyclopropylphenyl)oxy]-N-(4,4-difluorotetrahydro-1H-pyrrol-3-yl)-3,4-dihydro-2H-benzo[b][1,4]oxazine-4-carboxamide hydrochloride (JD-5303077)

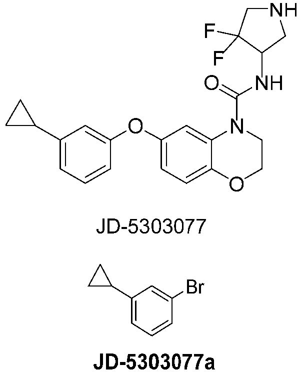

Following the synthetic method of Example 52, compound **JD-5303047a** was replaced with **JD-5303077a** to obtain 6-[(3-cyclopropylphenyl)oxy]-N-(4,4-difluorotetrahydro-1*H*-pyrrol-3-yl)-3,4-dihydro-2*H*-benzo[*b*][1,4]oxazine-4-carboxamide hydrochloride **(JD-5303077)** (3.84 mg, 9.24 µmol, yield: 47.7%, purity: 100.00%) as an off-white solid.

LC-MS [415+H]⁺ = 416.

¹H NMR: (400 MHz, DMSO-*d*₆) δ = 0.64 (dd, *J* = 4.94, 2.06 Hz, 2 H), 0.89 - 0.99 (m, 2 H), 1.23 (s, 1 H) 1.83 - 1.91 (m, 1 H), 3.62 - 3.71 (m, 2 H), 3.71 - 3.79 (m, 2 H), 3.82 (br dd, *J* = 5.44, 3.19 Hz, 1 H), 4.13 - 4.27 (m, 2 H), 4.67 - 4.81 (m, 1 H), 6.62 - 6.71 (m, 3 H), 6.76 (d, *J=* 7.75 Hz, 1 H), 6.88 (d, *J=* 8.88 Hz, 1 H), 7.19 (t, *J=* 7.82 Hz, 1 H), 7.42 (d, *J* = 2.75 Hz, 1 H), 7.53 (d, *J=* 8.25 Hz, 1 H), 9.68 (br d, *J=* 2.00 Hz, 2 H).

### Example 67: Preparation of N-(piperidin-3-yl)-6-{methyl[(4-methyl-1,3-thiazol-5-yl)methyl]amino}-3,4-dihydro-2H-benzo[b][1,4]oxazine-4-carboxamide formate (JD-5303079)

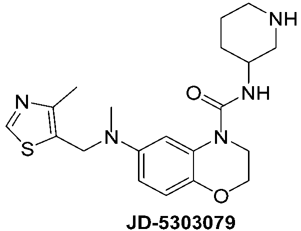

### Step 1: Preparation of tert-butyl 6-{methyl[(4-methyl-1,3-thiazol-5-yl)methyl]amino}-3,4-dihydro-2H-benzo[b][1,4]oxazine-4-carboxylate (JD-5303079-1)

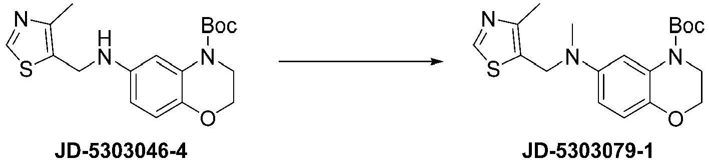

*tert*-Butyl 6-{[(4-methyl-1,3-thiazol-5-yl)methyl]amino}-3,4-dihydro-2*H-*benzo[*b*][1,4]oxazine-4-carboxylate **(JD-5303046-4)** (350 mg, 0.97 mmol, 1.0 eq) was dissolved in DMF (4.0 mL), then NaH (60%) (232 mg, 5.8 mmol, 6.0 eq) was added thereto at 0°C, and the mixture was stirred for 0.5 hours. Mel (824 mg, 5.8 mmol, 6.0 eq) was then added thereto, and the mixture was stirred at room temperature for 2.5 hours. The reaction mixture was quenched with saturated NH₄Cl solution (10 mL) at 0°C and extracted with EtOAc (30 mL, 10 mL * 3). The combined organic phases were washed with water (30 mL, 10 mL * 3) and brine (10 mL), dried over anhydrous sodium sulfate, and concentrated under reduced pressure to obtain *tert*-butyl 6-{methyl[(4-methyl-1,3-thiazol-5-yl)methyl]amino}-3,4-dihydro-2*H*-benzo[*b*][1,4]oxazine-4-carboxylate **(JD-5303079-1)** (350 mg, crude product) as a yellow solid.

LC-MS ([M+H]⁺) = 376.2.

### Step 2: Preparation of 6-{methyl[(4-methyl-1,3-thiazol-5-yl)methyl]amino}-3,4-dihydro-2H-benzo[b][1,4]oxazine (JD-5303079-2)

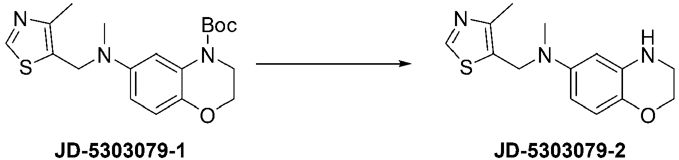

*tert*-Butyl 6- {methyl[(4-methyl-1,3-thiazol-5-yl)methyl]amino}-3,4-dihydro-2*H-*benzo[*b*][1,4]oxazine-4-carboxylate **(JD-5303079-1)** (350 mg, 0.93 mmol, 1.0 eq) was dissolved in DCM (3.0 mL), followed by the addition of dioxane/HCl (2.33 mL, 9.32 mmol, 10.0 eq) (4 N) at room temperature. The reaction mixture was concentrated under reduced pressure to obtain a residue. The residue was added with sodium hydroxide solution (15%) (3 mL) to adjust the pH to 10, and extracted with DCM (20 mL, 10 mL * 2). The combined organic phases were washed with brine (10 mL), dried over anhydrous sodium sulfate, and concentrated under reduced pressure to obtain 6-{methyl[(4-methyl-1,3-thiazol-5-yl)methyl]amino}-3,4-dihydro-2*H*-benzo[*b*][1,4]oxazine **(JD-5303079-2)** (200 mg, crude product) as a yellow solid.

LC-MS ([M+H]⁺) = 276.1.

### Step 3: Preparation of tert-butyl 3-{[(6-{methyl[(4-methyl-1,3-thiazol-5-yl)methyl]amino}-3,4-dihydro-2H-benzo[b][1,4]oxazin-4-yl)carbonyl]amino}piperidine-1-carboxylate (JD-5303079-3)

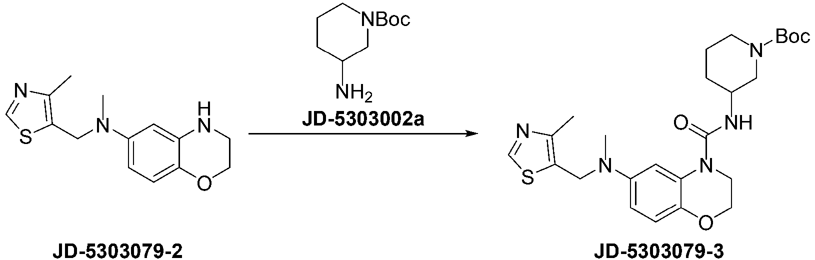

6-{Methyl[(4-methyl-1,3-thiazol-5-yl)methyl]amino}-3,4-dihydro-2*H-*benzo[*b*][1,4]oxazine **(JD-5303079-2)** (200 mg, 0.73 mmol, 1.0 eq) was dissolved in DCM (2.0 mL), followed by the addition of TEA (220 mg, 2.18 mmol, 3.0 eq) and (COCl₂)₃ (108 mg, 0.36 mmol, 0.5 eq), and the mixture was reacted at room temperature for 0.5 hours. A solution of 3A (174 mg, 0.87 mmol, 1.2 eq) in DCM (1.0 mL) was then added thereto, and the reaction system was stirred at room temperature for 11.5 hours. The reaction mixture was quenched with water (5 mL) at room temperature and extracted with dichloromethane (20 mL, 10.0 mL * 2). The combined organic phases were washed with brine (10 mL), dried over anhydrous sodium sulfate, and concentrated under reduced pressure to obtain a residue. The residue was purified by column chromatography (SiO₂, petroleum ether/ethyl acetate = 3/1 to 1/1) to obtain *tert*-butyl 3-{[(6-{methyl[(4-methyl-1,3-thiazol-5-yl)methyl]amino}-3,4-dihydro-2*H-*benzo[*b*][1,4]oxazin-4-yl)carbonyl]amino}piperidine-1-carboxylate **(JD-5303079-3)** (80 mg, three-step yield: 16.5%, purity: 93%) as a yellow solid.

LC-MS ([M+H]⁺) = 502.4.

**¹H NMR** (300 MHz, DMSO-*d*₆) *δ* = 8.80 (s, 1H), 7.03 (d, *J* = 3.0 Hz, 1H), 6.73 (d, *J* = 8.7 Hz, 1H), 6.62 - 6.55 (m, 1H), 6.53 (dd, *J* = 9.0, 2.7 Hz, 1H), 4.54 (s, 2H), 4.12 - 4.07 (m, 2H), 3.78 - 3.45 (m,7H), 2.93 - 2.76 (m, 3H), 2.38 (s, 3H), 1.84 - 1.56 (m, 4H), 1.37 (s, 9H).

### Step 4: Preparation of N-(piperidin-3-yl)-6-{methyl[(4-methyl-1,3-thiazol-5-yl)methyl]amino}-3,4-dihydro-2H-benzo[b][1,4]oxazine-4-carboxamide formate (JD-5303079)

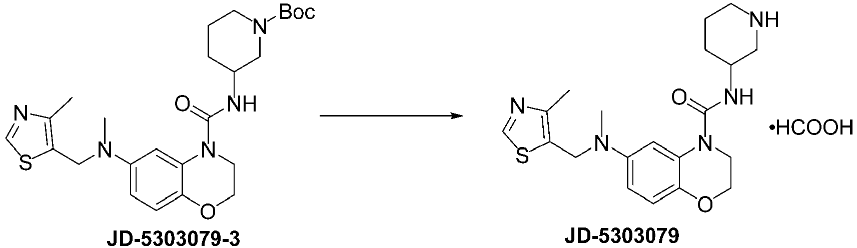

*tert*-Butyl 3-{[(6-{methyl[(4-methyl-1,3-thiazol-5-yl)methyl]amino}-3,4-dihydro-2*H*-benzo[*b*][1,4]oxazin-4-yl)carbonyl]amino}piperidine-1-carboxylate **(JD-5303079-3)** (80 mg, 0.16 mmol, 1.0 eq) was dissolved in DCM (3 mL), followed by the addition of dioxane/HCl (0.4 mL, 1.6 mmol, 10.0 eq, 4 N), and the mixture was reacted at room temperature for 2 hours. The reaction mixture was concentrated under reduced pressure to obtain a residue. The residue was added with sodium hydroxide solution (3 mL, 15%) to adjust the pH to 10, and extracted with DCM (10 mL * 2). The combined organic phases were washed with brine (10 mL), dried over anhydrous sodium sulfate, and concentrated under reduced pressure to obtain a residue. The residue was purified by prep-HPLC (column: WelchUlC18 150 * 25 mm * 5 mm; mobile phase: [water (0.1% FA)-ACN]; B%: 15% to 25%, 6 minutes) to obtain *N-*(piperidin-3-yl)-6-{methyl[(4-methyl-1,3-thiazol-5-yl)methyl]amino}-3,4-dihydro-2*H-*benzo[*b*][1,4]oxazine-4-carboxamide formate **(JD-5303079)** (25.4 mg, yield: 34.4%, purity: 98.4%) as a yellow solid.

LC-MS ([M-1.3FA+H]⁺) = 402.4.

**¹H NMR** (300 MHz, DMSO-*d*₆) δ = 8.80 (s, 1H), 8.30 (brs, 1H), 7.07 (d, *J* = 2.7 Hz, 1H), 6.82 (d, *J* = 7.2 Hz, 1H), 6.72 (d, *J* = 8.7 Hz, 1H), 6.52 (dd, *J* = 8.7, 2.7 Hz, 1H), 4.53 (s, 2H), 4.17 - 4.02 (m, 2H), 3.88 - 3.75 (m, 1H), 3.75 - 3.58 (m, 2H), 3.17 - 2.92 (m, 2H), 2.74 (s, 3H), 2.70 - 2.55 (m, 2H), 2.37 (s, 3H), 1.88 - 1.67 (m, 2H), 1.65 - 1.39 (m, 2H).

### Example 68: Preparation of N-[(1S,3S)-3-aminocyclohexyl]-6-{[(4-methyl-1,3-thiazol-5-yl)methyl]oxyl-3,4-dihydro-2H-benzo[b][1,4]oxazine-4-carboxamide (JD-5303080)

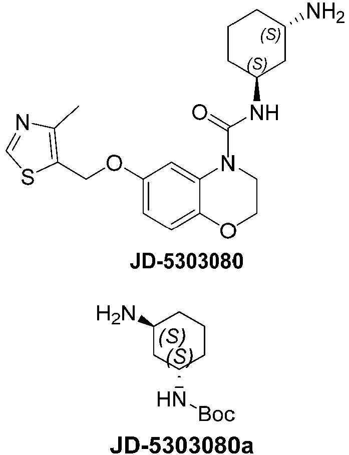

Following the synthetic method of Example 1, compound **JD-5303001d** was replaced with **JD-5303080a** to obtain *N*-[(1*S*,3*S*)-3-aminocyclohexyl]-6-{[(4-methyl-1,3-thiazol-5-yl)methyl]oxy}-3,4-dihydro-2*H*-benzo[*b*][1,4]oxazine-4-carboxamide **(JD-5303080)** (111 mg, 254 µmol, yield: 51.2%, purity: 99.92%, HCl) as an off-white solid.

LC-MS [415+H]⁺ = 403.2.

¹H NMR: (400 MHz, DMSO-*d*₆) δ ppm 1.21 - 1.42 (m, 2 H) 1.43 - 1.53 (m, 2 H) 1.62 (br s, 4 H) 2.38 (s, 3 H) 3.67 (br d, *J* = 4.63 Hz, 2 H) 3.81 - 3.99 (m, 2 H) 4.03 - 4.18 (m, 2 H) 5.16 (s, 2 H) 6.53 - 6.60 (m, 1 H) 6.61 - 6.72 (m, 1 H) 6.75 (d, *J=* 8.76 Hz, 1 H) 7.31 (d, *J*= 2.50 Hz, 1 H) 8.97 (s, 1 H).

### Example 69: Preparation of N-[(1R,3R)-3-aminocyclohexyl]-6-{[(4-methyl-1,3-thiazol-5-yl)methyl]oxy}-3,4-dihydro-2H-benzo[b][1,4]oxazine-4-carboxamide (JD-5303081)

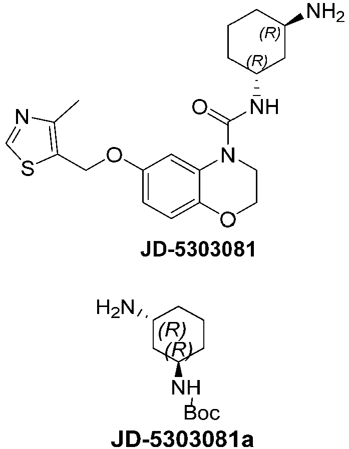

Following the synthetic method of Example 1, compound **JD-5303001d** was replaced with **JD-5303081a** to obtain *N*-[(1*R*,3*R*)-3-aminocyclohexyl]-6-{[(4-methyl-1,3-thiazol-5-yl)methyl]oxy}-3,4-dihydro-2*H*-benzo[*b*][1,4]oxazine-4-carboxamide **(JD-5303081)** (111 mg, 254 µmol, yield: 51.2%, purity: 98.43%, HCl) as an off-white solid.

LC-MS [415+H]⁺ = 403.2.

¹H NMR (400 MHz, DMSO-*d*₆) δ ppm 1.21 - 1.42 (m, 2 H) 1.43 - 1.53 (m, 2 H) 1.62 (br s, 4 H) 2.38 (s, 3 H) 3.67 (br d, *J* = 4.63 Hz, 2 H) 3.81 - 3.99 (m, 2 H) 4.03 - 4.18 (m, 2 H) 5.16 (s, 2 H) 6.53 - 6.60 (m, 1 H) 6.61 - 6.72 (m, 1 H) 6.75 (d, *J* = 8.76 Hz, 1 H) 7.31 (d, *J* = 2.50 Hz, 1 H) 8.97 (s, 1 H).

### Example 70: Preparation of 6-{[(2-fluorophenyl)methyl]oxy}-N-[3-(hydroxymethyl)piperidin-3-yl]-3,4-dihydro-2H-benzo[b][1,4]oxazine-4-carboxamide formate (JD-5303091)

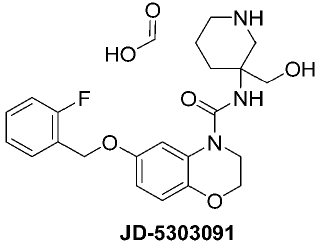

Following the synthetic method of Example 64, compound JD-5303001 c was replaced with JD-5303028a to obtain 6-{[(2-fluorophenyl)methyl]oxy}-*N*-[3-(hydroxymethyl)piperidin-3-yl]-3,4-dihydro-2*H*-benzo[*b*][1,4]oxazine-4-carboxamide formate (JD-5303091) (30 mg, 18.7%) as a white solid.

LCMS: 416.2 ([M-FA+H]⁺).

**¹H NMR (300 MHz, DMSO-*d*₆):** *δ* 8.29 (s, 1H), 7.53 (td, *J* = 7.5, 1.8 Hz, 1H), 7.47 - 7.33 (m, 2H), 7.28 - 7.20 (m, 2H), 6.78 (d, *J* = 9.0 Hz, 1H), 6.62 (dd, *J* = 9.0, 3.0 Hz, 1H), 6.23 (s, 1H), 5.04 (s, 2H), 4.21 - 4.06 (m, 2H), 3.83 - 3.59 (m, 3H), 3.50 - 3.25 (m, 2H), 3.03 - 2.86 (m, 1H), 2.75 - 2.51 (m, 2H), 2.12 (d, *J* = 12.6 Hz, 1H), 1.82 - 1.15 (m, 3H).

### Example 71: Preparation of N-[3-(hydroxymethyl)piperidin-3-yl]-6-{[(2-methyl-1,3-thiazol-5-yl)methyl]oxy}-3,4-dihydro-2H-benzo[b][1,4]oxazine-4-carboxamide (JD-5303109)

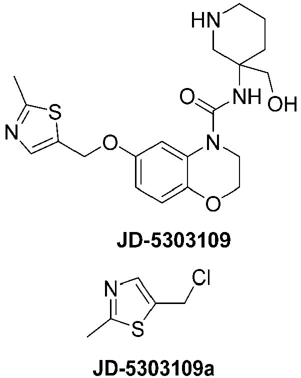

Following the synthetic method of Example 64, compound JD-5303001c was replaced with JD-5303109a to obtain *N*-[3-(hydroxymethyl)piperidin-3-yl]-6-{[(2-methyl-1,3-thiazol-5-yl)methyl]oxy}-3,4-dihydro-2*H*-benzo[*b*][1,4]oxazine-4-carboxamide (JD-5303109) (31.7 mg, yield: 26.8%, purity: 98.0%) as a white solid.

LC-MS ([M+H]⁺) = 419.1.

**¹H NMR** (300 MHz, DMSO-*d*₆) δ = 7.66 (s, 1H), 7.29 (d, *J* = 3.0 Hz, 1H), 6.79 (d, *J* = 9.0 Hz, 1H), 6.61 (dd, *J* = 9.0, 3.0 Hz, 1H), 6.11 (s, 1H), 5.26 - 5.10 (m, 2H), 4.78 (brs, 1H), 4.13 (t, *J* = 4.5 Hz, 2H), 3.80 - 3.60 (m, 3H), 3.50 - 3.37 (m, 2H), 3.03 (d, *J* = 12.0 Hz, 1H), 2.87 - 2.77 (m, 1H), 2.63 (s, 3H), 2.50 - 2.40 (m, 1H), 2.20 - 2.06 (m, 1H), 1.62 - 1.17 (m, 4H).

### Example 72: Preparation of N-[3-(hydroxymethyl)piperidin-3-yl]-6-({[2-(trifluoromethyl)pyridin-3-yl]methyl}oxy)-3,4-dihydro-2H-benzo[b][1,4]oxazine-4-carboxamide 2,2,2-trifluoroacetate (JD-5303110)

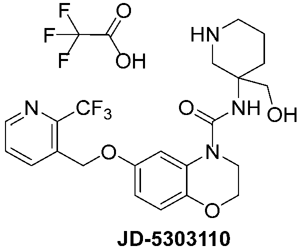

Following the synthetic method of Example 64, compound JD-5303001c was replaced with JD-5303031b to obtain *N*-[3-(hydroxymethyl)piperidin-3-yl]-6-({[2-(trifluoromethyl)pyridin-3-yl]methyl}oxy)-3,4-dihydro-2*H*-benzo[*b*][1,4]oxazine-4-carboxamide 2,2,2-trifluoroacetate (JD-5303110) (21.7 mg, yield: 12.3%, purity: 98.2%) as a white solid.

LC-MS ([M-TFA+H]⁺) = 467.2.

**¹H NMR** (300 MHz, DMSO-*d*₆) δ = 8.85 (d, *J* = 11.7 Hz, 1H), 8.72 (d, *J* = 4.5 Hz, 1H), 8.28 - 8.09 (m, 2H), 7.78 (dd, *J=* 8.1, 4.8 Hz, 1H), 7.44 (d, *J=* 2.7 Hz, 1H), 6.80 (d, *J* = 8.7 Hz, 1H), 6.63 (dd, *J* = 9.0, 3.0 Hz, 1H), 6.28 (s, 1H), 5.17 (s, 2H), 4.23 - 4.09 (m, 2H), 3.95 - 3.81 (m, 2H), 3.72 - 3.56 (m, 2H), 3.47 (d, *J=* 10.8 Hz, 1H), 3.23 - 3.08 (m, 1H), 3.02 - 2.79 (m, 2H), 2.12 - 1.98 (m, 1H), 1.92 - 1.50 (m, 3H).

### Example 73: Preparation of 6-{[(2.3-fluorophenyl)methyl]oxy}-N-[3-(hydroxymethyl)piperidin-3-yl]-3,4-dihydro-2H-benzo[b][1,4]oxazine-4-carboxamide (JD-5303107)

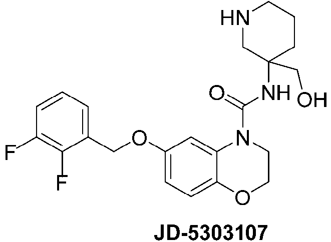

Following the synthetic method of Example 64, compound JD-5303001c was replaced with JD-5303036b to obtain 6-{[(2.3-fluorophenyl)methyl]oxy}-*N*-[3-(hydroxymethyl)piperidin-3-yl]-3,4-dihydro-2*H*-benzo[*b*][1,4]oxazine-4-carboxamide (JD-5303107) (35 mg, yield: 24.8%, purity: 95.1%) as a white solid.

LC-MS ([M+H]⁺) = 434.2.

**¹H NMR:** (300 MHz, DMSO-*d*₆) *δ* = 7.46 - 7.31 (m, 3H), 7.28 - 7.20 (m, 1H), 6.80 (d, *J* = 9.0 Hz, 1H), 6.64 (dd, *J* = 9.0, 3.0 Hz, 1H), 6.12 (s, 1H), 5.11 (s, 2H), 4.89 - 4.54 (m, 1H), 4.21 - 4.05 (m, 2H), 3.77 - 3.61 (m, 3H), 3.40 (d, *J* = 10.8 Hz, 1H), 2.97 (d, *J* = 12.3 Hz, 1H), 2.85 - 2.74 (m, 1H), 2.49 - 2.40 (m, 2H), 2.13 (d, *J* = 12.9 Hz, 1H), 1.53 - 1.23 (m, 3H).

### Example 74: Preparation of N-(4,4-difluorotetrahydro-1H-pyrrol-3-yl)-6-[(2-methylthiophen-3-yl)oxy]-3,4-dihydro-2H-benzo[b][1,4]oxazine-4-carboxamide 2,2,2-trifluoroacetate (JD-5303101)

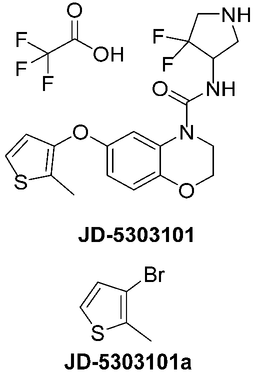

Following the synthetic method of Example 52, compound JD-5303047a was replaced with JD-5303101a to obtain *N*-(4,4-difluorotetrahydro-1*H*-pyrrol-3-yl)-6-[(2-methylthiophen-3-yl)oxy]-3,4-dihydro-2*H*-benzo[*b*][1,4]oxazine-4-carboxamide 2,2,2-trifluoroacetate (JD-5303101) (6 mg, yield: 26.5%, purity: 99.8%) as a white solid.

LC-MS ([M-TFA+H]⁺) = 396.1.

**¹H NMR** (300 MHz, DMSO-*d*₆) *δ* = 9.48 (brs, 2H), 7.41 (d, *J* = 8.4 Hz, 1H), 7.32 - 7.22 (m, 2H), 6.3 (d, *J* = 9.0 Hz, 1H), 6.73 (d, *J* = 5.4 Hz, 1H), 6.55 (dd, *J* = 9.0, 3.0 Hz, 1H), 4.92 - 4.68 (m, 1H), 4.28 - 4.09 (m, 2H), 3.92 - 3.60 (m, 5H), 3.42 - 3.24 (m, 1H), 2.23 (s, 3H).

### Example 75: Preparation of 6-[(cyclohexylmethyl)oxy]-N-[3-(hydroxymethyl)piperidin-3-yl]-3,4-dihydro-2H-benzo[b][1,4]oxazine-4-carboxamide (JD-5303103)

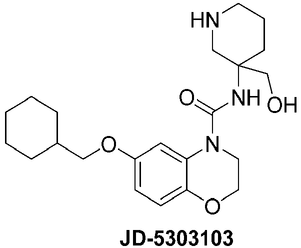

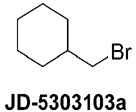

Following the synthetic method of Example 64, compound JD-5303001c was replaced with JD-5303103a to obtain 6-[(cyclohexylmethyl)oxy]-*N*-[3-(hydroxymethyl)piperidin-3-yl]-3,4-dihydro-2*H*-benzo[*b*][1,4]oxazine-4-carboxamide (JD-5303103) (30 mg, 45.7%) as a yellow solid.

LCMS: 404.4 ([M+H]⁺).

**¹H NMR (300 MHz, DMSO-*d*₆):** *δ* 7.22 (d, *J* = 2.9 Hz, 1H), 6.75 (d, *J* = 8.9 Hz, 1H), 6.52 (dd, *J* = 8.9, 2.9 Hz, 1H), 6.17 (s, 1H), 4.21 - 4.02 (m, 2H), 3.78 - 3.59 (m, 5H), 3.47 - 3.35 (m, 2H), 3.03 - 2.92 (m, 1H), 2.77 - 2.52 (m, 2H), 2.21 - 2.02 (m, 1H), 1.85 - 1.45 (m, 9H), 1.33 - 1.10 (m, 4H), 1.08 - 0.93 (m, 2H).

### Example 76: Preparation of 7-{[(2-fluorophenyl)methyl]oxy}-N-[3-(hydroxymethyl)piperidin-3-yl]-1,2,3,4-tetrahydroquinoline-1-carboxamide (JD-5303099)

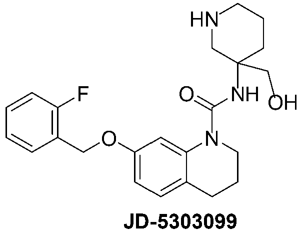

Following the synthetic method of Example 70, compound JD-5303001-1 was replaced with JD-5303072-1 to obtain 7-{[(2-fluorophenyl)methyl]oxy}-*N*-[3-(hydroxymethyl)piperidin-3-yl]-1,2,3,4-tetrahydroquinoline-1-carboxamide (JD-5303099) (5.8 mg, 24.8%) as a white solid.

**LCMS:** 414.2 ([M+H]⁺).

**¹H NMR (300 MHz, DMSO-*d*₆):** *δ* 8.53 - 8.00 (m, 1H), 7.58 - 7.49 (m, 1H), 7.47 - 7.38 (m, 1H), 7.31 - 7.19 (m, 3H), 7.02 (d, *J* = 8.4 Hz, 1H), 6.64 (dd, *J* = 8.4, 2.7 Hz, 1H), 6.02 (s, 1H), 5.06 (s, 2H), 3.76 - 3.51 (m, 4H), 3.49 - 3.40 (m, 1H), 3.13 - 3.02 (m, 1H), 2.88 - 2.71 (m, 2H), 2.64 (t, *J=* 6.3 Hz, 2H), 2.13 - 2.00 (m, 1H), 1.92 - 1.33 (m, 6H).

### Example 77: Preparation of N-(4,4-difluorotetrahydro-1H-pyrrol-3-yl)-6-[(3-methylphenyl)oxy]-3,4-dihydro-2H-benzo[b][1,4]oxazine-4-carboxamide hydrochloride (JD-5303083)

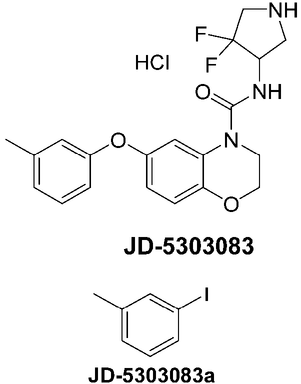

Following the synthetic method of Example 52, compound JD-5303047a was replaced with JD-5303083a to obtain *N*-(4,4-difluorotetrahydro-1*H*-pyrrol-3-yl)-6-[(3-methylphenyl)oxy]-3,4-dihydro-2*H*-benzo[*b*][1,4]oxazine-4-carboxamide hydrochloride (JD-5303083) (13.9 mg, 32.7 µmol, 80.1%, HCl) as an off-white solid.

LCMS [389+H]⁺ = 390.

¹H NMR (400 MHz, DMSO-*d*₆) δ = 2.26 (s, 3 H) 3.34 - 3.39 (m, 1 H) 3.57 - 3.89 (m, 5 H) 4.12 - 4.30 (m, 2 H) 4.66 - 4.82 (m, 1 H) 6.65 (dd, *J=* 8.82, 2.81 Hz, 1 H) 6.70 - 6.78 (m, 2 H) 6.84 - 6.91 (m, 2 H) 7.20 (t, *J=* 7.82 Hz, 1 H) 7.42 (d, *J=* 2.88 Hz, 1 H) 7.50 (d, *J* = 8.38 Hz, 1 H) 9.48 - 9.76 (m, 2 H).

### Example 78: Preparation ofN-(4,4-difluorotetrahydro-1H-pyrrol-3-yl)-6-(1,3-thiazol-5-yloxy)-3,4-dihydro-2H-benzo[b][1,4]oxazine-4-carboxamide trifluoroacetate (JD-5303088)

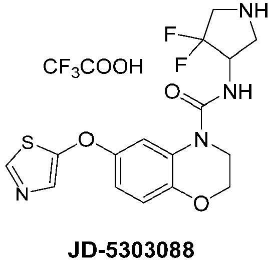

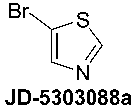

Following the synthetic method of Example 52, compound JD-5303047a was replaced with JD-5303088a to obtain *N*-(4,4-difluorotetrahydro-1*H*-pyrrol-3-yl)-6-(1,3-thiazol-5-yloxy)-3,4-dihydro-2*H*-benzo[*b*][1,4]oxazine-4-carboxamide trifluoroacetate (JD-5303088) (19.7 mg, 39.7 µmol, yield: 19.1%, FA) as an off-white solid.

LCMS [382+H]⁺ = 383.

¹H NMR: (400 MHz, DMSO-*d*₆) δ = ppm 3.38 (dd, *J* = 11.69, 9.69 Hz, 1 H) 3.67 - 3.90 (m, 5 H) 4.13 - 4.28 (m, 2 H) 4.72 - 4.88 (m, 1 H) 6.80 (dd, *J* = 8.88, 2.88 Hz, 1 H) 6.91 (d, *J* = 9.01 Hz, 1 H) 7.41 - 7.68 (m, 3 H) 8.68 (d, *J=* 0.88 Hz, 1 H) 9.41 - 9.90 (m, 2 H).

### Example 79: Preparation of N-[(1S,3R)-3-aminocyclohexyl]-6-[(4-methyl-1,3-thiazol-5-yl)oxy]-3,4-dihydro-2H-benzo[b][1,4]oxazine-4-carboxamide (JD-5303089)

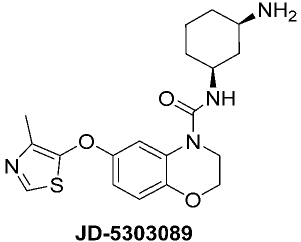

Following the synthetic method of Example 65, compound JD-5303001d was replaced with JD-5303022a to obtain *N*-[(1*S*,3*R*)-3-aminocyclohexyl]-6-[(4-methyl-1,3-thiazol-5-yl)oxy]-3,4-dihydro-2*H*-benzo[*b*][1,4]oxazine-4-carboxamide (JD-5303089) (16.1 mg, 25.3%) as a white solid.

**LCMS:** 389.2 ([M+H]⁺).

**¹H NMR (300 MHz, DMSO-*d*₆):** *δ* 8.70 (s, 1H), 8.18 - 7.57 (m, 2H), 7.48 (d, *J* = 3.0 Hz, 1H), 7.03 (d, *J* = 7.8 Hz, 1H), 6.84 (d, *J* = 9.0 Hz, 1H), 6.63 (dd, *J* = 8.7, 2.7 Hz, 1H), 4.21 - 4.10 (m, 2H), 3.74 - 3.65 (m, 2H), 3.58 - 3.50 (m, 1H), 3.05 - 2.94 (m, 1H), 2.22 (s, 3H), 2.13 - 2.02 (m, 1H), 1.91 - 1.83 (m, 1H), 1.78 - 1.68 (m, 2H), 1.34 - 1.16 (m, 4H).

### Example 80: Preparation of N-(4,4-difluorotetrahydro-1H-pyrrol-3-yl)-6-[(2-fluorophenyl)oxy]-3,4-dihydro-2H-benzo[b][1,4]oxazine-4-carboxamide hydrochloride (JD-5303092)

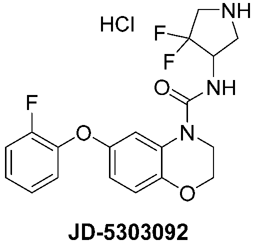

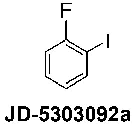

Following the synthetic method of Example 52, compound JD-5303047a was replaced with JD-5303092a to obtain *N*-(4,4-difluorotetrahydro-1*H*-pyrrol-3-yl)-6-[(2-fluorophenyl)oxy]-3,4-dihydro-2*H*-benzo[*b*][1,4]oxazine-4-carboxamide hydrochloride (JD-5303092) (19.2 mg, 44.2 µmol, yield: 14.5%, purity: 99.1%, HCl) as a white solid.

LCMS [393+H]⁺ = 394.

¹H NMR (400 MHz, DMSO-*d*₆) δ = ppm 3.36 - 3.42 (m, 1 H) 3.62 - 3.88 (m, 5 H) 4.12 - 4.28 (m, 2 H) 4.65 - 4.85 (m, 1 H) 6.65 (dd, *J* = 8.88, 2.88 Hz, 1 H) 6.88 (d, *J* = 8.88 Hz, 1 H) 7.03 - 7.10 (m, 1 H) 7.11 - 7.21 (m, 2 H) 7.30 - 7.38 (m, 1 H) 7.44 (d, *J* = 2.88 Hz, 1 H) 7.57 (br d, *J=* 8.13 Hz, 1 H) 9.57 - 10.03 (m, 2 H).

### Example 81: Preparation of N-(4,4-difluorotetrahydro-1H-pyrrol-3-yl)-6-{[2-(trifluoromethyl)pyridin-3-yl]oxy}-3,4-dihydro-2H-benzo[b][1,4]oxazine-4-carboxamide hydrochloride (JD-5303097)

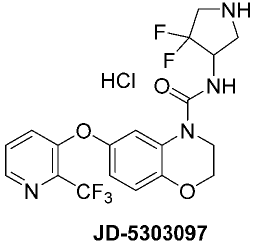

Following the synthetic method of Example 52, compound JD-5303047a was replaced with JD-5303031b to obtain *N*-(4,4-difluorotetrahydro-1*H*-pyrrol-3-yl)-6-{[2-(trifluoromethyl)pyridin-3-yl]oxy}-3,4-dihydro-2*H*-benzo[*b*][1,4]oxazine-4-carboxamide hydrochloride (JD-5303097) (70.0 mg, 145 µmol, 99.0%, HCl) as a white solid.

LC-MS [444+H]⁺ = 445.

**¹H NMR:** (400 MHz, DMSO-*d*₆) δ = 3.39 (br t, *J* = 9.69 Hz, 1 H), 3.62 - 3.72 (m, 2 H), 3.73 - 3.81 (m, 2 H), 3.82 - 3.91 (m, 1 H), 4.16 - 4.29 (m, 2 H), 4.67 - 4.82 (m, 1 H), 6.77 (dd, *J=* 8.76, 2.88 Hz, 1 H), 6.95 (d, *J* = 8.76 Hz, 1 H), 7.45 - 7.58 (m, 2 H), 7.61 - 7.77 (m, 2 H), 8.42 (d, *J* = 4.25 Hz, 1 H), 9.77 - 10.31 (m, 2 H).

### Example 82: Preparation of N-(4,4-difluorotetrahydro-1H-pyrrol-3-yl)-6-[(2,5-dimethylthiophen-3-yl)oxy]-3,4-dihydro-2H-benzo[b][1,4]oxazine-4-carboxamide (JD-5303098)

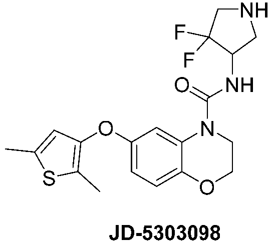

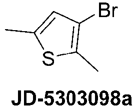

Following the synthetic method of Example 52, compound JD-5303047a was replaced with JD-5303098a to obtain *N*-(4,4-difluorotetrahydro-1*H*-pyrrol-3-yl)-6-[(2,5-dimethylthiophen-3-yl)oxy]-3,4-dihydro-2*H*-benzo[*b*][1,4]oxazine-4-carboxamide (JD-5303098) (9.3 mg, 29.3%) as a white solid.

**LCMS:** 410.1 ([M+H]⁺).

**¹H NMR (400 MHz, DMSO-*d*₆):** *δ* 7.23 (d, *J* = 3.2 Hz, 1H), 7.08 (d, *J* = 8.4 Hz, 1H), 6.81 (d, *J* = 8.8 Hz, 1H), 6.52 (dd, *J* = 8.8, 2.8 Hz, 1H), 6.44 (s, 1H), 4.37 - 4.26 (m, 1H), 4.18 - 4.12 (m, 2H), 3.76 - 3.68 (m, 2H), 3.27 - 3.20 (m, 2H), 3.04 - 2.90 (m, 1H), 2.83 - 2.75 (m, 1H), 2.32 (s, 3H), 2.15 (s, 3H).

### Example 83: Preparation of N-[3-(hydroxymethyl)piperidin-3-yl]-6-[(4-methyl-1,3-thiazol-5-yl)oxy]-3,4-dihydro-2H-benzo[b][1,4]oxazine-4-carboxamide (JD-5303090)

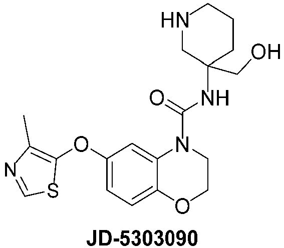

Following the synthetic method of Example 65, compound JD-5303001d was replaced with JD-5303074-6 to obtain *N*-[3-(hydroxymethyl)piperidin-3-yl]-6-[(4-methyl-1,3-thiazol-5-yl)oxy]-3,4-dihydro-2*H*-benzo[*b*][1,4]oxazine-4-carboxamide (JD-5303090) (20 mg, 42.8%) as a white solid.

**LCMS:** 405.1 ([M+H]⁺).

**¹H NMR (300 MHz, DMSO-*d*₆):** *δ* 8.65 (s, 1H), 7.47 (d, *J* = 3.0 Hz, 1H), 6.86 (d, *J* = 8.7 Hz, 1H), 6.65 (dd, *J* = 9.0, 3.0 Hz, 1H), 6.17 (s, 1H), 5.16 - 4.61 (m, 1H), 4.25 - 4.09 (m, 2H), 3.94 - 3.79 (m, 1H), 3.69 - 3.59 (m, 2H), 3.44 - 3.35 (m, 2H), 3.30 - 3.25 (m, 1H), 2.96 - 2.84 (m, 1H), 2.68 - 2.54 (m, 2H), 2.22 (s, 3H), 2.09 - 1.99 (m, 1H), 1.57 - 1.38 (m, 3H).

### Example 84: Preparation of N-(4,4-difluoropiperidin-3-yl)-6-{[(2-fluorophenyl)methyl]oxy}-3,4-dihydro-2H-benzo[b][1,4]oxazine-4-carboxamide (JD-5303104)

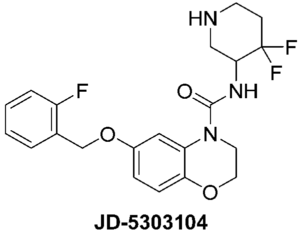

### Step 1: Preparation of tert-butyl ({1-[(benzyloxy)carbonyl]-4-hydroxypiperidin-3-yl}amino)formate (JD-5303104-2)

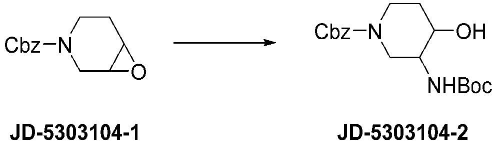

Benzyl hexahydro-2*H*-oxepino[2,3-*c*]pyridine-3-carboxylate **(JD-5303104-1)** (7.0 g, 30.01 mmol, 1.0 eq) was dissolved in EtOH (35.0 mL), then NH₃·H₂O (35.0 mL) was added thereto, and the mixture was reacted at 70°C in an autoclave for 15 hours. The reaction mixture was directly concentrated under reduced pressure to obtain a residue. The residue was dissolved in DCM (70.0 mL), followed by the addition of TEA (4.0 g, 39.01 mmol, 1.3 eq) and Boc₂O (7.2 g, 33.01 mmol, 1.1 eq), and the mixture was reacted at room temperature for 15 hours. The reaction mixture was concentrated under reduced pressure to obtain a crude product. The crude product was purified by column chromatography (SiO₂, petroleum ether/ethyl acetate = 1/1) to obtain *tert*-butyl ({1-[(benzyloxy)carbonyl]-4-hydroxypiperidin-3-yl}amino)formate **(JD-5303104-2)** (3.2 g, yield: 30.4%, purity: 92%) as a colorless oil.

LC-MS ([M-56+H]⁺) = 295.1.

**¹H NMR:** (300 MHz, CDCl₃) *δ* = 7.46 - 7.27 (m, 5H), 5.26 - 5.04 (m, 2H), 4.68 (brs, 1H), 4.25 - 4.00 (m, 1H), 3.99 - 3.78 (m, 1H), 3.74 - 3.57 (m, 1H), 3.54 - 3.30 (m, 1H), 3.25 - 2.80 (m, 2H), 2.05 - 1.86 (m, 1H), 1.67 - 1.32 (m, 10H).

### Step 2: Preparation of tert-butyl ({1-[(benzyloxy)carbonyl]-4-oxopiperidin-3-yl}amino)formate (JD-5303104-3)

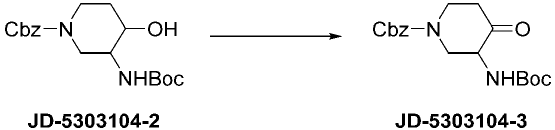

*tert*-Butyl ({1-[(benzyloxy)carbonyl]-4-hydroxypiperidin-3-yl}amino)formate **(JD-5303104-2)** (3.2 g, 9.13 mmol, 1.0 eq) was dissolved in DCM (35.0 mL), then DMP (5.8 g, 13.70 mmol, 1.5 eq) was added thereto, and the mixture was reacted at room temperature for 15 hours. The reaction mixture was quenched with NaHCO₃ (40 mL) and Na₂S₂O₃ (10 mL), and extracted with DCM (150 mL, 50 mL * 3). The combined organic phases were washed with brine (50 mL), dried over anhydrous sodium sulfate, and concentrated under reduced pressure to obtain a residue. The residue was purified by column chromatography (SiO₂, petroleum ether/ethyl acetate = 8/1) to obtain *tert*-butyl ({1-[(benzyloxy)carbonyl]-4-oxopiperidin-3-yl}amino)formate **(JD-5303104-3)** (2.7 g, yield: 84.9%, purity: 90%) as a yellow oil.

LC-MS ([M-56+H]⁺) = 293.1.

**¹H NMR:** (400 MHz, CDCl₃) *δ* = 7.52 - 7.30 (m, 5H), 5.44 (s, 1H), 5.29 - 5.10 (m, 2H), 4.97 - 4.80 (m, 1H), 4.51 (s, 1H), 4.30 (s, 1H), 3.22 - 3.00 (m, 1H), 2.73 (t, *J=* 11.6 Hz, 1H), 2.67 - 2.45 (m, 2H), 1.45 (s, 9H).

### Step 3: Preparation of tert-butyl bis[({1-[(benzyloxy)carbonyl]-4,4-difluoropiperidin-3-yl}amino)formate] (JD-5303104-4)

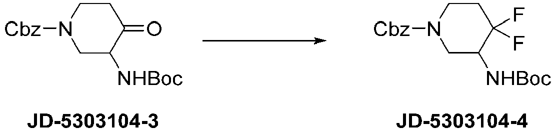

*tert*-Butyl ({1-[(benzyloxy)carbonyl]-4-oxopiperidin-3-yl}amino)formate **(JD-5303104-3)** (300 mg, 0.86 mmol, 1.0 eq) was dissolved in DCM (5.0 mL), and DAST (1.4 g, 8.61 mmol, 10.0 eq) was slowly added thereto under a N₂ atmosphere at 0°C. The mixture was reacted at 30°C for 72 hours. The reaction mixture was quenched with saturated NaHCO₃ aqueous solution (40 mL) at room temperature and extracted with DCM (60 mL, 20 mL * 3). The combined organic phases were washed with brine (20 mL), dried over anhydrous sodium sulfate, and concentrated under reduced pressure to obtain a residue. The residue was purified by column chromatography (SiO₂, petroleum ether/ethyl acetate = 10/1) to obtain *tert*-butyl bis[({1-[(benzyloxy)carbonyl]-4,4-difluoropiperidin-3-yl}amino)formate] **(JD-5303104-4)** (150 mg, yield: 47.0%, purity: 89%) as a yellow oil.

LC-MS ([M-56+H]⁺) = 315.1.

**¹H NMR:** (300 MHz, CDCl₃) *δ* = 7.43 - 7.29 (m, 5H), 5.26 - 5.04 (m, 2H), 4.84 - 4.66 (m, 1H), 4.13 - 3.95 (m, 2H), 3.27 - 2.95 (m, 2H), 2.25 - 2.07 (m, 1H), 2.05 - 1.82 (m, 1H), 1.45 (s, 9H).

### Step 4: Preparation of tert-butyl bis[({1-[(benzyloxy)carbonyl]-4,4-difluoropiperidin-3-yl}amino)formate] (JD-5303104-5)

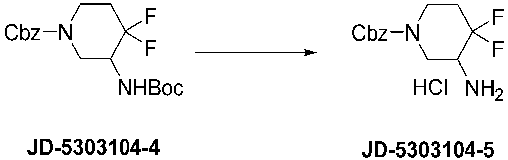

*tert-Butyl* bis[({1-[(benzyloxy)carbonyl]-4,4-difluoropiperidin-3-yl}amino)formate] **(JD-5303104-4)** (150 mg, 0.41 mmol, 1.0 eq) was dissolved in DCM (3.0 mL), then dioxane/HCl (1.01 mL, 4.05 mmol, 10.0 eq) was added thereto, and the mixture was reacted at room temperature for 15 hours. The reaction mixture was directly concentrated under reduced pressure to obtain *tert*-butyl bis[({1-[(benzyloxy)carbonyl]-4,4-difluoropiperidin-3-yl}amino)formate] **(JD-5303104-5)** (150 mg, crude product) as a yellow solid.

LC-MS ([M-HCl+H]⁺) = 271.1.

### Step 5: Preparation of benzyl 4,4-difluoro-3-{[(6-{[(2-fluorophenyl)methyl]oxy}-3,4-dihydro-2H-benzo[b][1,4]oxazin-4-yl)carbonyl]amino}piperidine-1-carboxylate (JD-5303104-6)

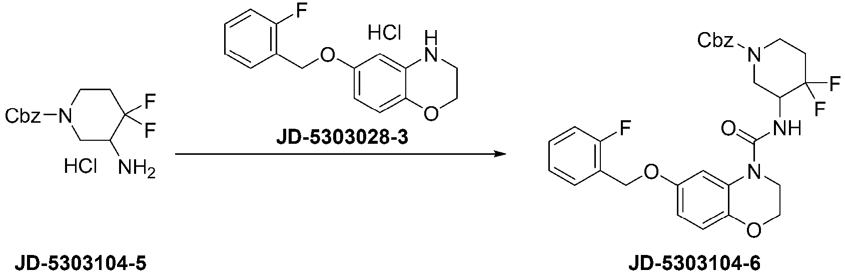

*tert-*Butyl bis[({1-[(benzyloxy)carbonyl]-4,4-difluoropiperidin-3-yl}amino)formate] **(JD-5303104-5)** (40 mg, 0.14 mmol, 1.0 eq) was dissolved in DCM (4.0 mL), followed by the addition of TEA (137 mg, 1.35 mmol, 10.0 eq) and (COCl2)₃ (20 mg, 0.07 mmol, 0.5 eq), and the reaction mixture was stirred at 30°C for 0.5 hours. A solution of **70-5** (50 mg crude, 0.14 mmol, 1.0 eq) in DCM (1.0 mL) was then added thereto, and the reaction system was stirred at 30°C for 14.5 hours. The reaction mixture was quenched with water (10 mL) at room temperature and extracted with DCM (20 mL, 10 mL * 2). The combined organic phases were washed with brine (10 mL), dried over anhydrous sodium sulfate, and concentrated under reduced pressure to obtain a residue. The residue was purified by column chromatography (SiO₂, petroleum ether/ethyl acetate = 3/1) to obtain benzyl 4,4-difluoro-3-{[(6-{[(2-fluorophenyl)methyl]oxyl-3,4-dihydro-2*H*-benzo[*b*][1,4]oxazin-4-yl)carbonyl]amino}piperidine-1-carboxylate **(JD-5303104-6)** (40 mg, two-step yield: 53.3%, purity: 85%) as a white solid.

LC-MS ([M+H]⁺) = 556.2.

### Step 6: Preparation of N-(4,4-difluoropiperidin-3-yl)-6-{[(2-fluorophenyl)methyl]oxy}-3,4-dihydro-2H-benzo[b][1,4]oxazine-4-carboxamide (JD-5303104)

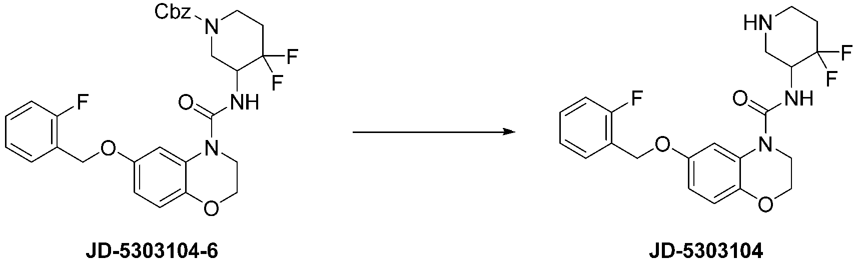

Benzyl 4,4-difluoro-3-{[(6-{[(2-fluorophenyl)methyl]oxy}-3,4-dihydro-2*H-*benzo[*b*][1,4]oxazin-4-yl)carbonyl]amino}piperidine-1-carboxylate **(JD-5303104-6)** (40 mg, 0.07 mmol, 1.0 eq) was dissolved in DCM (3.0 mL), then TMSI (72 mg, 0.36 mmol, 5.0 eq) was added thereto, and the mixture was stirred at room temperature for 3 hours. The reaction mixture was quenched with water (5 mL) and extracted with DCM (20 mL, 10 mL * 2). The aqueous phase was then added with saturated sodium bicarbonate solution to adjust the pH to 10, and extracted with DCM (20 mL, 10 mL * 2). The combined organic phases were washed with brine (10 mL), dried over anhydrous sodium sulfate, and concentrated under reduced pressure to obtain a residue. The residue was purified by prep-TLC (DCM/MeOH = 15/1) to obtain N-(4,4-difluoropiperidin-3-yl)-6- {[(2-fluorophenyl)methyl]oxyl-3,4-dihydro-2H-benzo[b][1,4]oxazine-4-carboxamide **(JD-5303104)** (6.6 mg, yield: 21.8%, purity: 95.2%) as a white solid.

LC-MS ([M+H]⁺) = 422.1.

**¹H NMR:** (300 MHz, CD₃OD) *δ* = 7.56 - 7.43 (m, 1H), 7.42 - 7.29 (m, 1H), 7.23 - 7.04 (m, 3H), 6.81 (d, *J =* 9.0 Hz, 1H), 6.68 (dd, *J =* 9.0, 3.0 Hz, 1H), 5.05 (s, 2H), 4.38 - 4.19 (m, 2H), 4.17 - 4.00 (m, 2H), 3.57 - 3.43 (m, 1H), 3.21 - 3.02 (m, 2H), 2.90 - 2.68 (m, 2H), 2.30 - 1.88 (m, 2H).

### Example 85: Preparation ofN-[3-(formamido)cyclohexyl]-6-{[(4-methyl-1,3-thiazol-5-yl)methyl]oxy}-3,4-dihydro-2H-benzo[b][[1,4]oxazine-4-carboxamide (JD-5303100)

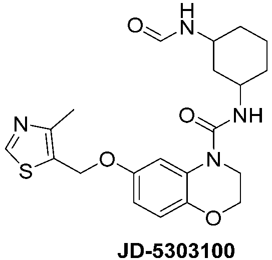

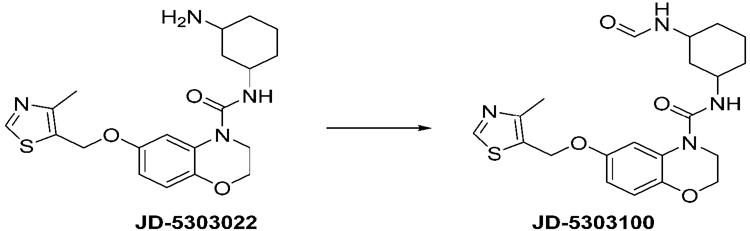

To DCM (2.0 mL) were added compound 1 (100 mg, 0.25 mmol, 1.0 eq), HCOOH (65 mg, 1.24 mmol, 5.0 eq), HATU (471 mg, 1.24 mmol, 5.0 eq), and DIEA (321 mg, 2.48 mmol, 10.0 eq). The mixture was stirred at room temperature for 15 hours, diluted with K₂CO₃ solution (5 mL), and extracted with DCM (10 mL * 2). The combined organic phases were washed with brine (10 mL), dried over Na₂SO₄, filtered, and concentrated under reduced pressure to obtain a residue, which was purified by TLC (DCM/MeOH = 8/1) to obtain JD-5303100 (70 mg, yield: 65.4%) as a yellow solid.

**LCMS:** 431.2 ([M+H]⁺).

**¹H NMR (300 MHz, DMSO-*d*₆):** *δ* 8.97 (s, 1H), 8.18 (d, *J* = 8.1 Hz, 1H), 7.97 - 7.91 (m, 1H), 7.32 (d, *J* = 3.0 Hz, 1H), 6.79 - 6.68 (m, 2H), 6.58 (dd, *J =* 8.7, 2.7 Hz, 1H), 5.16 (s, 2H), 4.14 - 4.04 (m, 3H), 3.99 - 3.75 (m, 1H), 3.69 - 3.63 (m, 2H), 2.38 (s, 3H), 1.77 - 1.34 (m, 8H).

### Example 86: Preparation of (2S)-2-{[(6-{[(2-fluorophenyl)methyl]oxy}-3,4-dihydro-2H-benzo[b][1,4]oxazin-4-yl)carbonyl]amino}-3-hydroxypropanamide (JD-5303084)

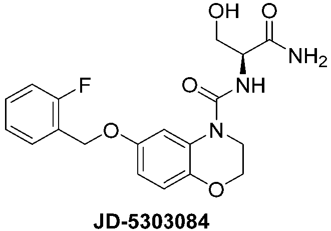

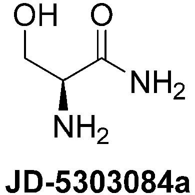

Following the synthetic method of Example 33, compound JD-5303028b was replaced with JD-5303084a to obtain (2S)-2-{[(6-{[(2-fluorophenyl)methyl]oxy}-3,4-dihydro-2*H-*benzo[*b*][1,4]oxazin-4-yl)carbonyl]amino}-3-hydroxypropanamide (JD-5303084) (82 mg, 65.2%) as a white solid.

**LCMS:** 390.1 ([M+H]⁺).

**¹H NMR (400 MHz, DMSO-*d*₆):** *δ* 7.60 - 7.35 (m, 4H), 7.30 - 7.19 (m, 2H), 7.13 (s, 1H), 6.80 (d, *J* = 8.8 Hz, 1H), 6.71 (d, *J* = 7.6 Hz, 1H), 6.64 (dd, *J* = 9.2, 3.2 Hz, 1H), 5.10 - 5.00 (m, 2H), 4.94 (t, *J =* 5.6 Hz, 1H), 4.25 - 4.08 (m, 3H), 3.85 - 3.61 (m, 4H).

### Example 87: Preparation of N-[(1S,3R)-3-aminocyclohexyl]-6-{[(2-fluorophenyl)methyl]oxy}-3,4-dihydro-2H-benzo[b][1,4]oxazine-4-carboxamide (JD-5303086)

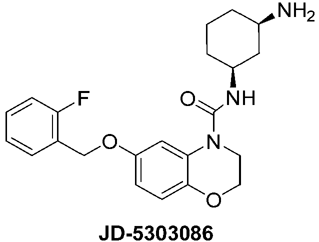

Following the synthetic method of Example 33, compound JD-5303028b was replaced with JD-5303022a to obtain *N*-[(1*S*,3*R*)-3-aminocyclohexyl]-6-{[(2-fluorophenyl)methyl]oxy}-3,4-dihydro-2*H*-benzo[*b*][1,4]oxazine-4-carboxamide (JD-5303086) (43.7 mg, 55.0%) as a white solid.

**LCMS:** 400.3 ([M+H]⁺).

**¹H NMR (400 MHz, DMSO-*d*₆):** *δ* 7.62 - 7.32 (m, 5H), 7.27 - 7.20 (m, 2H), 6.97 (d, *J* = 7.6 Hz, 1H), 6.76 (d, *J =* 8.8 Hz, 1H), 6.59 (dd, *J =* 8.8, 2.8 Hz, 1H), 5.02 (s, 2H), 4.75 - 4.05 (m, 2H), 3.75 - 3.52 (m, 3H), 3.05 - 2.94 (m, 1H), 2.11 (d, *J =* 11.6 Hz, 1H), 1.92 - 1.71 (m, 3H), 1.39 - 1.08 (m, 4H).

### Example 88: Preparation of N-[(1S,3R)-3-aminocyclohexyl]-6-{[(2.6-difluorophenyl)methyl]oxy}-3,4-dihydro-2H-benzo[b][1,4]oxazine-4-carboxamide (JD-5303087)

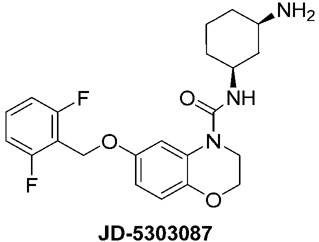

Following the synthetic method of Example 22, JD-5303001d was replaced with JD-5303035a to obtain *N*-[(1*S*,3*R*)-3-aminocyclohexyl]-6-{[(2.6-difluorophenyl)methyl]oxyl-3,4-dihydro-2*H*-benzo[*b*][1,4]oxazine-4-carboxamide (JD-5303087) (78.2 mg, 69.3%) as a white solid.

**LCMS:** 418.3 ([M+H]⁺).

**¹H NMR (300 MHz, DMSO-*d*₆):** *δ* 7.58 - 7.46 (m, 1H), 7.37 (d, *J* = 2.7 Hz, 1H), 7.17 (t, *J* = 7.8 Hz, 2H), 6.94 (d, *J =* 7.8 Hz, 1H), 6.76 (d, *J* = 9.0 Hz, 1H), 6.59 (dd, *J* = 8.7, 2.7 Hz, 1H), 4.99 (s, 2H), 4.11 (t, *J =* 4.5 Hz, 2H), 3.70 - 3.63 (m, 2H), 3.61 - 3.41 (m, 3H), 2.86 - 2.72 (m, 1H), 2.00 (d, *J =* 12.0 Hz, 1H), 1.83 - 1.64 (m, 3H), 1.34 - 1.06 (m, 4H).

### Example 89: Preparation of N-[3-(hydroxymethyl)-2-oxopiperidin-3-yl]-6-{[(4-methyl-1,3-thiazol-5-yl)methyl]oxy}-3,4-dihydro-2H-benzo[b][1,4]oxazine-4-carboxamide (JD-5303095)

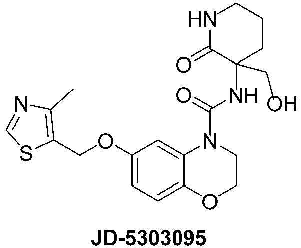

### Step 1: Preparation of ethyl 3-azido-2-oxopiperidine-3-carboxylate (JD-5303095-2)

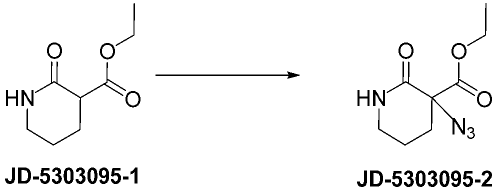

To a solution of compound ethyl 2-oxopiperidine-3-carboxylate **(JD-5303095-1)** (3.0 g, 17.52 mmol, 1.0 eq) in DCM (30 mL) were added DBU (2.94 g, 19.28 mmol, 1.1 eq) and TsN₃ (3.80 g, 19.28 mmol, 1.1 eq). The mixture was stirred at room temperature for 72 hours, diluted with water (15 mL), and extracted with DCM (15 mL * 2). The combined organic phases were washed with brine (10 mL), dried over Na₂SO₄, filtered, and concentrated under reduced pressure to obtain a residue. The residue was purified by silica gel column chromatography (DCM) to obtain ethyl 3-azido-2-oxopiperidine-3-carboxylate **(JD-5303095-2)** (3.0 g, 80.6%) as a yellow solid.

**LCMS:** 213.1 ([M+H]⁺).

**¹H NMR (400 MHz, CDCl₃):** *δ* 6.36 (s, 1H), 4.41 - 4.22 (m, 2H), 3.46 - 3.33 (m, 2H), 2.26 - 2.14 (m, 1H), 1.97 - 1.82 (m, 3H), 1.33 (t, *J* = 7.2 Hz, 3H).

### Step 2: Preparation of ethyl 3-amino-2-oxopiperidine-3-carboxylate (JD-5303095-3)

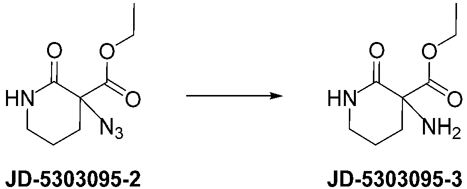

To a solution of compound ethyl 3-azido-2-oxopiperidine-3-carboxylate **(JD-5303095-2)** (1.0 g, 4.71 mmol, 1.0 eq) in toluene (7 mL) was added PPh₃ (989 mg, 3.77 mmol, 0.8 eq). After 5 minutes, 5% HCl (7 mL) was added thereto. The mixture was stirred at room temperature for 12 hours, diluted with water (15 mL), and extracted with DCM (10 mL * 2). The aqueous phase was added with NaOH solution to adjust the pH to 9, and extracted with DCM/MeOH (10/1) (11 mL * 3). The combined organic phases were washed with brine (10 mL), dried over Na₂SO₄, filtered, and concentrated under reduced pressure to obtain compound ethyl 3-amino-2-oxopiperidine-3-carboxylate **(JD-5303095-3)** (160 mg, crude product) as a yellow oil.

**LCMS:** 187.1 ([M+H]⁺).

### Step 3: Preparation of ethyl 3-{[(6-{[(4-methyl-1,3-thiazol-5-yl)methyl]oxy}-3,4-dihydro-2H-benzo[b][1,4]oxazin-4-yl)carbonyl]amino}-2-oxopiperidine-3-carboxylate (JD-5303095-4)

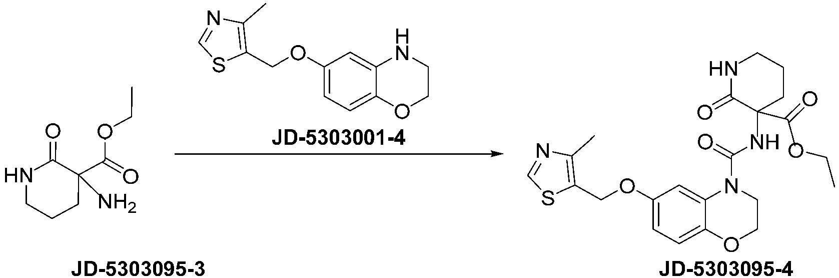

6-{[(4-Methyl-1,3-thiazol-5-yl)methyl]oxy}-3,4-dihydro-2*H*-benzo[*b*][1,4]oxazine **(JD-5303001-4)** (120 mg, 0.46 mmol, 1.0 eq) and TEA (231 mg, 2.29 mmol, 5.0 eq) were dissolved in DCM (4.0 mL), followed by the addition of (COCl₂)₃ (68 mg, 0.23 mmol, 0.5 eq), and the mixture was reacted at room temperature for 0.5 hours. A solution of ethyl 3-amino-2-oxopiperidine-3-carboxylate (JD-5303095-3) (127 mg, 0.69 mmol, 1.4 eq) in DCM (0.5 mL) was then added thereto, and the mixture was stirred at 40°C for 15 hours. The reaction system was quenched with water (10 mL) and extracted with DCM (20 mL, 10 mL * 2). The combined organic phases were washed with brine (10 mL), dried over anhydrous sodium sulfate, and concentrated under reduced pressure to obtain a residue. The residue was purified by column chromatography (SiO₂, dichloromethane/methanol = 50/1) to obtain ethyl 3-{[(6-{[(4-methyl-1,3-thiazol-5-yl)methyl]oxy}-3,4-dihydro-2*H*-benzo[*b*][1,4]oxazin-4-yl)carbonyl] amino }-2-oxopiperidine-3-carboxylate (JD-5303095-4) (120 mg, yield: 55.3%, purity: 90%) as a white solid.

**LCMS:** 475.2 ([M+H]⁺).

**¹H NMR (400 MHz, CDCl₃):** *δ* 8.71 (s, 1H), 7.22 (d, *J =* 2.7 Hz, 1H), 7.14 (s, 1H), 6.85 (d, *J* = 9.0 Hz, 1H), 6.68 (dd, *J* = 9.0, 3.0 Hz, 1H), 6.04 (d, *J* = 3.0 Hz, 1H), 5.16 (s, 2H), 4.37 - 4.14 (m, 4H), 3.93 - 3.75 (m, 2H), 3.66 - 3.56 (m, 1H), 3.47 - 3.33 (m, 1H), 2.60 - 2.47 (m, 4H), 2.37 - 2.20 (m, 2H), 1.95 - 1.87 (m, 1H), 1.28 (t, *J =* 7.2 Hz, 3H).

### Step 4: Preparation ofN-[3-(hydroxymethyl)-2-oxopiperidin-3-yl]-6-{[(4-methyl-1,3-thiazol-5-yl)methyl]oxy}-3,4-dihydro-2H-benzo[b][1,4]oxazine-4-carboxamide (JD-5303095)

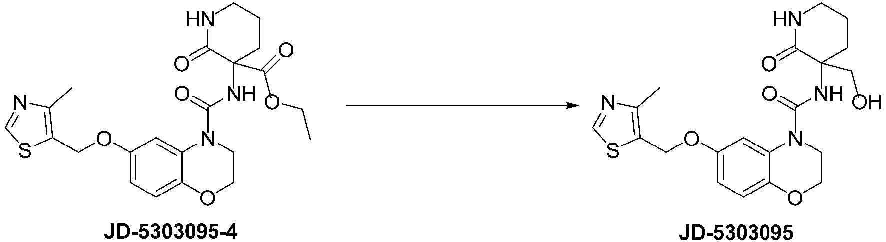

Compound ethyl 3-{[(6-{[(4-methyl-1,3-thiazol-5-yl)methyl]oxy}-3,4-dihydro-2*H-*benzo[*b*][1,4]oxazin-4-yl)carbonyl]amino}-2-oxopiperidine-3-carboxylate **(JD-5303095-4)** (120 mg, 0.25 mmol, 1.0 eq) was dissolved in THF (3 mL) with stirring, and LiAlH₄ (1.0 M, THF) (0.75 mL, 0.75 mmol, 3.0 eq) was added thereto at 0°C. The mixture was stirred at 0°C for 2 hours, quenched with 15% NaOH solution (0.1 mL) at 0°C, dried over Na₂SO₄, filtered, and the filtrate was concentrated to obtain a residue. The residue was purified by prep-TLC (DCM/MeOH = 8/1) to obtain JD-5303095 (15 mg, 13.7%) as a white solid.

**LCMS:** 433.1 ([M+H]⁺).

**¹H NMR (300 MHz, DMSO-*d*₆):** *δ* 8.98 (s, 1H), 7.53 (d, *J* = 2.7 Hz, 1H), 7.32 (d, *J* = 2.7 Hz, 1H), 6.79 (d, *J =* 2.7 Hz, 1H), 6.70 - 6.58 (m, 2H), 5.27 - 5.04 (m, 3H), 4.11 (t, *J =* 4.2 Hz, 2H), 3.77 - 3.58 (m, 3H), 3.56 - 3.49 (m, 1H), 3.24 - 3.13 (m, 1H), 3.12 - 3.03 (m, 1H), 2.39 (s, 3H), 2.25 - 2.09 (m, 2H), 1.75 - 1.69 (m, 1H), 1.45 - 1.40 (m, 1H).

### Example 90: Preparation of 6- {[(2-fluorophenyl)methyl]oxy}-N-[3-(hydroxymethyl)-2-oxopiperidin-3-yl]-3,4-dihydro-2H-benzo[b][1,4]oxazine-4-carboxamide (JD-5303102)

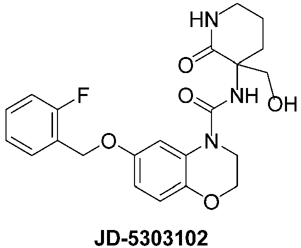

Following the synthetic method of Example 89, compound JD-5303001-4 was replaced with JD-5303028-3 to obtain 6-{[(2-fluorophenyl)methyl]oxy}-N-[3-(hydroxymethyl)-2-oxopiperidin-3-yl]-3,4-dihydro-2*H*-benzo[*b*][1,4]oxazine-4-carboxamide (JD-5303102) (40 mg, 46.3%) as a white solid.

**LCMS:** 430.2 ([M+H]⁺).

**¹H NMR (300 MHz, DMSO-*d*₆):** *δ* 7.59 - 7.48 (m, 2H), 7.48 - 7.38 (m, 1H), 7.34 (d, *J =* 3.0 Hz, 1H), 7.30 - 7.17 (m, 2H), 6.79 (d, *J =* 8.7 Hz, 1H), 6.69 - 6.58 (m, 2H), 5.14 (t, *J* = 6.3 Hz, 1H), 5.04 (s, 2H), 4.11 (t, *J =* 4.5 Hz, 2H), 3.78 - 3.48 (m, 4H), 3.28 - 3.14 (m, 1H), 3.14 - 3.00 (m, 1H), 2.23 - 2.02 (m, 2H), 1.87 - 1.64 (m, 2H).

### Example 91: Preparation ofN-[3-(hydroxymethyl)piperidin-3-yl]-7-{[(4-methyl-1,3-thiazol-5-yl)methyl]oxy}-1,2,3,4-tetrahydroquinoline-1-carboxamide (JD-5303094)

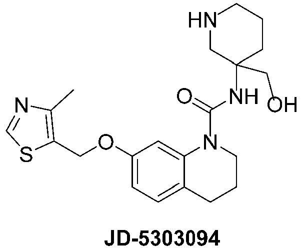

Following the synthetic method of Example 76, compound JD-5303028a was replaced with JD-5303001d to obtain *N*-[3-(hydroxymethyl)piperidin-3-yl]-7-{[(4-methyl-1,3-thiazol-5-yl)methyl]oxy}-1,2,3,4-tetrahydroquinoline-1-carboxamide (JD-5303094) (7.5 mg, 5.0%) as a white solid.

**LCMS:** 417.2 ([M+H]⁺).

**¹H NMR (400 MHz, DMSO-*d*₆):** *δ* 8.98 (s, 1H), 7.26 (d, *J =* 2.4 Hz, 1H), 7.03 (d, *J* = 8.4 Hz, 1H), 6.64 (dd, *J* = 8.0, 2.4 Hz, 1H), 5.98 (s, 1H), 5.21 (s, 2H), 5.15 - 4.92 (m, 1H), 3.68 - 3.62 (m, 2H), 3.58 - 3.51 (m, 2H), 3.44 (d, *J =* 10.8 Hz, 1H), 3.06 - 2.97 (m, 1H), 2.78 - 2.72 (m, 1H), 2.72 - 2.66 (m, 1H), 2.64 (t, *J* = 6.8 Hz, 2H), 2.40 (s, 3H), 2.12 - 2.05 (m, 1H), 1.85 - 1.77 (m, 2H), 1.73 - 1.37 (m, 4H).

### Example 92: Preparation ofN-[3-(methoxymethyl)piperidin-3-yl]-6-{[(4-methyl-1,3-thiazol-5-yl)methyl]oxy}-3,4-dihydro-2H-benzo[b][1,4]oxazine-4-carboxamide (JD-5303093)

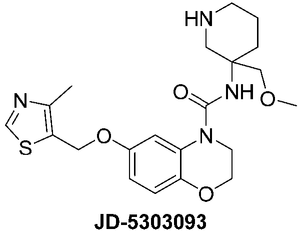

### Step 1: Preparation of methyl 3-(methoxymethyl)-1-{[(2-methylprop-2-yl)oxy]carbonyl}piperidine-3-carboxylate (JD-5303093-2)

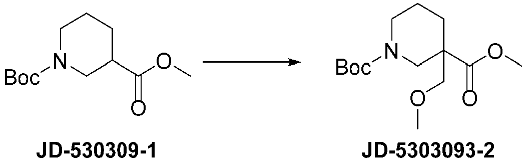

To a solution of compound methyl 1-{[(2-methylprop-2-yl)oxy]carbonyl}piperidine-3-carboxylate **(JD-530309-1)** (500 mg, 2.055 mmol, 1.0 eq) in THF (8 mL) was added LDA (2.0 M in THF) (1.13 mL, 2.26 mmol, 1.1 eq), and the mixture was stirred under a N₂ atmosphere at -65°C for 0.5 hours. MOMBr (308 mg, 2.46 mmol, 1.2 eq) was then added thereto, and the mixture was stirred at room temperature for 15 hours. The reaction mixture was diluted with saturated NH₄Cl solution (15 mL) and extracted with EtOAc (10 mL × 2). The combined organic phases were washed with brine (15 mL), dried over Na₂SO₄, filtered, and concentrated under reduced pressure to obtain a residue. The residue was purified by silica gel column chromatography (petroleum ether/ethyl acetate = 20/1) to obtain compound methyl 3-(methoxymethyl)-1-{[(2-methylprop-2-yl)oxy]carbonyl}piperidine-3-carboxylate **(JD-5303093-2)** (500 mg, 84.6%) as a yellow oil.

**LCMS:** 232.1 ([M-56+H]⁺).

**¹H NMR (300 MHz, CDCl₃):** *δ* 3.80 - 3.65 (m, 4H), 3.58 - 3.23 (m, 8H), 2.03 - 1.82 (m, 1H), 1.73 - 1.64 (m, 1H), 1.62 - 1.52 (m, 2H), 1.46 (s, 9H).

### Step 2: Preparation of 3-(methoxymethyl)-1-{[(2-methylprop-2-yl)oxy]carbonyl}piperidine-3-carboxylic acid (JD-5303093-3)

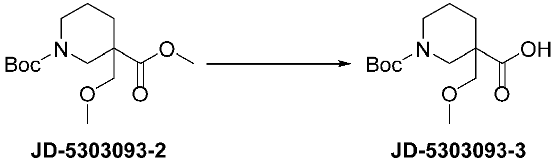

To a solution of compound methyl 3-(methoxymethyl)-1-{[(2-methylprop-2-yl)oxy]carbonyl}piperidine-3-carboxylate **(JD-5303093-2)** (500 mg, 1.74 mmol, 1.0 eq) in THF (3 mL) and H₂O (3 mL) was added LiOH·H₂O (219 mg, 5.22 mmol, 3.0 eq). The mixture was stirred at room temperature for 15 hours. The reaction mixture was diluted with water (10 mL) and extracted with ethyl acetate (8 mL * 2). The aqueous phase was added with KHSO₄ solution to adjust the pH to 3, and extracted with ethyl acetate (8 mL * 3). The combined organic phases were washed with brine (10 mL), dried over Na₂SO₄, filtered, and concentrated under reduced pressure to obtain compound 3-(methoxymethyl)-1-{[(2-methylprop-2-yl)oxy]carbonyl}piperidine-3-carboxylic acid **(JD-5303093-3)** (420 mg, crude product) as a colorless oil.

**LCMS:** 218.3 ([M-56+H]⁺).

### Step 3: Preparation of benzyl {[3-(methoxymethyl)-1-{[(2-methylprop-2-yl)oxy]carbonyl}piperidin-3-yl]amino}formate (JD-5303093-4)

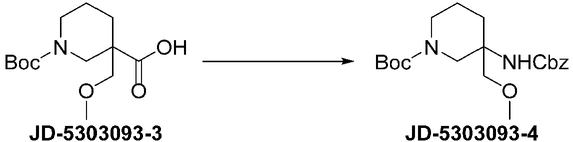

To a solution of compound 3-(methoxymethyl)-1-{[(2-methylprop-2-yl)oxy]carbonyl}piperidine-3-carboxylic acid **(JD-5303093-3)** (420 mg, 1.54 mmol, 1.0 eq) in toluene (4 mL) and BnOH (0.8 mL) were added TEA (311 mg, 3.08 mmol, 2.0 eq) and DPPA (550 mg, 2.00 mmol, 1.3 eq). The mixture was then stirred at 90°C for 15 hours. The reaction mixture was diluted with water (8 mL) and extracted with ethyl acetate (8 mL * 2). The combined organic phases were washed with brine (8 mL), dried over Na₂SO₄, filtered, and concentrated under reduced pressure to obtain a residue. The residue was purified by silica gel column chromatography (petroleum ether/ethyl acetate = 15/1) to obtain compound benzyl {[3-(methoxymethyl)-1-{[(2-methylprop-2-yl)oxy]carbonyl}piperidin-3-yl]amino}formate **(JD-5303093-4)** (650 mg, crude product) as a colorless oil.

**LCMS**: 323.1 ([M-56+H]⁺).

### Step 4: Preparation of tert-butyl 3-amino-3-(methoxymethyl)piperidine-1-carboxylate (JD-5303093-5)

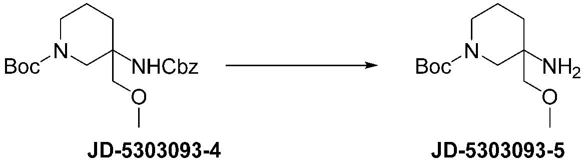

To a solution of compound *tert-*butyl 3-amino-3-(methoxymethyl)piperidine-1-carboxylate **(JD-5303093-5)** (650 mg, crude product, 1.54 mmol, 1.0 eq) in MeOH (5 mL) was added Pd/C (65 mg, 10%). The mixture was then stirred under a H₂ atmosphere at room temperature for 15 hours. The reaction mixture was filtered, and the filtrate was concentrated to obtain a crude oil (480 mg), which was diluted with water (5 mL) and DCM (5 mL). The mixture was added with 1 N HCl to adjust the pH to 3, and extracted with DCM (5 mL * 2). The aqueous phase was added with Na₂CO₃ solution to adjust the pH to 8, and extracted with DCM (5 mL * 3). The combined organic phases were washed with brine (5 mL), dried over Na₂SO₄, filtered, and concentrated under reduced pressure to obtain compound *tert-*butyl 3-amino-3-(methoxymethyl)piperidine-1-carboxylate (JD-5303093-5) (220 mg, 51.7% over three steps) as a colorless oil.

**LCMS:** 245.2 ([M+H]⁺).

Following the synthetic method of Example 1, compound JD-3503001d was replaced with JD-5303093-5 to obtain *N*-[3-(methoxymethyl)piperidin-3-yl]-6- {[(4-methyl-1,3-thiazol-5-yl)methyl]oxy}-3,4-dihydro-2*H*-benzo[*b*][1,4]oxazine-4-carboxamide (JD-5303093) (53 mg, 65.4%) as a yellow solid.

**LCMS:** 433.2 ([M+H]⁺).

**¹H NMR (300 MHz, DMSO-*d*₆):** *δ* 8.98 (s, 1H), 7.32 (d, *J* = 3.0 Hz, 1H), 6.78 (d, *J* = 9.0 Hz, 1H), 6.63 (dd, *J* = 9.0, 3.0 Hz, 1H), 6.40 (s, 1H), 5.17 (s, 2H), 4.23 - 4.06 (m, 2H), 3.87 - 3.77 (m, 1H), 3.75 - 3.58 (m, 3H), 3.46 (d, *J =* 9.0 Hz, 1H), 3.33 (s, 1H), 3.28 (s, 3H), 3.17 - 3.00 (m, 1H), 2.90 - 2.68 (m, 2H), 2.39 (s, 3H), 2.22 - 2.18 (m, 1H), 1.83 - 1.47 (m, 3H).

### Example 93: Preparation of 6-{[(2-fluorophenyl)methyl]oxy}-N-[3-(methoxymethyl)piperidin-3-yl]-3,4-dihydro-2H-benzo[b][1,4]oxazine-4-carboxamide (JD-5303096)

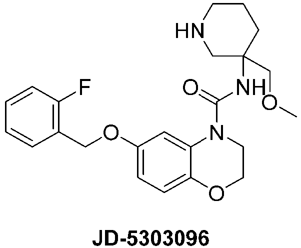

Following the synthetic method of Example 92, JD-3503001c was replaced with JD-5303028a to obtain 6-{[(2-fluorophenyl)methyl]oxy}-*N*-[3-(methoxymethyl)piperidin-3-yl]-3,4-dihydro-2*H*-benzo[*b*][1,4]oxazine-4-carboxamide (JD-5303096) (51 mg, 39.4%) as a white solid.

**LCMS:** 430.2 ([M+H]⁺).

**¹H NMR (300 MHz, DMSO-*d*₆):** *δ* 8.28 (brs, 1H), 7.53 (td, *J* = 7.2, 1.5 Hz, 1H), 7.46 - 7.37 (m, 1H), 7.34 (d, *J =* 2.7 Hz, 1H), 7.29 - 7.16 (m, 2H), 6.78 (d, *J* = 9.0 Hz, 1H), 6.63 (dd, *J =* 9.0, 3.0 Hz, 1H), 6.41 (s, 1H), 5.03 (s, 2H), 4.24 - 4.03 (m, 2H), 3.90 - 3.77 (m, 1H), 3.75 - 3.56 (m, 3H), 3.46 (d, *J =* 9.3 Hz, 1H), 3.28 (s, 3H), 3.15 - 3.00 (m, 1H), 2.90 - 2.65 (m, 2H), 2.22 - 2.08 (m, 1H), 1.86 - 1.43 (m, 3H).

### Example 94: Preparation of N-[3-amino-1-(hydroxymethyl)cyclohexyl]-6-{[(4-methyl-1,3-thiazol-5-yl)methyl]oxyl-3,4-dihydro-2H-benzo[b][1,4]oxazine-4-carboxamide (JD-5303108)

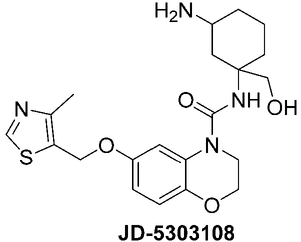

### Step 1: Preparation of benzyl [(2,4-dioxo-1,3-diazaspiro[4.5]decan-7-yl)amino]formate (JD-5303108-2)

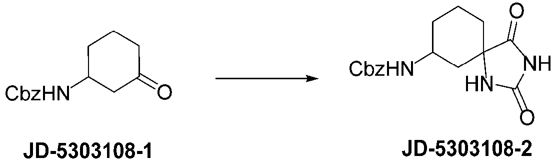

Benzyl (3-oxocyclohexyl)carbamate **(JD-5303108-1)** (5.0 g, 20.22 mmol, 1.0 eq) was dissolved in MeOH (30 mL) and H₂O (30 mL), followed by the addition of (NH₄)₂CO₃ (5.8 g, 60.66 mmol, 3.0 eq) and TMSCN (4.0 g, 40.44 mmol, 2.0 eq), and the mixture was reacted at 80°C for 15 hours. The reaction mixture was concentrated under reduced pressure to obtain a residue. The residue was filtered, and the filter cake was collected to obtain benzyl [(2,4-dioxo-1,3-diazaspiro[4.5]decan-7-yl)amino]formate **(JD-5303108-2)** (5.5 g, crude product) as a yellow solid.

LC-MS ([M+H]⁺) = 318.1.

### Step 2: Preparation of tert-butyl 7-(7,7-dimethyl-3,5-dioxo-1-phenyl-4-aza-2,6-dioxaoctan-4-yl)-1-{[(2-methylprop-2-yl)oxy]carbonyl}-2,4-dioxo-1,3-diazaspiro[4.5]decane-3-carboxylate (JD-5303108-3)

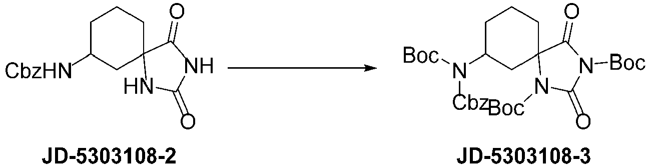

Benzyl (2,4-dioxo-1,3-diazaspiro[4.5]decan-7-yl)carbamate **(JD-5303108-2)** (5.5 g, 17.33 mmol, 1.0 eq) was dissolved in dioxane (60 mL), followed by the addition of TEA (10.5 g, 103.99 mmol, 6.0 eq), (Boc)₂O (18.9 g, 86.66 mmol, 5.0 eq), and DMAP (212 mg, 1.73 mmol, 0.1 eq), and the mixture was reacted at 70°C for 15 hours. The reaction mixture was concentrated under reduced pressure to obtain a residue. The residue was purified by column chromatography (SiO₂, petroleum ether/ethyl acetate = 20/1) to obtain *tert-*butyl 7-(7,7-dimethyl-3,5-dioxo-1-phenyl-4-aza-2,6-dioxaoctan-4-yl)-1-{[(2-methylprop-2-yl)oxy]carbonyl}-2,4-dioxo-1,3-diazaspiro[4.5]decane-3-carboxylate **(JD-5303108-3)** (6.2 g, two-step yield: 49.6%, purity: 94%) as a yellow oil.

LC-MS ([M+H₂O]⁺) = 635.3.

**¹H NMR:** (300 MHz, CDCl₃) *δ* = 7.45 - 7.28 (m, 5 H), 5.35 - 5.24 (m, 2 H), 4.49 - 4.44 (m, 1 H), 2.71 (t, *J =* 6.3 Hz, 1 H), 2.61 - 2.50 (m, 1 H), 2.14 (d, *J* = 10.8 Hz, 1 H), 2.06 - 1.95 (m, 1 H), 1.89 - 1.76 (m, 2 H), 1.58 - 1.55 (m, 2 H), 1.54 - 1.47 (m, 18 H), 1.46 - 1.41 (m, 9 H).

### Step 3: Preparation of 1-amino-3-(7,7-dimethyl-3,5-dioxo-1-phenyl-4-aza-2,6-dioxaoctan-4-yl)cyclohexane-1-carboxylic acid (JD-5303108-4)

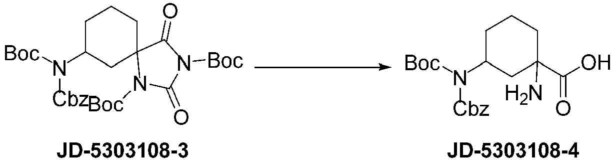

*tert-*Butyl 7-(7,7-dimethyl-3,5-dioxo-1-phenyl-4-aza-2,6-dioxaoctan-4-yl)-1-{[(2-methylprop-2-yl)oxy]carbonyl}-2,4-dioxo-1,3-diazaspiro[4.5]decane-3-carboxylate **(JD-5303108-3)** (6.2 g, 10.04 mmol, 1.0 eq) was dissolved in THF (30 mL) and H₂O (30 mL), followed by the addition of LiOH·H₂O (3.37 g, 80.30 mmol, 8.0 eq), and the mixture was reacted at room temperature for 15 hours. The reaction mixture was concentrated under reduced pressure to obtain 1-amino-3-(7,7-dimethyl-3,5-dioxo-1-phenyl-4-aza-2,6-dioxaoctan-4-yl)cyclohexane-1-carboxylic acid **(JD-5303108-4)** (7.0 g, crude product) as a white solid.

LC-MS ([M-H]⁻) = 391.2.

### Step 4: Preparation of 1-amino-3-{[(benzyloxy)carbonyl]amino}cyclohexane-1-carboxylic acid (JD-5303108-5)

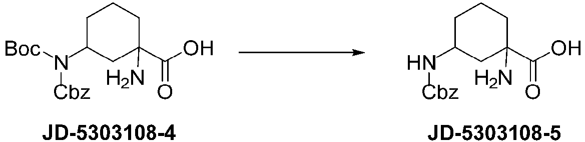

1-Amino-3-(7,7-dimethyl-3,5-dioxo-1-phenyl-4-aza-2,6-dioxaoctan-4-yl)cyclohexane-1-carboxylic acid **(JD-5303108-4)** (7.0 g crude, 10.04 mmol, 1.0 eq) was suspended in DCM (50 mL), then HCl/dioxane (4.0 M) (75.3 mL, 301.2 mmol, 30.0 eq) was added thereto, and the mixture was reacted at room temperature for 15 hours. The reaction mixture was concentrated under reduced pressure to obtain 1-amino-3-(7,7-dimethyl-3,5-dioxo-1-phenyl-4-aza-2,6-dioxaoctan-4-yl)cyclohexane-1-carboxylic acid **(JD-5303108-5)** (8.0 g, crude product) as a white solid.

LC-MS ([M+H]⁺) = 293.1.

### Step 5: Preparation of methyl 1-amino-3-{[(benzyloxy)carbonyl]amino}cyclohexane-1-carboxylate (JD-5303108-6)

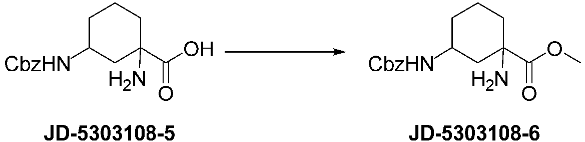

1-Amino-3-(7,7-dimethyl-3,5-dioxo-1-phenyl-4-aza-2,6-dioxaoctan-4-yl)cyclohexane-1-carboxylic acid **(JD-5303108-5)** (4.0 g crude, 5.02 mmol, 1.0 eq) was dissolved in MeOH (50 mL), followed by the addition of MeSO₃H (2.5 mL), and the mixture was reacted at 65°C for 15 hours. The reaction mixture was added with saturated sodium carbonate solution to adjust the pH to 9, and concentrated under reduced pressure to obtain a residue. The residue was washed with dichloromethane/methanol (10/1) (90 mL, 30.0 mL * 3). The mixture was filtered, and the collected filtrate was concentrated under reduced pressure to obtain a residue. The residue was purified by column chromatography (SiO₂, petroleum ether/ethyl acetate = 80/1) to obtain methyl 1-amino-3-{[(benzyloxy)carbonyl]amino}cyclohexane-1-carboxylate **(JD-5303108-6)** (170 mg, three-step yield: 11.1%, purity: 90%) as a yellow oil.

LC-MS ([M+H]⁺) = 307.1.

**¹H NMR:** (300 MHz, CDCl₃) *δ* = 7.38 - 7.31 (m, 5 H), 5.07 (s, 2 H), 4.63 (d, *J* = 6.3 Hz, 1 H), 4.04 - 3.89 (m, 1 H), 3.70 (s, 3 H), 2.02 -1.79 (m, 3 H), 1.70 - 1.56 (m, 6 H), 1.20 - 1.07 (m, 1 H).

### Step 6: Preparation of benzyl {[3-(methoxycarbonyl)-3-{[(6-{[(4-methyl-1,3-thiazol-5-yl)methyl]oxy}-3,4-dihydro-2H-benzo[b][1,4]oxazin-4-yl)carbonyl]amino}cyclohexyl]amino}formate (JD-5303108-7)

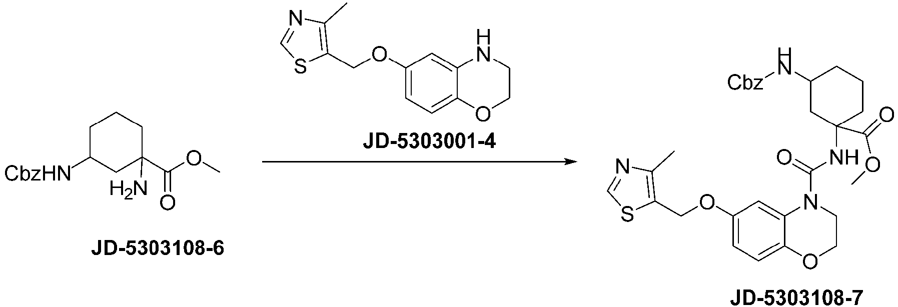

Methyl 1-amino-3-{[(benzyloxy)carbonyl]amino}cyclohexane-1-carboxylate **(JD-5303108-6)** (140 mg, 0.46 mmol, 1.2 eq) and TEA (193 mg, 1.91 mmol, 5.0 eq) were dissolved in DCM (2.0 mL), followed by the addition of (COCl₂)₃ (57 mg, 0.19 mmol, 0.5 eq), and the mixture was reacted at room temperature for 0.5 hours. A solution of **Int-1** (100 mg, 0.38 mmol, 1.0 eq) in DCM (1 mL) was then added thereto, and the mixture was stirred at 40°C for 15 hours. The reaction system was quenched with water (5 mL) and extracted with DCM (10 mL, 5 mL * 2). The combined organic phases were washed with brine (5 mL), dried over anhydrous sodium sulfate, and concentrated under reduced pressure to obtain a residue. The residue was purified by column chromatography (SiO₂, petroleum ether/ethyl acetate = 1/1) to obtain benzyl {[3-(methoxycarbonyl)-3-{[(6-{[(4-methyl-1,3-thiazol-5-yl)methyl]oxy}-3,4-dihydro-2*H*-benzo[*b*][1,4]oxazin-4-yl)carbonyl]amino}cyclohexyl]amino}formate **(JD-5303108-7)** (150 mg, yield: 66.1%, purity: 90%) as a yellow solid.

LC-MS ([M+H]⁺) = 595.2.

### Step 7: Preparation of methyl 3-amino-1-{[(6-{[(4-methyl-1,3-thiazol-5-yl)methyl]oxy}-3,4-dihydro-2H-benzo[b][1,4]oxazin-4-yl)carbonyl]amino}cyclohexane-1-carboxylate (JD-5303108-8)

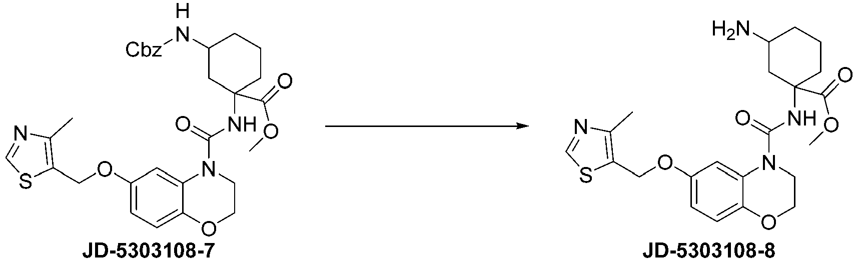

Benzyl {[3-(methoxycarbonyl)-3-{[(6-{[(4-methyl-1,3-thiazol-5-yl)methyl]oxy}-3,4-dihydro-2*H*-benzo[*b*][1,4]oxazin-4-yl)carbonyl]amino } cyclohexyl]amino}formate **(JD-5303108-7)** (150 mg, 0.25 mmol, 1.0 eq) was dissolved in DCM (5 mL), then TMSI (151 mg, 0.75 mmol, 3.0 eq) was added thereto, and the mixture was stirred at room temperature for 4 hours. The reaction mixture was quenched with water (5 mL) and extracted with DCM (10 mL, 5 mL * 2). The aqueous phase was then added with saturated sodium bicarbonate solution to adjust the pH to 9, and extracted with DCM/MeOH (8/1) (27 mL, 9 mL * 3). The combined organic phases were washed with brine (10 mL), dried over anhydrous sodium sulfate, and concentrated under reduced pressure to obtain a residue. The residue was purified by column chromatography (SiO₂, dichloromethane/methanol = 20/1) to obtain methyl 3-amino-1-{[(6-{[(4-methyl-1,3-thiazol-5-yl)methyl]oxy}-3,4-dihydro-2*H-*benzo[*b*][1,4]oxazin-4-yl)carbonyl]amino}cyclohexane-1-carboxylate **(JD-5303108-8)** (60 mg, yield: 51.7%, purity: 90%) as a yellow solid.

LC-MS ([M+H]⁺) = 461.2.

### Step 8: Preparation of N-[3-amino-1-(hydroxymethyl)cyclohexyl]-6-{[(4-methyl-1,3-thiazol-5-yl)methyl]oxy}-3,4-dihydro-2H-benzo[b][1,4]oxazine-4-carboxamide (JD-5303108)

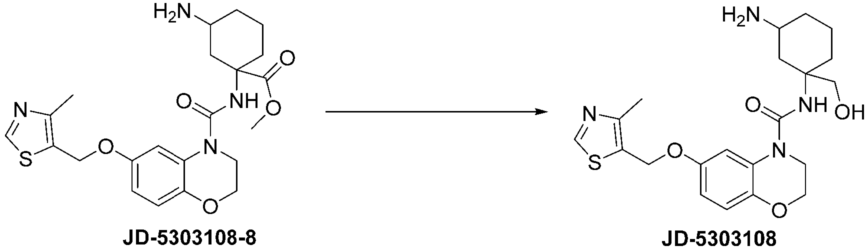

**53-8** (60 mg, 0.13 mmol, 1.0 eq) was dissolved in THF (5 mL), and the mixture was cooled to 0°C. LiAlH₄ (1.0 M in THF) (0.26 mL, 0.26 mmol, 2.0 eq) was then added thereto, and the mixture was reacted at 0°C for 1 hour. The reaction mixture was quenched by dropwise addition of NaOH solution (15%) (0.1 mL) at 0°C and dried over Na₂SO₄. The mixture was filtered, and the collected filtrate was concentrated under reduced pressure to obtain a residue. The residue was purified by prep-HPLC (column: WelchUlC18 150 * 25 mm * 5 mm; mobile phase: [water (0.1% TFA)-ACN]; B%: 25% to 45%, 6 minutes) to obtain a product. The product was dissolved in water (5 mL), then added with saturated sodium hydroxide aqueous solution to adjust the pH to 9, and extracted with DCM (5 mL * 2). The combined organic phases were washed with brine (5 mL), dried over anhydrous sodium sulfate, and concentrated under reduced pressure to obtain **JD01-LHNY-53** (4.5 mg, yield: 8.0%, purity: 95.3%) as a white solid.

LC-MS ([M+H]⁺) = 433.3.

**¹H NMR:** (300 MHz, DeOD) *δ* = 8.89 (s, 1 H), 7.17 (d, *J =* 3.0 Hz, 1 H), 6.82 (d, *J* = 9.0 Hz, 1 H), 6.69 (dd, *J* = 9.0, 3.0 Hz, 1 H), 5.19 (s, 2 H), 4.22 - 4.15 (m, 2 H), 3.87 - 3.60 (m, 4 H), 2.93 - 2.80 (m, 1 H), 2.53 - 2.41 (m, 4 H), 2.12 (d, *J =* 13.2 Hz, 1 H), 1.93 (d, *J* = 11.7 Hz, 1 H), 1.74 - 1.63 (m, 1 H), 1.60 - 1.45 (m, 1 H), 1.26 - 1.09 (m, 3 H).

### Example 95: Preparation of N-[3-amino-1-(hydroxymethyl)cyclohexyl]-6-{[(2-fluorophenyl)methyl]oxy}-3,4-dihydro-2H-benzo[b][1,4]oxazine-4-carboxamide (JD-5303111)

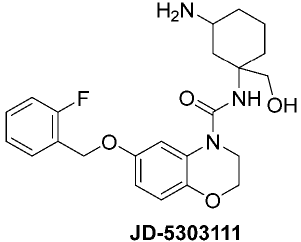

Following the synthetic method of Example 94, compound JD-5303001-4 was replaced with JD-5303028-3 to obtain *N*-[3-amino-1-(hydroxymethyl)cyclohexyl]-6-{[(2-fluorophenyl)methyl]oxyl-3,4-dihydro-2*H*-benzo[b][1,4]oxazine-4-carboxamide (JD-5303111) (19 mg, yield: 20.2%, purity: 95.9%) as a white solid.

LC-MS ([M+H]⁺) = 430.3.

**¹H NMR:** (300 MHz, DMSO-*d*₆) *δ* = 7.53 (td, *J* = 7.5, 1.5 Hz, 1 H), 7.48 - 7.34 (m, 1 H), 7.30 - 7.14 (m, 3 H), 6.77 (d, *J* = 9.0 Hz, 1 H), 6.61 (dd, *J =* 8.7, 2.7 Hz, 1 H), 6.28 - 5.80 (m, 2 H), 5.02 (s, 2 H), 4.23 - 4.00 (m, 2 H), 3.87 - 3.70 (m, 1 H), 3.68 - 3.57 (m, 1 H), 3.55 - 3.45 (m, 2 H), 3.38 (s, 2 H), 3.08 - 2.88 (m, 1 H), 2.14 - 1.95 (m, 1 H), 1.93 - 1.74 (m, 1 H), 1.70 - 1.38 (m, 2 H), 1.35 - 0.97 (m, 4 H).

### Example 96: Preparation of benzyl 4,4-difluoro-3-{[(6-{[(4-methyl-1,3-thiazol-5-yl)methyl]oxy}-3,4-dihydro-2H-benzo[b][1,4]oxazin-4-yl)carbonyl]amino}piperidine-1-carboxylate (JD-5303105)

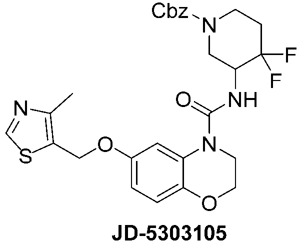

Following Step 5 of the synthetic method of Example 84, compound JD-5303104-5 was replaced with JD-5303001-4 to obtain benzyl 4,4-difluoro-3-{[(6-{[(4-methyl-1,3-thiazol-5-yl)methyl]oxy}-3,4-dihydro-2*H*-benzo[*b*][1,4]oxazin-4-yl)carbonyl]amino}piperidine-1-carboxylate (JD-5303105) (95 mg, yield: 69.4%, purity: 99%) as a white solid.

LC-MS ([M+H]⁺) = 559.2.

**¹H NMR:** (300 MHz, DMSO-*d*₆) *δ* = 8.87 (s, 1H), 7.42 - 7.23 (m, 5H), 7.17 (d,*J* = 3.0 Hz, 1H), 6.81 (d, *J =* 9.0 Hz, 1H), 6.66 (dd, *J =* 9.0, 2.7 Hz, 1H), 5.16 (s, 2H), 5.12 (s, 2H), 4.38 - 4.19 (m, 2H), 4.17 - 3.95 (m, 4H), 3.57 - 3.40 (m, 1H), 3.28 - 3.06 (m, 2H), 2.42 (s, 3H), 2.30 - 1.90 (m, 2H).

### Example 97: Preparation ofN-(4,4-difluoropiperidin-3-yl)-6-{[(4-methyl-1,3-thiazol-5-yl)methyl]oxy}-3,4-dihydro-2H-benzo[b][1,4]oxazine-4-carboxamide (JD-5303106)

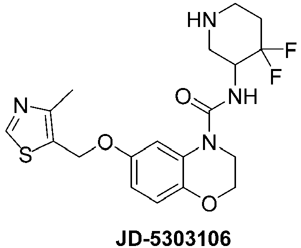

Following the synthetic method of Example 84, compound JD-5303104-5 was replaced with JD-5303001-4 to obtain *N*-(4,4-difluoropiperidin-3-yl)-6-{[(4-methyl-1,3-thiazol-5-yl)methyl]oxy}-3,4-dihydro-2H-benzo[b][1,4]oxazine-4-carboxamide (JD-5303106) (10 mg, yield: 18.8%, purity: 99%) as a white solid.

LC-MS ([M+H]⁺) = 425.2.

**¹H NMR:** (300 MHz, DMSO-*d*₆) *δ* = 8.98 (s, 1H), 7.27 (d, *J =* 3.0 Hz, 1H), 6.96 (d, *J* = 9.0 Hz, 1H), 6.79 (d, *J* = 9.0 Hz, 1H), 6.62 (dd, *J* = 9.0, 3.0 Hz, 1H), 5.16 (s, 2H), 4.30 - 3.98 (m, 3H), 3.97 - 3.82 (m, 1H), 3.60 - 3.45 (m, 1H), 2.98 - 2.80 (m, 2H), 2.70 - 2.56 (m, 2H), 2.38 (s, 3H), 2.17 - 1.73 (m, 2H).

### Example 98: Preparation of N-methyl-N-[3-(methylamino)cyclohexyl]-6-{[(4-methyl-1,3-thiazol-5-yl)methyl]oxy}-3,4-dihydro-2H-benzo[b][1,4]oxazine-4-carboxamide (JD-5303082)

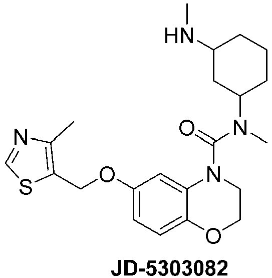

### Step 1: Preparation of N-methyl-N-[3-(methylamino)cyclohexyl]-6-{[(4-methyl-1,3-thiazol-5-yl)methyl]oxy}-3,4-dihydro-2H-benzo[b][1,4]oxazine-4-carboxamide (JD-5303082)

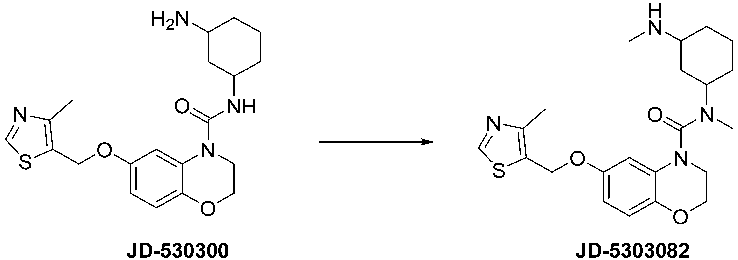

*N*-(3-Aminocyclohexyl)-6-{[(4-methyl-1,3-thiazol-5-yl)methyl]oxy}-3,4-dihydro-2*H*-benzo[*b*][1,4]oxazine-4-carboxamide **(JD-5303008)** (100 mg, 0.25 mmol, 1.0 eq) was dissolved in MeOH (2 mL), then (HCHO)ₙ (11 mg, 0.37 mmol, 1.5 eq) was added thereto, and the mixture was reacted at room temperature for 0.5 hours. NaBH₃CN (31 mg, 0.5 mmol, 2.0 eq) was then added thereto, and the mixture was stirred at 50°C for 11.5 hours. The reaction mixture was concentrated to obtain a crude product, which was diluted with water (8 mL) and extracted with dichloromethane (20 mL, 10 mL * 2). The combined organic phases were washed with brine (8 mL), dried over anhydrous sodium sulfate, and concentrated under reduced pressure to obtain a residue. The residue was purified by column chromatography (SiO₂, dichloromethane/methanol = 30/1 to 10/1) to obtain N-methyl-N-[3-(methylamino)cyclohexyl]-6-{[(4-methyl-1,3-thiazol-5-yl)methyl]oxy}-3,4-dihydro-2*H-*benzo[*b*][1,4]oxazine-4-carboxamide **(JD-5303082)** (12.9 mg, yield: 12.1%, purity: 97%) as a yellow solid.

LC-MS ([M+H]⁺) = 431.2.

**¹H NMR** (300 MHz, DMSO-*d*₆) *δ* = 10.43 (brs, 1H), 8.97 (s, 1H), 7.34 (d, *J =* 3.0 Hz, 1H), 6.76 (d, *J* = 8.7 Hz, 1H), 6.70 (d, *J* = 5.7 Hz, 1H), 6.60 (dd, *J* = 9.0, 3.0 Hz, 1H), 5.17 (s, 2H), 4.18 - 4.00 (m, 3H), 3.79 - 3.60 (m, 2H), 2.73 - 2.56 (m, 6H), 2.39 (s, 3H), 2.10 - 1.41 (m, 8H).

### Comparative Example 1: Preparation of N-(piperidin-3-yl)-7-{[(4-methyl-1,3-thiazol-5-yl)methyl]oxy}-2,3-dihydro-1H-pyrido[2,3-b][1,4]oxazine-1-carboxamide trifluoroacetate (JD-5303065)

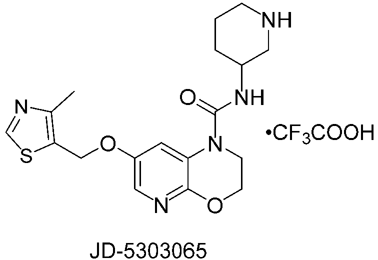

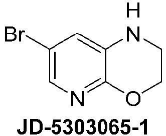

Following the synthetic method of Example 1, compound **JD-5303001-1** was replaced with **JD-5303065-1** to obtain *N*-(piperidin-3-yl)-7-{[(4-methyl-1,3-thiazol-5-yl)methyl]oxy}-2,3-dihydro-1*H*-pyrido[2,3-*b*][1,4]oxazine-1-carboxamide trifluoroacetate **(JD-5303065)** (1.42 mg, 3.57 µmol, yield: 34.9%, purity: 98%) as a yellow gum.

LCMS [389+H]⁺ = 390.

¹H NMR (400 MHz, DMSO-*d*₆) δ ppm 1.47 - 1.70 (m, 2 H) 1.82 - 1.95 (m, 2 H) 2.40 (s, 3 H) 2.70 - 2.84 (m, 2 H) 3.18 (br d, *J =* 11.51 Hz, 1 H) 3.28 - 3.31 (m, 1 H) 3.64 - 3.77 (m, 2 H) 3.84 - 3.96 (m, 1 H) 4.20 - 4.32 (m, 2 H) 5.27 (s, 2 H) 7.09 (br d, *J =* 7.13 Hz, 1 H) 7.62 (d, *J =* 2.63 Hz, 1 H) 7.94 (d, *J =* 2.75 Hz, 1 H) 8.55 (br s, 2 H) 9.00 (s, 1 H).

### Comparative Example 2: Preparation of N-(piperidin-3-yl)-6-{[(4-methyl-1,3-thiazol-5-yl)methyl]oxy}-3,4-dihydro-2H-pyrido[3,2-b][1,4]oxazine-4-carboxamide (JD-5303066)

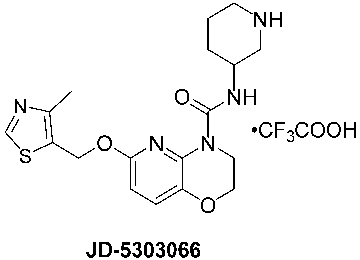

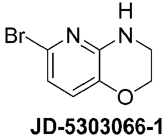

Following the synthetic method of Example 1, compound **JD-5303001-1** was replaced with **JD-5303066-1** to obtain *N*-(piperidin-3-yl)-7-{[(4-methyl-1,3-thiazol-5-yl)methyl]oxy}-2,3-dihydro-1*H*-pyrido[2,3-*b*][1,4]oxazine-1-carboxamide trifluoroacetate **(JD-5303066)** (22.4 mg, purity: 98.83%) as a yellow gum.

LC-MS [389+H]⁺ = 390.

**¹H NMR:** (400 MHz, METHANOL-*d*₄) *δ* = 1.55 (qd, *J =* 11.63, 3.38 Hz, 1 H), 1.63 - 1.74 (m, 1 H) 1.81 - 1.90 (m, 1 H), 1.98 - 2.08 (m, 1 H), 2.42 (s, 3 H), 2.81 (br d, *J* = 11.13 Hz, 2 H), 3.19 (br d, *J =* 12.01 Hz, 1 H), 3.42 (br d, *J=* 10.26 Hz, 1 H), 3.89 (br dd, *J =* 6.44, 3.06 Hz, 1 H), 3.95 - 4.04 (m, 2 H), 4.12 - 4.23 (m, 2 H), 5.41 (d, *J =* 3.38 Hz, 2 H), 6.53 (d, *J =* 8.63 Hz, 1 H), 7.39 (d, *J =* 8.63 Hz, 1 H), 8.54 - 8.83 (m, 2 H), 9.01 (s, 1 H), 9.26 (d, *J =* 6.75 Hz, 1 H).

### Bioassay Example

### 1. hTRPM3 calcium flux assay

In this assay, the FLIPR^{®} Calcium 5 Assay Kit was utilized to screen small molecules. HeLa cells transfected with human TRPM3 protein for approximately 24 hours were seeded in a 96-well plate and cultured until reaching appropriate density. The culture medium was discarded, and a cell-permeable calcium indicator (FLIPR Calcium 5 Assay Kit) prepared in extracellular buffer was added. The cells were then co-incubated at 37°C with 5% CO₂ for 50 minutes. Subsequently, small molecule solutions at varying concentrations, along with a positive inhibitor, were added to the 96-well plate, with a blank control included. The incubation continued for another 15 minutes. The small molecules were prepared as a 10 mM stock solution in DMSO and diluted to the desired concentrations using a dosing solvent (10% DMSO + 90% HBSS). The blank control consisted of extracellular buffer containing an equivalent concentration of the solvent DMSO. All steps were performed under light-protected conditions. After incubation, the 96-well plate was placed in a FlexStation 3 multimode microplate reader. A pre-loaded sample plate containing the TRPM3-specific agonist pregnenolone sulfate (PregS) was installed in the injector of the microplate reader, with a set concentration of 60 µM (final concentration in wells of 15 µM). The test program was configured to measure cellular fluorescence with excitation at 485 nm and emission at 525 nm. Each well was monitored for 90 seconds, with a data acquisition interval of 1.52 seconds. After recording baseline fluorescence for 25 seconds, the positive agonist PregS (final concentration of 15 µM) was added via the injector, and the remaining data points were collected. Upon completion of the plate measurements, the quantified fluorescence intensity change curves generated by the instrument were used to calculate the fluorescence increase before and after agonist addition for all wells. The values were normalized against the fluorescence increase of the corresponding blank control wells to determine the inhibition rate of fluorescence intensity under different small molecule concentrations. A curve-fitting model (Y=100/(1+10^((LogIC50-X)*HillSlope))) was applied to generate inhibition curves for concentration logarithm versus inhibition rate. All graphs were generated by GraphPad Prism version 8.0.

**Table 1: Results of hTRPM3 calcium flux assay**

| Compound | IC₅₀ | Compound | IC₅₀ |
|---|---|---|---|
| JD-5303001 | B | JD-5303054 | B |
| JD-5303002 | A | JD-5303055 | A |
| JD-5303003 | A | JD-5303056 | A |
| JD-5303004 | A | JD-5303057 | A |
| JD-5303005 | B | JD-5303058 | A |
| JD-5303006 | A | JD-5303059 | A |
| JD-5303007 | A | JD-5303060 | A |
| JD-5303008 | A | JD-5303072 | B |
| JD-5303009 | A | JD-5303073 | B |
| JD-5303010 | A | JD-5303074 | A |
| JD-5303011 | B | JD-5303075 | A |
| JD-5303012 | B | JD-5303075A | A |
| JD-5303013 | A | JD-5303075B | B |
| JD-5303014 | A | JD-5303076 | A |
| JD-5303015 | B | JD-5303077 | B |
| JD-5303016 | B | JD-5303079 | B |
| JD-5303017 | B | JD-5303080 | A |
| JD-5303018 | A | JD-5303081 | A |
| JD-5303019 | A | JD-5303082 | A |
| JD-5303020 | B | JD-5303083 | B |
| JD-5303021 | B | JD-5303084 | A |
| JD-5303022 | A | JD-5303086 | B |
| JD-5303023 | B | JD-5303087 | B |
| JD-5303024 | B | JD-5303088 | B |
| JD-5303025 | B | JD-5303089 | B |
| JD-5303026 | B | JD-5303090 | A |
| JD-5303027 | B | JD-5303091 | A |
| JD-5303028 | B | JD-5303092 | B |
| JD-5303029 | B | JD-5303093 | A |
| JD-5303030 | A | JD-5303094 | A |
| JD-5303031 | A | JD-5303095 | A |
| JD-5303032 | B | JD-5303096 | A |
| JD-5303033 | B | JD-5303097 | A |
| JD-5303034 | A | JD-5303098 | B |
| JD-5303035 | A | JD-5303099 | B |
| JD-5303036 | A | JD-5303100 | A |
| JD-5303037 | B | JD-5303101 | B |
| JD-5303038 | B | JD-5303102 | A |
| JD-5303039 | A | JD-5303103 | B |
| JD-5303040 | B | JD-5303104 | A |
| JD-5303041 | A | JD-5303105 | B |
| JD-5303042 | B | JD-5303106 | A |
| JD-5303043 | B | JD-5303107 | A |
| JD-5303044 | B | JD-5303108 | B |
| JD-5303049 | A | JD-5303109 | A |
| JD-5303050 | A | JD-5303110 | A |
| JD-5303051 | A | JD-5303111 | A |
| JD-5303052 | B | JD-5303065 of Comparative Example 1 | C |
| JD-5303053 | B | JD-5303066 of Comparative Example 2 | C |

| | | | |
|---|---|---|---|
| "A": IC₅₀ ≤ 1 µM; "B": 1 µM < IC₅₀ ≤ 10 µM; "C": IC₅₀ > 10 µM. | | | |

The test results demonstrated that the compounds of the present disclosure exhibited good hTRPM3 inhibition, with some compounds achieving 1 µM hTRPM3 inhibition.

## Claims

1. A compound I, a stereoisomer thereof, or a pharmaceutically acceptable salt thereof, 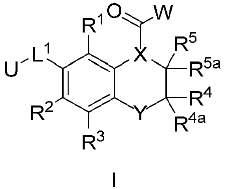 wherein
X is N or CH;
Y is O or -CH₂-;
L¹ is -L^{a}-L^{b}-L^{c}-L^{d}-;
L^{a}, L^{b}, L^{c}, and L^{d} are each independently a direct bond, 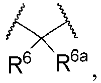
O, S, or 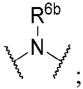 and L^{a}, L^{b}, L°, and L^{d} are not simultaneously a direct bond; the number of O, S**,** and 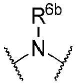 in L^{a}, L^{b}, L^{c}, and L^{d} is 1 or 2;
R⁶, R^{6a}, and R^{6b} are each independently H, C₁-C₆ alkyl, or C₁-C₆ alkyl substituted by one or more R⁶⁻¹;
each R⁶⁻¹ is independently hydroxyl, halogen, amino, -NHC₁-C₆ alkyl, or -N(C₁-C₆ alkyl)₂;
U is 3- to 12-membered heterocycloalkyl, C₆-C₁₀ aryl, 5- to 10-membered heteroaryl, 3-to 8-membered cycloalkyl, C₆-C₁₀ aryl substituted by one or more R⁹⁻¹, 5- to 10-membered heteroaryl substituted by one or more R⁹⁻², or 3- to 8-membered cycloalkyl substituted by one or more R^{9-a};
R⁹⁻¹, R⁹⁻², and R^{9-a} are each independently halogen, cyano, hydroxyl, C₁-C₆ alkyl, C₁-C₆ alkoxy, 3- to 8-membered cycloalkyl, 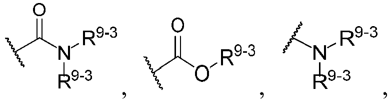 oxo, -O-(3- to 8-membered cycloalkyl), or C₁-C₆ alkyl substituted by one or more R⁹⁻⁴;
each R⁹⁻³ is independently H, C₁-C₆ alkyl, or 3- to 8-membered cycloalkyl;
each R⁹⁻⁴ is independently hydroxyl, halogen, or C₁-C₆ alkoxy;
R¹, R², and R³ are each independently H, halogen, cyano, C₁-C₆ alkyl, C₁-C₆ alkoxy, or C₁-C₆ alkyl substituted by one or more halogens;
R⁴, R^{4a}, R^{5a}, and R⁵ are each independently H, C₁-C₆ alkyl, C₁-C₆ alkoxy, 3- to 8-membered cycloalkyl, or C₁-C₆ alkyl substituted by one or more halogens;
or, R⁴ and R^{4a} together with the carbon atom to which they are attached form a 3- to 8-membered cycloalkyl ring;
W is L²R⁷;
L² is a direct bond, -CH₂-, -NH-, or -N(C₁-C₆ alkyl)-;
R⁷ is 3- to 8-membered cycloalkyl, 3- to 12-membered heterocycloalkyl, 5- to 10-membered heteroaryl, 3- to 12-membered heterocycloalkyl substituted by one or more R⁷⁻¹, 5-to 10-membered heteroaryl substituted by one or more R⁷⁻², C₁-C₆ alkyl substituted by one or more R⁷⁻³, or 3- to 8-membered cycloalkyl substituted by one or more R⁷⁻⁴;
R⁷⁻¹, R⁷⁻², and R⁷⁻⁴ are each independently halogen, hydroxyl, oxo, amino, -NH(C₁-C₆ alkyl), -N(C₁-C₆ alkyl)₂, C₁-C₆ alkyl, C₁-C₆ alkoxy, 3- to 8-membered cycloalkyl, -O-(3- to 8-membered cycloalkyl), 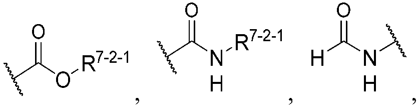 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, amino, C₁-C₆ alkoxy, C₂-C₆ alkenyl, C₂-C₆ alkynyl, -NH(C₁-C₆ alkyl), -N(C₁-C₆ alkyl)₂, 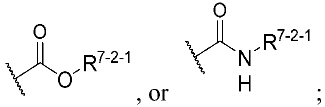
each R⁷⁻³ is independently halogen, hydroxyl, amino, C₁-C₆ alkoxy, -NH(C₁-C₆ alkyl), - N(C₁-C₆ alkyl)₂, 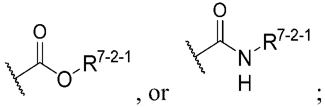
each R⁷⁻²⁻¹ is independently H, C₁-C₆ alkyl, 3- to 8-membered cycloalkyl, or C₁-C₆ alkyl substituted by one or more C₆-C₁₀ aryl;
the heteroatom in the 3- to 12-membered heterocycloalkyl is selected from one or more of N, O, and S; the number of heteroatoms is 1, 2, or 3;
the heteroatom in the 5- to 10-membered heteroaryl is selected from one or more of N, O, and S; the number of heteroatoms is 1, 2, or 3.

2. The compound I, the stereoisomer thereof, or the pharmaceutically acceptable salt thereof according to claim 1, wherein
L¹ is 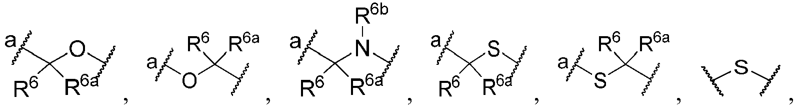 or 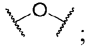 wherein the a-end is attached to U;
U is 3- to 12-membered heterocycloalkyl, C₆-C₁₀ aryl, 5- to 10-membered heteroaryl, C₆-C₁₀ aryl substituted by one or more R⁹⁻¹, or 5- to 10-membered heteroaryl substituted by one or more R⁹⁻²;
R⁹⁻¹ and R⁹⁻² are each independently halogen, cyano, hydroxyl, C₁-C₆ alkyl, C₁-C₆ alkoxy, 3- to 8-membered cycloalkyl, 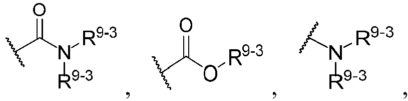 oxo, -O-(3- to 8-membered cycloalkyl), or C₁-C₆ alkyl substituted by one or more R⁹⁻⁴;
R⁷⁻¹, R⁷⁻², and R⁷⁻⁴ are each independently halogen, hydroxyl, oxo, amino, -NH(C₁-C₆ alkyl), -N(C₁-C₆ alkyl)₂, C₁-C₆ alkyl, C₁-C₆ alkoxy, 3- to 8-membered cycloalkyl, -O-(3- to 8-membered cycloalkyl), 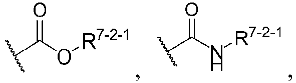 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, amino, C₂-C₆ alkenyl, C₂-C₆ alkynyl, -NHC₁-C₆ alkyl, -N(C₁-C₆ alkyl)₂, 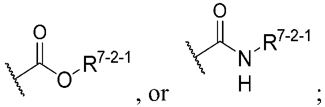
each R⁷⁻²⁻¹ is independently H, C₁-C₆ alkyl, or 3- to 8-membered cycloalkyl.

3. The compound I, the stereoisomer thereof, or the pharmaceutically acceptable salt thereof according to claim 1 or 2, wherein the compound I satisfies one or more of the following conditions:
(1) each C₁-C₆ alkyl is independently methyl, ethyl, *n*-propyl, isopropyl, *n*-butyl, isobutyl, or *tert-*butyl*,* preferably methyl;
(2) each C₂-C₆ alkenyl is independently vinyl, propenyl, allyl, butenyl, or pentenyl, preferably vinyl;
(3) each C₂-C₆ alkynyl is independently ethynyl, propynyl, propargyl, butynyl, or pentynyl;
(4) each C₁-C₆ alkoxy is independently methoxy, ethoxy, *n*-propoxy, isopropoxy, *n*-butoxy, isobutoxy, or *tert*-butoxy, preferably methoxy;
(5) each halogen is independently fluorine, chlorine, bromine, or iodine;
(6) the heteroatom in each 3- to 12-membered heterocycloalkyl is independently N; the number of heteroatoms may independently be 1 or 2;
(7) each 3- to 12-membered heterocycloalkyl is independently 4- to 10-membered heterocycloalkyl;
(8) each 3- to 12-membered heterocycloalkyl is independently a monocyclic ring or a bicyclic ring, wherein the bicyclic ring may be a bridged ring or a spiro ring;
(9) each C₆-C₁₀ aryl is independently phenyl or naphthyl, preferably phenyl;
(10) each 5- to 10-membered heteroaryl is independently 5- to 6-membered heteroaryl;
(11) the heteroatom in each 5- to 10-membered heteroaryl is independently N, S, or O; the number of heteroatoms may independently be 1 or 2;
(12) each 5- to 10-membered heteroaryl is independently a monocyclic ring or a bicyclic ring;
(13) each 3- to 8-membered cycloalkyl is independently cyclopropyl, cyclobutyl, cyclopentyl, or cyclohexyl, preferably cyclopropyl or cyclobutyl;
(14) the pharmaceutically acceptable salt is hydrochloride, formate, or trifluoroacetate.

4. The compound I, the stereoisomer thereof, or the pharmaceutically acceptable salt thereof according to claim 1 or 2, wherein the compound I satisfies one or more of the following conditions:
(1) X is N;
(2) Y is O;
(3) L¹ is 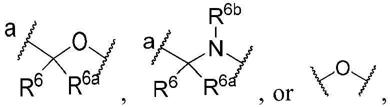 preferably 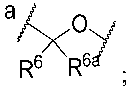 wherein the a-end is attached to U;
(4) R⁶, R^{6a}, and R^{6b} are each independently H or C₁-C₆ alkyl, preferably H;
(5) U is C₆-C₁₀ aryl, 5- to 10-membered heteroaryl, C₆-C₁₀ aryl substituted by one or more R⁹⁻¹, or 5- to 10-membered heteroaryl substituted by one or more R⁹⁻²;
(6) R⁹⁻¹ and R⁹⁻² are each independently halogen, hydroxyl, C₁-C₆ alkyl, C₁-C₆ alkoxy, 3-to 8-membered cycloalkyl, 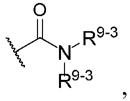 or C₁-C₆ alkyl substituted by one or more R⁹⁻⁴;
(7) each R⁹⁻³ is independently H or C₁-C₆ alkyl, preferably H;
(8) R⁹⁻⁴ is halogen;
(9) R¹, R², and R³ are each independently H or halogen; preferably, R¹ and R³ are H, and R² is H or halogen;
(10) R^{4a} is H;
(11) R⁵ and R^{5a} are H;
(12) R⁴ is H or C₁-C₆ alkyl, preferably H;
(13) L² is a direct bond, -CH₂-, or -NH-, preferably -NH-;
(14) R⁷ is 3- to 12-membered heterocycloalkyl, 5- to 10-membered heteroaryl, 3- to 12-membered heterocycloalkyl substituted by one or more R⁷⁻¹, 5- to 10-membered heteroaryl substituted by one or more R⁷⁻², or 3- to 8-membered cycloalkyl substituted by one or more R⁷⁻⁴;
preferably, R⁷ is 3- to 12-membered heterocycloalkyl or 3- to 12-membered heterocycloalkyl substituted by one or more R⁷⁻¹;
more preferably, R⁷ is piperidinyl, tetrahydropyrrolyl, tetrahydropyrrolyl substituted by one or more halogens, or piperidinyl substituted by one or more halogens;
further preferably, R⁷ is piperidinyl substituted by 1 or 2 F or tetrahydropyrrolyl substituted by 1 or 2 F;
(15) R⁷⁻¹, R⁷⁻², and R⁷⁻⁴ are each independently halogen, amino, -NHC₁-C₆ alkyl, -N(C₁-C₆ alkyl)₂, C₁-C₆ alkyl, 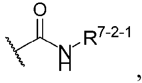 C₁-C₆ alkyl substituted by one or more R⁷⁻¹⁻¹, or C₁-C₆ alkoxy substituted by one or more R⁷⁻¹⁻²;
(16) R⁷⁻¹⁻¹ and R⁷⁻¹⁻² are each independently halogen, hydroxyl, C₂-C₆ alkenyl, 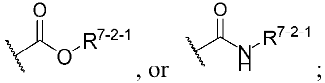
(17) each R⁷⁻²⁻¹ is independently H or C₁-C₆ alkyl; and
(18) when L² is a direct bond, R⁷ is 3- to 12-membered heterocycloalkyl or 3- to 12-membered heterocycloalkyl substituted by one or more R⁷⁻¹; the 3- to 12-membered heterocycloalkyl is preferably a spiro ring; preferably, the heteroatom in the 3- to 12-membered heterocycloalkyl may be N, and the number of heteroatoms may be 1 or 2; more preferably, the N atom in the 3- to 12-membered heterocycloalkyl is attached to L².

5. The compound I, the stereoisomer thereof, or the pharmaceutically acceptable salt thereof according to claim 1 or 2, wherein the compound I satisfies one or more of the following conditions:
(1) L¹ is 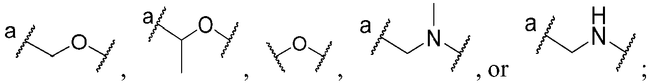 wherein the a-end is attached to U;
(2) U is 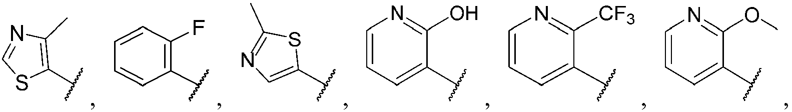 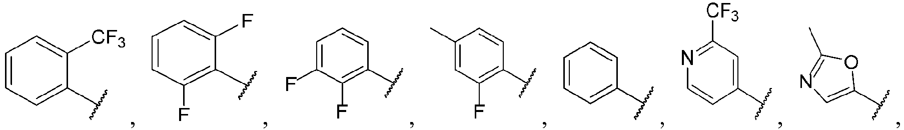 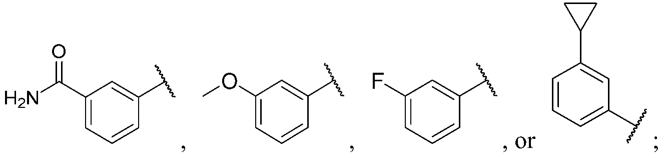 and
(3) W is 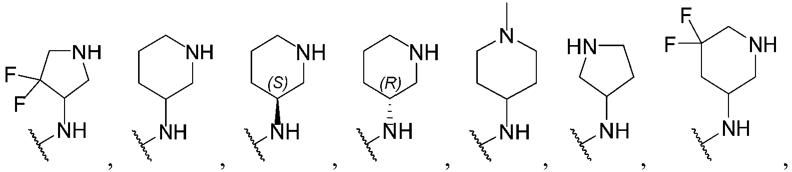 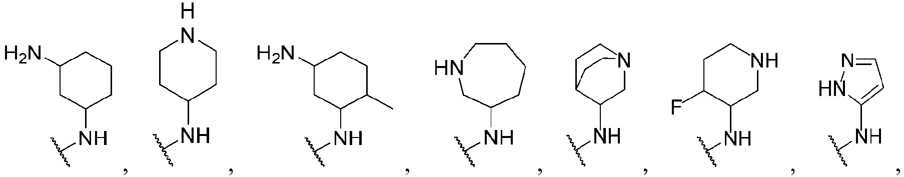 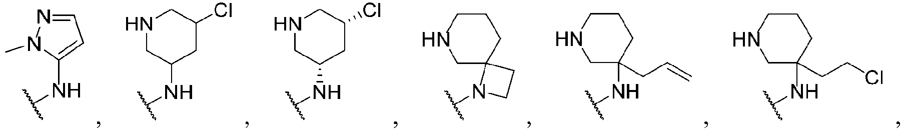 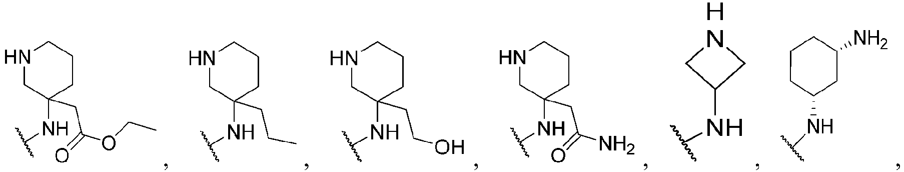 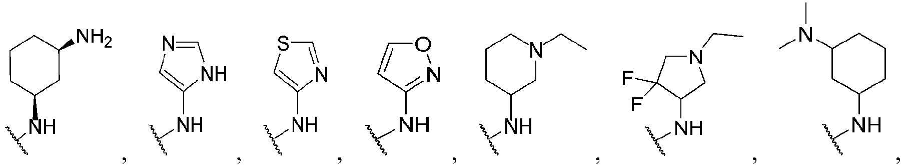 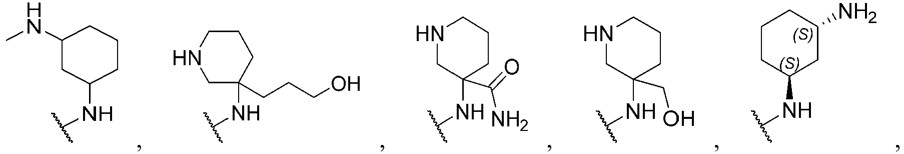 or

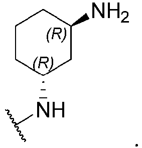

6. The compound I, the stereoisomer thereof, or the pharmaceutically acceptable salt thereof according to claim 1 or 2, wherein
X is N or CH;
Y is O or -CH₂-;
L¹ is 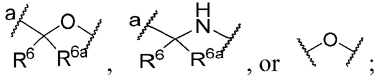 wherein the a-end is attached to U;
R⁶, R^{6a}, and R^{6b} are each independently H or C₁-C₆ alkyl;
U is C₆-C₁₀ aryl, 5- to 10-membered heteroaryl, C₆-C₁₀ aryl substituted by one or more R⁹⁻¹, or 5- to 10-membered heteroaryl substituted by one or more R⁹⁻²;
R⁹⁻¹ and R⁹⁻² are each independently halogen, hydroxyl, C₁-C₆ alkyl, C₁-C₆ alkoxy, 3- to 8-membered cycloalkyl, 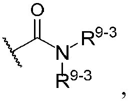 or C₁-C₆ alkyl substituted by one or more halogens;
each R⁹⁻³ is independently H or C₁-C₆ alkyl;
R¹, R², and R³ are each independently H, halogen, or C₁-C₆ alkyl;
R⁴ and R^{4a} are each independently H or C₁-C₆ alkyl;
or, R⁴ and R^{4a} together with the carbon atom to which they are attached form a 3- to 8-membered cycloalkyl ring;
W is L²R⁷;
L² is a direct bond, -CH₂-, or -NH-;
R⁷ is 3- to 12-membered heterocycloalkyl, 5- to 10-membered heteroaryl, 3- to 12-membered heterocycloalkyl substituted by one or more R⁷⁻¹, 5- to 10-membered heteroaryl substituted by one or more R⁷⁻², or 3- to 8-membered cycloalkyl substituted by one or more R⁷⁻⁴;
R⁷⁻¹, R⁷⁻², and R⁷⁻⁴ are each independently halogen, amino, -NHC₁-C₆ alkyl, C₁-C₆ alkyl, -N(C₁-C₆ alkyl)₂, 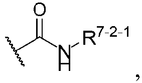 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, C₂-C₆ alkenyl, 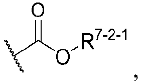 or 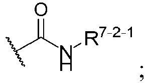
each R⁷⁻²⁻¹ is independently H or C₁-C₆ alkyl;
the heteroatom in the 3- to 12-membered heterocycloalkyl is selected from one or more of N, O, and S; the number of heteroatoms is 1, 2, or 3;
the heteroatom in the 5- to 10-membered heteroaryl is selected from one or more of N, O, and S; the number of heteroatoms is 1, 2, or 3.

7. The compound I, the stereoisomer thereof, or the pharmaceutically acceptable salt thereof according to claim 1 or 2, wherein the compound I satisfies one or more of the following conditions:
(1) the number of O, S, and 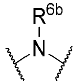 in L^{a}, L^{b} , L^{c}, and L^{d} is 1;
(2) U is 3- to 8-membered cycloalkyl;
(3) R^{9-a} is independently halogen, hydroxyl, C₁-C₆ alkyl, C₁-C₆ alkoxy, 3- to 8-membered cycloalkyl, 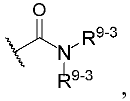 or C₁-C₆ alkyl substituted by one or more R⁹⁻⁴;
(4) L² is a direct bond, -NH-, or -N(CH₃)-;
(5) R⁷ is C₁-C₆ alkyl substituted by one or more R⁷⁻³;
(6) R⁷⁻³ is hydroxyl or 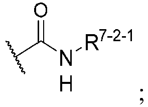
(7) R⁷⁻¹, R⁷⁻², and R⁷⁻⁴ are each 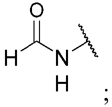
(8) R⁷⁻¹⁻¹ and R⁷⁻¹⁻² are each independently C₁-C₆ alkoxy; and
(9) each R⁷⁻²⁻¹ is independently 3- to 8-membered cycloalkyl or C₁-C₆ alkyl substituted by one or more C₆-C₁₀ aryl.

8. The compound I, the stereoisomer thereof, or the pharmaceutically acceptable salt thereof according to claim 1 or 2, wherein the compound I satisfies one or two of the following conditions:
(1) U is 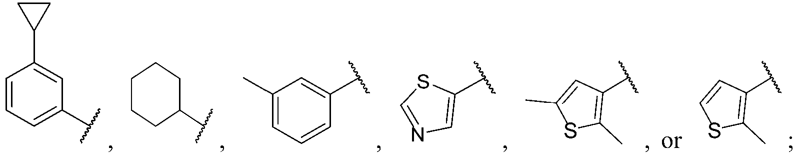 and
(2) W is

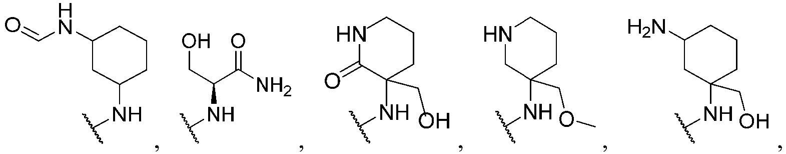

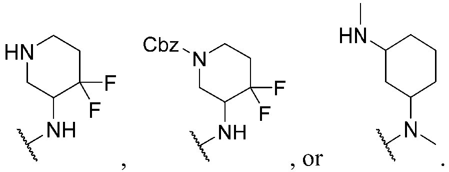

9. The compound I, the stereoisomer thereof, or the pharmaceutically acceptable salt thereof according to claim 1 or 2, wherein the compound I satisfies any one of the following schemes:
scheme 1:
X is N or CH;
Y is O or -CH₂-;
L¹ is 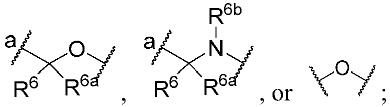 wherein the a-end is attached to U;
R⁶, R^{6a}, and R^{6b} are each independently H or C₁-C₆ alkyl;
U is C₆-C₁₀ aryl, 5- to 10-membered heteroaryl, 3- to 8-membered cycloalkyl, C₆-C₁₀ aryl substituted by one or more R⁹⁻¹, or 5- to 10-membered heteroaryl substituted by one or more R⁹⁻²;
R⁹⁻¹ and R⁹⁻² are each independently halogen, hydroxyl, C₁-C₆ alkyl, C₁-C₆ alkoxy, 3- to 8-membered cycloalkyl, 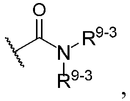 or C₁-C₆ alkyl substituted by one or more halogens;
each R⁹⁻³ is independently H or C₁-C₆ alkyl;
R¹, R², and R³ are each independently H, halogen, or C₁-C₆ alkyl;
R⁴ and R^{4a} are each independently H or C₁-C₆ alkyl;
or, R⁴ and R^{4a} together with the carbon atom to which they are attached form a 3- to 8-membered cycloalkyl ring;
R⁵ and R^{5a} are H;
W is L²R⁷;
L² is a direct bond, -CH₂-, or -NH-;
R⁷ is 3- to 12-membered heterocycloalkyl, 5- to 10-membered heteroaryl, 3- to 12-membered heterocycloalkyl substituted by one or more R⁷⁻¹, 5- to 10-membered heteroaryl substituted by one or more R⁷⁻², C₁-C₆ alkyl substituted by one or more R⁷⁻³, or 3- to 8-membered cycloalkyl substituted by one or more R⁷⁻⁴;
R⁷⁻¹, R⁷⁻², and R⁷⁻⁴ are each independently halogen, amino, -NHC₁-C₆ alkyl, C₁-C₆ alkyl, -N(C₁-C₆ alkyl)₂, 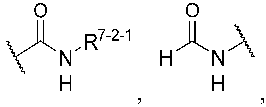 C₁-C₆ alkyl substituted by one or more R⁷⁻¹⁻¹, or C₁-C₆ alkoxy substituted by one or more R⁷⁻¹⁻²;
R⁷⁻¹⁻¹ and R⁷⁻¹⁻² are each independently halogen, hydroxyl, C₁-C₆ alkoxy, C₂-C₆ alkenyl, 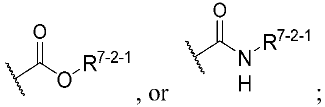
each R⁷⁻³ is independently hydroxyl or 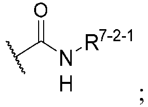 each R⁷⁻²⁻¹ is independently H, C₁-C₆ alkyl, or C₁-C₆ alkyl substituted by one or more C₆-C₁₀ aryl;
the heteroatom in the 3- to 12-membered heterocycloalkyl is selected from one or more of N, O, and S; the number of heteroatoms is 1, 2, or 3;
the heteroatom in the 5- to 10-membered heteroaryl is selected from one or more of N, O, and S; the number of heteroatoms is 1, 2, or 3;
scheme 2:
the compound I is compound I-1, I-2, or I-3,

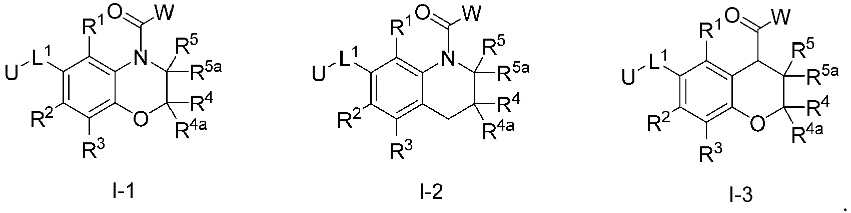

10. The compound I, the stereoisomer thereof, or the pharmaceutically acceptable salt thereof according to claim 1 or 2, wherein the compound I or the stereoisomer thereof is selected from any one of the following compounds: 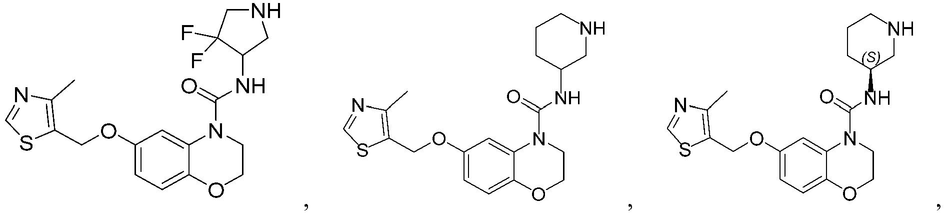 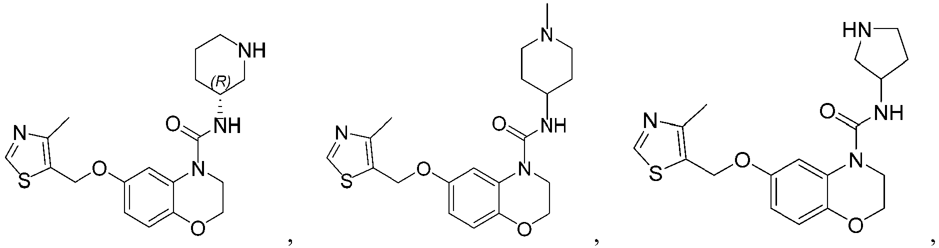 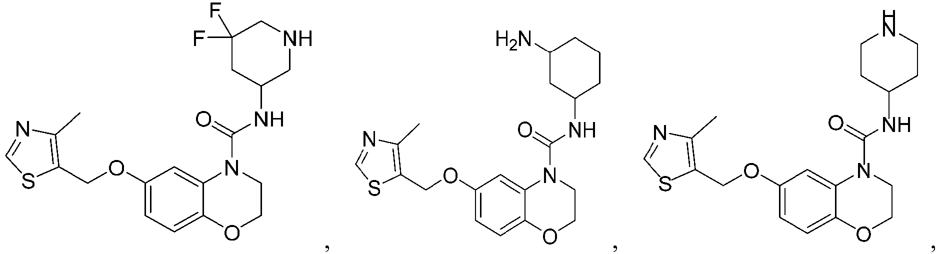 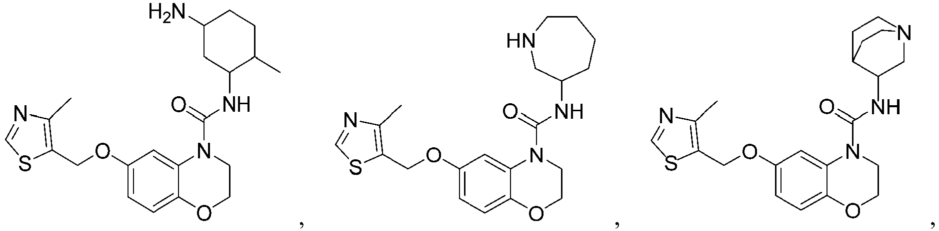 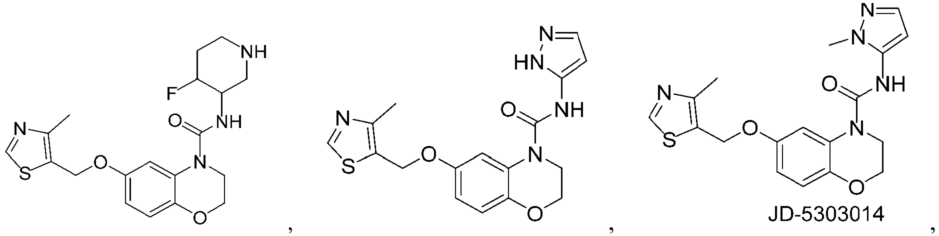 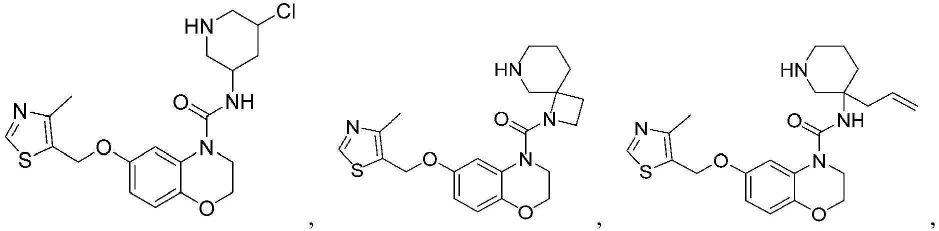 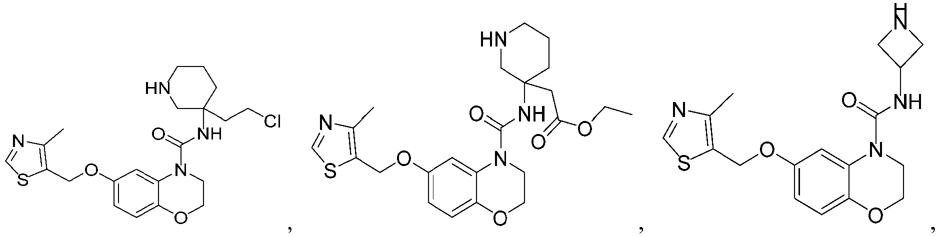 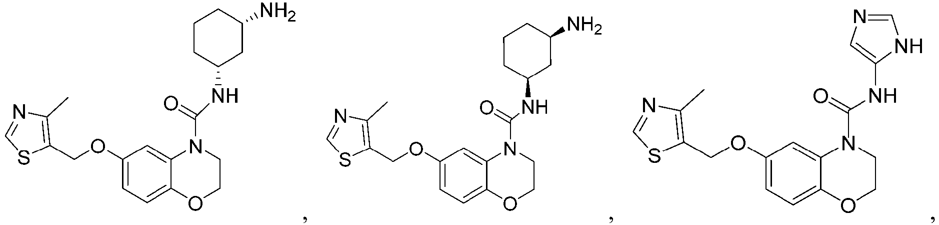 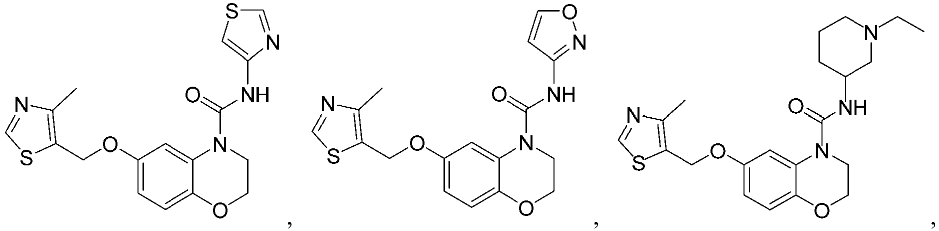 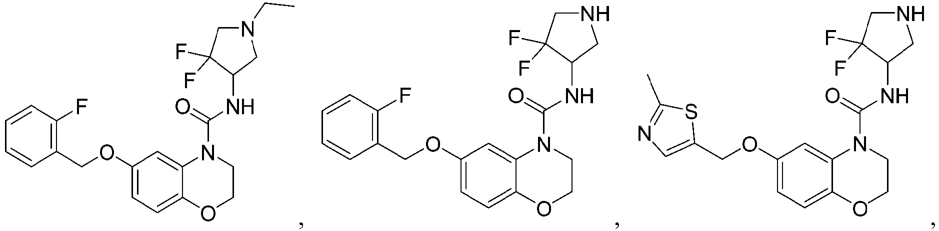 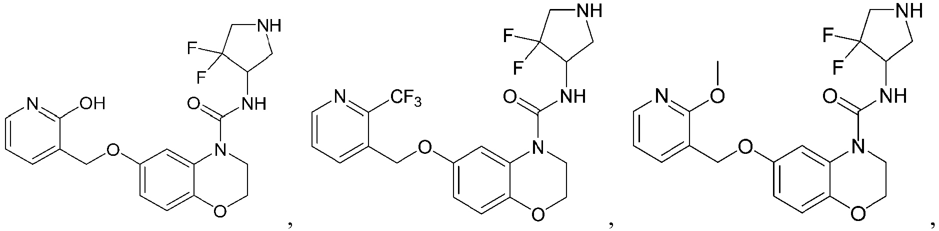 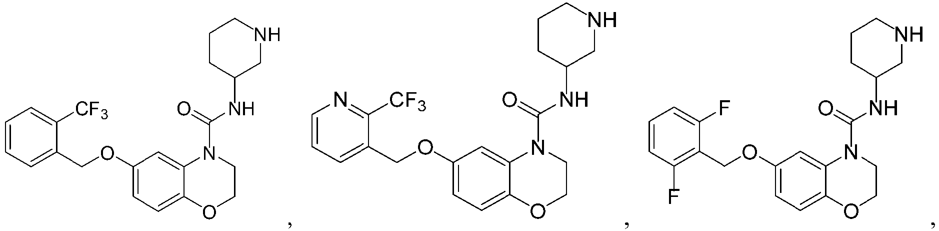 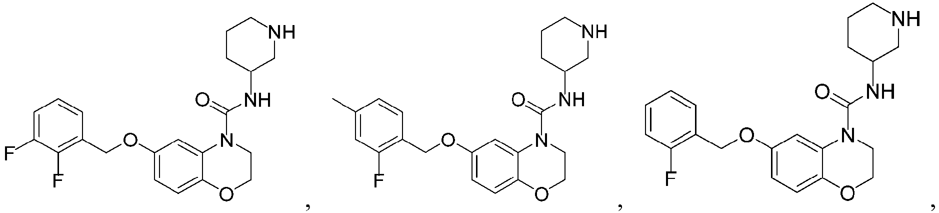 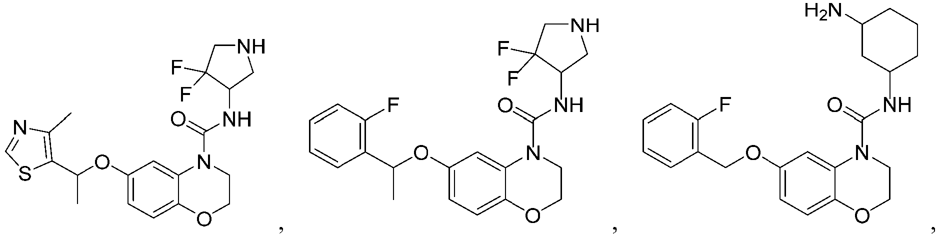 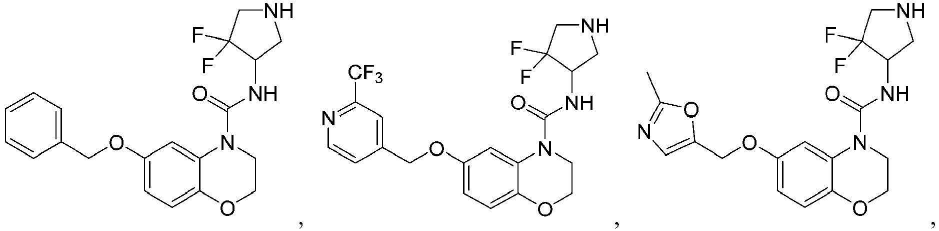 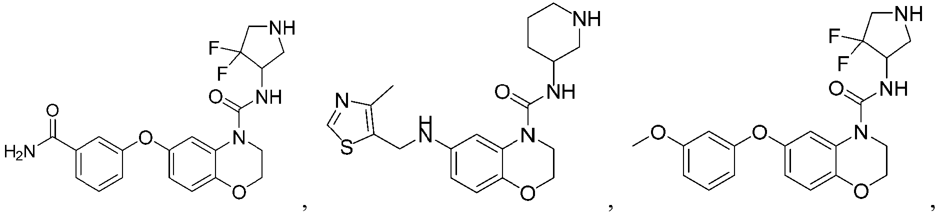 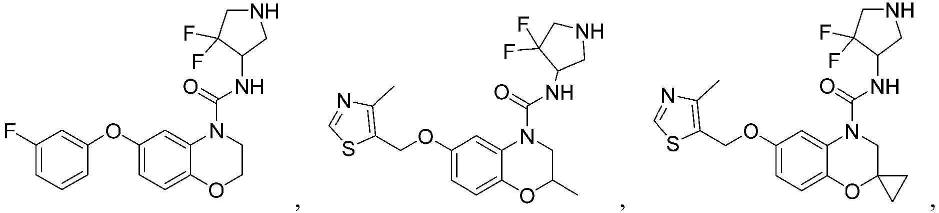 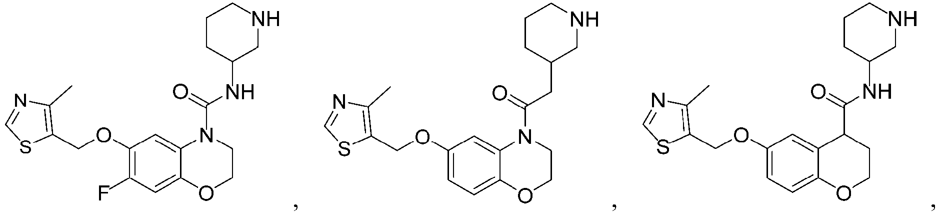 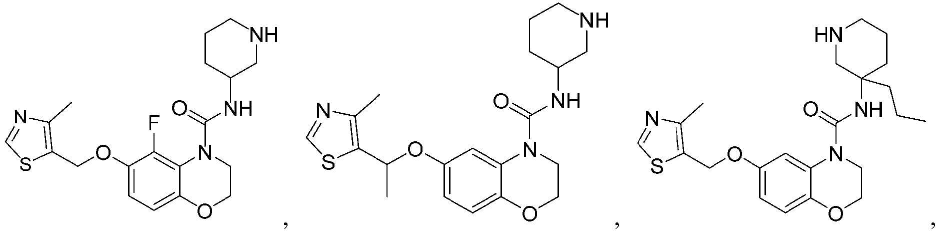 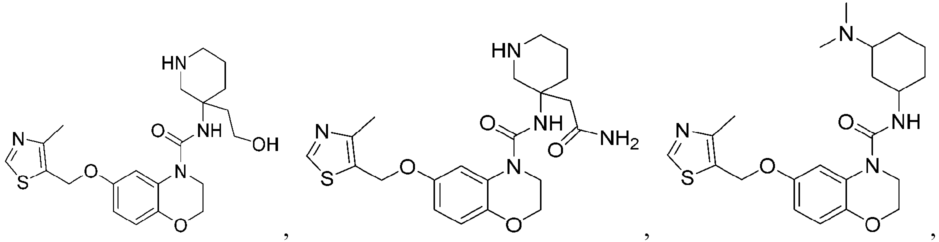 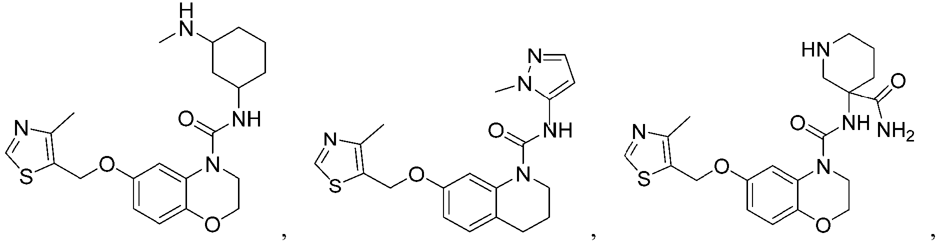 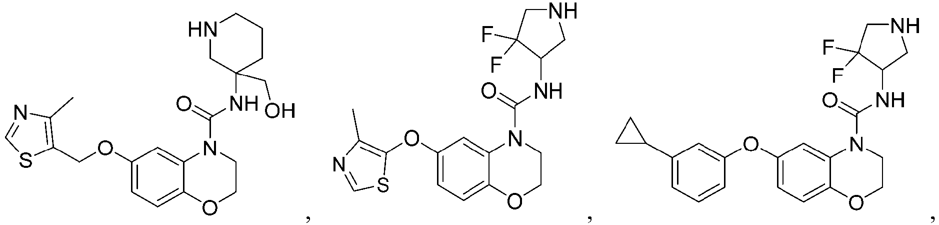 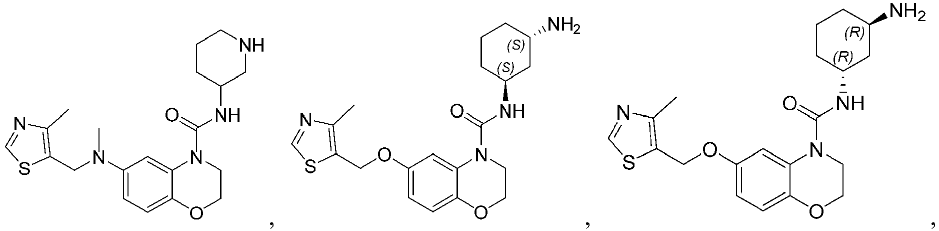 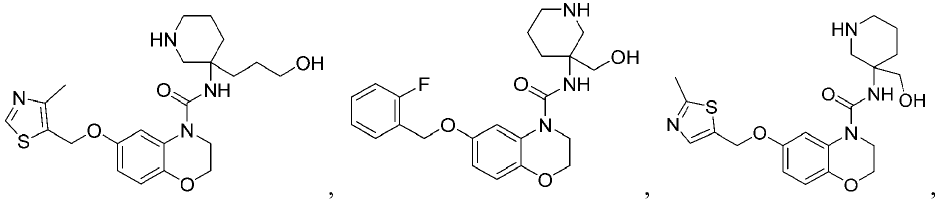 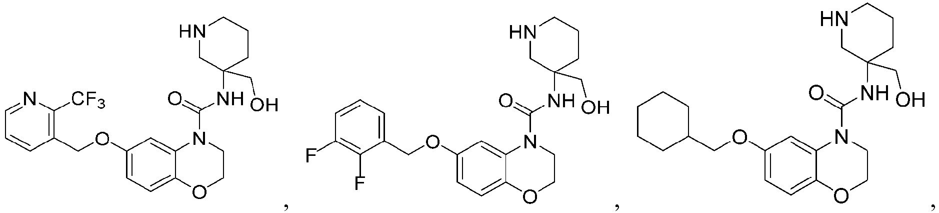 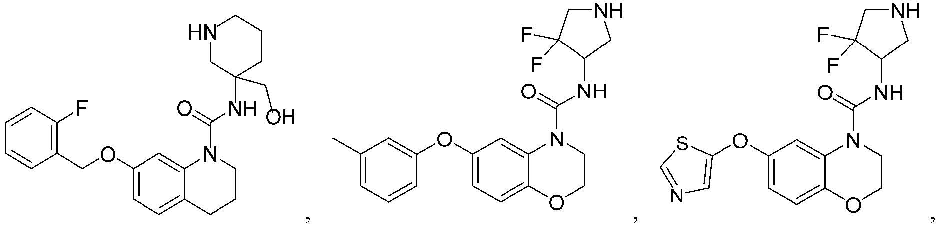 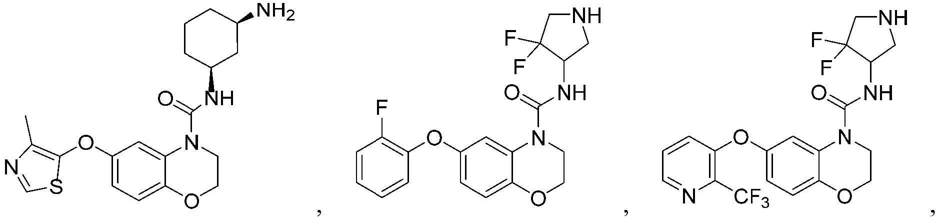 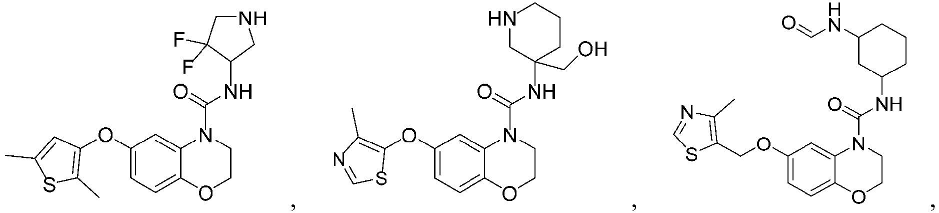 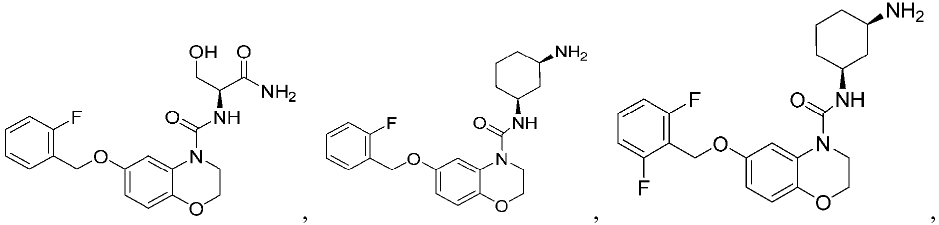 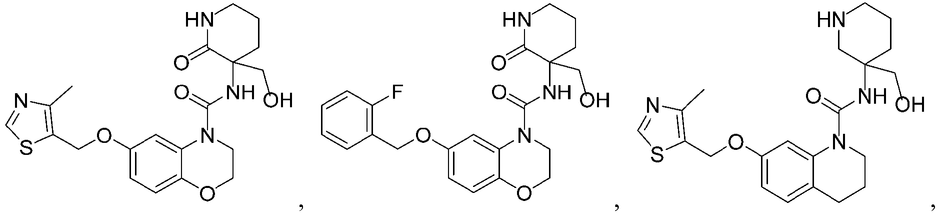 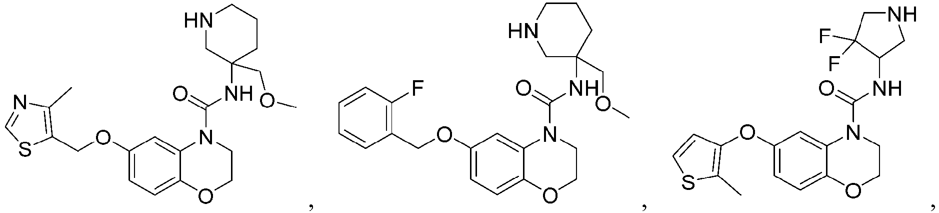 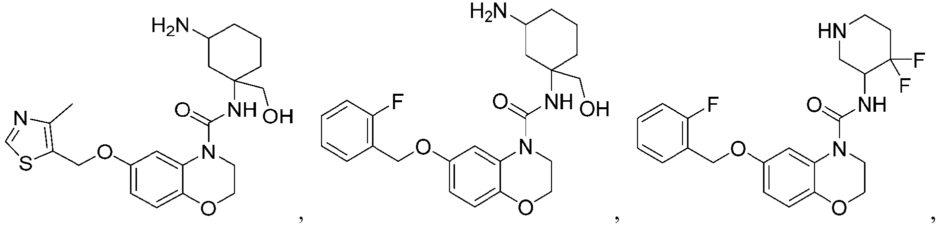 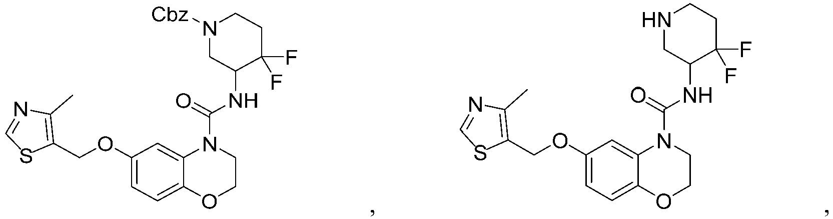 and 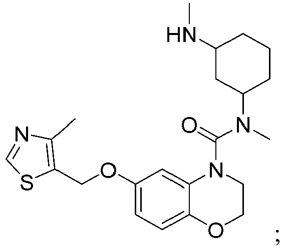
the pharmaceutically acceptable salt of the compound I is selected from any one of the following compounds:

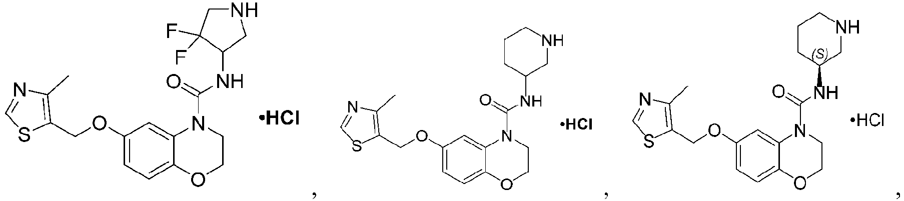

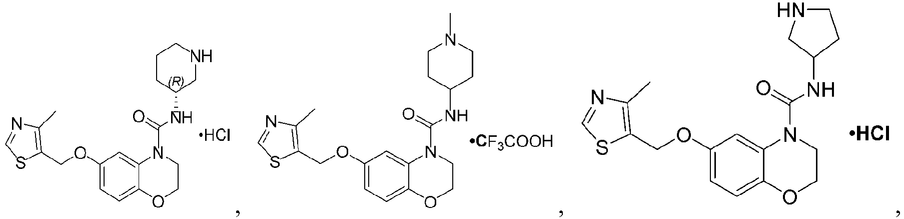

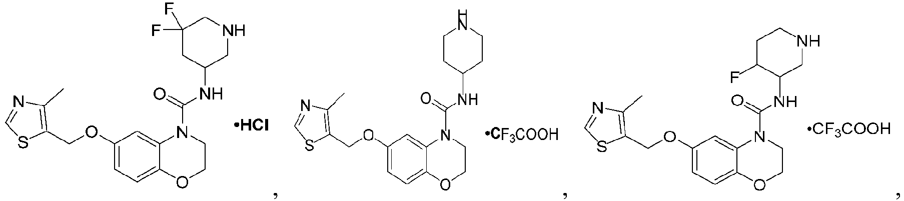

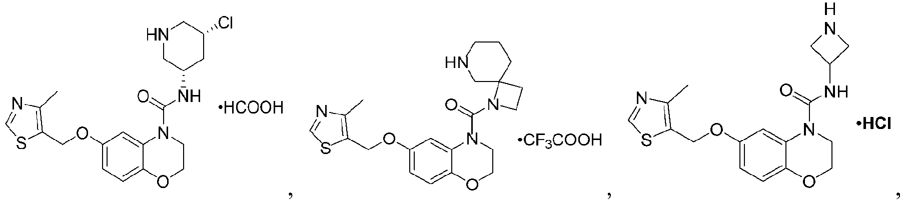

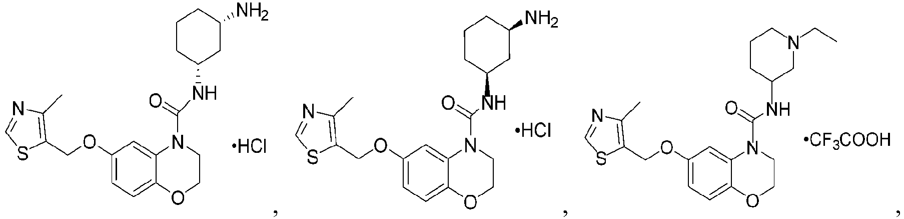

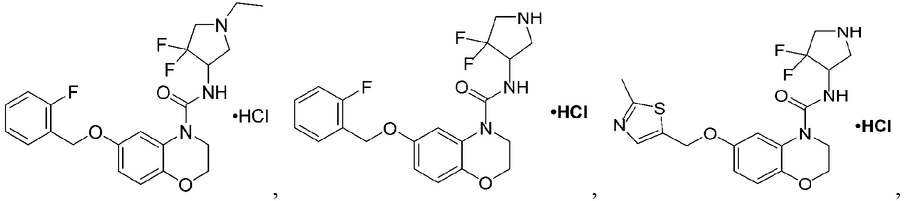

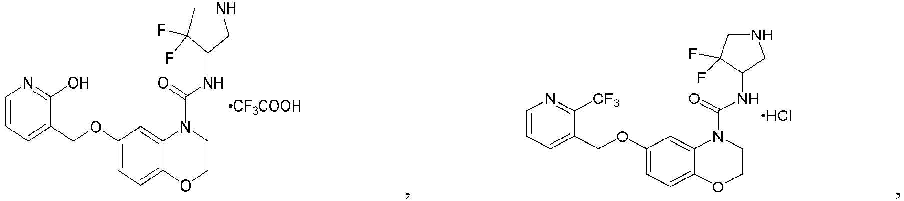

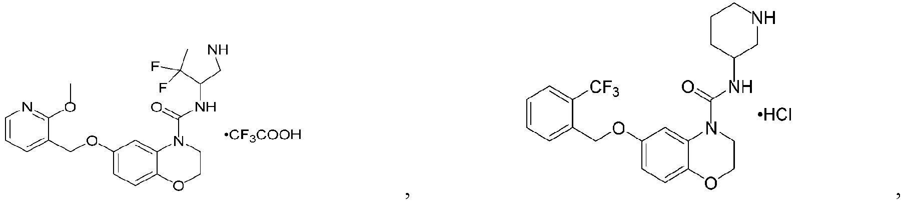

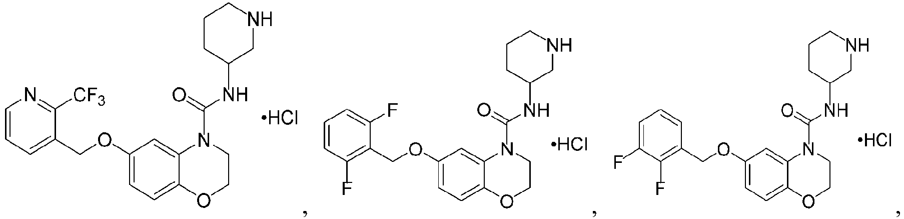

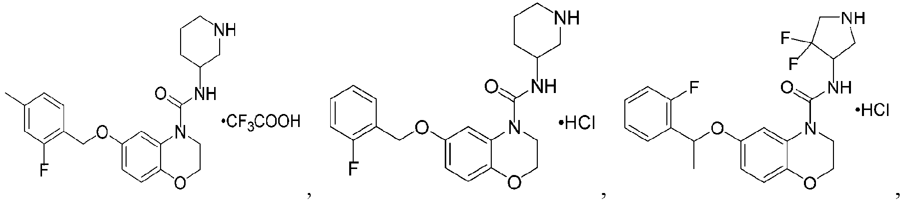

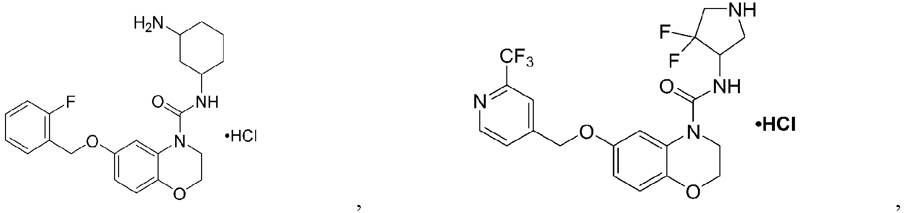

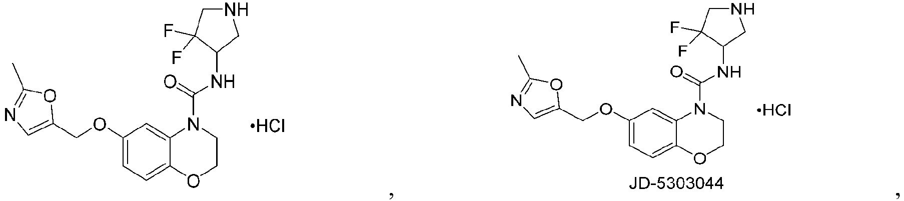

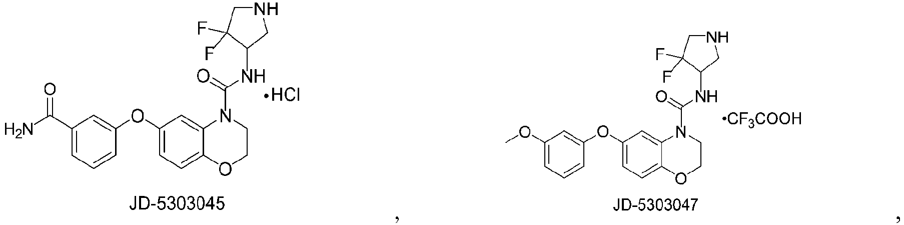

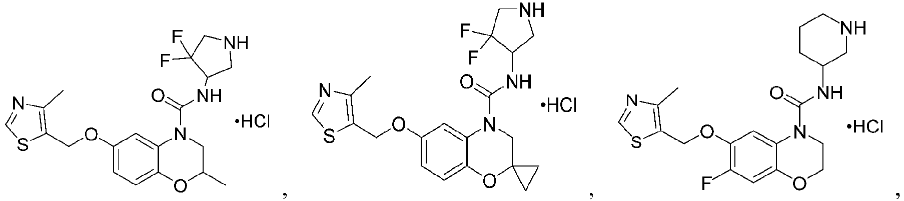

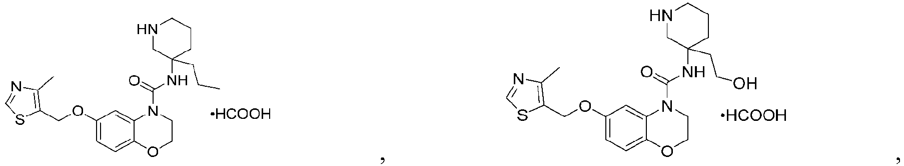

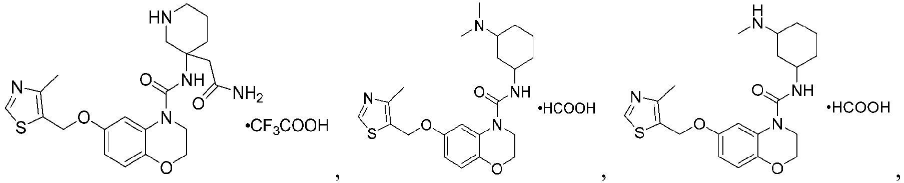

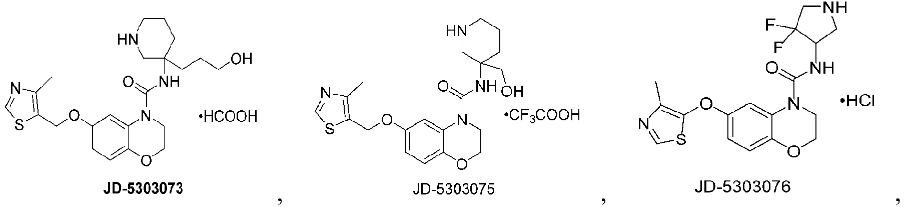

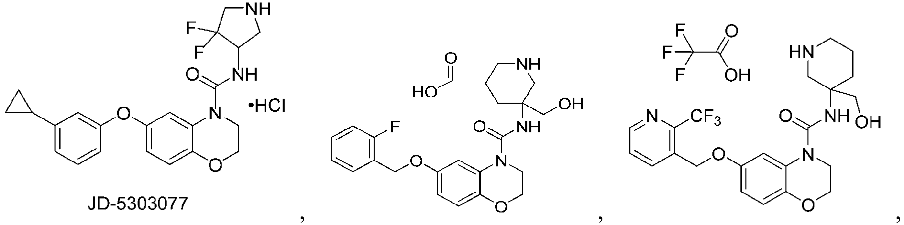

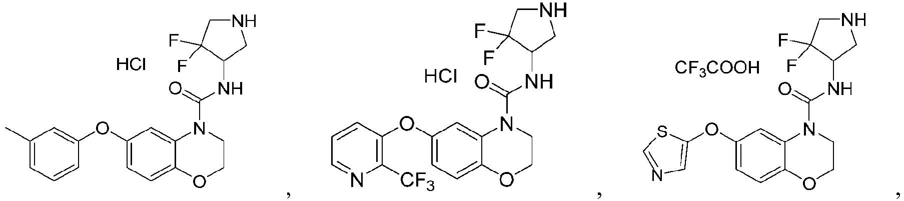

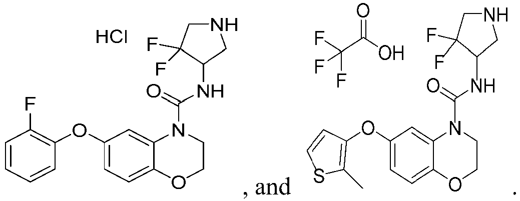

11. A compound II, 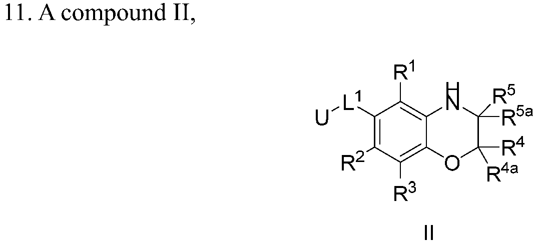 wherein R¹, R², R³, R⁴, R^{4a}, R⁵, R^{5a}, L¹, and U are as defined in any one of claims 1 to 10.

12. A pharmaceutical composition comprising the compound I, the stereoisomer thereof, or the pharmaceutically acceptable salt thereof according to any one of claims 1 to 10, and at least one pharmaceutically acceptable excipient.

13. Use of the compound I, the stereoisomer thereof, or the pharmaceutically acceptable salt thereof according to any one of claims 1 to 10, or the pharmaceutical composition according to claim 12 in the manufacture of a medicament for treating pain; wherein the pain is preferably inflammatory pain.

14. Use of the compound I, the stereoisomer thereof, or the pharmaceutically acceptable salt thereof according to any one of claims 1 to 10, or the pharmaceutical composition according to claim 12 in the manufacture of a medicament for preventing and/or treating a TRPM3-mediated disease; wherein the TRPM3-mediated disease is preferably pain, more preferably inflammatory pain.
